# EUROPEAN PATENT SPECIFICATION

(11) **EP 3 897 855 B1**
(45) Date of publication and mention of the grant of the patent: **07.06.2023**
(21) Application number: 19845855.6
(22) Date of filing: 20.12.2019
(51) Int. Cl.: A61P 35/00, C07D 401/12, C07D 401/14, C07D 405/14, C07D 413/14, A61K 31/497, A61K 31/444

(54) **KIF18A INHIBITORS**
KIF18A-INHIBITOREN
INHIBITEURS DE KIF18A

(30) Priority: 20.12.2018 US 201862783069 P
(43) Date of publication of application: 27.10.2021
(73) Proprietor: Amgen Inc., Thousand Oaks, CA 91320-1799 (US)
(72) Inventor: TAMAYO, Nuria A., Thousand Oaks, 91320-1799 (US); BANERJEE, Abhisek, Thousand Oaks, California 91320-1799 (US); BROWN, James Alexander, Thousand Oaks, California 91320-1799 (US); FROHN, Michael J., Thousand Oaks, California 91320-1799 (US); CHEN, Jian Jeffrey, Thousand Oaks, California 91320-1799 (US); LI, Kexue, Thousand Oaks, California 91320-1799 (US); LIU, Qingyian, Thousand Oaks, California 91320-1799 (US); LOW, Jonathan Dante, Thousand Oaks, California 91320-1799 (US); MA, Vu, Thousand Oaks, California 91320-1799 (US); PETTUS, Liping H., Thousand Oaks, California 91320-1799 (US); WALTON, Mary Catherine, Thousand Oaks, California 91320-1799 (US); MINATTI, Ana Elena, Thousand Oaks, California 91320-1799 (US); BOURBEAU, Matthew Paul, Thousand Oaks, California 91320-1799 (US); JIA, Lei, Thousand Oaks, California 91320-1799 (US); NGUYEN, Thomas T., Thousand Oaks, California 91320-1799 (US); NISHIMURA, Nobuko, Thousand Oaks, California 91320-1799 (US); XUE, Qiufen May, Thousand Oaks, California 91320-1799 (US); ALLEN, John Gordon, Thousand Oaks, California 91320-1799 (US)
(74) Representative: Murgitroyd & Company
(86) International application number: PCT/US2019/068172
(87) International publication number: WO 2020/132651

(56) References cited:
- WO-A1-2011/085261
- GIRGIS A S ET AL: "Novel synthesis of nicotinamide derivatives of cytotoxic properties", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 14, no. 13, 1 July 2006 (2006-07-01), pages 4466-4476, XP027992717, ISSN: 0968-0896 [retrieved on 2006-07-01]
- JOACHIM BRAUN ET AL: "Synthesis and Biological Evaluation of Optimized Inhibitors of the Mitotic Kinesin Kif18A", ACS CHEMICAL BIOLOGY, vol. 10, no. 2, 26 November 2014 (2014-11-26), pages 554-560, XP055671701, ISSN: 1554-8929, DOI: 10.1021/cb500789h

## Description

The invention relates to the field of pharmaceutical agents and, more specifically, is directed to compounds and compositions useful for modulating KIF18A, and to uses and methods for managing cell proliferation and for treating cancer.

### BACKGROUND OF THE INVENTION

Cancer is one of the most widespread diseases afflicting mankind and a major cause of death worldwide. In an effort to find an effective treatment or a cure for one or more of the many different cancers, over the last couple of decades, numerous groups have invested a tremendous amount of time, effort and financial resources. However, to date, of the available cancer treatments and therapies, only a few offer any considerable degree of success.

Cancer is often characterized by unregulated cell proliferation. Damage to one or more genes, responsible for the cellular pathways, which control progress of proliferation through the cell cycle and centrosome cycle, can cause the loss of normal regulation of cell proliferation. These deregulated genes can code for various tumor suppressor or oncogene proteins, which participate in a cascade of events, leading to unchecked cell-cycling progression and cell proliferation. Various kinase and kinesin proteins have been identified, which play key roles in cell cycle and mitotic regulation and progression of normal dividing cells and cancer cells.

Kinesins are molecular motors that play important roles in cell division and intracellular vesicle and organelle transport. Mitotic kinesin plays roles in several aspects of spindle assembly, chromosome segregation, centrosome separation and dynamics (reviewed in O. Rath and F. Kozielski, Nature Review Cancer, 12:527-39, 2012). Human kinesins are categorized into 14 subfamilies based on sequence homology within the so called "motor domain", this domains ATPase activity drives unidirectional movement along microtubules (MTs). The non-motor domain of these proteins is responsible for cargo attachment; a "cargo" can include any one of a variety of different membranous organelles, signal transduction scaffolding systems, and chromosomes. Kinesins use the energy of ATP hydrolysis to move cargo along polarized microtubules. Thus, kinesins are often called "plus-end" or "minus-end" directed motors.

*KIF18A* gene belongs to Kinesin-8 subfamily and is a plus-end-directed motor. KIF18A is believed to influence dynamics at the plus end of kinetochore microtubules to control correct chromosome positioning and spindle tension. Depletion of human KIF18A leads to longer spindles, increased chromosome oscillation at metaphase, and activation of the mitotic spindle assembly checkpoint in HeLa cervical cancer cells (MI Mayr et al, Current Biology 17, 488-98, 2007). KIF18A appears to be viable target for the treatment of cancer. KIF18A is overexpressed in various types of cancers, including but not limited to colon, breast, lung, pancreas, prostate, bladder, head, neck, cervix, and ovarian cancers. Further, genetic deletion or knockdown, or inhibition of KIF 18A effects mitotic spindle apparatus in cancer cell lines. Particularly, inhibition of KIF18A has been found to induce mitotic cell arrest, a known vulnerability that can promote cell death in mitosis via apoptosis, mitotic catastrophe, or multipolarity driven lethality or death after mitotic slippage in interphase. Accordingly, there has been a strong interest in finding inhibitors of KIF18A proteins.

Thus, the inhibition of KIF18A ATPase activity is a promising approach for the development of novel anti-cancer agents.

WO2011085261 discloses benzamide derivatives as anti-cancer agents.

### SUMMARY OF THE INVENTION

The present invention provides a new class of compounds useful for modulating KIF18A protein alone or in a bound complex with microtubules for treating KIF18A-mediated conditions and/or diseases, including cancer, inflammation, or ciliopathologies.

The scope of the invention is defined by the claims. Any references in the description to methods of treatment refer to the compounds, pharmaceutical compositions and medicaments of the present invention for use in a method for treatment of the human (or animal) body by therapy (or for diagnosis).

The compounds provided by the invention have MT-based KIF18A modulatory activity and, in particular, KIF18A inhibitory activity. To this end, the invention also provides the use of these compounds, as well as pharmaceutically acceptable salts thereof, in the preparation and manufacture of a pharmaceutical composition or medicament for therapeutic, prophylactic, acute or chronic treatment of KIF18A mediated diseases and disorders, including without limitation, cancer. Thus, the compounds of the invention are useful in the manufacture of anti-cancer medicaments. The invention also provides processes for making compounds of Formula I, as well as intermediates useful in such processes.

In embodiment 1, the present invention provides a compound of Formula (I), A compound of formula I: 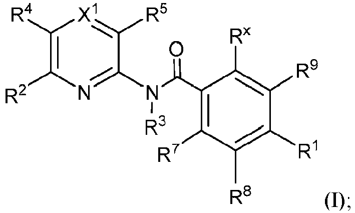 or any pharmaceutically-acceptable salt thereof, wherein:
X¹ is N or -CR⁶;
R¹ is a group -Z-R¹² wherein Z is -C₀₋₄alk-, -NR¹¹-, -NR¹¹SO₂-C₀₋₄alk-, -SO₂NR¹¹-C₀₋₄alk-, -NR¹¹SO₂NR¹¹-, -NR¹¹SO₂NR¹¹-C(=O)-O-, -C₀₋₄alk-S(=O)(=NH)-, C₀₋₄alk-NR¹¹-S(=O)(=NH), -C₀₋₄alk-S-, -C₀₋₄alk-S(=O)-, -C₀₋₄alk-SO₂-, C₀₋₄alk-O-, -P-, -P(=O), -P(=O)₂, -(C=O)-, -(C=O)NR¹¹-, -C=N(OH)-, or - NR¹¹(C=O); or
the group -Z-R¹² is -N=S(=O)-(R¹²)₂, wherein the two R¹² pair can alternatively combine with the sulfur atom attached to each of them to form a saturated or partially-saturated 3-, 4-, 5-, or 6-membered monocyclic ring containing 0, 1, 2 or 3 N atoms and 0, 1, or 2 atoms selected from O and S;
R² is halo or a group -Y-R¹³, wherein Y is -C₀₋₄alk-, -N(C₀₋₁alk)-C₀₋₄alk-, -C(=O)NR^{a}R^{a}(C₁₋₄alk)-, -O-C₀₋₄alk-, -S-, -S=O, -S(=O)₂-, -SO₂N(C₀₋₁alk)-C₀₋₄alk-, -N(C₀₋₁alk)-SO₂-C₀₋₄alk-, -C₀₋₄alk-S(=O)(=NH)-, -(C=O)-, -C₀₋₄alk-(C=O)-O-; or
the group -Y-R¹³ is -N=S(=O)-(R¹³)₂, wherein the two R¹³ pair can alternatively combine with the sulfur atom attached to each of them to form a saturated or partially-saturated 3-, 4-, 5-, or 6-membered monocyclic ring containing 0, 1, 2 or 3 N atoms and 0, 1, or 2 atoms selected from O and S;
R³ is H, methyl, or ethyl;
R⁴ is H, halo, CN, C₁₋₄alk, or C₁₋₄haloalk;
R⁵ is H, halo, C₁₋₈alk, or C₁₋₄haloalk;
R⁶ is H, halo, CN, C₁₋₈alk, C₁₋₄haloalk, -O-C₀₋₆alk-, or R^{6a};
R⁷ is H, halo, C₁₋₈alk, or C₁₋₄haloalk,
R⁸ is H, halo, C₁₋₈alk, or C₁₋₄haloalk,
R⁹ is H, halo, C₁₋₈alk, or C₁₋₄haloalk,
R^{x} is selected from the group consisting of 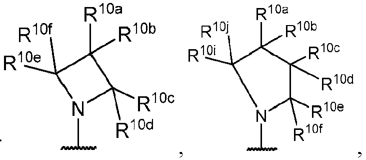 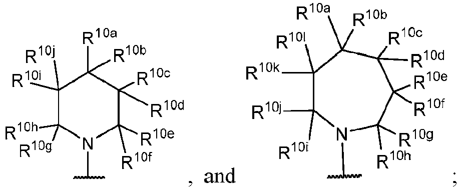
Each of R^{10a}, R^{10b}, R^{10c}, R^{10d}, R^{10e}, R^{10f,} R^{10g}, R^{10h}, R¹⁰ⁱ, and R^{10j} is H, halo, R^{10k}, or R¹⁰¹;
or alternatively, each of R^{10a} and R^{10b} pair, R^{10c} and R^{10d} pair, R^{10e} and R^{10f} pair, R^{10g} and R^{10h} pair, or R¹⁰ⁱ and R^{10j} pair, independently, can combine with the carbon atom attached to each of them to form a saturated or partially-saturated 3-, 4-, 5-, 6-membered monocyclic ring spiro to the R^{x} ring; wherein said 3-, 4-, 5-, 6-membered monocyclic ring contains 0, 1, 2 or 3 N atoms and 0, 1, or 2 atoms selected from O and S, and further wherein said 3-, 4-, 5-, 6-membered monocyclic ring is substituted by 0, 1, 2 or 3 group(s) selected from F, Cl, Br, C₁₋₆alk, C₁₋₄haloalk, -OR^{a}, -OC₁₋₄haloalk, CN, -NR^{a}R^{a}, or oxo;
R¹¹ is H or C₁₋₈alk;
R¹² is H, R^{12a}, orR^{12b};
R¹³ is R^{13a} or R^{13b};
R^{6a}, R^{10k}, R^{12a}, and R^{13a} is independently, at each instance, selected from the group consisting of a saturated, partially-saturated or unsaturated 3-, 4-, 5-, 6-, or 7-membered monocyclic or 4-, 5-, 6-, 7-, 8-, 9-, 10-, 11-, or 12-membered bicyclic ring containing 0, 1, 2 or 3 N atoms and 0, 1, or 2 atoms selected from O and S, which is substituted by 0, 1, 2 or 3 group(s) selected from F, Cl, Br, C₁₋₆alk, C₁₋₄haloalk, -OR^{a}, -OC₁₋₄haloalk, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆alkNR^{a}R^{a}, -OC₂₋₆alkOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkNR^{a}R^{a}, -NR^{a}C₂₋₆alkOR^{a}, -C₁₋₆alkNR^{a}R^{a}, -C₁₋₆alkOR^{a}, -C₁₋₆alkN(R^{a})C(=O)R^{b}, -C₁₋₆alkOC(=O)R^{b}, -C₁₋₆alkC(=O)NR^{a}R^{a}, -C₁₋₆alkC(=O)OR^{a}, R¹⁴, and oxo;
R¹⁰¹, R^{12b}, and R^{13b} is independently, at each instance, selected from the group consisting of C₁₋₆alk substituted by 0, 1, 2, 3, 4, or 5 group(s) selected from F, Cl, Br, -C(=O)OR^{a}, -OR^{a}, -C₁₋₂haloalk, -OC₁₋₄haloalk, CN, NH₂, NH(CH₃), or N(CH₃)₂;
R¹⁴ is independently, at each instance, selected from the group consisting of a saturated, partially-saturated or unsaturated 3-, 4-, 5-, 6-, or 7-membered monocyclic or 4-, 5-, 6-, 7-, 8-, 9-, 10-, 11-, or 12-membered bicyclic ring containing 0, 1, 2 or 3 N atoms and 0, 1, or 2 atoms selected from O and S, which is substituted by 0, 1, 2 or 3 group(s) selected from F, Cl, Br, C₁₋₆alk, C₁₋₄haloalk, -OR^{a}, -OC₁₋₄haloalk, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆alkNR^{a}R^{a}, -OC₂₋₆alkOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkNR^{a}R^{a}, -NR^{a}C₂₋₆alkOR^{a}, -C₁₋₆alkNR^{a}R^{a}, -C₁₋₆alkOR^{a}, -C₁₋₆alkN(R^{a})C(=O)R^{b}, -C₁₋₆alkOC(=O)R^{b}, -C₁₋₆alkC(=O)NR^{a}R^{a}, -C₁₋₆alkC(=O)OR^{a}, and oxo;
R^{a} is independently, at each instance, H or R^{b}; and
R^{b} is independently, at each instance, C₁₋₆alk, phenyl, or benzyl, wherein the C₁₋₆alk is being substituted by 0, 1, 2 or 3 substituents selected from halo, -OH, -OC₁₋₄alk, -NH₂, -NHC₁₋₄alk, -OC(=O)C₁₋₄alk, or -N(C₁₋₄alk)C₁₋₄alk; and the phenyl or benzyl is being substituted by 0, 1, 2 or 3 substituents selected from halo, C₁₋₄alk, C₁₋₃haloalk, -OH, -OC₁₋₄alk, -NH₂, -NHC₁₋₄alk, -OC(=O)C₁₋₄alk, or -N(C₁₋₄alk)C₁₋₄alk.

In embodiment 2, the present invention provides compounds wherein R^{x} is

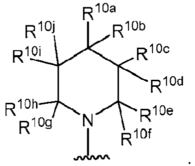

In embodiment 3, the present invention provides compounds wherein X¹ is -CR⁶; having the formula (Ia):

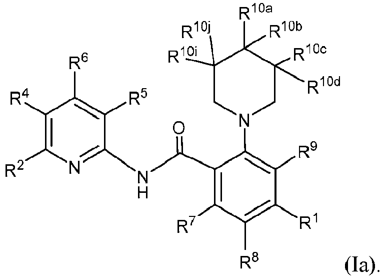

In embodiment 4, the present invention provides compounds wherein X¹ is N; having the formula (Ib):

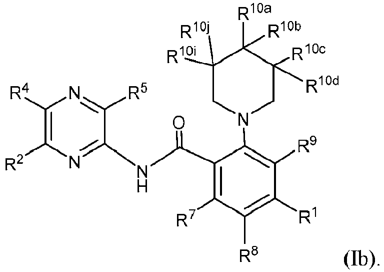

In embodiment 5, the present invention provides compounds in accordance with any of the above embodiments, or pharmaceutically acceptable salts thereof, wherein R³ is H or methyl.

In embodiment 6, the present invention provides compounds in accordance with any of the above embodiments, or pharmaceutically acceptable salts thereof, wherein each of R^{10c}, R^{10d}, R^{10e}, R^{10f}, R^{10g}, R^{10h}, R¹⁰ⁱ, and R^{10j} is H, halo, C₁₋₆alk, or C₁₋₄haloalk; and each of R^{10a} and R^{10b} pair combine with the carbon atom attached to each of them form a saturated 3-, 4-, or 5-membered monocyclic ring spiro to the R^{x} ring; wherein said ring contains 0, 1, 2 or 3 N atoms and 0 or 1 atoms selected from 0 and S.

In embodiment 7, the present invention provides compounds in accordance with any of the above embodiments, or pharmaceutically acceptable salts thereof, wherein each of R^{10c}, R^{10d}, R^{10e}, R^{10f}, R^{10g}, R^{10h}, R¹⁰ⁱ, and R^{10j} is H, methyl, or ethyl; and each of R^{10a} and R^{10b} pair combine with the carbon atom attached to each of them form a cyclopropyl, cyclobutyl, or cyclopentyl ring spiro to the R^{x} ring.

In embodiment 8, the present invention provides compounds in accordance with any of the above embodiments, or pharmaceutically acceptable salts thereof, wherein R^{x} is selected from:

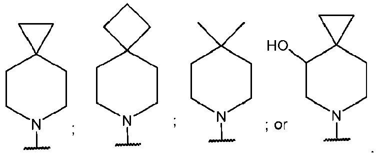

In embodiment 9, the present invention provides compounds in accordance with any of the above embodiments, or pharmaceutically acceptable salts thereof, wherein R^{x} is

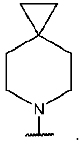

In embodiment 10, the present invention provides compounds in accordance with any of the above embodiments, or pharmaceutically acceptable salts thereof, wherein Z is absent, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-,-(CH₂)₀₋₂-S(=O)-, (CH₃CH)-S(=O)-, -(CH₂)₀₋₂-SO₂-, -O-, -P(=O), -(C=O)-, or -NH(C=O)-.

In embodiment 11, the present invention provides compounds in accordance with any of the above embodiments, or pharmaceutically acceptable salts thereof, wherein the group -Z-R¹² is -N=S(=O)-(R¹²)₂, wherein the two R¹² pair can alternatively combine with the sulfur atom attached to each of them to form a saturated or partially-saturated 3-, 4-, 5-, or 6-membered monocyclic ring containing 0, 1, 2 or 3 N atoms and 0, 1, or 2 atoms selected from O and S; which is selected from:

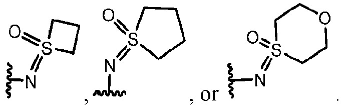

In embodiment 12, the present invention provides compounds in accordance with any of the above embodiments, or pharmaceutically acceptable salts thereof, wherein R¹ is -Z-R¹², wherein Z is absent, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-, -(CH₂)₀₋₂-S(=O)-, (CH₃CH)-S(=O)-, -(CH₂)₀₋₂-SO₂-, -O-, -P(=O), -(C=O)-, or -NH(C=O); and R¹² is selected from:
(a) H,
(b) cyclopropyl, cyclobutyl, cyclopentyl, cyclohexyl, oxiranyl, oxetanyl, tetrahydrofuranyl, azetidinyl, imidazolyl, morpholinyl, pyrrolidinyl, piperazinyl, 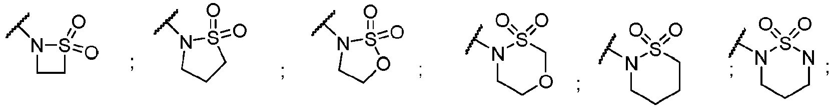 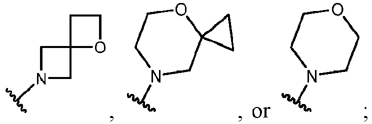 wherein each said ring is substituted by 0, 1, 2 or 3 group(s) selected from wherein each ring is substituted by 0, 1, 2 or 3 OH, F, methyl, -CH₂OH, -C(=O)OCH₃, -C(=O)OC(CH₃)₃, NH₂, CN, and oxo; or C₁₋₆alk substituted by 0, 1, 2 or 3 OH, F, -C(=O)OCH₃, -NH₂, -NH(CH₃), or -N(CH₃)₂.

In embodiment 13, the present invention provides compounds in accordance with any of the above embodiments, or pharmaceutically acceptable salts thereof, wherein R¹ is a group -Z-R¹², wherein Z is -NHSO₂- or -SO₂NH-; and R¹² is oxetanyl, cyclopropyl, or R¹² is C₁₋₆alk substituted by 0, 1, 2 or 3 OH group(s).

In embodiment 14, the present invention provides compounds in accordance with any of the above embodiments, or pharmaceutically acceptable salts thereof, wherein R¹ is a group -Z-R¹², wherein Z is -NHSO₂- and R¹² is -CH₂-CH₂-OH.

In embodiment 15, the present invention provides compounds in accordance with any of the above embodiments, or pharmaceutically acceptable salts thereof, wherein
R² is halo or a group -Y-R¹³, wherein Y is absent, -SO₂NH-(CH₂)₀₋₄-, NH-, -NH-SO₂-(CH)₂)₀₋₄-, -O-(CH₂)₀₋₄, -O-(CH(CH₃))-, -(CH₂)₀₋₄-S(=O)(=NH)-, -(C=O)-, -(CH₂)₀₋₄-(C=O)-O-, or -(CH₂)₁₋₄;
R¹³ is a saturated, partially-saturated or unsaturated 3-, 4-, 5-, 6-, or 7-membered monocyclic or 8-, 9-, 10-, 11-, or 12-membered bicyclic ring containing 0, 1, 2 or 3 N atoms and 0 or 1 atoms selected from O and S, which is substituted by 0, 1, 2 or 3 group(s) selected from F, Cl, Br, C₁₋₆alk, C₁₋₄haloalk, -OH, -OC₁₋₄haloalk, CN, R¹⁴, and oxo; or; R¹³ is C₁₋₆alk substituted by 0, 1, 2, 3, 4, or 5 group(s) selected from F, Cl, Br, -OH, -OC₁₋₄haloalk, or CN.

In embodiment 16, the present invention provides compounds in accordance with any of the above embodiments, or pharmaceutically acceptable salts thereof, wherein R² is a saturated 5- or 6-membered monocyclic ring wherein each said ring contains 0, 1, or 2 N atoms and 0 or 1 O atom, and wherein each said ring is substituted by 0, 1, 2 or 3 group(s) selected from F, Cl, Br, C₁₋₆alk, C₁₋₄haloalk, -OH, -OCH₃, -OC₁₋₄haloalk, CN, R¹⁴, and oxo.

In embodiment 17, the present invention provides compounds in accordance with any of the above embodiments, or pharmaceutically acceptable salts thereof, wherein R² is:
(a) F, Br;
(b) a group -Y-R¹³, wherein Y is absent; and R¹³ is morpholinyl, piperidinyl, azetidinyl, pyrrolidinyl, cyclopropyl, cyclobutyl, cyclopentyl, cyclohexyl, piperazinyl, tetrahydrofuranyl, tetrahydropyranyl, pyridinyl, pyrimidinyl, 3,6-dihydro-2H-pyranyl, 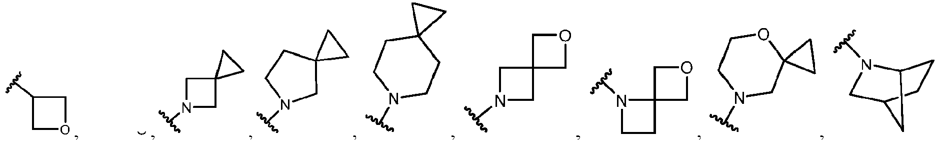 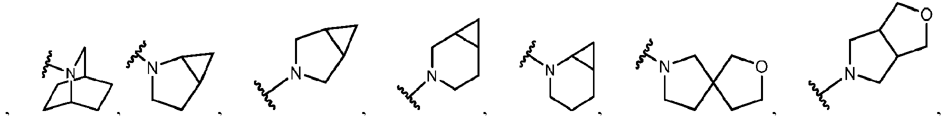 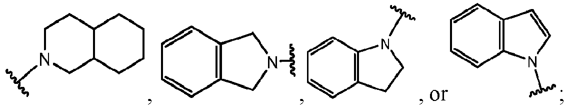 wherein each said ring is substituted by 0, 1, 2 or 3 group(s) selected from F, Cl, Br, methyl, CF₃, CH₂OH, CH(CH₃)OH, C(CH₃)₂OH, -OH, -OCHF₂, CN, oxo, or cyclopropyl; or
(c) a group -Y-R¹³, wherein Y is absent, -SO₂NH-, NH, -O-, S(=O)(=NH)- , -O-(CH₂), -O-(CH(CH₃))-, C(=O)-, C(=O)-O-, -CH₂C(=O)-O-, or -CH₂-; and wherein R¹³ is 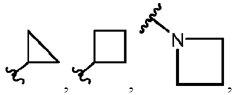 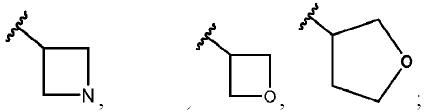 wherein each ring is substituted by 0, 1, 2, 3, 4, or 5 group(s) selected from F, Cl, Br, methyl, CF₃, -OH, or CN; or R¹³ is H or C₁₋₆alk substituted by 0, 1, 2, 3, 4, or 5 group(s) selected from F, Cl, Br, methyl, CF₃, -OH, or CN.

In embodiment 18, the present invention provides compounds in accordance with embodiments 1-17, or pharmaceutically acceptable salts thereof, wherein R² is (a) halo; (b) a group -Y-R¹³, wherein Y is absent; and R¹³ is morpholinyl, piperidinyl, azetidinyl, pyrrolidinyl, cyclopropyl, cyclobutyl, cyclopentyl, cyclohexyl, piperazinyl, tetrahydrofuranyl, 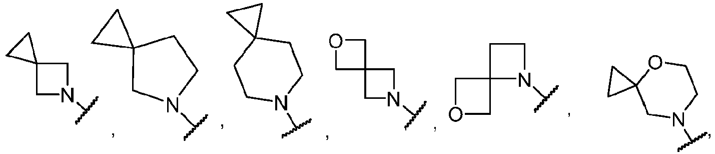 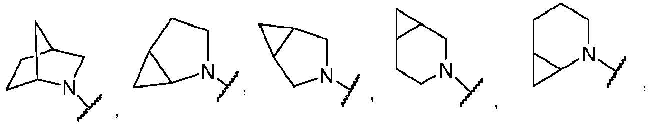 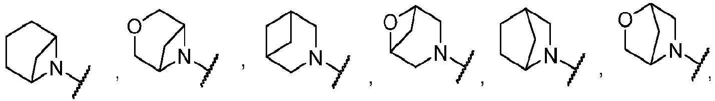 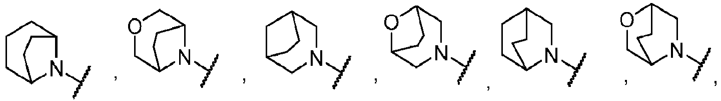 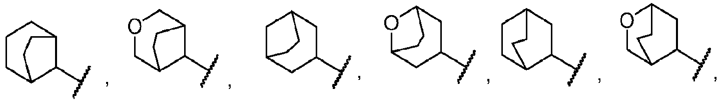 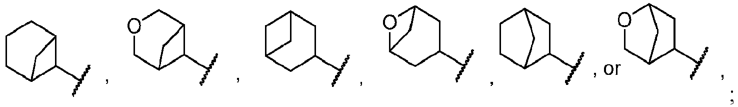 wherein each said ring is substituted by 0, 1, 2 or 3 group(s) selected from F, Cl, Br, methyl, CF₃, -OH, -OCHF₂, CN, and oxo; or (c) a group -Y-R¹³, wherein Y is NH, -O-, -O-(CH₂)-, -O-(CH₂)-(CH₂)-, or -O-(CH₂)-(CH₂)-(CH₂)-, and wherein R¹³ is 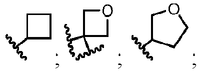 ; or R¹³ is C₁₋₆alk substituted by 0, 1, 2, 3, 4, or 5 group(s) selected from F, Cl, Br, methyl, CF₃, -OH, or CN.

In embodiment 19, the present invention provides compounds in accordance with any of the above embodiments, or pharmaceutically acceptable salts thereof, wherein R² is

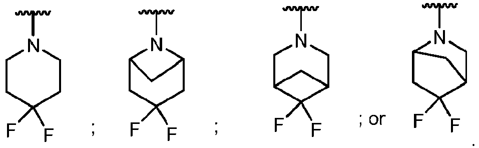

In embodiment 20, the present invention provides compounds in accordance with any of the above embodiments, or pharmaceutically acceptable salts thereof, wherein R² is morpholinyl or piperidinyl substituted by 0, 1, 2 or 3 group(s) selected from F, Cl, Br, methyl, CF₃, -OH, -OCHF₂, CN, or oxo.

In embodiment 21, the present invention provides compounds in accordance with any of the above embodiments, or pharmaceutically acceptable salts thereof, wherein R² is morpholinyl substituted by 1, 2 or 3 methyl group(s).

In embodiment 22, the present invention provides compounds in accordance with any of the above embodiments, or pharmaceutically acceptable salts thereof, wherein R⁴ is selected from H, F, methyl, CN, or Br.

In embodiment 23, the present invention provides compounds in accordance with any of the above embodiments, or pharmaceutically acceptable salts thereof, wherein R⁴ is H.

In embodiment 24, the present invention provides compounds in accordance with any of the above embodiments, or pharmaceutically acceptable salts thereof, wherein R⁵ is H.

In embodiment 25, the present invention provides compounds in accordance with any of the above embodiments, or pharmaceutically acceptable salts thereof, wherein R⁶ is H, methyl, cyclopropyl, CN, CF₃, or azetidinyl.

In embodiment 26, the present invention provides compounds in accordance with any of the above embodiments, or pharmaceutically acceptable salts thereof, wherein R⁷ is H.

In embodiment 27, the present invention provides compounds in accordance with any of the above embodiments, or pharmaceutically acceptable salts thereof, wherein R⁸ is H or F.

In embodiment 28, the present invention provides compounds in accordance with any of the above embodiments, or pharmaceutically acceptable salts thereof, wherein R⁹ is H or F.

In embodiment 29, the present invention provides a compound, or pharmaceutically acceptable salts thereof, selected from:

| Ex. # | Chemical Structure | Name |
|---|---|---|
| 1 | 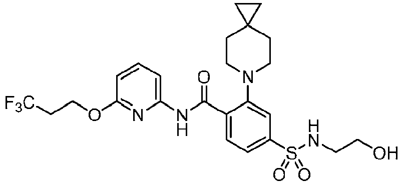 | 4-(N-(2-Hydroxyethyl)sulfamoyl)-2-(6-azaspiro[2.5]octan-6-yl)-N-(6-(3,3,3-trifluoropropoxy)pyridin-2-yl)benzamide |
| 1-1 | 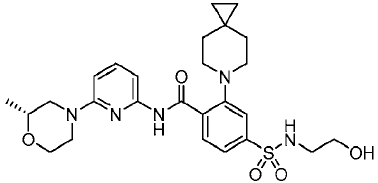 | (*R*)-4-(*N*-(2-Hydroxyethyl)sulfamoyl)-*N*-(6-(2-methylmorpholino)pyridin-2-yl)-2-(6-azaspiro[2.5]octan-6-yl)benzamide |
| 1-2 | 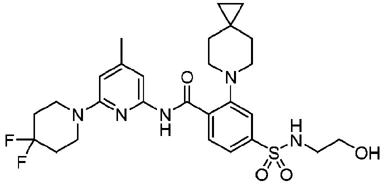 | *N*-(6-(4,4-Diffuoropiperidin-1-yl)-4-methylpyridin-2-yl)-4-(*N*-(2-hydroxyethyl)sulfamoyl)-2-(6-azaspiro[2.5]octan-6-yl)benzamide |
| 2 | 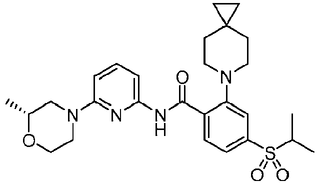 | (*R*)-4-(Isopropylsulfonyl)-*N*-(6-(2-methylmorpholino)pyridin-2-yl)-2-(6-azaspiro [2.5]octan-6-yl)benzamide |
| 2-7 | 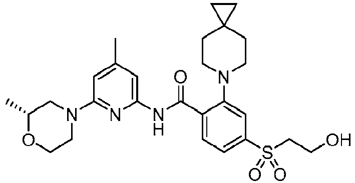 | (*R*)-4-((2-Hydroxyethyl)sulfonyl)-*N*-(4-methyl-6-(2-methylmorpholinolpyridin-2-yl)-2-(6-azaspiro[2.5]octan-6-yl)benzamide |
| 3 | 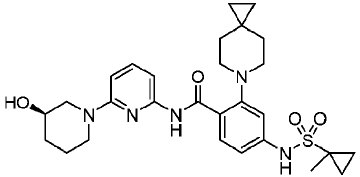 | (*R*)-*N-*(6-(3-Hydroxypiperidin-1-yl)pyridin-2-yl)-4-((1-methylcyclopropane)-1-sulfonamido)-2-(6-azaspiro[2.5]octan-6-yl)benzamide |
| 4 | 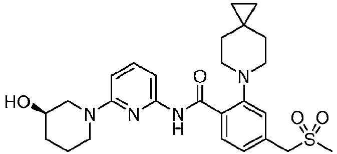 | (*R*)-*N*(6-(3-hydroxypiperidin-1-yl)pyridin-2-yl)-4-((methylsulfonyl)methyl)-2-(6-azaspiro[2.5]octan-6-yl)benzamide |
| 5 | 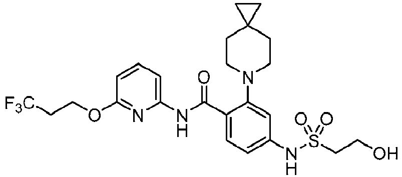 | 4-((2-Hydroxyethyl)sulfonamido)-2-(6-azaspiro[2.5]octan-6-yl)-N-(6-(3,3,3-trifluoropropoxy)pyridin-2-yl)benzamide |
| 6 | 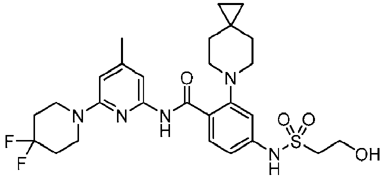 | *N*-(6-(4,4-Diffuoropiperidin-1-yl)-4-methylpyridin-2-yl)-4-((2-hydroxyethyl)sulfonamido)-2-(6-azaspiro[2.5]octan-6-yl)benzamide |
| 7-1 | 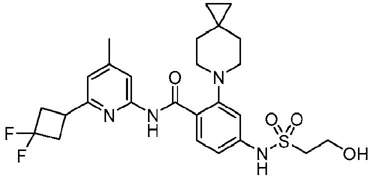 | *N*-(6-(3,3-Difluorocyclobutyl)-4-methylpyridin-2-yl)-4-((2-hydroxyethyl)sulfonamido)-2-(6-azaspiro[2.5]octan-6-yl)benzamide |
| 7-12 | 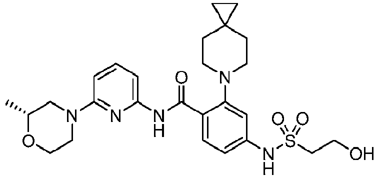 | (*R*)-4-((2-Hydroxyethyl)sulfonamido)-*N*-(6-(2-methylmorpholino)pyridin-2-yl)-2-(6-azaspiro[2.5]octan-6-yl)benzamide |
| 7-33 | 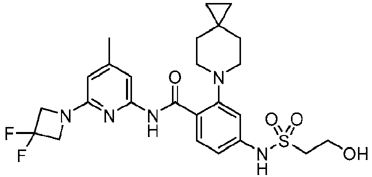 | *N*-(6-(3,3-Difluoroazetidin-1-yl)-4-methylpyridin-2-yl)-4-((2-hydroxyethyl)sulfonamido)-2-(6-azaspiro[2.5]octan-6-yl)benzamide |
| 7-44 | 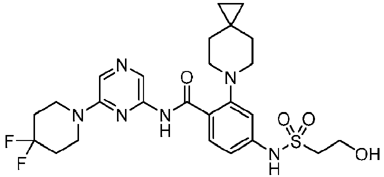 | *N*-(6-(4,4-Difluoropiperidin-1-yl)pyrazin-2-yl)-4-((2-hydroxyethyl)sulfonamido)-2-(6-azaspiro[2.5]octan-6-yl)benzamide |
| 8-1 | 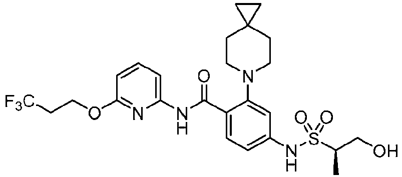 | (*R*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide |
| 8-2 | 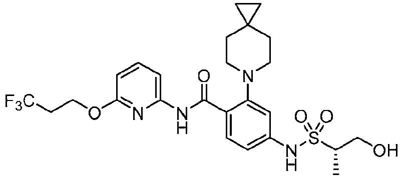 | (*S*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide |
| 9-7 | 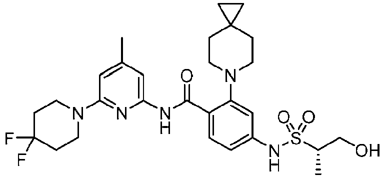 | (*S*)-*N*-(6-(4,4-Difluoropiperidin-1-yl)-4-methylpyridin-2-yl)-4-((2-hydroxy-1-methylethyl)sulfonamido)-2-(6-azaspiro[2.5]octan-6-yl)benzamide |
| 9-8 | 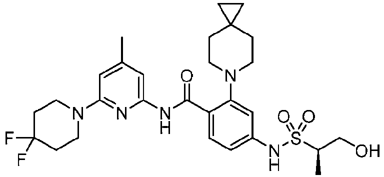 | (*R*)-*N-*(6-(4,4-Difluoropiperidin-1-yl)-4-methylpyridin-2-yl)-4-((2-hydroxy-1-methylethyl)sulfonamido)-2-(6-azaspiro[2.5]octan-6-yl)benzamide |
| 10 | 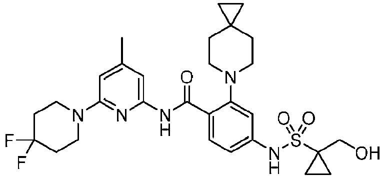 | *N*-(6-(4,4-Diffuoropiperidin-1-yl)-4-methylpyridin-2-yl)-4-((1-(hydroxymethyl)cyclopropane)-1-sulfonamido)-2-(6-azaspiro[2.5]octan-6-yl)benzamide |
| 11 | 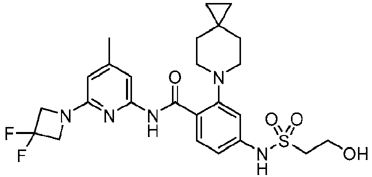 | *N*-(6-(3,3-Difluoroazetidin-1-yl)-4-methylpyridin-2-yl)-4-((2-hydroxyethyl)sulfonamido)-2-(6-azaspiro[2.5]octan-6-yl)benzamide |
| 14 | 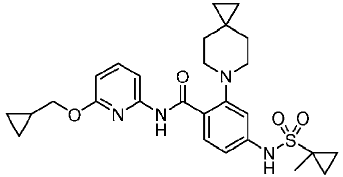 | *N*-(6-(Cyclopropylmethoxy)pyridin-2-yl)-4-((1-methylcyclopropane)-1-sulfonamido)-2-(6-azaspiro[2.5]octan-6-yl)benzamide |
| 16 | 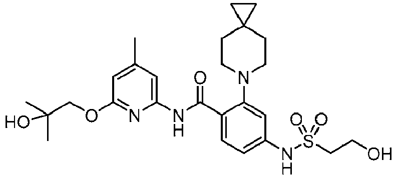 | *N*-(6-(2-Hydroxy-2-methylpropoxy)-4-methylpyridin-2-yl)-4-((2-hydroxyethyl)sulfonamido)-2-(6-azaspiro[2.5]octan-6-yl)benzamide |
| 17 | 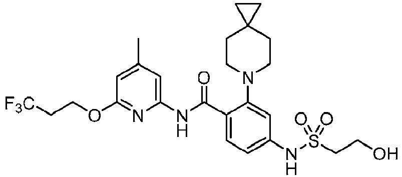 | 4-((2-Hydroxyethyl)sulfonamido)-*N*-(4-methyl-6-(3,3,3-trifluoropropoxy)pyridin-2-yl)-2-(6-azaspiro[2.5]octan-6-yl)benzamide |
| 21 | 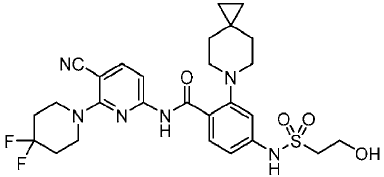 | *N*-(5-Cyano-6-(4,4-difluoropiperidin-1-yl)pyridin-2-yl)-4-((2-hydroxyethyl)sulfonamido)-2-(6-azaspiro[2.5]octan-6-yl)benzamide |
| 22 | 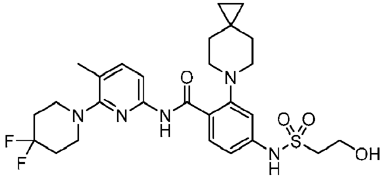 | *N*-(6-(4,4-Difluoropiperidin-1-yl)-5-methylpyridin-2-yl)-4-((2-hydroxyethyl)sulfonamido)-2-(6-azaspiro[2.5]octan-6-yl)benzamide |
| 23 | 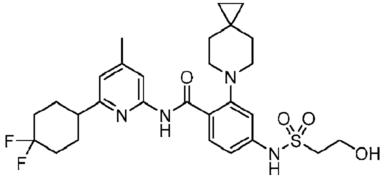 | *N*-(6-(4,4-difluorocyclohexyl)-4-methylpyridin-2-yl)-4-((2-hydroxyethyl)sulfonamido)-2-(6-azaspiro[2.5]octan-6-yl)benzamide |
| 24 | 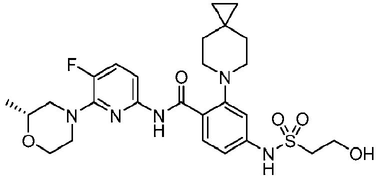 | (*R*)-*N*-(5-Fluoro-6-(2-methylmorpholino)pyridin-2-yl)-4-((2-hydroxyethyl)sulfonamido)-2-(6-azaspiro[2.5]octan-6-yl)benzamide |
| 25 | 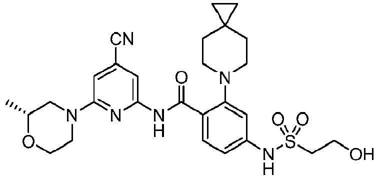 | (R)-*N*-(4-Cyano-6-(2-methylmorpholino)pyridin-2-yl)-4-((2-hydroxyethyl)sulfonamido)-2-(6-azaspiro[2.5]octan-6-yl)benzamide |
| 27-1 | 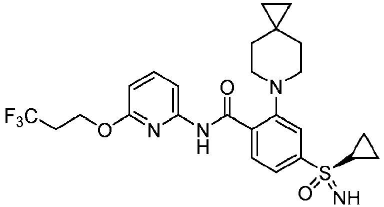 | 2-(6-Azaspiro[2.5]octan-6-yl)-4-(*S-*cyclopropylsulfonimidoyl)-*N*-(6-(3,3,3-trifluoropropoxy)-2-pyridinyl)benzamide |
| 27-2 | 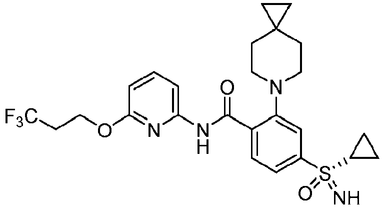 | 2-(6-Azaspiro[2.5]octan-6-yl)-4-(*R-*cyclopropylsulfonimidoyl)-*N*-(6-(3,3,3-trifluoropropoxy)-2-pyridinyl)benzamide |

or any pharmaceutically-acceptable salt thereof.

In embodiment 30, the present invention provides pharmaceutical compositions comprising a compound, or pharmaceutically acceptable salts thereof, in accordance with any one of embodiments 1-29, and a pharmaceutically acceptable diluent or carrier.

In embodiment 31, the present invention provides a method of treating a condition that may be treated with KIF18a inhibitors, the method comprising administering to a patient in need thereof a therapeutically effective amount of the compound in accordance with embodiments 1-29, or the composition according to embodiment 30.

In embodiment 32, the present invention provides the method of embodiment 31, wherein said condition is cancer selected from the group consisting of (a) a solid or hematologically derived tumor selected from cancer of the cancer of the bladder, endometrial, lung squamous cell, breast, colon, kidney, liver, lung, small cell lung cancer, esophagus, gall-bladder, brain, head and neck, ovary, pancreas, stomach, cervix, thyroid, prostate and skin, (b) a hematopoietic tumor of lymphoid lineage selected from leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma, (c) a hematopoietic tumor of myeloid lineage selected from acute and chronic myelogenous leukemias, myelodysplastic syndrome and promyelocytic leukemia (d) a tumor of mesenchymal origin selected from fibrosarcoma and rhabdomyosarcoma, (e) a tumor of the central and peripheral nervous system selected from astrocytoma, neuroblastoma, glioma and schwannoma, or (f) a melanoma, seminoma, teratocarcinoma, osteosarcoma, xenoderoma pigmentosum, keratoctanthoma, thyroid follicular cancer or Kaposi's sarcoma.

In a sub-embodiment 32a, the present invention provides the method of embodiment 31, wherein said condition is cancer selected from the group consisting of melanoma, prostate cancer, cervical cancer, breast cancer, colon cancer, sarcoma, or leukemia. See: Zhang C. et. al., "Kif18A is involved in human breast carcinogenesis", Carcinogenesis, 2010 Sep;31(9):1676-84. doi: 10.1093/carcin/bgq134. Epub 2010 Jul 1. See also: (1) https://www.proteinatlas.org/ENSG00000121621-KIF18A/pathology; (2) Nagahara, M. et. al., "Kinesin 18A expression: clinical relevance to colorectal cancer progression", Int. J. Cancer: 129, 2543-2552 (2011) VC 2011 UIC; and (3) Yu, Y. et. al., "The Role of Kinesin Family Proteins in Tumorigenesis and Progression - Potential Biomarkers and Molecular Targets for Cancer Therapy", Cancer 2010;116:5150-60. VC 2010 American Cancer Society.

In embodiment 33, the present invention provides a method of reducing the size of a solid tumor in a subject, the method comprising administering to the subject in need thereof a therapeutically effective amount of the compound in accordance with embodiments 1-29, or the composition according to embodiment 30.

In embodiment 34, the present invention provides a method of treating a cell proliferation disorder in a subject, the method comprising administering to the subject in need thereof a therapeutically effective amount of the compound in accordance with embodiments embodiments 1-29, or the composition according to embodiment 30.

In embodiment 35, the present invention provides a method of inhibiting KIF18A in a cell, comprising contacting the cell with a compound, or pharmaceutically acceptable salts thereof, in accordance with embodiments embodiments 1-29, or the composition according to embodiment 30.

In embodiment 36, the invention provides a method of preparing a compound of Formula (I) as described herein.

In embodiment 37, the invention provides an intermediate compound used in the method of preparing a compound of Formula (I) as described herein.

### DETAILED DESCRIPTION OF THE INVENTION

The present invention includes all pharmaceutically acceptable isotopically-labelled compounds of the present invention wherein one or more atoms are replaced by atoms having the same atomic number, but an atomic mass or mass number different from the atomic mass or mass number which predominates in nature.

Examples of isotopes suitable for inclusion in the compounds of the invention include, but are not limited to, isotopes of hydrogen, such as ²H and ³H, carbon, such as ¹¹C, ¹³C and ¹⁴C, chlorine, such as ³⁸Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of the present invention, for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the present invention can generally be prepared by conventional techniques known to those skilled in the art or by processes analogous to those described in the accompanying Examples and Preparations using an appropriate isotopically-labeled reagent in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

Specific embodiments of the present invention include the compounds exemplified in the Examples below and their pharmaceutically acceptable salts, complexes, solvates, polymorphs, stereoisomers, metabolites, prodrugs, and other derivatives thereof.

Unless otherwise specified, the following definitions apply to terms found in the specification and claims:

"C_{α-β}alk" means an alkyl group comprising a minimum of α and a maximum of β carbon atoms in a branched or linear relationship or any combination of the three, wherein α and β represent integers. The alkyl groups described in this section may also contain one or two double or triple bonds. A designation of C₀alk indicates a direct bond. Examples of C₁₋₆alkyl include, but are not limited to the following:

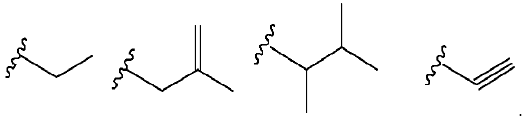

"Benzo group", alone or in combination, means the divalent radical C₄H₄=, one representation of which is -CH=CH-CH=CH-, that when vicinally attached to another ring forms a benzene-like ring--for example tetrahydronaphthylene, indole and the like.

The terms "oxo" and "thioxo" represent the groups =0 (as in carbonyl) and =S (as in thiocarbonyl), respectively.

"Halo" or "halogen" means a halogen atom selected from F, Cl, Br and I.

"C_{α-β}haloalk" means an alk group, as described above, wherein any number --at least one-- of the hydrogen atoms attached to the alk chain are replaced by F, Cl, Br or I.

The group N(R^{a})R^{a} and the like include substituents where the two R^{a} groups together form a ring, optionally including a N, O or S atom, and include groups such as:

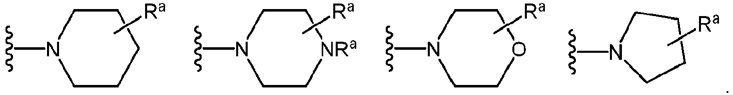

The group N(C_{α-β}alk) C_{α-β}palk, wherein α and β are as defined above, include substituents where the two C_{α-β}alk groups together form a ring, optionally including a N, O or S atom, and include groups such as

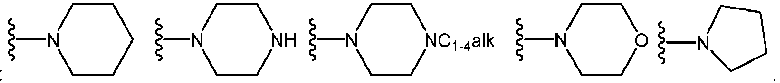

"Bicyclic ring" means a group that features two joined rings. A bicyclic ring can be carbocyclic (all of the ring atoms are carbons), or heterocyclic (the rings atoms consist, for example, 1, 2 or 3 heteroatoms, such as N, O, or S, in addition to carbon atoms). The two rings can both be aliphatic (*e.g*. decalin and norbomane), or can be aromatic (*e.g.*naphthalene), or a combination of aliphatic and aromatic (*e.g*. tetralin). Bicyclic rings include (a) spirocyclic compounds, wherein the two rings share only one single atom, the spiro atom, which is usually a quaternary carbon. Examples of spirocyclic compound include, but are not limited to: 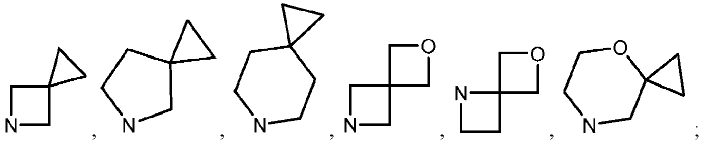 (b) fused bicyclic compounds, wherein two rings share two adjacent atoms. In other words, the rings share one covalent bond, i.e. the bridgehead atoms are directly connected (e.g. α-thujene and decalin). Examples of fused bicyclic rings include, but are not limited to: 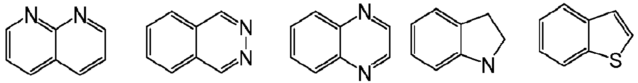 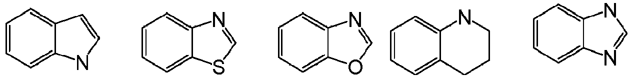 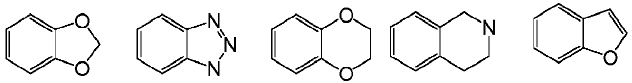 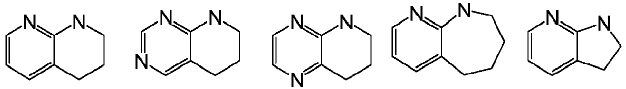 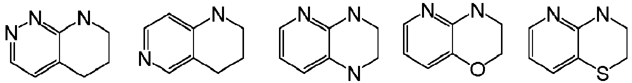 ; and (c) bridged bicyclic compounds, wherein the two rings share three or more atoms, separating the two bridgehead atoms by a bridge containing at least one atom. For example, norbornane, also known as bicyclo[2.2.1]heptane, can be thought of as a pair of cyclopentane rings each sharing three of their five carbon atoms. Examples of bridged bicyclic rings include, but are not limited to: 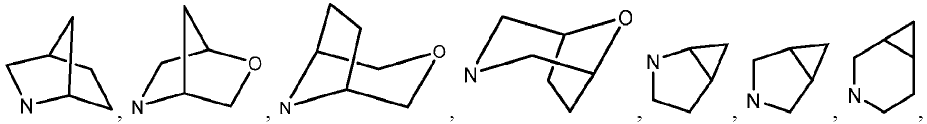 or

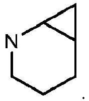

"Carbocycle" or "Carbocyclic" means a ring comprising by itself or in combination with other terms, represents, unless otherwise stated, cyclic version of "C_{α-β}alk". Examples of carbocycle include cyclopentyl, cyclohexyl, 1-cyclohexenyl, 3-cyclohexenyl, cycloheptyl, cyclobutylene, cyclohexylene and the like.

"Heterocycle" or "Heterocyclic" means a ring comprising at least one carbon atom and at least one other atom selected from N, O and S. Examples of heterocycles that may be found in the claims include, but are not limited to, the following: 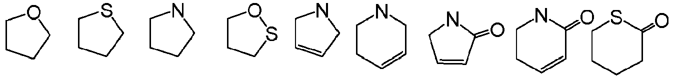 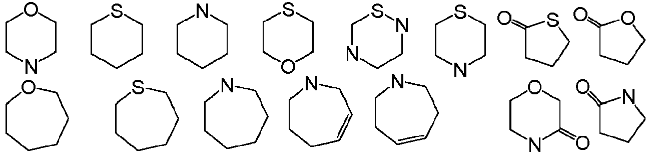 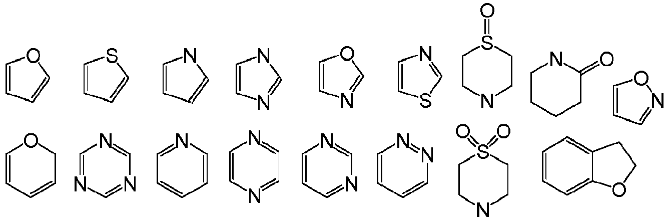 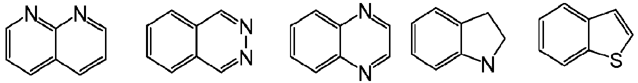 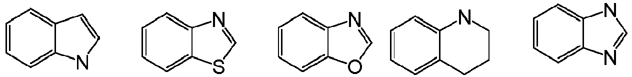 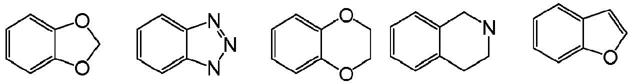 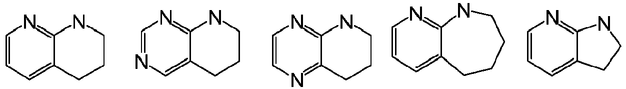 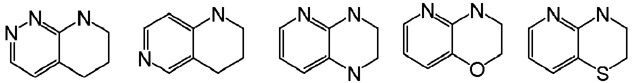 and

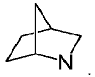

"Pharmaceutically-acceptable salt" means a salt prepared by conventional means, and are well known by those skilled in the art. The "pharmacologically acceptable salts" include basic salts of inorganic and organic acids, including but not limited to hydrochloric acid, hydrobromic acid, sulfuric acid, phosphoric acid, methane sulfonic acid, ethane sulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. When compounds of the invention include an acidic function such as a carboxy group, then suitable pharmaceutically acceptable cation pairs for the carboxy group are well known to those skilled in the art and include alkaline, alkaline earth, ammonium, quaternary ammonium cations and the like. For additional examples of "pharmacologically acceptable salts," *see infra* and Berge et al., J. Pharm. Sci. 66:1 (1977).

"Saturated, partially-saturated or unsaturated" includes substituents saturated with hydrogens, substituents completely unsaturated with hydrogens and substituents partially saturated with hydrogens.

"Leaving group" generally refers to groups readily displaceable by a nucleophile, such as an amine, a thiol or an alcohol nucleophile. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate.

"Protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. Preferred protecting groups are indicated herein where appropriate. Examples of amino protecting groups include, but are not limited to, aralkyl, substituted aralkyl, cycloalkenylalkyl and substituted cycloalkenyl alkyl, allyl, substituted allyl, acyl, alkoxycarbonyl, aralkoxycarbonyl, silyl and the like. Examples of aralkyl include, but are not limited to, benzyl, ortho-methylbenzyl, trityl and benzhydryl, which can be optionally substituted with halogen, alkyl, alkoxy, hydroxy, nitro, acylamino, acyl and the like, and salts, such as phosphonium and ammonium salts. Examples of aryl groups include phenyl, naphthyl, indanyl, anthracenyl, 9-(9-phenylfluorenyl), phenanthrenyl, durenyl and the like. Examples of cycloalkenylalkyl or substituted cycloalkylenylalkyl radicals, preferably have 6-10 carbon atoms, include, but are not limited to, cyclohexenyl methyl and the like. Suitable acyl, alkoxycarbonyl and aralkoxycarbonyl groups include benzyloxycarbonyl, t-butoxycarbonyl, iso-butoxycarbonyl, benzoyl, substituted benzoyl, butyryl, acetyl, trifluoroacetyl, trichloro acetyl, phthaloyl and the like. A mixture of protecting groups can be used to protect the same amino group, such as a primary amino group can be protected by both an aralkyl group and an aralkoxycarbonyl group. Amino protecting groups can also form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, such as nitrophthalimidyl. Amino groups may also be protected against undesired reactions, such as oxidation, through the formation of an addition salt, such as hydrochloride, toluene sulfonic acid, trifluoroacetic acid and the like. Many of the amino protecting groups are also suitable for protecting carboxy, hydroxy and mercapto groups. For example, aralkyl groups. Alkyl groups are also suitable groups for protecting hydroxy and mercapto groups, such as tert-butyl.

Silyl protecting groups are silicon atoms optionally substituted by one or more alkyl, aryl and aralkyl groups. Suitable silyl protecting groups include, but are not limited to, trimethylsilyl, triethylsilyl, triisopropylsilyl, tert-butyldimethylsilyl, dimethylphenylsilyl, 1,2-bis(dimethylsilyl)benzene, 1,2-bis(dimethylsilyl)ethane and diphenylmethylsilyl. Silylation of an amino groups provide mono- or di-silylamino groups. Silylation of aminoalcohol compounds can lead to a N,N,O-trisilyl derivative. Removal of the silyl function from a silyl ether function is readily accomplished by treatment with, for example, a metal hydroxide or ammonium fluoride reagent, either as a discrete reaction step or in situ during a reaction with the alcohol group. Suitable silylating agents are, for example, trimethylsilyl chloride, tert-butyl-dimethylsilyl chloride, phenyldimethylsilyl chloride, diphenylmethyl silyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art of organic chemistry including amino acid/amino acid ester or aminoalcohol chemistry.

Protecting groups are removed under conditions which will not affect the remaining portion of the molecule. These methods are well known in the art and include acid hydrolysis, hydrogenolysis and the like. A preferred method involves removal of a protecting group, such as removal of a benzyloxycarbonyl group by hydrogenolysis utilizing palladium on carbon in a suitable solvent system such as an alcohol, acetic acid, and the like or mixtures thereof. A t-butoxycarbonyl protecting group can be removed utilizing an inorganic or organic acid, such as HCl or trifluoroacetic acid, in a suitable solvent system, such as dioxane or methylene chloride. The resulting amino salt can readily be neutralized to yield the free amine. Carboxy protecting group, such as methyl, ethyl, benzyl, tert-butyl, 4-methoxyphenylmethyl and the like, can be removed under hydrolysis and hydrogenolysis conditions well known to those skilled in the art.

It should be noted that compounds of the invention may contain groups that may exist in tautomeric forms, such as cyclic and acyclic amidine and guanidine groups, heteroatom substituted heteroaryl groups (Y' = 0, S, NR), and the like, which are illustrated in the following examples: 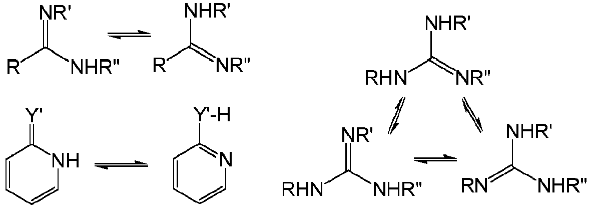 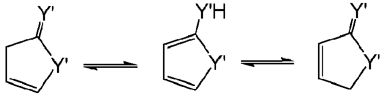 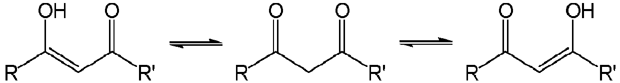 and though one form is named, described, displayed and/or claimed herein, all the tautomeric forms are intended to be inherently included in such name, description, display and/or claim.

Prodrugs of the compounds of this invention are also contemplated by this invention. A prodrug is an active or inactive compound that is modified chemically through in vivo physiological action, such as hydrolysis, metabolism and the like, into a compound of this invention following administration of the prodrug to a patient. The suitability and techniques involved in making and using prodrugs are well known by those skilled in the art. For a general discussion of prodrugs involving esters see Svensson and Tunek Drug Metabolism Reviews 165 (1988) and Bundgaard Design of Prodrugs, Elsevier (1985). Examples of a masked carboxylate anion include a variety of esters, such as alkyl (for example, methyl, ethyl), cycloalkyl (for example, cyclohexyl), aralkyl (for example, benzyl, p-methoxybenzyl), and alkylcarbonyloxyalkyl (for example, pivaloyloxymethyl). Amines have been masked as arylcarbonyloxymethyl substituted derivatives which are cleaved by esterases in vivo releasing the free drug and formaldehyde (Bungaard J. Med. Chem. 2503 (1989)). Also, drugs containing an acidic NH group, such as imidazole, imide, indole and the like, have been masked with N-acyloxymethyl groups (Bundgaard Design of Prodrugs, Elsevier (1985)). Hydroxy groups have been masked as esters and ethers. EP 039,051 (Sloan and Little, 4/11/81) discloses Mannich-base hydroxamic acid prodrugs, their preparation and use.

The specification and claims contain listing of species using the language "selected from ... and . . " and "is . . . or . . ." (sometimes referred to as Markush groups). When this language is used in this application, unless otherwise stated it is meant to include the group as a whole, or any single members thereof, or any subgroups thereof. The use of this language is merely for shorthand purposes and is not meant in any way to limit the removal of individual elements or subgroups as needed.

### PHARMACEUTICAL COMPOSITIONS, DOSING, AND ROUTES OF ADMINISTRATION

Also provided herein are pharmaceutical compositions that includes a compound as disclosed herein, together with a pharmaceutically acceptable excipient, such as, for example, a diluent or carrier. Compounds and pharmaceutical compositions suitable for use in the present invention include those wherein the compound can be administered in an effective amount to achieve its intended purpose. Administration of the compound described in more detail below.

Suitable pharmaceutical formulations can be determined by the skilled artisan depending on the route of administration and the desired dosage. See, e.g., Remington's Pharmaceutical Sciences, 1435-712 (18th ed., Mack Publishing Co, Easton, Pennsylvania, 1990). Formulations may influence the physical state, stability, rate of in vivo release and rate of in vivo clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein as well as the pharmacokinetic data obtainable through animal or human clinical trials.

The phrases "pharmaceutically acceptable" or "pharmacologically acceptable" refer to molecular entities and compositions that do not produce adverse, allergic, or other untoward reactions when administered to an animal or a human. As used herein, "pharmaceutically acceptable e" includes any and all solvents, dispersion media, coatings, antibacterial and antifungal agents, isotonic and absorption delaying agents and the like. The use of such excipients for pharmaceutically active substances is well known in the art. Except insofar as any conventional media or agent is incompatible with the therapeutic compositions, its use in therapeutic compositions is contemplated. Supplementary active ingredients also can be incorporated into the compositions. In exemplary embodiments, the formulation may comprise corn syrup solids, high-oleic safflower oil, coconut oil, soy oil, L-leucine, calcium phosphate tribasic, L-tyrosine, L-proline, L-lysine acetate, DATEM (an emulsifier), L-glutamine, L-valine, potassium phosphate dibasic, L-isoleucine, L-arginine, L-alanine, glycine, L-asparagine monohydrate, L-serine, potassium citrate, L-threonine, sodium citrate, magnesium chloride, L-histidine, L-methionine, ascorbic acid, calcium carbonate, L-glutamic acid, L-cystine dihydrochloride, L-tryptophan, L-aspartic acid, choline chloride, taurine, m-inositol, ferrous sulfate, ascorbyl palmitate, zinc sulfate, L-carnitine, alpha-tocopheryl acetate, sodium chloride, niacinamide, mixed tocopherols, calcium pantothenate, cupric sulfate, thiamine chloride hydrochloride, vitamin A palmitate, manganese sulfate, riboflavin, pyridoxine hydrochloride, folic acid, beta-carotene, potassium iodide, phylloquinone, biotin, sodium selenate, chromium chloride, sodium molybdate, vitamin D3 and cyanocobalamin.

The compound can be present in a pharmaceutical composition as a pharmaceutically acceptable salt. As used herein, "pharmaceutically acceptable salts" include, for example base addition salts and acid addition salts.

Pharmaceutically acceptable base addition salts may be formed with metals or amines, such as alkali and alkaline earth metals or organic amines. Pharmaceutically acceptable salts of compounds may also be prepared with a pharmaceutically acceptable cation. Suitable pharmaceutically acceptable cations are well known to those skilled in the art and include alkaline, alkaline earth, ammonium and quaternary ammonium cations. Carbonates or hydrogen carbonates are also possible. Examples of metals used as cations are sodium, potassium, magnesium, ammonium, calcium, or ferric, and the like. Examples of suitable amines include isopropylamine, trimethylamine, histidine, N,N'-dibenzylethylenediamine, chloroprocaine, choline, diethanolamine, dicyclohexylamine, ethylenediamine, N-methylglucamine, and procaine.

Pharmaceutically acceptable acid addition salts include inorganic or organic acid salts. Examples of suitable acid salts include the hydrochlorides, formates, acetates, citrates, salicylates, nitrates, phosphates. Other suitable pharmaceutically acceptable salts are well known to those skilled in the art and include, for example, formic, acetic, citric, oxalic, tartaric, or mandelic acids, hydrochloric acid, hydrobromic acid, sulfuric acid or phosphoric acid; with organic carboxylic, sulfonic, sulfo or phospho acids or N-substituted sulfamic acids, for example acetic acid, trifluoroacetic acid (TFA), propionic acid, glycolic acid, succinic acid, maleic acid, hydroxymaleic acid, methylmaleic acid, fumaric acid, malic acid, tartaric acid, lactic acid, oxalic acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid; and with amino acids, such as the 20 alpha amino acids involved in the synthesis of proteins in nature, for example glutamic acid or aspartic acid, and also with phenylacetic acid, methane sulfonic acid, ethanesulfonic acid, 2-hydroxyethanesulfonic acid, ethane 1,2-disulfonic acid, benzenesulfonic acid, 4-methylbenzenesulfonic acid, naphthalene 2-sulfonic acid, naphthalene 1,5-disulfonic acid, 2- or 3-phosphoglycerate, glucose 6-phosphate, N-cyclohexylsulfamic acid (with the formation of cyclamates), or with other acid organic compounds, such as ascorbic acid.

Pharmaceutical compositions containing the compounds disclosed herein can be manufactured in a conventional manner, e.g., by conventional mixing, dissolving, granulating, dragee-making, levigating, emulsifying, encapsulating, entrapping, or lyophilizing processes. Proper formulation is dependent upon the route of administration chosen.

For oral administration, suitable compositions can be formulated readily by combining a compound disclosed herein with pharmaceutically acceptable excipients such as carriers well known in the art. Such excipients and carriers enable the present compounds to be formulated as tablets, pills, dragees, capsules, liquids, gels, syrups, slurries, suspensions and the like, for oral ingestion by a patient to be treated. Pharmaceutical preparations for oral use can be obtained by adding a compound as disclosed herein with a solid excipient, optionally grinding a resulting mixture, and processing the mixture of granules, after adding suitable auxiliaries, if desired, to obtain tablets or dragee cores. Suitable excipients include, for example, fillers and cellulose preparations. If desired, disintegrating agents can be added. Pharmaceutically acceptable ingredients are well known for the various types of formulation and may be for example binders (e.g., natural or synthetic polymers), lubricants, surfactants, sweetening and flavoring agents, coating materials, preservatives, dyes, thickeners, adjuvants, antimicrobial agents, antioxidants and carriers for the various formulation types.

When a therapeutically effective amount of a compound disclosed herein is administered orally, the composition typically is in the form of a solid (e.g., tablet, capsule, pill, powder, or troche) or a liquid formulation (e.g., aqueous suspension, solution, elixir, or syrup).

When administered in tablet form, the composition can additionally contain a functional solid and/or solid carrier, such as a gelatin or an adjuvant. The tablet, capsule, and powder can contain about 1 to about 95% compound, and preferably from about 15 to about 90% compound.

When administered in liquid or suspension form, a functional liquid and/or a liquid carrier such as water, petroleum, or oils of animal or plant origin can be added. The liquid form of the composition can further contain physiological saline solution, sugar alcohol solutions, dextrose or other saccharide solutions, or glycols. When administered in liquid or suspension form, the composition can contain about 0.5 to about 90% by weight of a compound disclosed herein, and preferably about 1 to about 50% of a compound disclosed herein. In one embodiment contemplated, the liquid carrier is non-aqueous or substantially non-aqueous. For administration in liquid form, the composition may be supplied as a rapidly-dissolving solid formulation for dissolution or suspension immediately prior to administration.

When a therapeutically effective amount of a compound disclosed herein is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, in addition to a compound disclosed herein, an isotonic vehicle. Such compositions may be prepared for administration as solutions of free base or pharmacologically acceptable salts in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations can optionally contain a preservative to prevent the growth of microorganisms.

Injectable compositions can include sterile aqueous solutions, suspensions, or dispersions and sterile powders for the extemporaneous preparation of sterile injectable solutions, suspensions, or dispersions. In all embodiments the form must be sterile and must be fluid to the extent that easy syringability exists. It must be stable under the conditions of manufacture and storage and must resist the contaminating action of microorganisms, such as bacteria and fungi, by optional inclusion of a preservative. The carrier can be a solvent or dispersion medium containing, for example, water, ethanol, polyol (e.g., glycerol, propylene glycol, and liquid polyethylene glycol, and the like), suitable mixtures thereof, and vegetable oils. In one embodiment contemplated, the carrier is non-aqueous or substantially non-aqueous. The proper fluidity can be maintained, for example, by the use of a coating, such as lecithin, by the maintenance of the required particle size of the compound in the embodiment of dispersion and by the use of surfactants. The prevention of the action of microorganisms can be brought about by various antibacterial and antifungal agents, for example, parabens, chlorobutanol, phenol, sorbic acid, thimerosal, and the like. In many embodiments, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the embodiment of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Slow release or sustained release formulations may also be prepared in order to achieve a controlled release of the active compound in contact with the body fluids in the GI tract, and to provide a substantially constant and effective level of the active compound in the blood plasma. For example, release can be controlled by one or more of dissolution, diffusion, and ion-exchange. In addition, the slow release approach may enhance absorption via saturable or limiting pathways within the GI tract. For example, the compound may be embedded for this purpose in a polymer matrix of a biological degradable polymer, a water-soluble polymer or a mixture of both, and optionally suitable surfactants. Embedding can mean in this context the incorporation of micro-particles in a matrix of polymers. Controlled release formulations are also obtained through encapsulation of dispersed micro-particles or emulsified micro-droplets via known dispersion or emulsion coating technologies.

For administration by inhalation, compounds of the present invention are conveniently delivered in the form of an aerosol spray presentation from pressurized packs or a nebulizer, with the use of a suitable propellant. In the embodiment of a pressurized aerosol, the dosage unit can be determined by providing a valve to deliver a metered amount. Capsules and cartridges of, e.g., gelatin, for use in an inhaler or insufflator can be formulated containing a powder mix of the compound and a suitable powder base such as lactose or starch.

The compounds disclosed herein can be formulated for parenteral administration by injection (e.g., by bolus injection or continuous infusion). Formulations for injection can be presented in unit dosage form (e.g., in ampules or in multidose containers), with an added preservative. The compositions can take such forms as suspensions, solutions, or emulsions in oily or aqueous vehicles, and can contain formulatory agents such as suspending, stabilizing, and/or dispersing agents.

Pharmaceutical formulations for parenteral administration include aqueous solutions of the compounds in water-soluble form. Additionally, suspensions of the compounds can be prepared as appropriate oily injection suspensions. Suitable lipophilic solvents or vehicles include fatty oils or synthetic fatty acid esters. Aqueous injection suspensions can contain substances which increase the viscosity of the suspension. Optionally, the suspension also can contain suitable stabilizers or agents that increase the solubility of the compounds and allow for the preparation of highly concentrated solutions. Alternatively, a present composition can be in powder form for constitution with a suitable vehicle (e.g., sterile pyrogen-free water) before use.

Compounds disclosed herein also can be formulated in rectal compositions, such as suppositories or retention enemas (e.g., containing conventional suppository bases). In addition to the formulations described previously, the compounds also can be formulated as a depot preparation. Such long-acting formulations can be administered by implantation (e.g., subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

In particular, a compound disclosed herein can be administered orally, buccally, or sublingually in the form of tablets containing excipients, such as starch or lactose, or in capsules or ovules, either alone or in admixture with excipients, or in the form of elixirs or suspensions containing flavoring or coloring agents. Such liquid preparations can be prepared with pharmaceutically acceptable additives, such as suspending agents. A compound also can be injected parenterally, for example, intravenously, intramuscularly, subcutaneously, or intracoronarily. For parenteral administration, the compound is best used in the form of a sterile aqueous solution which can contain other substances, for example, salts, or sugar alcohols, such as mannitol, or glucose, to make the solution isotonic with blood.

For veterinary use, a compound disclosed herein is administered as a suitably acceptable formulation in accordance with normal veterinary practice. The veterinarian can readily determine the dosing regimen and route of administration that is most appropriate for a particular animal.

In some embodiments, all the necessary components for the treatment of KIF 18A-related disorder using a compound as disclosed herein either alone or in combination with another agent or intervention traditionally used for the treatment of such disease may be packaged into a kit. Specifically, the present invention provides a kit for use in the therapeutic intervention of the disease comprising a packaged set of medicaments that include the compound disclosed herein as well as buffers and other components for preparing deliverable forms of said medicaments, and/or devices for delivering such medicaments, and/or any agents that are used in combination therapy with the compound disclosed herein, and/or instructions for the treatment of the disease packaged with the medicaments. The instructions may be fixed in any tangible medium, such as printed paper, or a computer readable magnetic or optical medium, or instructions to reference a remote computer data source such as a world wide web page accessible via the internet.

A "therapeutically effective amount" means an amount effective to treat or to prevent development of, or to alleviate the existing symptoms of, the subject being treated. Determination of the effective amounts is well within the capability of those skilled in the art, especially in light of the detailed disclosure provided herein. Generally, a "therapeutically effective dose" refers to that amount of the compound that results in achieving the desired effect. For example, in one preferred embodiment, a therapeutically effective amount of a compound disclosed herein decreases KIF18A activity by at least 5%, compared to control, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, or at least 90%.

The amount of compound administered can be dependent on the subject being treated, on the subject's age, health, sex, and weight, the kind of concurrent treatment (if any), severity of the affliction, the nature of the effect desired, the manner and frequency of treatment, and the judgment of the prescribing physician. The frequency of dosing also can be dependent on pharmacodynamic effects on arterial oxygen pressures. While individual needs vary, determination of optimal ranges of effective amounts of the compound is within the skill of the art. Such doses may be administered in a single dose or it may be divided into multiple doses.

The terms "cancer" and "cancerous" when used herein refer to or describe the physiological condition in mammals that is typically characterized by unregulated cell growth. Examples of cancer include, without limitation, carcinoma, lymphoma, sarcoma, blastoma and leukemia. More particular examples of such cancers include squamous cell carcinoma, lung cancer, pancreatic cancer, cervical cancer, bladder cancer, hepatoma, breast cancer, colon carcinoma, and head and neck cancer, ovarian cancer, and endometrial cancer. While the term "cancer" as used herein is not limited to any one specific form of the disease, it is believed that the methods of the invention will be particularly effective for cancers which are found to be accompanied by unregulated levels of KIF18A or dependent on KIF 18A for proper chromosome segregation and survival in the mammal.

The terms "treat", "treating" and "treatment" as used herein refer to therapy, including without limitation, curative therapy, prophylactic therapy, and preventative therapy. Prophylactic treatment generally constitutes either preventing the onset of disorders altogether or delaying the onset of a pre-clinically evident stage of disorders in individuals.

The term "patient", "subject", or "mammal" as used herein refers to any "patient", "subject", or "mammal" including humans, cows, horses, dogs and cats. In one embodiment of the invention, the mammal is a human.

The term "comprising" is meant to be open ended, including the indicated component(s) but not excluding other elements.

The terms "Formula I" include any sub formulas.

### METHODS OF USING KIF18A INHIBITORS

The present disclosure provides compounds having MT-based KIF18A modulatory activity in general, and inhibitory activity in particular. In one embodiment of the invention, there is provided a method of modulating KIF 18A protein in a subject, the method comprising administering to the subject an effective dosage amount of a compound of Formulas I. As such, the compounds of the invention may be used to treat cellular proliferation disorders, including uncontrolled cell growth, aberrant cell cycle regulation, centrosome abnormalities (structural and or numeric, fragmentation). Other diseases or disorders associated with the accumulation of extra centrosomes (>2) include human papillomavirus (HPV) infection, including HPV-associated neoplasias. The compounds are also useful for cilia-related dieases as well as ablating haploid germ cell population which could be used as a male contraceptive.

In addition, compounds of the invention are useful for, but not limited to, the prevention or treatment of cancer and other KIF 18A-mediated diseases or disorders. For example, compounds of the invention would be useful for the treatment of various solid and hematologically derived tumors, such as carcinomas, including, without limitation, cancer of the bladder, breast, colon, kidney, liver, lung (including squamous cell and small cell lung cancer), esophagus, gall-bladder, ovary, pancreas, stomach, cervix, thyroid, prostate, and skin (including squamous cell carcinoma); hematopoietic tumors of lymphoid lineage (including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias, myelodysplastic syndrome and promyelocytic leukemia); tumors of mesenchymal origin (including fibrosarcoma and rhabdomyosarcoma, and other sarcomas, e.g. soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma).

The compounds of the invention are also useful in the treatment of cancer related indications such as solid tumors, sarcomas (especially Ewing's sarcoma and osteosarcoma), retinoblastoma, rhabdomyosarcomas, neuroblastoma, hematopoietic malignancies, including leukemia and lymphoma, tumor- induced pleural or pericardial effusions, and malignant ascites.

Based on the ability to modulate kinesin impacting angiogenesis, the compounds of the invention are also useful in treatment and therapy of proliferative diseases. Particularly, these compounds can be used for the treatment of an inflammatory disease, especially of manifestations at the locomotor apparatus, such as various inflammatory rheumatoid diseases, especially chronic polyarthritis including rheumatoid arthritis, juvenile arthritis or psoriasis arthropathy; paraneoplastic syndrome or tumor-induced inflammatory diseases, turbid effusions, collagenosis, such as systemic Lupus erythematosus, poly-myositis, dermato-myositis, systemic sclerodermia or mixed collagenosis; postinfectious arthritis (where no living pathogenic organism can be found at or in the affected part of the body), seronegative spondylarthritis, such as spondylitis ankylosans; vasculitis, sarcoidosis, or arthrosis; or further any combinations thereof.

The compounds of the invention can also be used as active agents against such disease states as arthritis, atherosclerosis, psoriasis, hemangiomas, myocardial angiogenesis, coronary and cerebral collaterals, ischemic limb angiogenesis, wound healing, peptic ulcer Helicobacter related diseases, fractures, cat scratch fever, rubeosis, neovascular glaucoma and retinopathies such as those associated with diabetic retinopathy or macular degeneration. In addition, some of these compounds can be used as active agents against solid tumors, malignant ascites, hematopoietic cancers and hyperproliferative disorders such as thyroid hyperplasia (especially Grave's disease), and cysts (such as hypervascularity of ovarian stroma, characteristic of polycystic ovarian syndrome (Stein- Leventhal syndrome)) since such diseases require a proliferation of blood vessel cells for growth and/or metastasis.

Besides being useful for human treatment, these compounds are useful for veterinary treatment of companion animals, exotic animals and farm animals, including mammals, rodents, and the like. For example, animals including horses, dogs, and cats may be treated with compounds provided by the invention.

### COMBINATIONS

While the compounds of the invention can be dosed or administered as the sole active pharmaceutical agent, they can also be used in combination with one or more compounds of the invention or in conjunction with other agents. When administered as a combination, the therapeutic agents can be formulated as separate compositions that are administered simultaneously or sequentially at different times, or the therapeutic agents can be given as a single composition.

The phrase "co-therapy" (or "combination-therapy"), in defining use of a compound of the present invention and another pharmaceutical agent, is intended to embrace administration of each agent in a sequential manner in a regimen that will provide beneficial effects of the drug combination, and is intended as well to embrace co-administration of these agents in a substantially simultaneous manner, such as in a single capsule having a fixed ratio of these active agents or in multiple, separate capsules for each agent.

Specifically, the administration of compounds of the present invention may be in conjunction with additional therapies known to those skilled in the art in the prevention or treatment of cancer, such as with radiation therapy, small molecule targeted agents (e.g. PARP inhibitors, kinase inhibitors), therapeutic antibodies (e.g. naked and drug-conjugate) immunotherapy antibodies (checkpoint inhibitors, bi-specific T-cell engagers) with neoplastic or cytotoxic agents.

If formulated as a fixed dose, such combination products employ the compounds of this invention within the accepted dosage ranges. Compounds of Formula I may also be administered sequentially with known anticancer or cytotoxic agents when a combination formulation is inappropriate. The invention is not limited in the sequence of administration; compounds of the invention may be administered either prior to, simultaneous with or after administration of the known anticancer or cytotoxic agent.

There are large numbers of anticancer agents available in commercial use, in clinical evaluation and in pre-clinical development, which would be selected for treatment of neoplasia by combination drug chemotherapy. Such agents fall into several major categories such as antibiotic-type agents, alkylating and alkylating-like agents, antimitotic agents, targeted small molecule agents, antimetabolite agents, hormonal agents, immunological agents, anti-angiogenic agents, interferon-type agents and a category of miscellaneous agents.

The present disclosure also provides methods for combination therapies in which an agent known to modulate other pathways, or other components of the same pathway, or even overlapping sets of target enzymes are used in combination with a compound of the present disclosure, or a pharmaceutically acceptable salt thereof. In one aspect, such therapy includes but is not limited to the combination of one or more compounds of the disclosure with chemotherapeutic agents, therapeutic antibodies, targeted small molecule agents, and radiation treatment, to provide a synergistic or additive therapeutic effect.

Many chemotherapeutics are presently known in the art and can be used in combination with the compounds of the disclosure. In some embodiments, the chemotherapeutic is selected from the group consisting of antimitotic agents, alkylating agents, anti-metabolites, intercalating antibiotics, growth factor inhibitors, cell cycle inhibitors, enzymes, topoisomerase inhibitors, biological response modifiers, anti-hormones, angiogenesis inhibitors, and anti-androgens. Non-limiting examples are chemotherapeutic agents, cytotoxic agents, and non-peptide small molecules such as Gleevec^{®} (Imatinib Mesylate), Kyprolis^{®} (carfilzomib), Velcade^{®} (bortezomib), Casodex (bicalutamide), Iressa^{®} (gefitinib), and Adriamycin as well as a host of chemotherapeutic agents. Non-limiting examples of chemotherapeutic agents include alkylating agents such as thiotepa and cyclosphosphamide (CYTOXANTM); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, CasodexTM, chromomycins, dactinomycin, daunorubicin, detorubicin, 6-diazo-5-oxo- L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel and docetaxel, Nab-paclitaxel; retinoic acid; esperamicins; capecitabine; and pharmaceutically acceptable salts, acids or derivatives of any of the above.

Also included as suitable chemotherapeutic cell conditioners are anti-hormonal agents that act to regulate or inhibit hormone action on tumors such as anti-estrogens including for example tamoxifen, (NolvadexTM), raloxifene, aromatase inhibiting 4(5)- imidazoles, 4-hydroxytamoxifen, trioxifene, keoxifene, LY 117018, onapristone, and toremifene (Fareston); and anti-androgens such as flutamide, nilutamide, bicalutamide, leuprolide, and goserelin; chlorambucil; gemcitabine; 6-thioguanine; mercaptopurine; methotrexate; platinum analogs such as cisplatin, oxaliplatin, carboplatin; etoposide (VP-16); ifosfamide; mitomycin C; mitoxantrone; vinblastine vincristine; vinorelbine; navelbine; novantrone; teniposide; daunomycin; aminopterin; xeloda; ibandronate; topotecan; camptothecin-11 (CPT-11); topoisomerase inhibitor RFS 2000; difluoromethylomithine (DMFO),

Where desired, the compounds or pharmaceutical composition of the present disclosure can be used in combination with commonly prescribed anti-cancer drugs such as Herceptin^{®}, Avastin^{®}, Erbitux^{®}, Rituxan^{®}, Taxol^{®}, Abraxane, Arimidex^{®}, Taxotere^{®}, ABVD, AVICINE, Abagovomab, Acridine carboxamide, Adecatumumab, 17-N-Allylamino-17-demethoxygeldanamycin, Alpharadin, Alvocidib, 3-Aminopyridine-2-carboxaldehyde thiosemicarbazone, Amonafide, Anthracenedione, Anti-CD22 immunotoxins, Antineoplastic, Antitumorigenic herbs, Apaziquone, Atiprimod, Azathioprine, Belotecan, Bendamustine, BIBW 2992, Biricodar, Brostallicin, Bryostatin, Buthionine sulfoximine, CBV (chemotherapy), Calyculin, cell-cycle nonspecific antineoplastic agents, Dichloroacetic acid, Discodermolide, Elsamitrucin, Enocitabine, Epothilone, Eribulin, Everolimus, Exatecan, Exisulind, Ferruginol, Forodesine, Fosfestrol, ICE chemotherapy regimen, IT-101, Imexon, Imiquimod, Indolocarbazole, Irofulven, Laniquidar, Larotaxel, Lenalidomide, Lucanthone, Lurtotecan, Mafosfamide, Mitozolomide, Nafoxidine, Nedaplatin, Olaparib, Talazoparib, Niraparib, Ortataxel, PAC-1, Pawpaw, Pixantrone, Proteasome inhibitor, Rebeccamycin, Resiquimod, Rubitecan, SN-38, Salinosporamide A, Sapacitabine, Stanford V, Swainsonine, Talaporfin, Tariquidar, Tegafur-uracil, Temodar, Tesetaxel, Triplatin tetranitrate, Tris(2-chloroethyl)amine, Troxacitabine, Uramustine, Vadimezan, Vinflunine, ZD6126 or Zosuquidar, CDK4/6 inhibitors (Palbociclib. Ibrance; Ribociclib, Kisqali; Abemaciclib, Verzenio).

This disclosure further relates to a method for using the compounds or pharmaceutical compositions provided herein, in combination with radiation therapy for inhibiting abnormal cell growth or treating the hyperproliferative disorder in the mammal. Techniques for administering radiation therapy are known in the art, and these techniques can be used in the combination therapy described herein. The administration of the compound of the disclosure in this combination therapy can be determined as described herein.

Radiation therapy can be administered through one of several methods, or a combination of methods, including without limitation external-beam therapy, internal radiation therapy, implant radiation, stereotactic radiosurgery, systemic radiation therapy, radiotherapy and permanent or temporary interstitial brachytherapy. The term "brachytherapy," as used herein, refers to radiation therapy delivered by a spatially confined radioactive material inserted into the body at or near a tumor or other proliferative tissue disease site. The term is intended without limitation to include exposure to radioactive isotopes (e.g. At-211, I-131, I-125, Y-90, Re-186, Re-188, Sm- 153, Bi-212, P-32, and radioactive isotopes of Lu). Suitable radiation sources for use as a cell conditioner of the present disclosure include both solids and liquids. By way of non-limiting example, the radiation source can be a radionuclide, such as I-125, I-131, Yb-169, Ir-192 as a solid source, I-125 as a solid source, or other radionuclides that emit photons, beta particles, gamma radiation, or other therapeutic rays. The radioactive material can also be a fluid made from any solution of radionuclide(s), e.g., a solution of I-125 or I-131, or a radioactive fluid can be produced using a slurry of a suitable fluid containing small particles of solid radionuclides, such as Au-198, Y-90. Moreover, the radionuclide(s) can be embodied in a gel or radioactive micro spheres.

The compounds or pharmaceutical compositions of the disclosure can be used in combination with an amount of one or more substances selected from anti- angiogenesis agents, signal transduction inhibitors, antiproliferative agents, glycolysis inhibitors, or autophagy inhibitors.

Anti-angiogenesis agents, such as MMP-2 (matrix-metalloproteinase 2) inhibitors, MMP-9 (matrix-metalloprotienase 9) inhibitors, and COX-11 (cyclooxygenase 11) inhibitors, can be used in conjunction with a compound of the disclosure and pharmaceutical compositions described herein. Anti-angiogenesis agents include, for example, rapamycin, temsirolimus (CCI-779), everolimus (RAD001), sorafenib, sunitinib, and bevacizumab. Examples of useful COX-II inhibitors include alecoxib, valdecoxib, and rofecoxib. Examples of useful matrix metalloproteinase inhibitors are described in WO 96/33172 WO 96/27583 European Patent Publication EP0818442, European Patent Publication EP1004578 , WO 98/07697, WO 98/03516, WO 98/34918, WO 98/34915, WO 98/33768, WO 98/30566, European Patent Publication 606046, European Patent Publication 931 788, WO 90/05719, WO 99/52910, WO 99/52889, WO 99/29667, WO1999007675 , European Patent Publication EP1786785, European Patent Publication No. EP1181017, United States Publication No. US20090012085 , United States Publication US5863 949, United States Publication US5861 510, and European Patent Publication EP0780386. Preferred MMP-2 and MMP-9 inhibitors are those that have little or no activity inhibiting MMP-1. More preferred, are those that selectively inhibit MMP-2 and/or AMP-9 relative to the other matrix- metalloproteinases (i. e., MAP-1, MMP-3, MMP-4, MMP-5, MMP-6, MMP- 7, MMP- 8, MMP-10, MMP-11, MMP-12, andMMP-13). Some specific examples of MMP inhibitors useful in the disclosure are AG-3340, RO 32-3555, and RS 13-0830.

The present compounds may also be used in co-therapies with other anti-neoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflomithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-N1, interferon alfa-n3, interferon alfacon-1, interferon alpha, natural, interferon beta, interferon beta-1a, interferon beta-1b, interferon gamma, natural interferon gamma-1a, interferon gamma-1b, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburiembodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, VIRULIZIN, zinostatin stimalamer, or zoledronic acid; abarelix; AE 941 (Aeterna), ambamustine, antisense oligonucleotide, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techniclone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

The compounds of the invention may further be used with VEGFR inhibitors. Other compounds described in the following patents and patent applications can be used in combination therapy: US 6,258,812, US 2003/0105091, WO 01/37820, US 6,235,764, WO 01/32651, US 6,630,500, US 6,515,004, US 6,713,485, US 5,521,184, US 5,770,599, US 5,747,498, WO 02/68406, WO 02/66470, WO 02/55501, WO 04/05279, WO 04/07481, WO 04/07458, WO 04/09784, WO 02/59110, WO 99/45009, WO 00/59509, WO 99/61422, US 5,990,141, WO 00/12089, and WO 00/02871.

In some embodiments, the combination comprises a composition of the present invention in combination with at least one anti-angiogenic agent. Agents are inclusive of, but not limited to, in vitro synthetically prepared chemical compositions, antibodies, antigen binding regions, radionuclides, and combinations and conjugates thereof. An agent can be an agonist, antagonist, allosteric modulator, toxin or, more generally, may act to inhibit or stimulate its target (e.g., receptor or enzyme activation or inhibition), and thereby promote cell death or arrest cell growth.

Exemplary anti-angiogenic agents include ERBITUX^{™} (IMC-C225), KDR (kinase domain receptor) inhibitory agents (e.g., antibodies and antigen binding regions that specifically bind to the kinase domain receptor), anti-VEGF agents (e.g., antibodies or antigen binding regions that specifically bind VEGF, or soluble VEGF receptors or a ligand binding region thereof) such as AVASTIN^{™} or VEGF-TRAP^{™}, and anti-VEGF receptor agents (e.g., antibodies or antigen binding regions that specifically bind thereto), EGFR inhibitory agents (e.g., antibodies or antigen binding regions that specifically bind thereto) such as Vectibix (panitumumab), IRESSA^{™} (gefitinib), TARCEVA^{™} (erlotinib), anti-Ang1 and anti-Ang2 agents (e.g., antibodies or antigen binding regions specifically binding thereto or to their receptors, e.g., Tie2/Tek), and anti-Tie2 kinase inhibitory agents (e.g., antibodies or antigen binding regions that specifically bind thereto). The pharmaceutical compositions of the present invention can also include one or more agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met".

Other anti-angiogenic agents include Campath, IL-8, B-FGF, Tek antagonists (Ceretti et al., U.S. Publication No. 2003/0162712; U.S. Patent No. 6,413,932), anti-TWEAK agents (e.g., specifically binding antibodies or antigen binding regions, or soluble TWEAK receptor antagonists; see, Wiley, U.S. Patent No. 6,727,225), ADAM distintegrin domain to antagonize the binding of integrin to its ligands (Fanslow et al., U.S. Publication No. 2002/0042368), specifically binding anti-eph receptor and/or anti-ephrin antibodies or antigen binding regions (U.S. Patent Nos. 5,981,245; 5,728,813; 5,969,110; 6,596,852; 6,232,447; 6,057,124 and patent family members thereof), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto).

Additional anti-angiogenic/anti-tumor agents include: SD-7784 (Pfizer, USA); cilengitide.(Merck KGaA, Germany, EPO 770622); pegaptanib octasodium, (Gilead Sciences, USA); Alphastatin, (BioActa, UK); M-PGA, (Celgene, USA, US 5712291); ilomastat, (Arriva, USA, US 5892112); emaxanib, (Pfizer, USA, US 5792783); vatalanib, (Novartis, Switzerland); 2-methoxyestradiol, (EntreMed, USA); TLC ELL-12, (Elan, Ireland); anecortave acetate, (Alcon, USA); alpha-D148 Mab, (Amgen, USA); CEP-7055,(Cephalon, USA); anti-Vn Mab, (Crucell, Netherlands) DAC:antiangiogenic, (ConjuChem, Canada); Angiocidin, (InKine Pharmaceutical, USA); KM-2550, (KyowaHakko, Japan); SU-0879, (Pfizer, USA); CGP-79787, (Novartis, Switzerland, EP 970070); ARGENT technology, (Ariad, USA); YIGSR-Stealth, (Johnson & Johnson, USA); fibrinogen-E fragment, (BioActa, UK); angiogenesis inhibitor, (Trigen, UK); TBC-1635, (Encysive Pharmaceuticals, USA); SC-236, (Pfizer, USA); ABT-567, (Abbott, USA); Metastatin, (EntreMed, USA); angiogenesis inhibitor, (Tripep, Sweden); maspin, (Sosei, Japan); 2-methoxyestradiol, (Oncology Sciences Corporation, USA); ER-68203-00, (IVAX, USA); Benefin, (Lane Labs, USA); Tz-93, (Tsumura, Japan); TAN-1120, (Takeda, Japan); FR-111142, (Fujisawa, Japan, JP 02233610); platelet factor 4, (RepliGen, USA, EP 407122); vascular endothelial growth factor antagonist, (Borean, Denmark); bevacizumab (pINN), (Genentech, USA); angiogenesis inhibitors, (SUGEN, USA); XL 784, (Exelixis, USA); XL 647, (Exelixis, USA); MAb, alpha5beta3 integrin, second generation, (Applied Molecular Evolution, USA and MedImmune, USA); gene therapy, retinopathy, (Oxford BioMedica, UK); enzastaurin hydrochloride (USAN), (Lilly, USA); CEP 7055, (Cephalon, USA and Sanofi-Synthelabo, France); BC 1, (Genoa Institute of Cancer Research, Italy); angiogenesis inhibitor, (Alchemia, Australia); VEGF antagonist, (Regeneron, USA); rBPI 21 and BPI-derived antiangiogenic, (XOMA, USA); PI 88, (Progen, Australia); cilengitide (pINN), (Merck KGaA, German; Munich Technical University, Germany, Scripps Clinic and Research Foundation, USA); cetuximab (INN), (Aventis, France); AVE 8062, (Ajinomoto, Japan); AS 1404, (Cancer Research Laboratory, New Zealand); SG 292, (Telios, USA); Endostatin, (Boston Childrens Hospital, USA); ATN 161, (Attenuon, USA); ANGIOSTATIN, (Boston Childrens Hospital, USA); 2-methoxyestradiol, (Boston Childrens Hospital, USA); ZD 6474, (AstraZeneca, UK); ZD 6126, (Angiogene Pharmaceuticals, UK); PPI 2458, (Praecis, USA); AZD 9935, (AstraZeneca, UK); AZD 2171, (AstraZeneca, UK); vatalanib (pINN), (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); vaccine, gene-based, VEGF-2, (Scripps Clinic and Research Foundation, USA); SPV5.2, (Supratek, Canada); SDX 103, (University of California at San Diego, USA); PX 478, (ProlX, USA); METASTATIN, (EntreMed, USA); troponin I, (Harvard University, USA); SU 6668, (SUGEN, USA); OXI 4503, (OXiGENE, USA); o-guanidines, ( Dimensional Pharmaceuticals, USA); motuporamine C, (British Columbia University, Canada); CDP 791, (Celltech Group, UK); atiprimod (pINN), (GlaxoSmithKline, UK); E 7820, (Eisai, Japan); CYC 381, (Harvard University, USA); AE 941, (Aetema, Canada); vaccine, angiogenesis, (EntreMed, USA); urokinase plasminogen activator inhibitor, (Dendreon, USA); oglufanide (pINN), (Melmotte, USA); HIF-1alfa inhibitors, (Xenova, UK); CEP 5214, (Cephalon, USA); BAY RES 2622, (Bayer, Germany); Angiocidin, (InKine, USA); A6, (Angstrom, USA); KR 31372, (Korea Research Institute of Chemical Technology, South Korea); GW 2286, (GlaxoSmithKline, UK); EHT 0101, (ExonHit, France); CP 868596, (Pfizer, USA); CP 564959, (OSI, USA); CP 547632, (Pfizer, USA); 786034, (GlaxoSmithKline, UK); KRN 633, (Kirin Brewery, Japan); drug delivery system, intraocular, 2-methoxyestradiol, (EntreMed, USA); anginex, (Maastricht University, Netherlands, and Minnesota University, USA); ABT 510, (Abbott, USA); AAL 993, (Novartis, Switzerland); VEGI, (ProteomTech, USA); tumor necrosis factor-alpha inhibitors, (National Institute on Aging, USA); SU 11248, (Pfizer, USA and SUGEN USA); ABT 518, (Abbott, USA); YH16, (Yantai Rongchang, China); S-3APG, (Boston Childrens Hospital, USA and EntreMed, USA); MAb, KDR, (ImClone Systems, USA); MAb, alpha5 betal, (Protein Design, USA); KDRkinase inhibitor, (Celltech Group, UK, and Johnson & Johnson, USA); GFB 116, (South Florida University, USA and Yale University, USA); CS 706, (Sankyo, Japan); combretastatin A4 prodrug, (Arizona State University, USA); chondroitinase AC, (IBEX, Canada); BAY RES 2690, (Bayer, Germany); AGM 1470, (Harvard University, USA, Takeda, Japan, and TAP, USA); AG 13925, (Agouron, USA); Tetrathiomolybdate, (University of Michigan, USA); GCS 100, (Wayne State University, USA) CV 247, (Ivy Medical, UK); CKD 732, (Chong Kun Dang, South Korea); MAb, vascular endothelium growth factor, (Xenova, UK); irsogladine (INN), (Nippon Shinyaku, Japan); RG 13577, (Aventis, France); WX 360, (Wilex, Germany); squalamine (pINN), (Genaera, USA); RPI 4610, (Sima, USA); cancer therapy, (Marinova, Australia); heparanase inhibitors, (InSight, Israel); KL 3106, (Kolon, South Korea); Honokiol, (Emory University, USA); ZK CDK, (Schering AG, Germany); ZK Angio, (Schering AG, Germany); ZK 229561, (Novartis, Switzerland, and Schering AG, Germany); XMP 300, (XOMA, USA); VGA 1102, (Taisho, Japan); VEGF receptor modulators, (Pharmacopeia, USA); VE-cadherin-2 antagonists , (ImClone Systems, USA); Vasostatin, (National Institutes of Health, USA);vaccine, Flk-1, (ImClone Systems, USA); TZ 93, (Tsumura, Japan); TumStatin, (Beth Israel Hospital, USA); truncated soluble FLT 1 (vascular endothelial growth factor receptor 1), (Merck & Co, USA); Tie-2 ligands, (Regeneron, USA); and, thrombospondin 1 inhibitor, (Allegheny Health, Education and Research Foundation, USA).

Autophagy inhibitors include, but are not limited to chloroquine, 3- methyladenine, hydroxychloroquine (Plaquenil^{™}), bafilomycin A1, 5-amino-4- imidazole carboxamide riboside (AICAR), okadaic acid, autophagy-suppressive algal toxins which inhibit protein phosphatases of type 2A or type 1, analogues of cAMP, and drugs which elevate cAMP levels such as adenosine, LY204002, N6-mercaptopurine riboside, and vinblastine. In addition, antisense or siRNA that inhibits expression of proteins including but not limited to ATG5 (which are implicated in autophagy), may also be used.

Additional pharmaceutically active compounds/agents that can be used in the treatment of cancers and that can be used in combination with one or more compound of the present invention include: epoetin alfa; darbepoetin alfa; panitumumab; pegfilgrastim; palifermin; filgrastim; denosumab; ancestim; AMG 102; AMG 386; AMG 479; AMG 655; AMG 745; AMG 951; and AMG 706, or a pharmaceutically acceptable salt thereof.

In certain embodiments, a composition provided herein is conjointly administered with a chemotherapeutic agent. Suitable chemotherapeutic agents may include, natural products such as vinca alkaloids (e.g., vinblastine, vincristine, and vinorelbine), paclitaxel, epidipodophyllotoxins (e.g., etoposide and teniposide), antibiotics (e.g., dactinomycin (actinomycin D), daunorubicin, doxorubicin, and idarubicin), anthracyclines, mitoxantrone, bleomycins, plicamycin (mithramycin), mitomycin, enzymes (e.g., L-asparaginase which systemically metabolizes L-asparagine and deprives cells which do not have the capacity to synthesize their own asparagine), antiplatelet agents, antiproliferative/antimitotic alkylating agents such as nitrogen mustards (e.g., mechlorethamine, cyclophosphamide and analogs, melphalan, and chlorambucil), ethylenimines and methylmelamines (e.g., hexaamethylmelaamine and thiotepa), CDK inhibitors (e.g., seliciclib, UCN-01, P1446A-05, PD-0332991, dinaciclib, P27-00, AT-7519, RGB286638, and SCH727965), alkyl sulfonates (e.g., busulfan), nitrosoureas (e.g., carmustine (BCNU) and analogs, and streptozocin), trazenes-dacarbazinine (DTIC), antiproliferative/antimitotic antimetabolites such as folic acid analogs (e.g., methotrexate), pyrimidine analogs (e.g., fluorouracil, floxuridine, and cytarabine), purine analogs and related inhibitors (e.g., mercaptopurine, thioguanine, pentostatin and 2-chlorodeoxyadenosine), aromatase inhibitors (e.g., anastrozole, exemestane, and letrozole), and platinum coordination complexes (e.g., cisplatin and carboplatin), procarbazine, hydroxyurea, mitotane, aminoglutethimide, histone deacetylase (HDAC) inhibitors (e.g., trichostatin, sodium butyrate, apicidan, suberoyl anilide hydroamic acid, vorinostat, LBH 589, romidepsin, ACY-1215, and panobinostat), mTor inhibitors (e.g., temsirolimus, everolimus, ridaforolimus, and sirolimus), KSP(Eg5) inhibitors (e.g., Array 520), DNA binding agents (e.g., Zalypsis), PI3K delta inhibitor (e.g., GS-1101 and TGR-1202), PI3K delta and gamma inhibitor (e.g., CAL-130), multi-kinase inhibitor (e.g., TG02 and sorafenib), hormones (e.g., estrogen) and hormone agonists such as leutinizing hormone releasing hormone (LHRH) agonists (e.g., goserelin, leuprolide and triptorelin), BAFF-neutralizing antibody (e.g., LY2127399), IKK inhibitors, p38MAPK inhibitors, anti-IL-6 (e.g., CNTO328), telomerase inhibitors (e.g., GRN 163L), aurora kinase inhibitors (e.g., MLN8237, AMG 900, AZD-1152), cell surface monoclonal antibodies (e.g., anti-CD38 (HUMAX-CD38), anti-CS1 (e.g., elotuzumab), HSP90 inhibitors (e.g., 17 AAG and KOS 953), P13K / Akt inhibitors (e.g., perifosine), Akt inhibitor (e.g., GSK-2141795), PKC inhibitors (e.g., enzastaurin), FTIs (e.g., Zarnestra^{™}), anti-CD138 (e.g., BT062), Torc1/2 specific kinase inhibitor (e.g., INK128), kinase inhibitor (e.g., GS-1101), ER/UPR targeting agent (e.g., MKC-3946), cFMS inhibitor (e.g., ARRY-382), JAK1/2 inhibitor (e.g., CYT387), PARP inhibitor (e.g., olaparib, Talazoparib, Niraparib veliparib (ABT-888)), BCL-2 antagonist. Other chemotherapeutic agents may include mechlorethamine, camptothecin, ifosfamide, tamoxifen, raloxifene, gemcitabine, navelbine, sorafenib, or any analog or derivative variant of the foregoing.

The compounds of the present invention may also be used in combination with radiation therapy, hormone therapy, surgery and immunotherapy, which therapies are well known to those skilled in the art.

In certain embodiments, a pharmaceutical composition provided herein is conjointly administered with a steroid. Suitable steroids may include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts and/or derivatives thereof.In a particular embodiment, the compounds of the present invention can also be used in combination with additional pharmaceutically active agents that treat nausea. Examples of agents that can be used to treat nausea include: dronabinol; granisetron; metoclopramide; ondansetron; and prochlorperazine; or a pharmaceutically acceptable salt thereof.

The compounds or pharmaceutical compositions of the disclosure can also be used in combination with an amount of one or more substances selected from EGFR inhibitors, MEK inhibitors, PI3K inhibitors, AKT inhibitors, TOR inhibitors, and immune therapies, including anti-PD-1, anti-PDL-1, anti-CTLA4, anti-LAG1, and anti-OX40 agents, GITR agonists, CAR-T cells, and BiTEs.

EGFR inhibitors include, but are not limited to, small molecule antagonists, antibody inhibitors, or specific antisense nucleotide or siRNA. Useful antibody inhibitors of EGFR include cetuximab (Erbitux), panitumumab (Vectibix), zalutumumab, nimotuzumab, and matuzumab. Small molecule antagonists of EGFR include gefitinib, erlotinib (Tarceva), and most recently, lapatinib (TykerB). See e.g., Yan L, et. al., Pharmacogenetics and Pharmacogenomics In Oncology Therapeutic Antibody Development, BioTechniques 2005; 39(4): 565-8, and Paez J G, et. al., EGFR Mutations In Lung Cancer Correlation With Clinical Response To Gefitinib Therapy, Science 2004; 304(5676): 1497-500.

Non-limiting examples of small molecule EGFR inhibitors include any of the EGFR inhibitors described in the following patent publications, and all pharmaceutically acceptable salts and solvates of said EGFR inhibitors: European Patent Application EP 520722, published Dec. 30, 1992; European Patent Application EP 566226, published Oct. 20, 1993; PCT International Publication WO 96/33980, published Oct. 31, 1996; U.S. Pat. No. 5,747,498, issued May 5, 1998; PCT International Publication WO 96/30347, published Oct. 3, 1996; European Patent Application EP 787772, published Aug. 6, 1997; PCT International Publication WO 97/30034, published Aug. 21, 1997; PCT International Publication WO 97/30044, published Aug. 21, 1997; PCT International Publication WO 97/38994, published Oct. 23, 1997; PCT International Publication WO 97/49688, published Dec. 31, 1997; European Patent Application EP 837063, published Apr. 22, 1998; PCT International Publication WO 98/02434, published Jan. 22, 1998; PCT International Publication WO 97/38983, published Oct. 23, 1997; PCT International Publication WO 95/19774, published Jul. 27, 1995; PCT International Publication WO 95/19970, published Jul. 27, 1995; PCT International Publication WO 97/13771, published Apr. 17, 1997; PCT International Publication WO 98/02437, published Jan. 22, 1998; PCT International Publication WO 98/02438, published Jan. 22, 1998; PCT International Publication WO 97/32881, published Sep. 12, 1997; German Application DE 19629652, published Jan. 29, 1998; PCT International Publication WO 98/33798, published Aug. 6, 1998; PCT International Publication WO 97/32880, published Sep. 12, 1997; PCT International Publication WO 97/32880 published Sep. 12, 1997; European Patent Application EP 682027, published Nov. 15, 1995; PCT International Publication WO 97/02266, published Jan. 23, 197; PCT International Publication WO 97/27199, published Jul. 31, 1997; PCT International Publication WO 98/07726, published Feb. 26, 1998; PCT International Publication WO 97/34895, published Sep. 25, 1997; PCT International Publication WO 96/31510', published Oct. 10, 1996; PCT International Publication WO 98/14449, published Apr. 9, 1998; PCT International Publication WO 98/14450, published Apr. 9, 1998; PCT International Publication WO 98/14451, published Apr. 9, 1998; PCT International Publication WO 95/09847, published Apr. 13, 1995; PCT International Publication WO 97/19065, published May 29, 1997; PCT International Publication WO 98/17662, published Apr. 30, 1998; U.S. Pat. No. 5,789,427, issued Aug. 4, 1998; U.S. Pat. No. 5,650,415, issued Jul. 22, 1997; U.S. Pat. No. 5,656,643, issued Aug. 12, 1997; PCT International Publication WO 99/35146, published Jul. 15, 1999; PCT International Publication WO 99/35132, published Jul. 15, 1999; PCT International Publication WO 99/07701, published Feb. 18, 1999; and PCT International Publication WO 92/20642 published Nov. 26, 1992. Additional non-limiting examples of small molecule EGFR inhibitors include any of the EGFR inhibitors described in Traxler, P., 1998, Exp. Opin. Ther. Patents 8(12):1599-1625.

Antibody-based EGFR inhibitors include any anti-EGFR antibody or antibody fragment that can partially or completely block EGFR activation by its natural ligand. Non-limiting examples of antibody-based EGFR inhibitors include those described in Modjtahedi, H., et al., 1993, Br. J. Cancer 67:247-253; Teramoto, T., et al., 1996, Cancer 77:639-645; Goldstein et al., 1995, Clin. Cancer Res. 1:1311-1318; Huang, S. M., et al., 1999, Cancer Res. 15:59(8):1935-40; and Yang, X., et al., 1999, Cancer Res. 59:1236-1243. Thus, the EGFR inhibitor can be monoclonal antibody Mab E7.6.3 (Yang, 1999 supra), or Mab C225 (ATCC Accession No. HB-8508), or an antibody or antibody fragment having the binding specificity thereof.

MEK inhibitors include, but are not limited to, CI-1040, AZD6244, PD318088, PD98059, PD334581, RDEA119, ARRY-142886, ARRY-438162, and PD-325901.

PI3K inhibitors include, but are not limited to, wortmannin, 17-hydroxywortmannin analogs described in WO 06/044453, 4-[2-(1H-Indazol-4-yl)-6-[[4-(methylsulfonyl)piperazin-1-yl]methyl]thieno[3,2-d]pyrimidin-4-yl]morpholine (also known as GDC 0941 and described in PCT Publication Nos. WO 09/036,082 and WO 09/055,730), 2-Methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in PCT Publication No. WO 06/122806), (S)-1-(4-((2-(2-aminopyrimidin-5-yl)-7-methyl-4-morpholinothieno[3,2-d]pyrimidin-6-yl)methyl)piperazin-1-yl)-2-hydroxypropan-1-one (described in PCT Publication No. WO 2008/070740), LY294002 (2-(4-Morpholinyl)-8-phenyl-4H-1-benzopyran-4-one available from Axon Medchem), PI 103 hydrochloride (3-[4-(4-morpholinylpyrido-[3',2':4,5]furo[3,2-d]pyrimidin-2-yl]phenol hydrochloride available from Axon Medchem), PIK 75 (N'-[(1E)-(6-bromoimidazo[1,2-a]pyridin-3-yl)methylene]-N,2-dimethyl-5-nitrobenzenesulfono-hydrazide hydrochloride available from Axon Medchem), PIK 90 (N-(7,8-dimethoxy-2,3-dihydro-imidazo[1,2-c]quinazolin-5-yl)-nicotinamide available from Axon Medchem), GDC-0941 bismesylate (2-(1H-Indazol-4-yl)-6-(4-methanesulfonyl-piperazin-1-ylmethyl)-4-morpholin-4-yl-thieno[3,2-d]pyrimidine bismesylate available from Axon Medchem), AS-252424 (5-[1-[5-(4-Fluoro-2-hydroxy-phenyl)-furan-2-yl]-meth-(Z)-ylidene]-thiazolidine-2,4-dione available from Axon Medchem), and TGX-221 (7-Methyl-2-(4-morpholinyl)-9-[1-(phenylamino)ethyl]-4H-pyrido-[1,2-a]pyrimidin-4-one available from Axon Medchem), XL-765, and XL-147. Other PI3K inhibitors include demethoxyviridin, perifosine, CAL101, PX-866, BEZ235, SF1126, INK1117, IPI-145, BKM120, XL147, XL765, Palomid 529, GSK1059615, ZSTK474, PWT33597, IC87114, TG100-115, CAL263, PI-103, GNE-477, CUDC-907, and AEZS-136.

AKT inhibitors include, but are not limited to, Akt-1-1 (inhibits Akt1) (Barnett et al. (2005) Biochem. J., 385 (Pt. 2), 399-408); Akt-1-1,2 (inhibits Ak1 and 2) (Barnett et al. (2005) Biochem. J. 385 (Pt. 2), 399-408); API-59CJ-Ome (e.g., Jin et al. (2004) Br. J. Cancer 91, 1808-12); 1-H-imidazo[4,5-c]pyridinyl compounds (e.g., WO05011700); indole-3-carbinol and derivatives thereof (e.g., U.S. Pat. No. 6,656,963; Sarkar and Li (2004) J Nutr. 134(12 Suppl), 3493S-3498S); perifosine (e.g., interferes with Akt membrane localization; Dasmahapatra et al. (2004) Clin. Cancer Res. 10(15), 5242-52, 2004); phosphatidylinositol ether lipid analogues (e.g., Gills and Dennis (2004) Expert. Opin. Investig. Drugs 13, 787-97); and triciribine (TCN or API-2 or NCI identifier: NSC 154020; Yang et al. (2004) Cancer Res. 64, 4394-9).

TOR inhibitors include, but are not limited to, inhibitors include AP-23573, CCI-779, everolimus, RAD-001, rapamycin, temsirolimus, ATP-competitive TORC1/TORC2 inhibitors, including PI-103, PP242, PP30 and Torin 1. Other TOR inhibitors in FKBP12 enhancer; rapamycins and derivatives thereof, including: CCI-779 (temsirolimus), RAD001 (Everolimus; WO 9409010) and AP23573; rapalogs, e.g. as disclosed in WO 98/02441 and WO 01/14387, e.g. AP23573, AP23464, or AP23841; 40-(2-hydroxyethyl)rapamycin, 40-[3-hydroxy(hydroxymethyl)methylpropanoate]-rapamycin (also called CC1779), 40-epi-(tetrazolyt)-rapamycin (also called ABT578), 32-deoxorapamycin, 16-pentynyloxy-32(S)-dihydrorapanycin, and other derivatives disclosed in WO 05005434; derivatives disclosed in U.S. Pat. No. 5,258,389, WO 94/090101, WO 92/05179, U.S. Pat. No. 5,118,677, U.S. Pat. No. 5,118,678, U.S. Pat. No. 5,100,883, U.S. Pat. No. 5,151,413, U.S. Pat. No. 5,120,842, WO 93/111130, WO 94/02136, WO 94/02485, WO 95/14023, WO 94/02136, WO 95/16691, WO 96/41807, WO 96/41807 and U.S. Pat. No. 5,256,790; phosphorus-containing rapamycin derivatives (e.g., WO 05016252); 4H-1-benzopyran-4-one derivatives (e.g., U.S. Provisional Application No. 60/528,340).

Immune therapies include, but are not limited to, anti-PD-1 agents, anti-PDL-1 agents, anti-CTLA-4 agents, anti-LAG1 agents, and anti-OX40 agents. Exemplary anti-PD-1 antibodies and methods for their use are described by Goldberg et al., Blood 110(1):186-192 (2007), Thompson et al., Clin. Cancer Res. 13(6): 1757-1761 (2007), and Korman et al., International Application No. PCT/JP2006/309606 (publication no. WO 2006/121168 A1) include: Yervoy^{™} (ipilimumab) or Tremelimumab (to CTLA-4), galiximab (to B7.1). BMS-936558 (to PD-1), MK-3475 (to PD-1), AMP224 (to B7DC), BMS-936559 (to B7-H1), MPDL3280A (to B7-H1), MEDI-570 (to ICOS), AMG557 (to B7H2), MGA271 (to B7H3), IMP321 (to LAG-3), BMS-663513 (to CD137), PF-05082566 (to CD137), CDX-1127 (to CD27), anti-OX40 (Providence Health Services), huMAbOX40L (to OX40L), Atacicept (to TACI), CP-870893 (to CD40), Lucatumumab (to CD40), Dacetuzumab (to CD40), Muromonab-CD3 (to CD3), Ipilumumab (to CTLA-4). Immune therapies also include genetically engineered T-cells (e.g., CAR-T cells) and bispecific antibodies (e.g., BiTEs).

GITR agonists include, but are not limited to, GITR fusion proteins and anti-GITR antibodies (e.g., bivalent anti-GITR antibodies), such as, a GITR fusion protein described in U.S. Pat. No. 6,111,090box.c, European Patent No.: 090505B1, U.S. Pat. No. 8,586,023, PCT Publication Nos.: WO 2010/003118 and 2011/090754, or an anti-GITR antibody described, e.g., in U.S. Pat. No. 7,025,962, European Patent No.: 1947183B1, U.S. Pat. No. 7,812,135, U.S. Pat. No. 8,388,967, U.S. Pat. No. 8,591,886, European Patent No.: EP 1866339, PCT Publication No.: WO 2011/028683, PCT Publication No.: WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Pat. No. 7,618,632, and PCT Publication No.: WO 2011/051726.

The compounds described herein can be used in combination with the agents disclosed herein or other suitable agents, depending on the condition being treated. Hence, in some embodiments the one or more compounds of the disclosure will be co-administered with other agents as described above. When used in combination therapy, the compounds described herein are administered with the second agent simultaneously or separately. This administration in combination can include simultaneous administration of the two agents in the same dosage form, simultaneous administration in separate dosage forms, and separate administration. That is, a compound described herein and any of the agents described above can be formulated together in the same dosage form and administered simultaneously. Alternatively, a compound of the disclosure and any of the agents described above can be simultaneously administered, wherein both the agents are present in separate formulations. In another alternative, a compound of the present disclosure can be administered just followed by and any of the agents described above, or vice versa. In some embodiments of the separate administration protocol, a compound of the disclosure and any of the agents described above are administered a few minutes apart, or a few hours apart, or a few days apart.

As one aspect of the present invention contemplates the treatment of the disease/conditions with a combination of pharmaceutically active compounds that may be administered separately, the invention further relates to combining separate pharmaceutical compositions in kit form. The kit comprises two separate pharmaceutical compositions: a compound of the present invention, and a second pharmaceutical compound. The kit comprises a container for containing the separate compositions such as a divided bottle or a divided foil packet. Additional examples of containers include syringes, boxes, and bags. In some embodiments, the kit comprises directions for the use of the separate components. The kit form is particularly advantageous when the separate components are preferably administered in different dosage forms (e.g., oral and parenteral), are administered at different dosage intervals, or when titration of the individual components of the combination is desired by the prescribing health care professional.

### EXPERIMENTAL

Abbreviations: The following abbreviations may be used herein:

| | |
|---|---|
| ACN or MeCH | acetonitrile |
| AcOH | acetic acid |
| aq or aq. | aqueous |
| BOC or Boc | *tert*-butyloxycarbonyl |
| BrettPhos | 2-(dicyclohexylphosphino)3,6-dimethoxy-2',4',6'-triisopropyl-1,1'-biphenyl |
| cataCXium^{®} A | rac-((3*R*,5*R*,7*R*)-adamantan-1-yl)((3*S*,5*S*,7*S*-adamantan-1-yl)(butyl)phosphane |
| DABSO | 1,4-diazabicyclo[2.2.2]octane bis(sulfur dioxide) adduct |
| DCE | 1,2-dichloroethane |
| DCM | dichloromethane |
| DMAP | 4-dimethylaminopyridine |
| DMF | *N,N*-dimethylformamide |
| DMSO | dimethyl sulfoxide |
| Dppf, DPPF or dppf | 1,1'-bis(diphenylphosphino)ferrocene |
| eq or eq. or equiv. | Equivalent |
| ESI or ES | electrospray ionization |
| Et | Ethyl |
| Et₂O | diethyl ether |
| EtOH | ethyl alcohol |
| EtOAc | ethyl acetate |
| g | grams |
| h | hour |
| HATU | 1-[bis(dimethylamino)methylene]-1*H*-1,2,3-triazolo[4,5-*b*]pyridinium 3-oxid hexafluorophosphate |
| HPLC | high pressure liquid chromatography |
| IPA | isopropanol |
| iPr | Isopropyl |
| iPr₂NEt or DIPEA | *N*-ethyl diisopropylamine (Hiinig's base) |
| KOAc | potassium acetate |
| LDA | Lithium diisopropylamide |
| LC MS, LCMS, LC-MS or LC/MS | liquid chromatography mass spectroscopy |
| LG | leaving group (e.g., halogen, mesylate, triflate) |
| LHMDS | lithium bis(trimethylsilyl)amide |
| m/z | mass divided by charge |
| mCPBA or MCPBA | meta-chloroperoxybenzoic acid |
| Me | methyl |
| MeOH | methanol |
| Met | metal species for cross-coupling (e.g., MgX, ZnX, SnR₃, SiR₃, B(OR)₂) |
| Mg | milligrams |
| Min | minutes |
| mL | milliliters |
| MS | mass spectra |
| MsCl | methanesulfonyl chloride |
| MTBE | *tert*-butyl methyl ether |
| NMP | 1-methyl-2-pyrrolidine |
| *n*-BuLi | n-butyllithium |
| NMR | nuclear magnetic resonance |
| Pd₂(dba)₃ | tris(dibenzylideneacetone)dipalladium(0) |
| Pd(dppf)Cl₂·DCM | [1,1'-bis(diphenylphosphino)ferrocene]dichloropalladium(II), complex with dichloromethane |
| Pd(PPh₃)₄ | tetrakis(triphenylphosphine)palladium(0) |
| Ph | phenyl |
| Phen | 1,10-phenanthroline |
| PR or PG or Prot. group | protecting group |
| rbf | round-bottom flask |
| RP-HPLC | reverse phase high pressure liquid chromatography |
| RT or rt | room temperature |
| sat. or satd. | saturated |
| SFC | supercritical fluid chromatography |
| SPhos Pd G3 or SPhos G3 | (2-dicyclohexylphosphino-2',6'-dimethoxybiphenyl) [2-(2'-amino-1,1'-biphenyl)]palladium(II) methane sulfonate |
| TBAF | tetra-*n*-butylammonium fluoride |
| *t*-BuOH | *tert*-butanol |
| TATU | O-(7-azabenzotriazolyl)-tetramethyluronium tetrafluorobor |
| TEA or Et₃N | trimethylamine |
| TFA | trifluoroacetic acid |
| THF | tetrahydrofuran |
| TLC | thin layer chromatography |
| T₃P | 2,4,6-tripropyl-1,3,5,2,4,6-trioxatriphosphinane 2,4,6-trioxide |
| UV | ultraviolet |
| Xantphos | 4,5-bis(diphenylphosphino)-9,9-dimethylxanthene |
| XtalFluor-M | difluoro(morpholino)sulfonium tetrafluoroborate |

Unless otherwise noted, all materials were obtained from commercial suppliers and used without further purification. All parts are by weight and temperatures are in degrees centigrade unless otherwise indicated. All microwave assisted reactions were conducted with a Smith Synthesizer^{™} from Biotage^{™}. All compounds showed NMR spectra consistent with their assigned structures. Melting points were determined on a Buchi apparatus and are uncorrected. Mass spectral data was determined by electrospray ionization technique. All examples were purified to >90% purity as determined by high-performance liquid chromatography. Unless otherwise stated, reactions were run at room temperature.

In synthesizing compounds of the present invention, it may be desirable to use certain leaving groups. The term "leaving groups" ("LG") generally refer to groups that are displaceable by a nucleophile. Such leaving groups are known in the art. Examples of leaving groups include, but are not limited to, halides (e.g., I, Br, F, Cl), sulfonates (e.g., mesylate, tosylate), sulfides (e.g., SCH₃), N-hydroxysuccinimide, N-hydroxybenzotriazole, and the like. Examples of nucleophiles include, but are not limited to, amines, thiols, alcohols, Grignard reagents, anionic species (e.g., alkoxides, amides, carbanions) and the like.

The examples presented below illustrate specific embodiments of the present invention. These examples are meant to be representative and are not intended to limit the scope of the claims in any manner.

It is noted that when a percent (%) is used with regard to a liquid, it is a percent by volume with respect to the solution. When used with a solid, it is the percent with regard to the solid composition. Materials obtained from commercial suppliers were typically used without further purification. Reactions involving air or moisture sensitive reagents were typically performed under a nitrogen or argon atmosphere. Purity was measured using high performance liquid chromatography (HPLC) system with UV detection at 254 nm and 215 nm (System A: Agilent Zorbax Eclipse XDB-C8 4.6 x 150 mm, 5 µm, 5 to 100% CH₃CN in H₂O with 0.1% TFA for 15 min at 1.5 mL/min; System B: Zorbax SB-C8, 4.6 x 75 mm, 10 to 90% CH₃CN in H₂O with 0.1% formic acid for 12 min at 1.0 mL/min) (Agilent Technologies, Santa Clara, CA). Silica gel chromatography was generally performed with prepacked silica gel cartidges (Biotage, Uppsala, Sweden or Teledyne-Isco, Lincoln, NE). ¹H NMR spectra were recorded on a Bruker AV-400 (400 MHz) spectrometer (Bruker Corporation, Madison, WI) or a Varian (Agilent Technologies, Santa Clara, CA) 400 MHz spectrometer at ambient temperature. All observed protons are reported as parts per million (ppm) downfield from tetramethylsilane (TMS) or other internal reference in the appropriate solvent indicated. Data are reported as follows: chemical shift, multiplicity (s = singlet, d = doublet, t = triplet, q = quartet, br = broad, m = multiplet), coupling constants, and number of protons. Low-resolution mass spectral (MS) data were determined on an Agilent 1100 Series (Agilent Technologies, Santa Clara, CA) LC/MS with UV detection at 254 nm and 215 nm and a low resonance electrospray mode (ESI).

For the purpose of clarity in this general synthesis section, Compounds of Formula (I) as defined in the summary of the inventions can be schematically drawn to contain Ring Ar¹ and Ring Ar² as follows: 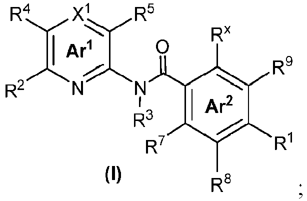 wherein the group -NR³-(C=O)- is a linker, ring Ar¹ is located to the left of the linker, and ring Ar² is located to the right of linker. Generally, compounds of Formula (I), can be synthesized via three general steps as follows:
Step 1: Preparation of Ring Ar¹ compound.
Step 2: Preparation of Ring Ar² compound.
Step 3: Coupling of Ring Ar¹ compound to Ring Ar² compound.

The generic Schemes A-E below are meant to provide guidance to the ordinarily skilled synthetic chemist, who will readily appreciate that the solvent, concentration, reagent, protecting group, order of synthetic steps, time, temperature, and the like can be modified as necessary, well within the skill and judgment of the ordinarily skilled artisan.

### SCHEME A

According to Scheme A, in one embodiment a compound of Formula (I) as disclosed herein can be synthesized as follows:
Step 1a: Preparation of Ring Ar¹ compound:

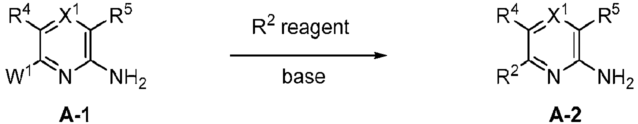

Step 1a: Preparation of Ring Ar¹ compound:: Compound **A-1**, wherein W¹ is a halogen, for example fluoro, chloro, or bromo can be reacted with an R² group containing agent in the presence of a suitable base, in a suitable organic solvent such as NMP, dioxane, acetonitrile, tetrahydrofuran, DMF, methylene chloride, and the like, to form compound **A-2.** Compound **A-1** is commercially available or can be synthesized by known methods by those skilled in the art. Examples of compound **A-1 i**nclude, but are not limited to, 6-fluoropyridin-2-amine, 6-fluoro-4-methylpyridin-2-amine, 6-chloro-5-methylpyridin-2-amine, 6-bromo-5-methylpyridin-2-amine, or 6-chloropyrazin-2-amine. Examples of R² reagents include, but not limited to (1) (R)-2-methylmorpholine, (2) 4,4-difluoropiperidine hydrochloride, (3) 3,3-difluoroazetidine hydrochloride, or (4) 3,3,3-trifluoropropan-1-ol. Examples of bases include, but are not limited to diisopropylethyl amine, potassium carbonate, or sodium hydride.

Step 1b: Preparation of Ring Ar¹ compound:

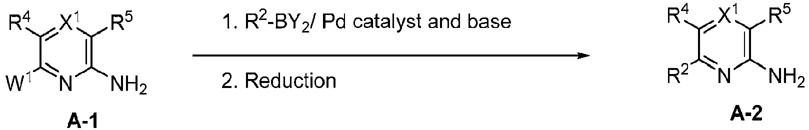

Alternatively, compound **A-1** as defined in step 1a, can be converted to compound **A-2,** as defined in step 1a, via Suzuki cross coupling reaction with a suitable organoboron R² reagent (R²-BY₂, wherein Y is an organic functional group) such as 2-(4,4-difluorocyclohex-1-en-1-yl)-4,4,5,5-tetramethyl-1,3,2-dioxaborolane or 2-(4-ffuorocyclopent-1-en-1-yl)-4,4,5,5-tetramethyl-1,3,2-dioxaborolane, and a suitable palladium catalyst and a base, such as PdCl₂(dppf)-DCM adduct and potassium phosphate tribasic. This step is followed by a reduction with a suitable palladium catalyst and a hydrogen source, such as Pd/C in the presence of hydrogen gas, to form compound **A-2.** This alternative Suzuki reaction can be used when the group R² is linked to the Ar¹ ring via a carbon-carbon bond.

Step 2a: Preparation of Ring Ar² compound:

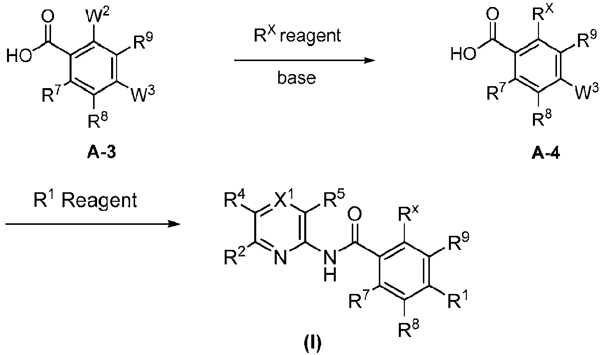

In Step 2a, Compound **A-3,** wherein each of W² and W³ is independently a halogen, for example fluoro, chloro, bromo, or iodo, can be reacted with an R^{x} reagent, such as (1) 6-azaspiro[2.5]octane hydrochloride, (2) 4,4-dimethylpiperidine hydrochloride, (3) 3,4,4-trimethylpiperidine hydrochloride, (4) 4-methyl-6-azaspiro[2.5]octane hydrochloride, or (5) 7-azaspiro[3.5]nonane hydrochloride, in a suitable organic solvent such as NMP, acetonitrile, tetrahydrofuran, DMF, methylene chloride, DMSO, and the like, to form Compound **A-4**.

Step 3a: Coupling of Ring Ar¹ compound to Ring Ar² compound followed bv introduction of R¹:

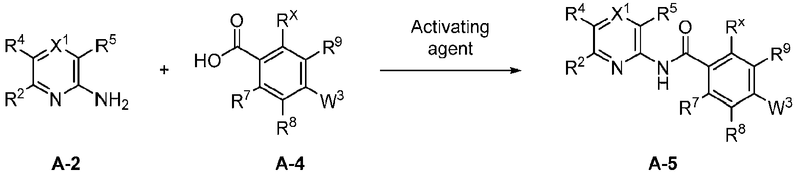

In Step 3a, compound **A-4,** which was obtained from Step 2a, can be reacted with an activating agent such as acid chloride (COCl)₂ or SOCl₂, in a suitable organic solvent such as tetrahydrofuran, methylene chloride, and the like, to form an acid chloride derivative, which can then react with compound **A-2** to form compound **A-5.** Alternatively, compound **A-2** can be directly coupled with compound **A-4,** which was obtained from Step 2a, in a suitable organic solvent such as acetonitrile, tetrahydrofuran, DMF, methylene chloride, and the like, in the presence of a coupling reagent, such as N, N'-diisopropylcarbodiimide, N-(3-dimethylaminopropyl)-N'-ethylcarbodiimide, benzotriazol-1-yl-oxytripyrrolidinophosphonium hexafluorophosphate, *O*-(benzotriazol-1-yl)-N,N,N',N'-tetramethyluronium hexafluorophosphate, carbonyl diimidazole, and polyphosphonic anhydride. Those ordinary skilled synthetic chemists will readily understand that other coupling agents can be used. Further manipulation of halogen group W³ by transformation reactions such as, metal-catalyzed sulfoamidation, sulfination, or sulfonylation, in a suitable organic solvent such as DMSO, acetonitrile, tetrahydrofuran, DMF, methylene chloride, and the like, in the presence of a metal catalyst and an R¹ reagent, such as (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropanoate, (5) 2-hydroxypropane-1-sulfonamide, (6) 2-hydroxyethane-1-sulfonamide, (7) ethyl iodoacetate, (8) 2-mercaptopropan-1-ol, (9) 2-mercapto-2-methylpropan-1-ol, (10) 2-aminoethan-1-ol, or (11) cyclopropanethiol can be used to form Compound (I). Those ordinary skilled chemists will readily understand that coupling reaction such as shown in Step 3a can be performed under various known conditions.

### SCHEME B

Step 1a or 1b: Preparation of Ring Ar¹ compound: see SCHEME A above
Step 2b: Preparation of Ring Ar² compound:

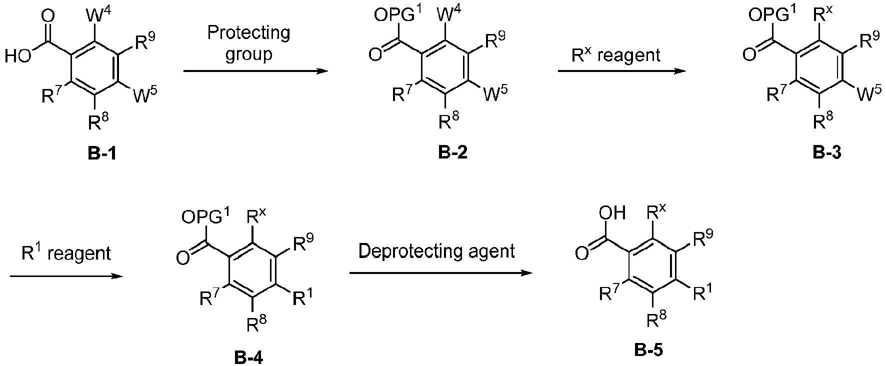

Scheme B provides an alternative method for formation of compounds of Formula (I) as disclosed herein. After Step 1a or Step 1b as described in Scheme A, the group R¹ can alternatively be introduced to Ring Ar² in Step 2b rather that in Step 3a as in Scheme A. According to Step 2b, compound **B-1**, wherein each of W⁴ and W⁵ is independently a halogen, for example fluoro, chloro, bromo, or iodo, can be reacted with an appropriate carboxylic acid protecting group (PGi reagent), such as methyl iodide in the presence of a base such as potassium carbonate, to form a methyl ester, or other appropriate protecting group to form other ester such as benzyl ester, in a suitable organic solvent such as NMP, acetonitrile, tetrahydrofuran, DMF, methylene chloride and the like, to form compound **B-2**, wherein each of W⁴ and W⁵ are as defined in compound **B-1.** Compound **B-2** can then be reacted with an R^{x} reagent, such as 6-azaspiro[2.5]octane, in a suitable organic solvent such as NMP, acetonitrile, tetrahydrofuran, DMF, methylene chloride, DMSO, and the like, to form compound **B-3**, wherein W⁵ is as defined in compound **B-1**. Compound **B-3** can then be reacted with an R¹ reagent by a transformation reaction such as, metal-catalyzed sulfoamidation, sulfination, or sulfonylation, in a suitable organic solvent such as DMSO, acetonitrile, tetrahydrofuran, DMF, and the like, in the presence of a metal catalyst, such as copper iodide, Pd₂(dba)₃ to form compound **B-4,** which can then be reacted further with an appropriate carboxylic acid deprotecting agent to form compound **B-5.** Appropriate carboxylic acid protecting groups and deprotection agents are known to those skilled in the art, e.g., as discussed in Greene's Protective Groups in Organic Synthesis.

Step 3b: Coupling of Ring Ar¹ compound to Ring Ar² compound:

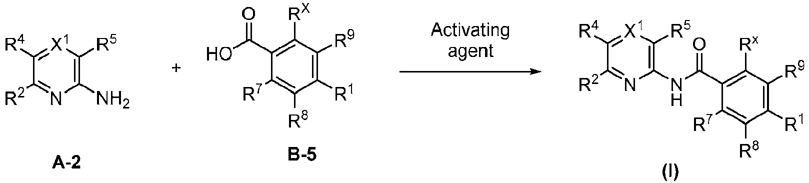

Step 3b is similar to the coupling reaction as described above in Step 3a.

### SCHEME C

Scheme C provides yet another alternative method for formation of compounds of Formula (I) as disclosed herein. According to Scheme C, Step 1a can be performed as described in Scheme A, followed by Step 2b as described in Scheme B.

Step 3c: Coupling of Ring Ar¹ compound to Ring Ar² compound:

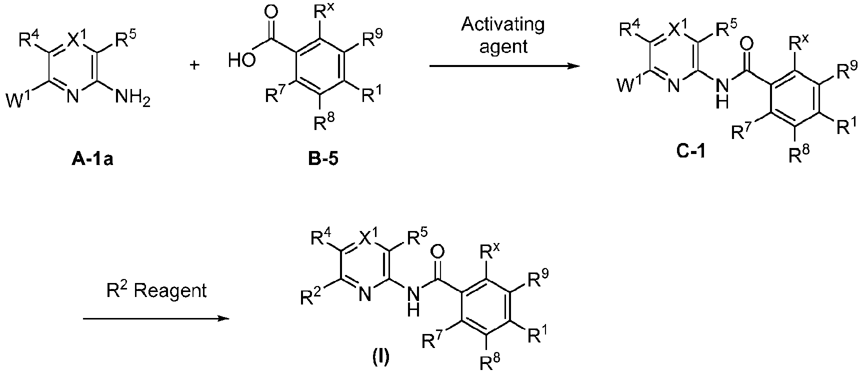

In Step 3c, compound **A-la,** which is compound **A-1** of Scheme A, wherein X¹ is N or CR⁶and W¹ is a halogen, for example fluoro or chloro, can be reacted with compound **B-5** obtained from step 2b of scheme B, in the presence of an activating agents under conditions similar to step 3a and 3b above, to form compound **C-1,** wherein W¹ is as defined in compound **A-la,** which then can be reacted with an R² group containing agent, such as (1) (*R*)-2-methylmorpholine, (2) 4,4-difluoropiperidine hydrochloride, (3) 3,3-difluoroazetidine hydrochloride, or (4) 3,3,3-trifluoropropan-1-ol, in the presence of a suitable base, such as diisopropylethyl amine, potassium carbonate, or sodium hydride, in a suitable organic solvent such as NMP, dioxane, acetonitrile, tetrahydrofuran, DMF, methylene chloride, and the like, to form compound of formula (**I**).

### SCHEME D

Scheme D provides yet another alternative method for formation of compounds of Formula (I) as disclosed herein. According to Scheme D, Step 1a or 1b can be performed as described in Scheme A, followed by Step 2b as described in Scheme B.

Step 3d: Coupling of Ring Ar¹ compound to Ring Ar² compound:

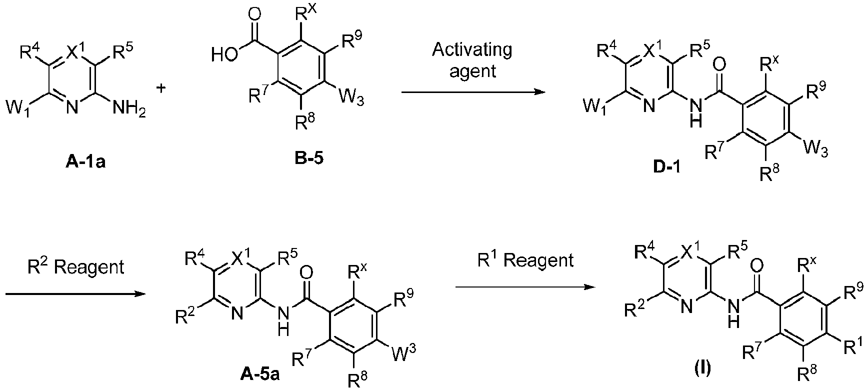

In Step 3d, compound **A-la,** which is compound **A-1** of Scheme A, wherein X¹ is N or CR⁶ and W¹ is a halogen, for example fluoro or chloro, can be reacted with compound **B-5** obtained from step 2a of scheme A, in the presence of a activating agent under conditions similar to step 3a and 3b above, to form compound **D-1,** wherein W¹ is as defined in compound **A-1a** and W³ is as defined in compound **B-5,** which then can be reacted with an R² group containing agent, such as (1) (*R*)-2-methylmorpholine, (2) 4,4-difluoropiperidine hydrochloride, (3) 3,3-difluoroazetidine hydrochloride, or (4) 3,3,3-trifluoropropan-1-ol, optionally in the presence of a suitable base, such as diisopropylethyl amine, potassium carbonate, or sodium hydride, in a suitable organic solvent such as NMP, dioxane, acetonitrile, tetrahydrofuran, DMF, and the like, to form compound **A-5a,** which is compound **A-5,** wherein X¹ is N and W³ is as defined in compound **B-5,** which can then be reacted with an R¹ group containing agent by a transformation reaction such as, metal-catalyzed sulfoamidation, sulfination, or sulfonylation, in a suitable organic solvent such as DMSO, dioxane, acetonitrile, tetrahydrofuran, DMF, and the like, in the presence of a metal catalyst, to form compound formula (I).

### SCHEME E

Scheme E provides yet another alternative method for formation of compounds of Formula (I) as disclosed herein. According to Scheme E, Step 1a or 1b can be performed as described in Scheme A to prepare compound **A-2.** Compound **E-1**, wherein W⁶ is a halogen, for example fluoro or chloro, which includes but is not limited to 2-fluoro-4-nitrobenzoic acid, 2,5-difluoro-4-nitrobenzoic acid, or 2,6-difluoro-4-nitrobenzoic acid, is commercially available or can be synthesized according to known methods by those skilled in the art.

Step 3e: Coupling of Ring Ar¹ compound to Ring Ar² compound

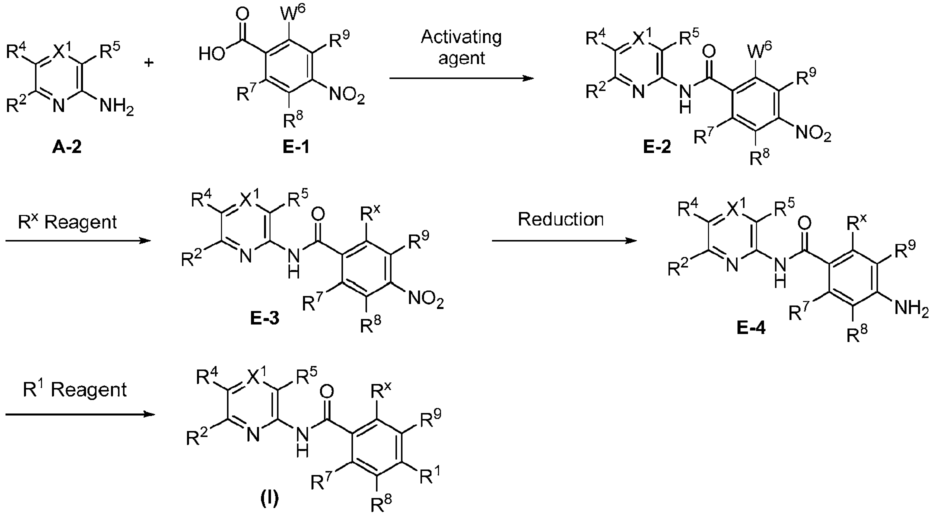

In step 3e, compound **A-2** can be reacted with compound **E-1**, the presence of an activation reagent under conditions similar to step 3a and 3b above, to form compound **E-2,** which then can be reacted with an R^{x} reagent in a similar way as described in Step 2a to form compound **E-3.** The nitro group on compound **E-4** can then be converted into an amino group by reacting with a reducing agent, which includes but is not limited to palladium on carbon and hydrogen gas, to form compound **E-4,** which can then be reacted with an R¹ reagent, such as (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropanoate, (5) 2-hydroxypropane-1-sulfonamide, (6) 2-hydroxyethane-1-sulfonamide, (7) ethyl iodoacetate, (8) 2-mercaptopropan-1-ol, (9) 2-mercapto-2-methylpropan-1-ol, (10) 2-aminoethan-1-ol, or (11) cyclopropanethiol, by a transformation reaction such as, metal-catalyzed sulfoamidation, sulfination, or sulfonylation, in a suitable organic solvent such as DMSO, dioxane, -acetonitrile, tetrahydrofuran, DMF, and the like, in the presence of a metal catalyst, to form compound (I).

### EXAMPLES

### Preparation of Synthetic Intermediates

### Ring Ar¹ Intermediates:

### Intermediate 1: 6-Amino-N-(tert-butyl)pyridine-2-sulfonamide

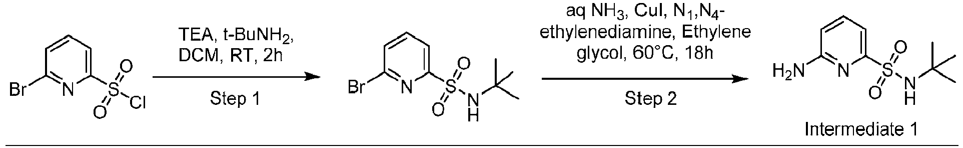

Step 1: To an ice-cold solution of 6-bromopyridine-2-sulfonyl chloride (0.50 g, 1.9 mmol, Suzhou sibian, China) in dichloromethane (10 mL), triethylamine (0.543 mL, 3.90 mmol) and *tert*-butylamine (0.310 mL, 2.92 mmol) were successively added under nitrogen atmosphere. The reaction mixture was stirred at room temperature for 1.5h. After completion, reaction mixture was quenched with water (10 mL) and the bi-phasic mixture was extracted with dichloromethane (3 x 15 mL). The combined organic extracts were washed with saturated brine solution (15 mL) and dried over anhydrous Na₂SO₄. The solution was filtered and concentrated under reduced pressure to give the crude material as a light-yellow oil. The crude material was absorbed onto a plug of silica gel and purified by Isolera-Biotage, eluted with 17 % to 22 % ethyl acetate in petroleum ether, to provide 6-bromo-N-(tert-butyl)pyridine-2-sulfonamide (0.35 g, 1.19 mmol, 61% yield) as an off white solid. ¹H NMR (400 MHz, Chloroform-*d*) δ 7.98 (dd, *J* = 7.6, 0.9 Hz, 1H), 7.75 (t, *J* = 7.8 Hz, 1H), 7.65 (dd, *J* = 8.0, 0.9 Hz, 1H), 4.96 (s, 1H), and 1.27 (s, 9H).

Step 2: In a 500 mL sealed tube, a mixture of 6-bromo-*N*-(*tert*-butyl)pyridine-2-sulfonamide (14.5 g, 49.5 mmol), *N1*,*N2*-dimethylethane-1,2-diamine (0.436 g, 4.95 mmol), K₂CO₃ (1.367 g, 9.89 mmol), and copper(I) iodide (0.471 g, 2.47 mmol) were taken up in aqueous ammonia (21%, 100 mL, 970 mmol) and ethylene glycol (100 mL). The tube was sealed with a screw cap under nitrogen atmosphere. The resulting reaction mixture was stirred at 60 °C for 18 h. The reaction mixture was allowed to cool to room temperature, diluted with water (150 mL) and extracted with EtOAc (3 x 100 mL). The combined organic extracts were washed with saturated brine solution (50 mL) and dried over anhydrous Na2S04. The solution was filtered and concentrated under reduced pressure to give the crude material as a light-yellow oil. The crude material was absorbed onto a plug of silica gel and purified by Isolera-Biotage, eluting with 5% to 6% methanol in chloroform, to provide 6-amino-*N*-(*tert*-butyl)pyridine-2-sulfonamide (6.94 g, 30.3 mmol, 61% yield) as an off white solid. ¹H NMR (400 MHz, DMSO-*d*₆) δ 7.54 (ddd, *J* = 8.4, 7.2, 1.3 Hz, 1H), 7.21 (s, 1H), 7.03 (dt, *J* = 7.3, 0.9 Hz, 1H), 6.59 (dt, *J* = 8.4, 1.0 Hz, 1H), 6.33 (s, 2H), and 1.12 (d, *J* = 1.3 Hz, 9H). *m*/*z* (ESI): 230.1 (M+H)⁺.

### Intermediate 2: 4-Methyl-6-morpholinopyridin-2-amine

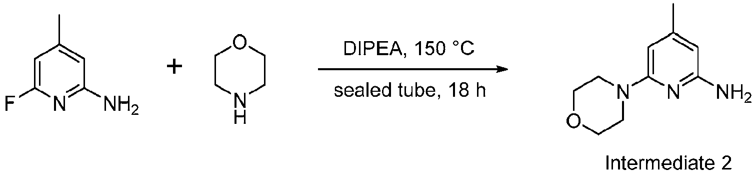

To a 250-mL pressure tube were added 6-fluoro-4-methylpyridin-2-amine (10.0 g, 79 mmol, Sibian chemicals, China), morpholine (8.29 g, 95 mmol), and DIPEA (41.5 mL, 238 mmol). The mixture was heated at 150 °C for 18 h. The reaction mixture was quenched with water (100 mL) and extracted with EtOAc (2 x 250 mL). The combined organic extracts were washed with brine (200 mL), dried over anhydrous Na₂SO₄, filtered, and concentrated under reduced pressure. The crude residue was absorbed onto a plug of silica gel and purified by flash chromatography through a Redi-Sep pre-packed silica gel column (40 g), eluting with a gradient of 1 % to 15 % EtOAc in hexanes, to give the title compound (8.5 g, 44.0 mmol, 56% yield) as a brown semisolid. ¹H NMR (400 MHz, DMSO-*d*₆) δ 5.75 (s, 1H), 5.67 (s, 1H), 5.44 (s, 2H), 3 65 (t, *J* = 8.4 Hz, 4H), 3.30 (t, *J* = 8.4 Hz, 4H), 2.06 (s, 3H). *m*/*z* (ESI): 194.2 (M+H)⁺.

### Intermediate 3: (R)-6-(2-Methylmorpholino)pyridin-2-amine

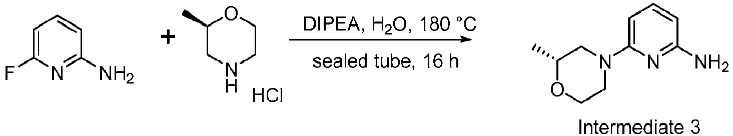

To a 500-mL pressure tube were added 6-fluoropyridin-2-amine (30.0 g, 268 mmol, Combi-Blocks, San Diego, CA), (*R*)-2-methylmorpholine hydrochloride (44.2 g, 321 mmol, F chemicals, China) and DIPEA (140 mL, 803 mmol) in water (60 mL). The mixture was heated at 180 °C for 18 h. The reaction mixture was cooled to room temperature and diluted with water (100 mL). The mixture was extracted with EtOAc (2 x 250 mL) and washed with brine (200 mL). The combined organic extracts were dried over anhydrous Na₂SO₄, filtered, and concentrated under reduced pressure. The crude residue was absorbed onto a plug of silica gel and purified by flash chromatography through a Redi-Sep pre-packed silica gel column (330 g), eluting with a gradient of 1 % to 20 % EtOAc in hexanes, to give the title compound (38.0 g, 197 mmol, 74% yield) as a yellow solid. ¹H NMR (400 MHz, DMSO-*d*₆): δ 7.20-7.14 (m, 1H), 5.89 (d, *J* = 8.0 Hz, 1H), 5.79 (d, *J* = 7.8 Hz, 1H), 5.53 (s, 2H), 4.07 - 3.97 (m, 1H), 3.98 - 3.82 (m, 2H), 3.55 - 3.45 (m, 2H), 2.71 - 2.58 (m, 1H), 2.35 - 2.29 (m, 1H), 1.13 (d, *J* = 6.2 Hz, 3H). *m*/*z* (ESI): 194.2 (M+H)⁺.

### Intermediate 4: (R)-4-Methyl-6-(2-methylmorpholino)pyridin-2-amine

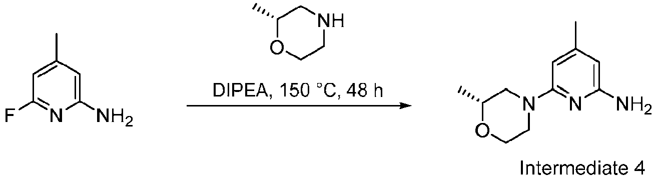

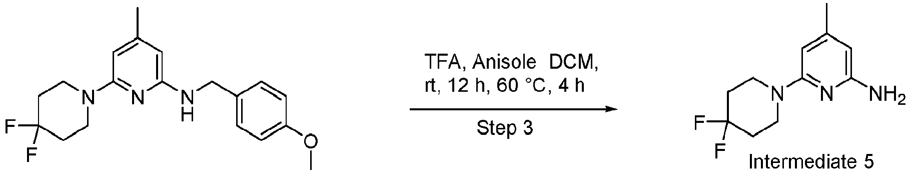

A mixture of 6-fluoro-4-methylpyridin-2-amine (75.0 g, 595 mmol), and (*R*)- 2-methylmorpholine hydrochloride (84 g, 832 mmol) in DIPEA (312 mL, 11.78 mol) was heated 150 °C in an autoclave (600 mL) for 48 h. The reaction mixture was quenched with water (1000 mL) and extracted with EtOAc (2 x 2500 mL). The combined organic layers were washed with brine solution (1000 mL), dried (Na₂SO₄), filtered, and concentrated under reduced pressure. The crude residue was purified by column chromatography over silica gel (60-120 mesh) using 30 to 100 % ethyl acetate in hexanes to give the title compound (62 g, 50% yield) as an off-white solid. ¹H NMR (300 MHz, DMSO-*d*₆): *δ* 5.74 (s, 1H), 5.63 (s, 1H), 5.43 (s, 2H), 3.98 (dt, *J* = 12.8, 2.5 Hz, 1H), 3.90 - 3.80 (m, 2H), 3.53 - 3.44 (m, 2H), 2.62 (ddd, *J* = 15.9, 8.0, 3.7 Hz, 1H), 2.29 (ddd, *J* = 13.4, 10.5, 3.1 Hz, 1H), 2.04 (s, 3H), 1.11 (d, *J* = 6.3 Hz, 3H). *m*/*z* (ESI): 208.1 (M+H)⁺.

**Table 1: The intermediates below were prepared following similar procedures for Intermediates 2-4:**

| Int. # | Chemical Structure | Name | LRMS: (ESI + ve ion) *m*/*z* |
|---|---|---|---|
| 4-1 | 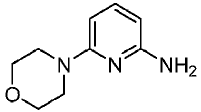 | 6-Morpholinopyridin-2-amine | 180.2 |
| 4-2 | 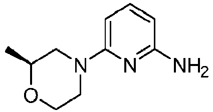 | (*S*)-6-(2-Methylmorpholino)pyridin-2-amine | 194.2 |
| 4-3 | 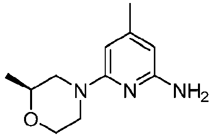 | (*S*)-4-Methyl-6-(2-methylmorpholino)pyridin-2-amine | 208.1 |
| 4-4 | 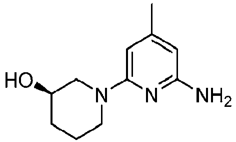 | (*R*)-1-(6-Amino-4-methylpyridin-2-yl)piperidin-3-ol | 208.1 |
| 4-5 | 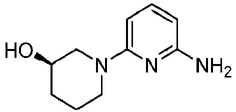 | (*R*)-1-(6-Aminopyridin-2-yl)piperidin-3-ol | 194.1 |
| 4-6 | 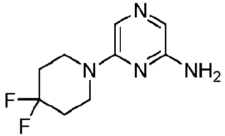 | 6-(4,4-Difluoropiperidin-1-yl)pyrazin-2-amine | 215.1 |

### Intermediate 5: 6-(4,4-Difluoropiperidin-1-yl)-4-methylpyridin-2-amine

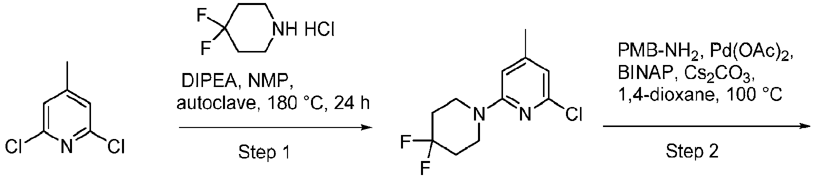

Step 1: To an autoclave was added 2,6-dichloro-4-methylpyridine (80 g, 490 mmol), 4,4-difluoropiperidine hydrochloride (86 g, 540 mmol), and DIPEA (342 mL, 1980 mmol) in NMP (800 mL). The reaction mixture was heated at 180 °C for 24 h. The reaction mixture was cooled to room temperature and basified to pH~9 using 10 % aqueous NaHCO₃ solution. The reaction mixture was extracted with ethyl acetate (2 x 1500 mL), washed with water (1500 mL), dried (Na₂SO₄), filtered, and concentrated under reduced pressure. The crude material was purified by column chromatography over silica gel (60-120 mesh) using 5-10 % ethyl acetate in hexanes to give the mixture of 2,6-dichloro-4-methylpyridine and 2-chloro-6-(4,4-difluoropiperidin-1-yl)-4-methylpyridine in 1:3 ratio (102 g) as a pale brown oil. This mixture (102 g) was further purified by reverse phase chromatography using 60% acetonitrile in water as an eluent to give 2-chloro-6-(4,4-difluoropiperidin-1-yl)-4-methylpyridine (70 g, 58% yield) as a pale brown liquid. ¹H NMR (400 MHz, DMSO-*d*₆): δ 6.76 (s, 1H), 6.57 (s, 1H), 3.66 (t, *J* = 5.6 Hz, 4H), 2.22 (s, 3H), 2.03 - 1.91 (m, 4H). *m*/*z* (ESI): 247.1 (M+H)⁺.

Step-2: To a solution of 2-chloro-6-(4,4-difluoropiperidin-1-yl)-4-methylpyridine (30.0 g, 122 mmol) in 1,4-dioxane (300 mL) were added (4-methoxyphenyl)methanamine (23.8 mL, 182 mmol) and Cs₂CO₃ (79 g, 240 mmol). The reaction mixture was degassed and purged with nitrogen for 30 min. BINAP (7.57 g, 12.2 mmol) and palladium(II)acetate (2.73 g, 12.2 mmol), were added to the reaction mixture and stirred at 100 °C for 16 h. The reaction mixture was cooled to room temperature, filtered through a CELITE^{®} bed, and washed with ethyl acetate (100 mL). The filtrate was concentrated under reduced pressure. The residue was extracted with EtOAc (2 x 500 mL), washed with water (500 mL) followed by brine (500 mL). The combined organic extracts were dried (Na₂SO₄), filtered, and concentrated under reduced pressure. The crude residue was purified by column chromatography over silica gel (60-120 mesh) using 5-8% ethyl acetate in hexanes to give 6-(4,4-difluoropiperidin-1-yl)-N-(4-methoxybenzyl)-4-methylpyridin-2-amine (48 g, 76% yield) as a yellow oil. ¹H NMR (400 MHz, DMSO-*d*₆): δ 7.22 (d, *J* = 7.2 Hz, 2H), 6.85 (d, *J* = 7.2 Hz, 2H), 6.64 (t, *J* = 6.0 Hz, 1H), 5.84 (s, 1H), 5.68 (s, 1H), 4.31 (d, *J* = 6.0 Hz, 2H), 3.71 (s, 3H), 3.56 (t, *J* = 5.6 Hz, 4H), 2.05 (s, 3H), 1.90 - 1.80 (m, 4H). *m*/*z* (ESI): 348.1 (M+H)⁺.

Step-3: To a solution of 6-(4,4-difluoropiperidin-1-yl)-N-(4-methoxybenzyl)-4-methylpyridin-2-amine (48.0 g, 138 mmol) in dry dichloromethane (480 mL) were added anisole (30.2 mL, 276 mmol) and TFA (240 mL, 3120 mmol). The reaction mixture was stirred at 55 °C for 4 h and concentrated under reduced pressure. The residue was dissolved in water (200 mL) and basified with 10% aqueous sodium bicarbonate solution to pH~8 and extracted with ethyl acetate (2 x 500 mL). The combined organic layers were washed with water (200 mL) followed by brine (200 mL), dried (Na₂SO₄), filtered, and concentrated under reduced pressure. The crude residue was purified by column chromatography over silica gel using 25 % to 35 % ethyl acetate in hexanes to give 6-(4,4-difluoropiperidin-1-yl)-4-methylpyridin-2-amine (LCMS ~85 %) as a brown oil. This material was further purified by reverse phase chromatography using 50-60 % acetonitrile in water to give 6-(4,4-difluoropiperidin-1-yl)-4-methylpyridin-2-amine (16.5 g, 72 mmol, 53% yield) as a brown oil. ¹H NMR (400 MHz, DMSO-*d*₆): δ 5.86 (s, 1H), 5.65 (s, 1H), 5.48 (s, 2H), 3.56 (t, J = 5.2 Hz, 4H), 2.06 (s, 3H), 1.96 - 1.87 (m, 4H). *m*/*z* (ESI): 228.2 (M+H)⁺.

**Table 2: The intermediates below were prepared following a similar procedure for Intermediate 5:**

| Int. # | Chemical Structure | Name | LRMS: (ESI + ve ion) *m*/*z* |
|---|---|---|---|
| 5-1 | 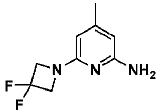 | 6-(3,3-Difluoroazetidin-1-yl)-4-methylpyridin-2-amine | 200.1 |

### Intermediate 6: 6-(3,3,3-Trifluoropropoxy)pyridin-2-amine

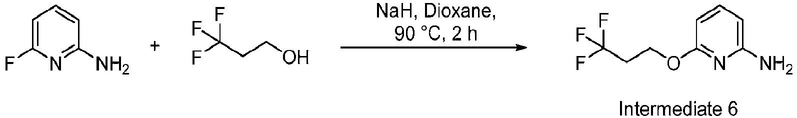

To a solution of 6-fluoropyridin-2-amine (50 g, 450 mmol, Combi-Blocks) in 1,4-dioxane (500 mL) was added 3,3,3-trifluoropropan-1-ol (102 g, 892 mmol, Apollo) under nitrogen atmosphere and the reaction was cooled to 0 °C. NaH (60% in mineral oil, 42.8 g, 1780 mmol) was added to the reaction mixture at 0 °C and the resulting mixture was stirred at 90 °C for 2 h. The reaction mixture was quenched with cold water (500 mL) and extracted with ethyl acetate (2 x 1000 mL). The combined organic extracts were dried (Na₂SO₄), filtered, and concentrated under reduced pressure. The crude residue was purified by column chromatography over silica gel (60-120 mesh) using 10% ethyl acetate in hexanes to give the title compound (45 g, 50 % yield) as a pale brown oil. ¹H NMR (400 MHz, DMSO-*d*₆): *δ* 7.30 - 7.26 (t, *J* = 7.8 Hz, 1H), 6.02 - 6.00 (dd, *J* = 7.8, 0.8 Hz, 1H), 5.89 - 5.86 (m, 3H), 4.36 - 4.33 (t, *J* = 6.2 Hz, 2H), 2.79 - 2.67 (qt, *J* = 11.5, 6.2 Hz, 2H). *m*/*z* (ESI): 207.1 (M+H)⁺.

### Intermediate 7: 6-(3,3-Difluorocyclobutyl)-4-methylpyridin-2-amine

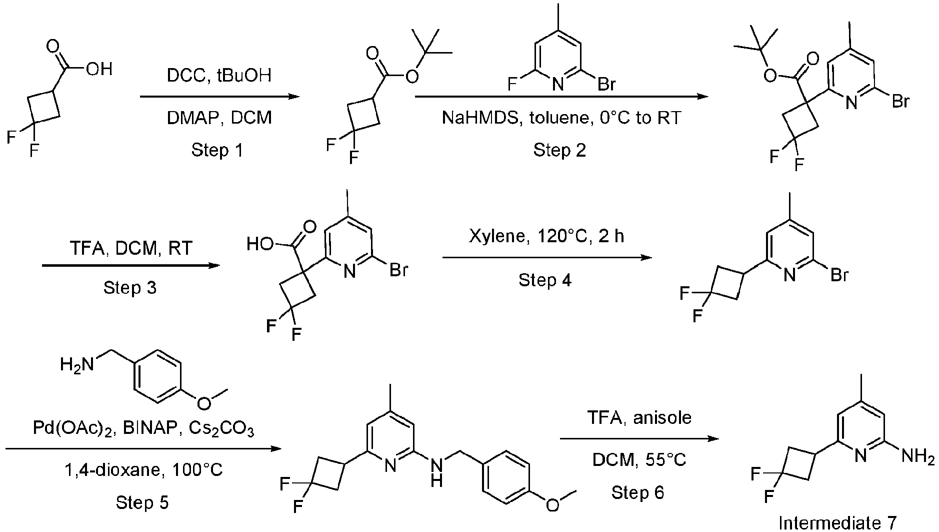

Step 1: To a solution of 3,3-difluorocyclobutane-1-carboxylic acid (3.0 g, 22. mmol, Combi-Blocks) and DMAP (0.269 g, 2.20 mmol) in dichloromethane (30 mL) were added tert-butanol (4.22 mL, 44.1 mmol) followed by dicyclohexyl carbodiimide (5.00 g, 24.2 mmol) at 0 °C. The reaction mixture was stirred at RT for 18 h before diethyl ether (20 mL) was added to the reaction mixture at 0 °C. The precipitated solid was filtered off, and the filtrate was washed with 1.5 N HCl (100 mL), water and brine, dried over Na₂SO₄ and concentrated under reduced pressure to provide tert-butyl 3,3-difluorocyclobutane-1-carboxylate (2.78 g, 14.5mmol, 65.6 % yield) as a clear oil. ¹H NMR (300 MHz, DMSO-*d*₆): δ ppm 2.93 (dddd, *J*=14.8, 7.5, 3.8, 2.1 Hz, 1 H), 2.63 - 2.87 (m, 4 H), 1.42 (d, *J*=2.3 Hz, 9 H).

Step 2: To a solution of 2-bromo-6-fluoro-4-methylpyridine (1.5 g, 7.9 mmol, TCI Chemicals) and *tert*-butyl 3,3-difluorocyclobutane-1-carboxylate (1.82 g, 9.47 mmol) in toluene (35 mL) was dropwise added sodium bis(trimethylsilyl)amide in THF (11.84 mL, 11.84 mmol, 1 M, Symax laboratories) at 0 °C and the reaction mixture was stirred for 20 min at 0 °C and 4 h at rt. The reaction mixture was quenched with a saturated aqueous solution of NH₄Cl (50 mL) and extracted with EtOAc (2 x 50 mL). The combined organic extracts were washed with brine, dried over Na₂SO₄, filtered, and concentrated. The residue was purified by flash column chromatography using a gradient of 0 % to 20 %, EtOAc in petroleum ether to provide tert-butyl 1-(6-bromo-4-methylpyridin-2-yl)-3,3-difluorocyclobutane-1-carboxylate (1.6 g, 3.2 mmol, 41 % yield) as a clear yellow oil. ¹H NMR (300 MHz, DMSO-*d*₆): δ ppm 7.48 (t, *J*=1.0 Hz, 1 H), 7.34 (t, *J*=1.0 Hz, 1 H), 3.15 - 3.31 (m, 2 H), 2.63 - 2.89 (m, 2 H), 2.34 (s, 3 H), 1.36 (s, 9 H). *m*/*z* (ESI): 362.0 (M+H)⁺.

Step 3: To a solution of *tert*-butyl 1-(6-bromo-4-methylpyridin-2-yl)-3,3-difluorocyclobutane-1-carboxylate (1.4 g, 2.8 mmol) in dichloromethane (24 mL) was added TFA (0.87 mL, 11.3 mmol) at RT. The reaction mixture was stirred for 18 h before it was concentrated to provide 1-(6-bromo-4-methylpyridin-2-yl)-3,3-difluorocyclobutane-1-carboxylic acid (0.90 g, 2.2 mmol, 77 % yield) as a brown sticky liquid. ¹H NMR (300 MHz, DMSO-*d*₆): δ ppm 7.47 (s, 1 H), 7.38 (s, 1 H), 3.25 (q, *J*=12.8 Hz, 2 H), 2.66 - 2.85 (m, 2 H), 2.33 (s, 3 H). *m*/*z* (ESI): 306.0 (M+H)⁺.

Step 4: A solution of 1-(6-bromo-4-methylpyridin-2-yl)-3,3-difluorocyclobutane-1-carboxylic acid (0.60 g, 1.4 mmol) in 2-xylene (7.0 mL) was stirred at 120 °C for 2 h. The reaction mixture was diluted with water (50 mL), extracted with EtOAc (2 x 50 mL), washed with water (50 mL) and brine, dried over Na₂SO₄, filtered, and concentrated. The concentrate was purified by flash column chromatography using a gradient of 0 % to 5 %, EtOAc in petroleum ether to provide 2-bromo-6-(3,3-difluorocyclobutyl)-4-methylpyridine (0.41 g, 1.2 mmol, 85 % yield) as light-yellow oil. ¹H NMR (300 MHz, DMSO-*d*₆): δ ppm 7.39 (s, 1 H), 7.25 (s, 1 H), 3.47 (qt, *J*=9.1, 4.5 Hz, 1 H), 2.73 - 2.97 (m, 4 H), 2.29 (s, 3 H). *m*/*z* (ESI): 262.0 (M+H)⁺.

Step 5: A mixture of 2-bromo-6-(3,3-difluorocyclobutyl)-4-methylpyridine (0.400 g, 1.53 mmol), (4-methoxyphenyl)methanamine (0.314 g, 2.29 mmol), Cs₂CO₃ (1.49 g, 4 58 mmol), BINAP (0.095 g, 0.153 mmol) and Pd(OAc)₂ (0.034 g, 0.15 mmol) in 1,4-dioxane (4 mL) was stirred at 100 °C for 16 h. The reaction mixture was filtered and diluted with EtOAc. The resulting solution was washed with water, and brine, dried over Na₂SO₄, filtered, and concentrated. The concentrate was purified by flash column chromatography using a gradient of 0% to 10 % ethyl acetate in petroleum ether to afford 6-(3,3-difluorocyclobutyl)-*N*-(4-methoxybenzyl)-4-methylpyridin-2-amine (0.360 g, 1.131 mmol, 74 % yield) as yellow oil. ¹H NMR (400 MHz, DMSO-*d*₆): δ ppm 7.22 - 7.31 (m, 2 H), 6.85 (dt, *J*=8.5, 2.1 Hz, 2 H), 6.26 (s, 1 H), 6.15 (s, 1 H), 4.38 (d, *J*=6.0 Hz, 2 H), 3.71 (s, 3 H), 3.14 - 3.22 (m, 1 H), 2.71 - 2.86 (m, 4 H), 209 (s, 3 H). *m*/*z* (ESI): 319.1 (M+H)⁺.

Step 6: To a mixture of 6-(3,3-difluorocyclobutyl)-*N*-(4-methoxybenzyl)-4-methylpyridin-2-amine (0.355 g, 1.12 mmol) and anisole (0.244 mL, 2.23 mmol) in dichloromethane (4 mL) was added trifluoroacetic acid (1.8 mL, 23 mmol) at 0 °C. The reaction mixture was stirred at 55 °C for 16 h before it was concentrated and then diluted with DCM (20 mL). The organic layer was washed with a saturate aqueous solution of NaHCO₃ solution (3 mL), water and brine, dried over Na₂SO₄, filtered, and concentrated. The concentrate was purified by flash column chromatography using a gradient of 10 % to 20 %, EtOAc in petroleum ether to provide 6-(3,3-difluorocyclobutyl)-4-methylpyridin-2-amine (0.090 g, 0.45 mmol, 41% yield) as a light-yellow oil. ¹H NMR (300 MHz, DMSO-*d*₆): δ ppm 6.27 (s, 1 H), 6.12 (s, 1 H), 3.18 (td, *J*=8.8, 3.1 Hz, 1 H), 2.69 - 2.85 (m, 4 H), 2.10 (d, *J*=2.1 Hz, 3 H). *m*/*z* (ESI): 199.1 (M+H)⁺.

### Intermediate 8: 5-Methyl-6-morpholinopyridin-2-amine

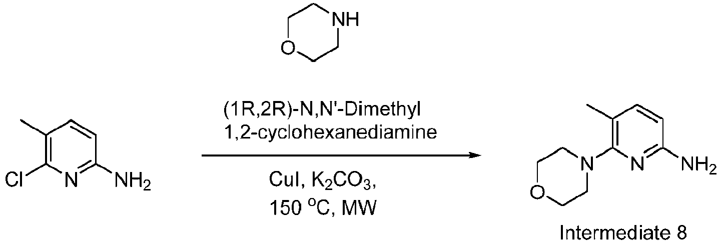

A mixture of 6-chloro-5-methylpyridin-2-amine (0.1 g, 0.70 mmol), morpholine (0.092 g, 1.05 mmol), K₂CO₃ (0.145 g, 1.052 mmol), copper(I) iodide (0.027 g, 0.140 mmol) and (*1R,2R*)*-N1,N2-*dimethylcyclohexane-1,2-diamine (0.020 g, 0.140 mmol) in DMF (2 mL) was heated in microwave at 150 °C for 4 h. Then the reaction mixture was filtered through a plug of celite and the filtrate was diluted with EtOAc, washed with water, brine, dried over Na₂SO₄, filtered, and concentrated. The concentrate was purified by flash column chromatography eluting with 15% ethyl acetate in petroleum ether to provide 5-methyl-6-morpholinopyridin-2-amine (10 mg, 0.052 mmol, 7 % yield) as pale-yellow solid. *m*/*z* (ESI): 194.2 (M+H)⁺.

### Preparation of Ring AR² Intermediates:

### Intermediate 9: 4-Iodo-2-(6-azaspiro[2.5]octan-6-yl)benzoic acid

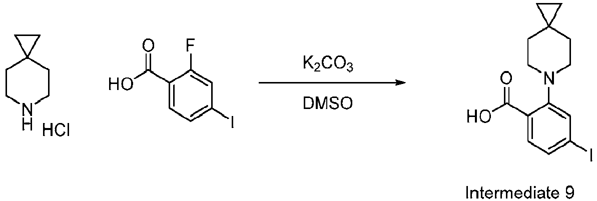

To a solution of 2-fluoro-4-iodobenzoic acid (300 g, 1.13 mol, Combi-Blocks, San Diego, CA) in DMSO (2.1 L) was added 6-azaspiro[2.5]octane hydrochloride (216 g, 1.47 mol, Wuxi AppTec) at 20 °C. Then K₂CO₃ (468 g, 3.38 mol) was added and the reaction solution was stirred at 140°C for 48 h under N₂. The reaction solution was slowly poured into ice water (4.20 L), then extracted with hexanes (2 L x 3). The water phase was taken and the pH was adjusted to 6 with HCl (2.00 mol/L, aq). A precipitate was obtained and collected by filtration. The solid was washed with water (700 mL x 3) and filtered. The moist solid was spread out on a large watch glass and dried in the air at 25 °C. 4-Iodo-2-(6-azaspiro[2.5]octan-6-yl)benzoic acid (280 g, 777 mmol, 68.9% yield) was obtained as a light yellow solid. 400 MHz DMSO-d₆ δ ppm 8.07 (s, 1H), 7.76 - 7.66 (m, 2H), 3.10 (t, *J* = 5.2 Hz, 4H), 1.55 (br s, 4H), 0.41 (s, 4H).

**Table 3: Intermediates 9-1 to 9-5 were prepared following a similar procedure for intermediate 9:**

| Int. # | Chemical Structure | Name | LRMS: (ESI + ve ion) *m*/*z* |
|---|---|---|---|
| 9-1 | 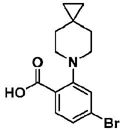 | 4-Bromo-2-(6-azaspiro[2.5]octan-6-yl)benzoic acid | 310.2/312.2 |
| 9-2 | 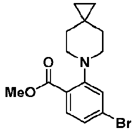 | Methyl 4-bromo-2-(6-azaspiro[2.5]octan-6-yl)benzoate | 324.1/326.1 |
| 9-3 | 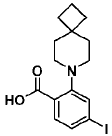 | 4-Iodo-2-(7-azaspiro[3.5]nonan-7-yl)benzoic acid | 372.0 |
| 9-4 | 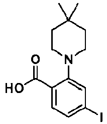 | 2-(4,4-Dimethylpiperidin-1-yl)-4-iodobenzoic acid | 360.0 |
| 9-5 | 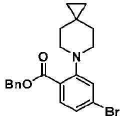 | Benzyl 4-bromo-2-(6-azaspiro[2.5]octan-6-yl)benzoate | 400.1/402.1 |

### Intermediate 10: 4-(Methylsulfonyl)-2-(6-azaspiro[2.5]octan-6-yl)benzoic acid

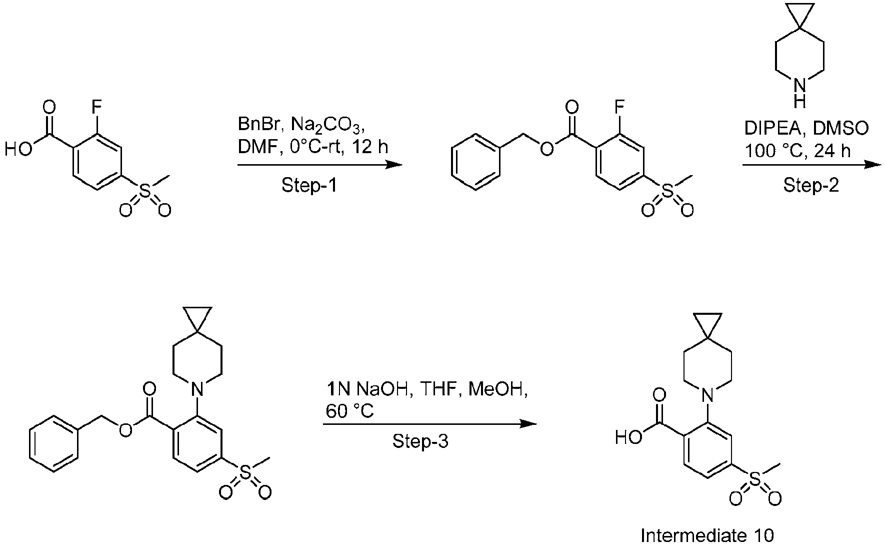

Step-1: To a solution of 2-fluoro-4-(methylsulfonyl)benzoic acid (90.0 g, 412 mmol) in DMF (1 L) was added benzyl bromide (78.1 g, 454 mmol) and sodium carbonate (52.5 g, 495 mmol) at 0 °C. The reaction mixture was stirred for 12 h at room temperature. The reaction mixture was quenched with water (1 L) and extracted with MTBE (3 × 1 L). The combined organic extracts were washed with brine (1 L), dried over Na₂SO₄, filtered, and concentrated under reduced pressure. The crude residue was purified by column chromatography over silica gel (230-400 mesh) using 0 to 30 % ethyl acetate in hexanes to give benzyl 2-fluoro-4-(methylsulfonyl)benzoate (100 g, 79% yield) as a white solid. ¹H NMR (300 MHz, DMSO-*d*₆): *δ* 8.16 (dd, *J* = 8.2, 6.9 Hz, 1H), 7.98 - 7.86 (m, 2H), 7.48 - 7.31 (m, 5H), 5.40 (s, 2H), 3.33 (s, 3H).

Step-2: To a solution of benzyl 2-fluoro-4-(methylsulfonyl)benzoate (55 g, 180 mmol) in dimethyl sulfoxide (550 mL) was added DIPEA (57.6 g, 446 mmol) followed by 6-azaspiro[2.5]octane (29.8 g, 268 mmol) and the reaction mixture was stirred at 100 °C for 24 h. The reaction mixture was quenched with water (1 L) and extracted with MTBE (3 × 1 L). The combined organic extracts were washed with brine (1 L), dried over Na₂SO₄, filtered, and concentrated under reduced pressure. The crude product was purified by column chromatography over silica gel (230-400 mesh) using 0 to 10% ethyl acetate in hexanes to give benzyl 4-(methylsulfonyl)-2-(6-azaspiro[2.5]octan-6-yl)benzoate (55 g, 77 % yield) as a white solid. ¹H NMR (400 MHz, DMSO-*d*₆): *δ* 7.76 (d, *J* = 8.0 Hz, 1H), 7.52 - 7.45 (m, 4H), 7.43 - 7.35 (m, 3H), 5.35 (s, 2H), 3.25 (s, 3H), 3.05 (t, *J* = 5.3 Hz, 4H), 1.36 (t, *J* = 5.3 Hz, 4H), 0.30 (s, 4H). *m*/*z* (ESI): 400.1 (M+H)⁺.

Step-3: To a solution of benzyl 4-(methylsulfonyl)-2-(6-azaspiro[2.5]octan-6-yl)benzoate (65 g, 160 mmol) in THF (108 mL) and methanol (36 mL) was added sodium hydroxide (1N, 407 mL) and the reaction mixture was stirred for 12 h at 60 °C. The reaction mixture was concentrated under reduced pressure to remove THF and methanol. The remaining aqueous solution was acidified to pH ~2 with 1.5 N HCl solution. The precipitate was filtered, washed with water (200 mL) followed by hexanes (200 mL), and dried under vacuum to give 4-(methylsulfonyl)-2-(6-azaspiro[2.5]octan-6-yl)benzoic acid (42 g, 83% yield) as an off-white solid. ¹H NMR (400 MHz, DMSO-*d*₆): *δ* 16.13 (s, 1H), 8.04 (d, *J* = 8.0 Hz, 1H), 7.99 (s, 1H), 7.75 (d, *J* = 8.0 Hz, 1H), 3.29 (s, 3H), 3.17 (b s, 4H), 1.55 (b s, 4H), 0.41 (s, 4H). *m*/*z* (ESI): 308.1 (M+H)⁺.

### Intermediate 11: 4-((1-Methylcyclopropane)-1-sulfonamido)-2-(6-azaspiro[2.5]octan-6-yl)benzoic acid

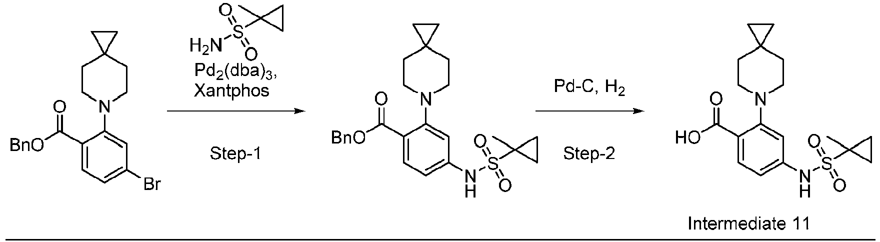

Step 1: To a 250-mL sealed tube were added benzyl 4-bromo-2-(6-azaspiro[2.5]octan-6-yl)benzoate (9 g, 22.48 mmol, Int. 9-5), 1-methylcyclopropane-1-sulfonamide (3.95 g, 29.2 mmol, Combi-Blocks, San Diego, CA) and K₂CO₃ (6.21 g, 45.0 mmol) in 1,4-dioxane (90 mL). The reaction was degassed and purged with nitrogen for 5 min. To this reaction mixture was added Xantphos (1.301 g, 2.248 mmol) followed by Pd₂(dba)₃ (1.029 g, 1.12 mmol) and the sealed tube was closed and stirred at 110 °C for 18 h. The reaction mixture was quenched with water (250 mL) and extracted with ethyl acetate (2 x 150 mL). The combined organic extracts were washed with water (100 mL), dried over Na₂SO₄, filtered, and concentrated under reduced pressure. The crude residue was purified by column chromatography over silica gel eluting with a gradient of 0% to 15 % EtOAc in hexanes to give benzyl 4-((1-methylcyclopropane)-1-sulfonamido)-2-(6-azaspiro[2.5]octan-6-yl)benzoate (6.1 g, 59% yield) as an orange oil. ¹H NMR (400 MHz, DMSO-*d*₆): *δ* 10.06 (s, 1H), 7.62 (d, *J* = 8.5 Hz, 1H), 7.48 - 7.31 (m, 5H), 6.98 (s, 1H), 6.81 (d, *J* = 8.5 Hz, 1H), 5.27 (s, 2H), 2.92 (t, *J* = 4.96 Hz, 4H), 1.40 - 1.30 (m, 7H), 1.16 (dd, *J* = 6.4, 4.7 Hz, 2H), 0.81 (dd, *J* = 6.4, 4.7 Hz, 2H), 0.28 (s, 4H). *m*/*z* (ESI): 455.2 (M+H)⁺.

Step 2: To a solution of benzyl 4-((1-methylcyclopropane)-1-sulfonamido)-2-(6-azaspiro[2.5]octan-6-yl)benzoate (2.1 g, 4.6 mmol) in MeOH (20 mL) and ethyl acetate (10 mL) was added 10% Pd-C (1.05 g, 50% wt/wt) under nitrogen atmosphere. The reaction mixture was degassed and stirring under hydrogen (1 atm, balloon pressure) for 4 h. The reaction mixture was filtered through a celite bed and washed with methanol (20 mL). The filtrate was concentrated under reduced pressure. The residue was triturated with diethyl ether (50 mL) to give 4-((1-methylcyclopropane)-1-sulfonamido)-2-(6-azaspiro[2.5]octan-6-yl)benzoic acid (1.2 g, 71% yield) as an off white solid. ¹H NMR (400 MHz, DMSO-*d₆*): *δ* 13.20 (s, 1H), 10.33 (s, 1H), 7.95 (d, *J* = 8.6 Hz, 1H), 7.41 (s, 1H), 7.20 (d, *J* = 8.6 Hz, 1H), 2.99 (s, 4H), 1.56 (s, 4H), 1.39 (s, 3H), 1.18 (t, *J* = 4.8 Hz, 2H), 0.83 (t, *J* = 4.7 Hz, 2H), 0.42 (s, 4H). *m*/*z* (ESI): 363.2 (M-H)⁺.

### Intermediate 12: 4-(N-(3-methyloxetan-3-yl)sulfamoyl)-2-(6-azaspiro[2.5]octan-6-yl)benzoic acid

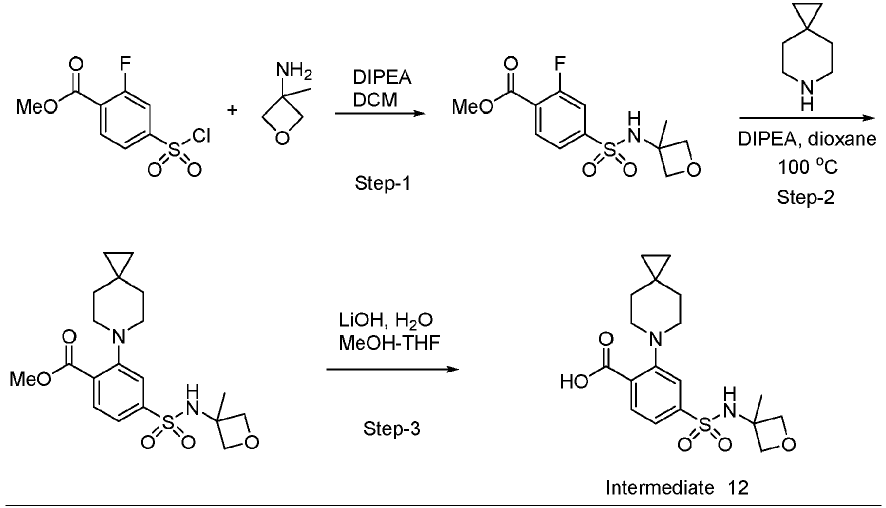

Step 1: To a solution of 3-methyl-3-oxetanamine hydrochloride (5.50 g, 44.5 mmol) and DIPEA (23.3 mL, 134 mmol) in DCM (200 mL) at 0 °C, methyl 4-(chlorosulfonyl)-2-fluorobenzoate (12.4 g, 49.0 mmol) was added and the mixture stirred from 0 °C to room temperature for 1 h. The mixture was diluted with 1.0 N HCl (200 mL) and extracted with dichloromethane (150 mL x 2). The combined organic layers were washed with brine, dried with anhydrous sodium sulfate, filtered, and concentrated under reduced pressure to afford crude product. The crude product was purified with Biotage SNAP 100 g column eluting with 0-30% EtOAc-EtOH (3:1) in heptane to afford methyl 2-fluoro-4-(N (3-methyloxetan-3-yl)sulfamoyl)benzoate (13.6 g, 44.8 mmol, 100 % yield) as a white solid. ¹H NMR (500 MHz, DMSO-d6) δ 8.67 (s, 1H), 8.11 (t, *J*=7.37 Hz, 1H), 7.76-7.82 (m, 1H), 7.69-7.76 (m, 1H), 4.56 (d, *J*=6.23 Hz, 2H), 4.18 (d, *J*=6.75 Hz, 2H), 3.90 (s, 3H), 1.42 (s, 3H).

Step 2: A mixture of DIPEA (16 mL, 93 mmol), 6-azaspiro[2.5]octane (6.22 g, 55.9 mmol), and methyl 2-fluoro-4-(*N*-(3-methyloxetan-3-yl)sulfamoyl)benzoate (14.1 g, 46.6 mmol) in anhydrous dioxane was stirred at 100 °C for 20 h. The mixture was cooled down to room temperature, quenched with water, and extracted with ethyl acetate. The combined organic extracts were washed with brine, dried, and evaporated to dryness under reduced pressure. The crude product was purified using the Biotage SNAP 340 g column eluting with 0-40% EtOAc-EtOH (3:1) in heptane to give methyl 4-(*N*-(3-methyloxetan-3-yl)sulfamoyl)-2-(6-azaspiro[2.5]octan-6-yl)benzoate (14.2 g, 35.9 mmol, 77 % yield) as an off-white solid. ¹H NMR (500 MHz, DMSO-d6) δ 8.42 (s, 1H), 7.72 (d, *J*=8.04 Hz, 1H), 7.47 (d, *J*=1.56 Hz, 1H), 7.36 (dd, *J*=1.82, 8.04 Hz, 1H), 4.55 (d, *J*=5.97 Hz, 2H), 4.14 (d, *J*=6.49 Hz, 2H), 3.85 (s, 3H), 3.02-3.09 (m, 4H), 1.44-1.50 (m, 4H), 1.42 (s, 3H), 0.35 (s, 4H).

Step 3: A mixture of methyl 4-(*N*-(3-methyloxetan-3-yl)sulfamoyl)-2-(6-azaspiro[2.5]octan-6-yl)benzoate (14.2 g, 35.9 mmol) and lithium hydroxide monohydrate (22.6 g, 538 mmol) in THF-water-MeOH (1:1:1, 300 mL) was stirred at room temperature for 16 h. The mixture was concentrated under reduced pressure to partially remove the organic solvent. The solution was acidified with 2N HCl to reach pH < 3. The precipitate was filtered and dried in air to give 4-(*N*-(3-methyloxetan-3-yl)sulfamoyl)-2-(6-azaspiro[2.5]octan-6-yl)benzoic acid (9.94 g, 26.1 mmol, 73 % yield) as a white solid. ¹H NMR (500 MHz, DMSO-*d*6) δ 851 (s, 1H), 8.04 (d, *J*=8.04 Hz, 1H), 7.89 (d, *J* = 1.30 Hz, 1H), 7.66 (dd, *J*=1.69, 8.17 Hz, 1H), 4.55 (d, *J*=6.23 Hz, 2H), 4.09-4.17 (m, 2H), 3.06-3.19 (m, 4H), 1.56 (t, *J* = 5.19 Hz, 4H), 1.40 (s, 3H), 0.36-0.46 (s, 4H).

### Intermediate 13: 4-(N-(tert-Butyl)sulfamoyl)-2-(6-azaspiro[2.5]octan-6-yl)benzoic acid

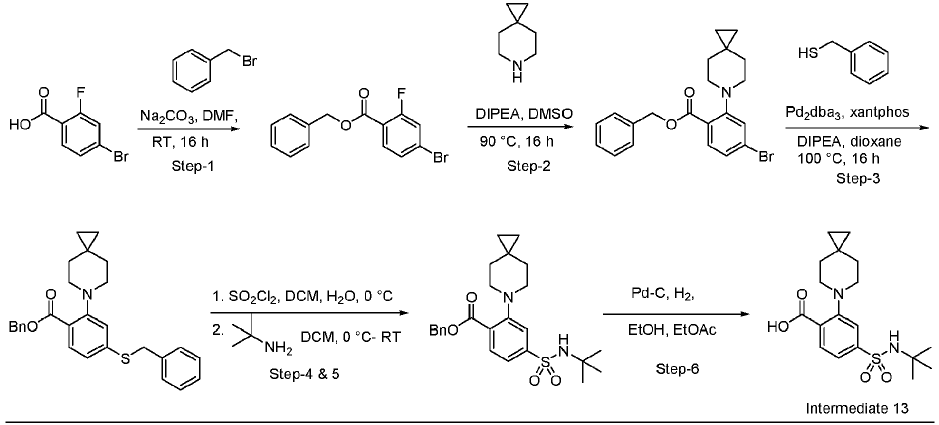

Step 1: To a solution of 4-bromo-2-fluorobenzoic acid (40 g, 180 mmol) in DMF (600 mL) were added sodium carbonate (23.2 g, 219 mmol) and benzyl bromide (22.8 mL, 192 mmol) at 0 °C. The reaction mixture was stirred at room temperature for 16 h before quenched with water (1.5 L) and extracted with ethyl acetate (3 x 500 mL). The combined organic extracts were washed with water (1500 mL) and brine (500 mL), dried over anhydrous Na₂SO₄, filtered, and concentrated under reduced pressure. The crude residue was purified by column chromatography over silica gel (60-120 mesh) using 10% ethyl acetate in hexanes to give benzyl 4-bromo-2-fluorobenzoate (50 g, 89% yield) as a pale yellow liquid.¹H NMR (400 MHz, Chloroform-*d*): δ 7.86 (dd, *J* = 8.0, 7.4 Hz, 1H), 7.50 - 7.35 (m, 7H), 5.40 (s, 2H).

Step 2: To a solution of benzyl 4-bromo-2-fluorobenzoate (30 g, 97 mmol) in DMSO (300 mL) were added 6-azaspiro[2.5]octane (15.1 g, 136 mmol) and DIPEA (33.9 mL, 194 mmol) at room temperature. The reaction mixture was stirred at 90 °C for 16 h. The reaction mixture was quenched with water (500 mL) and extracted with ethyl acetate (2 x 500 mL). The combined organic extracts were washed with brine (600 mL), dried over anhydrous Na₂SO₄, filtered, and concentrated under reduced pressure. The crude residue was purified by column chromatography over silica gel (60-120 mesh) using 0% to 10% of ethyl acetate in hexanes to give benzyl 4-bromo-2-(6-azaspiro[2.5]octan-6-yl)benzoate (25 g, 64% yield) as a pale-yellow oil. ¹H NMR (400 MHz, Chloroform-*d*): δ 7.61 (d, *J* = 8.4 Hz, 1H), 7.47 (d, *J* = 8.0 Hz, 2H), 7.43 - 7.35 (m, 3H), 7.22 (s, 1H), 7.09 (d, *J* = 8.4 Hz, 1H), 5.36 (s, 2H), 3.09 (t, *J* = 5.3 Hz, 4H), 1.49 (t, *J* = 5.3 Hz, 4H), 0.34 (s, 4H). *m*/*z* (ESI): 400.1 (M-H)⁺.

Step 3: A solution of benzyl 4-bromo-2-(6-azaspiro[2.5]octan-6-yl)benzoate (25 g, 62 mmol), DIPEA (21.8 mL, 125 mmol), xantphos (1.81 g, 3.12 mmol), Pd₂(dba)₃ (1.14 g, 1.25 mmol) and benzyl mercaptan (10 g, 81 mmol) in 1,4 dioxane (250 mL) degassed and purged with nitrogen for 15 min. The reaction mixture was heated at 100 °C for 16 h in a sealed pressure vessel. The reaction mixture was quenched with water (500 mL) and extracted with ethyl acetate (2 x 500 mL). The combined organic extracts were dried over anhydrous Na₂SO₄, filtered, and concentrated under reduced pressure. The crude residue was purified by column chromatography over silica gel (60-120 mesh) using 5% to 10% of ethyl acetate in hexanes to give benzyl 4-(benzylthio)-2-(6-azaspiro[2.5]octan-6-yl)benzoate (20 g, 72% yield) as a pale yellow liquid. ¹H NMR (400 MHz, DMSO-*d*₆): δ 7.57 - 7.53 (m, 1H), 7.48 - 7.26 (m, 10H), 6.93 - 6.87 (m, 2H), 5.27 (s, 2H), 4.32 (s, 2H), 2.95 - 2.87 (m, 4H), 1.35 (t, *J* = 5.3 Hz, 4H), 0.28 (s, 4H). *m*/*z* (ESI): 442.2 (M-H)⁺.

Step 4 & 5: To a solution of benzyl 4-(benzylthio)-2-(6-azaspiro[2.5]octan-6-yl)benzoate (20 g, 45 mmol) in DCM (160 mL) and water (40 mL), was added sulfuryl chloride (18.3 mL, 225 mmol) at 0 °C. The reaction mixture was stirred for 1 h before diluted with water (200 mL) and extracted with DCM (200 mL). The organic extract was dried over anhydrous Na₂SO₄, filtered, and cooled to 0 °C. tert-Butylamine (47.8 mL, 451 mmol) was added to the above solution. The reaction mixture was stirred at room temperature for 1 h before quenched with water (200 mL) and extracted with DCM (2 x 100 mL). The combined organic extracts were dried over anhydrous Na₂SO₄, filtered, and concentrated under reduced pressure. The crude residue was purified by column chromatography over silica gel (60-120 mesh) using 15 % ethyl acetate in hexanes to give benzyl 4-(*N*-(tert-butyl)sulfatnoyl)-2-(6-azaspiro[2.5]octan-6-yl)benzoate (12 g, 61 % yield) as a pale yellow solid. ¹H NMR (400 MHz, DMSO-*d*₆): δ 7.71 (d, *J* = 8.0 Hz, 1H), 7.63 (s, 1H), 7.55 - 7.46 (m, 3H), 7.45 7.33 (m, 4H), 5.33 (s, 2H), 3.01 (b s, 4H), 1.38 (b s, 4H), 1.10 (s, 9H), 0.31 (2, 4H). *m*/*z* (ESI): 457.2 (M-H)⁺.

Step 6: To a solution of benzyl 4-(*N*-(*tert*-butyl)sulfamoyl)-2-(6-azaspiro[2.5]octan-6-yl)benzoate (10 g, 21.9 mmol) in ethanol (50 mL) and ethyl acetate (50 mL) was added 10% palladium on carbon (4.66 g, 4.38 mmol) at room temperature under nitrogen atmosphere. The reaction mixture was degassed and stirred under hydrogen atmosphere (1 atm) at room temperature for 16 h. The reaction mixture was filtered through a celite bed and the filter bed was washed with ethyl acetate (200 mL). The filtrate was concentrated under reduced pressure. The crude residue was triturated with diethyl ether (200 mL) to give the title compound (6.0 g, 75% yield) as an off-white solid. ¹H NMR (400 MHz, DMSO-*d*₆): δ 8.06 (d, *J* = 8.2 Hz, 1H), 7.98 (s, 1H), 7.73 - 7.68 (m, 2H), 3.12 (t, *J* = 5.3 Hz, 4H), 1.57 (t, *J* = 5.3 Hz, 4H), 1.10 (s, 9H), 0.43 (s, 4H). *m*/*z* (ESI): 367.2 (M-H)⁺.

### Intermediate 14: 4-((Methylsulfonyl)methyl)-2-(6-azaspiro[2.5]octan-6-yl)benzoic acid

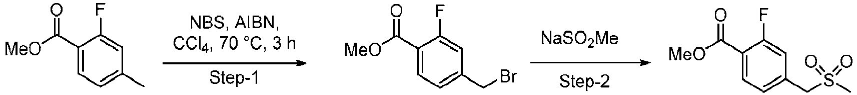

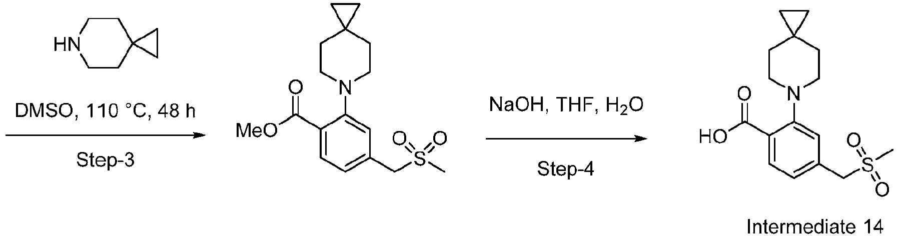

Step 1: To a solution of methyl 2-fluoro-4-methylbenzoate (10.0 g, 59.5 mmol) in carbon tetrachloride (200 mL) were added NBS (11.6 g, 65.4 mmol) and AIBN (0.976 g, 5.95 mmol) at rt. The reaction mixture was stirred at 70 °C for 3 h before quenched with water (250 mL) and extracted with dichloromethane (2 x 200 mL). The combined organic extracts were washed with brine (150 mL), dried over Na₂SO₄, filtered, and concentrated under reduced pressure to give methyl 2-fluoro-4-methylbenzoate (14.0 g, crude) as a pale yellow oil.¹H NMR (400 MHz, Chloroform-*d*): *δ* 8.04 - 7.88 (m, 1H), 7.27 - 7.13 (m, 2H), 4.46 (s, 2H), 3.96 (s, 3H).

Step 2: A mixture of methyl 2-fluoro-4-methylbenzoate (14.0 g, 56.7 mmol) and sodium methanesulfinate (12.15 g, 119 mmol) in DMF (42 mL) was irradiated in a microwave oven (Biotage initiator+) at 120 °C for 30 min. The reaction mixture was quenched with water (150 mL) and extracted with ethyl acetate (2 x 200 mL). The combined organic extracts were washed with saturated brine solution (150 mL), dried over Na₂SO₄, filtered, and concentrated under reduced pressure to give methyl 2-fluoro-4-((methylsulfonyl)methyl)benzoate (6 g, crude) as an off-white solid.

Step 3: A mixture of methyl 2-fluoro-4-((methylsulfonyl)methyl)benzoate (6.0 g, 24 mmol) and 6-azaspiro [2.5] octane (2.71 g, 24.4 mmol) in DMSO (30 mL) was irradiated in a microwave oven at 150 °C for 1 h. The reaction mixture was quenched with water (50 mL) and extracted with ethyl acetate (2 x 50 mL). The combined organic extracts were washed with brine (50 mL), dried over Na₂SO₄, filtered, and concentrated under reduced pressure to give methyl 4-((methylsulfonyl)methyl)-2-(6-azaspiro[2.5]octan-6-yl)benzoate (4.0 g, crude) as an off-white solid. The material as such was taken onto the next step without any further purification. ¹H NMR (400 MHz, Chloroform-*d*) *δ* 7.72 (d, *J* = 7.8 Hz, 1H), 7.11 (s, 1H), 6.96 (dd, *J* = 7.8, 1.6 Hz, 1H), 4.24 (s, 2H), 3.93 (s, 3H), 3.12 (t, *J* = 5.4 Hz, 4H), 2.78 (s, 3H), 1.55 (t, *J* = 5.5 Hz, 4H), 0.37 (s, 4H). *m*/*z* (ESI): 338.1 (M-H)⁺.

Step 4: To a solution of methyl 4-((methylsulfonyl)methyl)-2-(6-azaspiro[2.5]octan-6-yl)benzoate (2.0 g, 3.0 mmol) in THF (15 mL) was added sodium hydroxide (0.474 g, 11.8 mmol) in water (7 mL) and stirred at room temperature for 12 h. The reaction mixture was acidified with 1.5 N HCl solution to pH ~3 and extracted with ethyl acetate (5 x 20 mL). The combined organic extracts were washed with brine solution (20 mL), dried over Na₂SO₄, filtered, and concentrated under reduced pressure to give 4-((methylsulfonyl)methyl)-2-(6-azaspiro[2.5]octan-6-yl)benzoic acid (1.0 g, 10% yield over 3 steps) as a white solid. *m*/*z* (ESI): 324.1 (M-H)⁺.

### Intermediate 15: 4-((1-(tert-Butoxycarbonyl)azetidin-3-yl)sulfonyl)-2-(6-azaspiro[2.5]octan-6-yl)benzoic acid

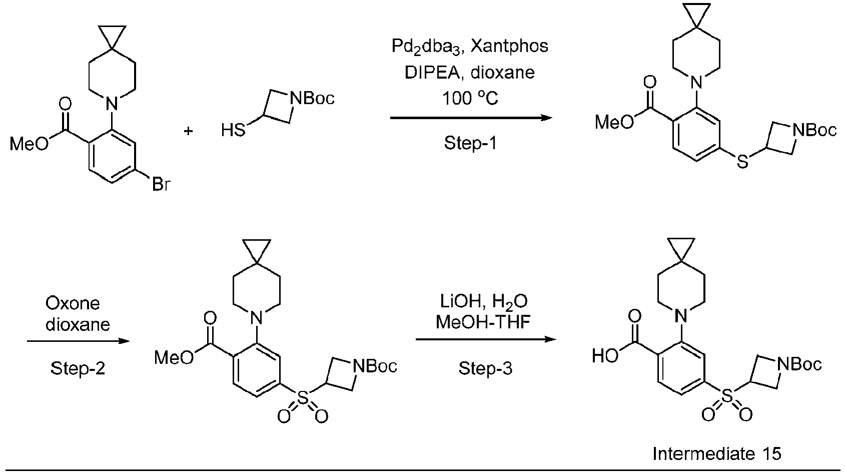

Step 1: A mixture of methyl 4-bromo-2-(6-azaspiro[2.5]octan-6-yl)benzoate (10.5 g, 32.4 mmol, Intermediate 9-2), DIPEA (8.37 mL, 64.8 mmol), Xantphos (1.87 g, 3.24 mmol) and Pd₂(dba)₃ (2.97 g, 3.24 mmol) in dioxane was bubbled with argon flow, then *tert*-butyl 3-mercaptoazetidine-1-carboxylate (7.66 mL, 40.5 mmol) was added. The mixture was stirred at 100 °C for 18 h. The mixture was cooled down to room temperature, concentrated and purified through a Biotage SNAP 340 g column eluting with a gradient of 0 % to 25 % EtOAc-EtOH (3: 1) in heptane to provide *tert*-butyl 3-((4-(methoxycarbonyl)-3-(6-azaspiro[2.5]octan-6-yl)phenyl)thio)azetidine-1-carboxylate (13.8 g, 32.0 mmol, 99 % yield) as a light-yellow sticky solid. ¹H NMR (500 MHz, DMSO-*d*₆) δ ppm 7.55 (d, *J*=8.04 Hz, 1H), 6.79 (s, 1H), 6.76 (d, *J*=8.14 Hz, 1H), 4.34-4.43 (m, 2H), 4.27-4.34 (m, 1H), 3.79 (s, 3H), 3.70 (dd, *J*=4.67, 8.56 Hz, 2H), 2.97-3.04 (m, 4H), 1.41-1.51 (m, 4H), 1.38 (s, 9H), 0.29-0.37 (m, 1H).

Step 2: To a solution of *tert*-butyl 3-((4-(methoxycarbonyl)-3-(6-azaspiro[2.5]octan-6-yl)phenyl)thio)azetidine-1-carboxylate (13.85 g, 32.0 mmol) in 1,4-dioxane (300 mL) was added Oxone monopersulfate (39.4 g, 64.0 mmol) in 150 mL water. The reaction mixture was stirred at room temperature for 5 h before adding 150 mL of ethyl acetate and 150 mL of water. This mixture was stirred for 10 min and the organic layer was separated, the aqueous layer was extracted with ethyl acetate. The combined organics were washed with brine, dried, filtered, and concentrated. The crude material was purified through a Biotage SNAP 340 g column eluting with a gradient of 0 % to 25 % EtOAc-EtOH (3:1) in heptane to give *tert*-butyl 3-((4-(methoxycarbonyl)-3-(6-azaspiro[2.5]octan-6-yl)phenyl)sulfonyl)azetidine-1-carboxylate (12.8 g, 27.5 mmol, 86 % yield) as an off-white solid. ¹H NMR (500 MHz, DMSO-*d*₆) δ 7.75 (d, *J*=8.04 Hz, 1H), 7.44-7.50 (m, 2H), 4.48-4.55 (m, 1H), 4.09 (br. s., 2H), 3.97-4.02 (m, 2H), 3.86 (s, 3H), 3.04-3.17 (m, 4H), 1.42-1.51 (m, 4H), 1.38 (s, 9H), 0.35 (s, 4H).

Step 3: A mixture of *tert*-butyl 3-((4-(methoxycarbonyl)-3-(6-azaspiro[2.5]octan-6-yl)phenyl)sulfonyl) azetidine-1-carboxylate (12 77 g, 27.5 mmol) and lithium hydroxide monohydrate (11.53 g, 275 mmol) in THF-water-MeOH (1:1:1, 230 mL) was stirred at room temperature for 15 h. The mixture was concentrated under reduced pressure to remove some organic solvent. The solution was acidified with 2N HCl to pH < 3. The precipitate was filtered and dried to give 4-((1-(*tert-*butoxycarbonyl)azetidin-3-yl)sulfonyl)-2-(6-azaspiro[2.5]octan-6-yl)benzoic acid (10.6 g, 23.5 mmol, 86 % yield) as an off-white solid. ¹H NMR (500 MHz, DMSO-*d*₆) δ ppm 8.03 (d, *J*=8.30 Hz, 1H), 7.93 (d, *J*=1.82 Hz, 1H), 7.72 (dd, *J*=1.69, 8.17 Hz, 1H), 4.48-4.60 (m, 1H), 4.10 (br. s., 2H), 3.99-4.06 (m, 2H), 3.14-3.22 (m, 4H), 1.49-1.59 (m, 4H), 1.38 (s, 9H), 0.41 (s, 4H).

### Intermediate 16: 4-(Cyclopropanesulfonimidoyl)-2-(6-azaspiro[2.5]octan-6-yl)benzoic acid

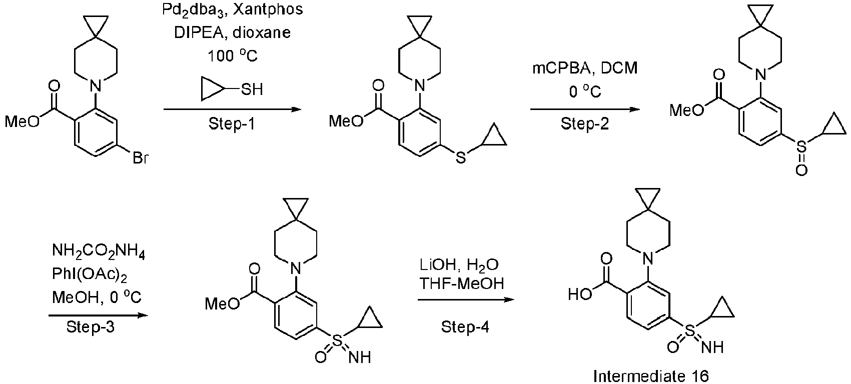

Step 1: To a mixture of methyl 4-bromo-2-(6-azaspiro[2.5]octan-6-yl)benzoate (3.69 g, 11.4 mmol, Intermediate 9-2), DIPEA (2.94 mL, 22.8 mmol), Xantphos (0.659 g, 1.14 mmol) and Pd₂(dba)₃ (1.04 g, 1.14 mmol) in 1,4-dioxane, was added cyclopropanethiol (0.928 mL, 12.5 mmol). The mixture was stirred at 100 °C for 16 h. The mixture was diluted with water and extracted with ethyl acetate. The organic extracts were washed with brine, dried, and concentrated. The mixture was purified through a Biotage SNAP 100 g column eluting with a gradient of 0 % to 20% EtOAc-EtOH (3:1) in heptane to provide methyl 4-(cyclopropylthio)-2-(6-azaspiro[2.5]octan-6-yl)benzoate (3.42 g, 10.8 mmol, 95 % yield) as a yellow oil. ¹H NMR (500 MHz, DMSO-*d*6) δ 7.57 (d, *J*=8.04 Hz, 1H), 7.00 (d, *J*=1.82 Hz, 1H), 6.93 (d, *J*=8.21 Hz, 1H), 3.79 (s, 3H), 2.95-3.04 (m, 4H), 2.26-2.36 (m, 1H), 1.43-1.49 (m, 4H), 1.07-1.16 (m, 2H), 0.55-0.66 (m, 2H), 0.33 (s, 4H).

Step 2: To a mixture of methyl 4-(cyclopropylthio)-2-(6-azaspiro[2.5]octan-6-yl)benzoate (3.35 g, 10.6 mmol) in DCM at 0 °C, was added 3-chloroperoxybenzoic acid (2.367 g, 13.72 mmol). The mixture was stirred at 0 °C for 30 min. The reaction was quenched with sat. NaHCO₃ and extracted with ethyl acetate. The organic extract was washed with brine, dried, and concentrated. The mixture was purified through a Biotage SNAP 100 g column eluting with a gradient of 0% to 40% EtOAc-EtOH (3: 1) in heptane to provide methyl 4-(cyclopropylsulfinyl)-2-(6-azaspiro[2.5]octan-6-yl)benzoate (3.34 g, 10.0 mmol, 95 % yield) as a yellow oil. ¹H NMR (500 MHz, DMSO-*d*₆) δ 7.70 (d, *J*=8.04 Hz, 1H), 7.30-7.47 (m, 1H), 7.22 (d, *J*=8.02 Hz, 1H), 3.84 (s, 3H), 3.03-3.11 (m, 4H), 2.52-2.55 (m, 1H), 1.43-1.51 (m, 4H), 0.88-1.01 (m, 3H), 0.77-0.88 (m, 1H), 0.34 (s, 4H).

Step 3: To a solution of methyl 4-(cyclopropylsulfinyl)-2-(6-azaspiro[2.5]octan-6-yl)benzoate (3.33 g, 9.99 mmol) in methanol were added iodobenzene diacetate (6.43 g, 20.0 mmol) and ammonium carbamate (3.12 g, 39.9 mmol) at 0 °C and the mixture was stirred at 0 °C for 1 h. The reaction was quenched with water and extracted with ethyl acetate and DCM. The combined organic extracts were washed with brine, dried, and concentrated. The mixture was purified through a Biotage SNAP 100 g column eluting with a gradient of 0% to 60% EtOAc-EtOH (3:1) in heptane to provide methyl 4-(cyclopropanesulfonimidoyl)-2-(6-azaspiro[2.5]octan-6-yl)benzoate (1.89 g, 5.42 mmol, 54.3 % yield) as an off-white solid. ¹H NMR (500 MHz, DMSO-*d*6) δ 7 70 (d, *J*=8 04 Hz, 1H), 7.53 (d, *J*=1.56 Hz, 1H), 7.43 (dd, *J*=1.56, 8.04 Hz, 1H), 4.29 (s, 1H), 3.85 (s, 3H), 3.01-3.13 (m, 4H), 2.67-2.75 (m, 1H), 1.43-1.51 (m, 4H), 1.05-1.14 (m, 1H), 0.87-1.00 (m, 3H), 0.35 (s, 4H).

Step 4: A mixture of methyl 4-(cyclopropanesulfonimidoyl)-2-(6-azaspiro[2.5]octan-6-yl)benzoate (1.89 g, 5.42 mmol) and lithium hydroxide monohydrate (0.683 g, 16.3 mmol) in THF-water-MeOH (2: 1: 1, 32 mL) was stirred at room temperature for 15 h. The mixture was concentrated under reduced pressure to remove some organic solvent. The solution was acidified with 2N HCl to pH < 3. The mixture was extracted with DCM (2x). The combined extracts were washed with brine, dried and concentrated to give 4-(cyclopropanesulfonimidoyl)-2-(6-azaspiro[2.5]octan-6-yl)benzoic acid (1.80 g, 5.38 mmol, 99 % yield) as an off-white solid. ¹H NMR (500 MHz, DMSO-*d*6) δ 8.08 (d, *J*=8.30 Hz, 1H), 8.01 (d, *J*=1.56 Hz, 1H), 7.78 (dd, *J*=1.56, 8.04 Hz, 1H), 4.44 (br. s., 1H), 3.16 (t, *J*=5.32 Hz, 4H), 2.74-2.81 (m, 1H), 1.58 (t, *J*=5.19 Hz, 4H), 1.11-1.22 (m, 1H), 0.89-1.04 (m, 3H), 0.43 (s, 4H).

### Compounds containing Ar¹ and Ar²

### Intermediate 17: N-(6-Fluoropyridin-2-yl)-4-((1-methylcyclopropane)-1-sulfonamido)-2-(6-azaspiro [2.5]octan-6-yl)benzamide

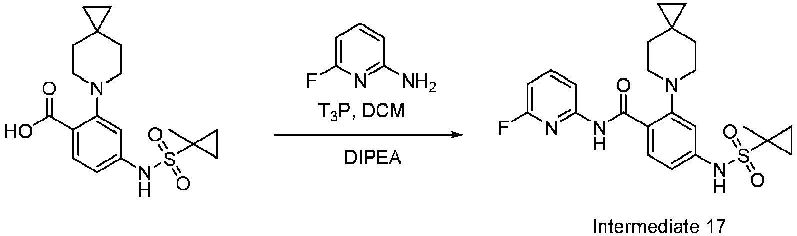

To a solution of 4-((1-methylcyclopropane)-1-sulfonamido)-2-(6-azaspiro[2.5]octan-6-yl)benzoic acid (1.0 g, 2.74 mmol, Intermediate 11) in DCM (20 mL) was added 6-fluoropyridin-2-amine (0.308 g, 2.74 mmol, Combi-Blocks), T₃P (50% in ethyl acetate, 1.3 mL, 4.1 mmol) and DIPEA (0.958 mL, 5.49 mmol). The resulting mixture was stirred at room temperature for 16 h. The reaction mixture was quenched with satd. NaHCO₃ solution (10 mL) and extracted with DCM (2 x 25 mL). The combined organic extracts were washed with brine (20 mL), dried over Na₂SO₄, filtered, and concentrated under reduced pressure. The crude residue was purified by column chromatography over silica gel using 20 % ethyl acetate in hexanes to give the title compound (0.65 g, 52 % yield) as an off-white solid. ¹H NMR (400 MHz, DMSO-*d*₆): δ 13.09 (s, 1H), 10.28 (s, 1H), 8.20 (dd, *J* = 8.0, 2.4 Hz, 1H), 8.12 - 7.92 (m, 2H), 7.36 (d, *J* = 2.2 Hz, 1H), 7.17 (dd, *J* = 8.6, 2.2 Hz, 1H), 6.89 (dd, *J* = 8.0, 2.4 Hz, 1H), 2.97 (t, *J* = 5.4 Hz, 4H), 1.79 - 1.56 (m, 4H), 1.41 (s, 3H), 1.32 - 1.14 (m, 2H), 0.90 - 0.79 (m, 2H), 0.41 (s, 4H). *m*/*z* (ESI): 459.1 (M-H)⁺.

**Table 4: Intermediates 18 - 36 were prepared following a similar procedure as described for intermediate 17**

| Int. # | Chemical Structure | Name | LRMS: (ESI + ve ion) *m*/*z* |
|---|---|---|---|
| 18 | 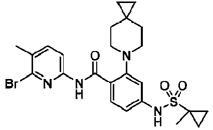 | *N*-(6-Bromo-5-methylpyridin-2-yl)-4-((1-methylcyclopropane)-1-sulfonamido)-2-(6-azaspiro[2.5]octan-6-yl)benzamide | 533.1/535.1 |
| 19 | 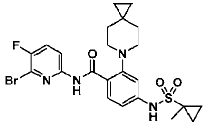 | *N*-(6-Bromo-5-fluoropyridin-2-yl)-4-((1-methylcyclopropane)-1-sulfonamido)-2-(6-azaspiro[2.5]octan-6-yl)benzamide | 537.0/539.0 |
| 20 | 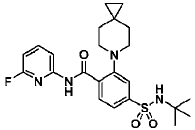 | 4-(*N*-(*tert*-Butyl)sulfamoyl)-N-(6-fluoropyridin-2-yl)-2-(6-azaspiro[2.5]octan-6-yl)benzamide | 461.2 |
| 21 | 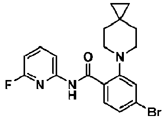 | 4-Bromo-*N*-(6-fluoropyridin-2-yl)-2-(6-azaspiro[2.5]octan-6-yl)benzamide | 406.0/408.0 |
| 22 | 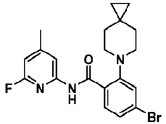 | 4-Bromo-*N*-(6-fluoro-4-methylpyridin-2-yl)-2-(6-azaspiro[2.5]octan-6-yl)benzamide | 418.1/420.1 |
| 23 | 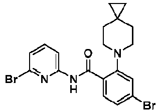 | 4-Bromo-*N*-(6-bromopyridin-2-yl)-2-(6-azaspiro[2.5]octan-6-yl)benzamide | 466.2/468.2 |
| 24 | 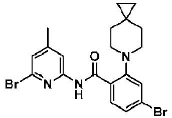 | 4-Bromo-*N*-(6-bromo-4-methylpyridin-2-yl)-2-(6-azaspiro[2.5]octan-6-yl)benzamide | 479.9/481.9 |
| 25 | 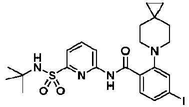 | *N*-(6-(*N*-(tert-Butyl)sulfamoyl)pyridin-2-yl)-4-iodo-2-(6-azaspiro[2.5]octan-6-yl)benzamide | 569.3 |
| 26 | 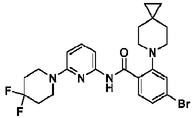 | 4-Bromo-*N*-(6-(4,4-difluoropiperidin-1-yl)pyridin-2-yl)-2-(6-azaspiro[2.5]octan-6-yl)benzamide | 505.2/507.2 |
| 27 | 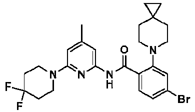 | 4-Bromo-*N*-(6-(4,4-difluoropiperidin-1-yl)-4-methylpyridin-2-yl)-2-(6-azaspiro[2.5]octan-6-yl)benzamide | 519.3/521.3 |
| 28 | 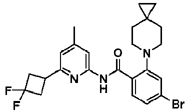 | 4-Bromo-*N*-(6-(3,3-difluorocyclobutyl)-4-methylpyridin-2-yl)-2-(6-azaspiro[2.5]octan-6-yl)benzamide | 490.1/492.1 |
| 29 | 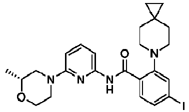 | (*R*)-4-Iodo-*N*-(6-(2-methylmorpholino)pyridin-2-yl)-2-(6-azaspiro[2.5]octan-6-yl)benzamide | 491.2/493.2 |
| 30 | 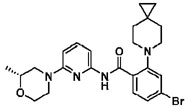 | (*R*)-4-Bromo-*N*-(6-(2-mettrylmorpholino)pyridin-2-yl)-2-(6-azaspiro[2.5]octan-6-yl)benzamide | 485.2/487.2 |
| 31 | 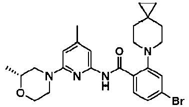 | (*R*)-4-Bromo-*N*-(4-methyl-6-(2-methylmorpholino)pyridin-2-yl)-2-(6-azaspiro[2.5]octan-6-yl)benzamide | 499.2/501.2 |
| 32 | 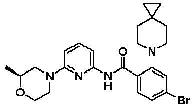 | (*S*)-4-Bromo-*N*-(6-(2-methylmorpholino)pyridin-2-yl)-2-(6-azaspiro[2.5]octan-6-yl)benzamide | 485.2/487.2 |
| 33 | 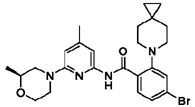 | (*S*)-4-Bromo-*N*-(4-methyl-6-(2-methylmorpholino)pyridin-2-yl)-2-(6-azaspiro[2.5]octan-6-yl)benzamide | 499.2/501.2 |
| 34 | 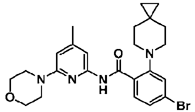 | 4-Bromo-*N*-(4-methyl-6-morpholinopyridin-2-yl)-2-(6-azaspiro[2.5]octan-6-yl)benzamide | 485.2/487.2 |
| 35 | 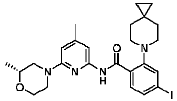 | (*R*)-4-Iodo-*N*-(4-methyl-6-(2-methylmorpholino)pyridin-2-yl)-2-(6-azaspiro[2.5]octan-6-yl)benzamide | 547.3 |
| 36 | 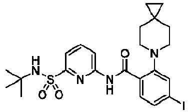 | *N*-(6-(*N*-(*tert*-Butyl)sulfamoyl)pyridin-2-yl)-4-iodo-2-(6-azaspiro[2.5]octan-6-yl)benzamide | 569.3 |
| 37 | 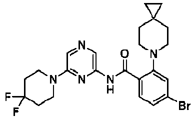 | 4-Bromo-*N*-(6-(4,4-difluoropiperidin-1-yl)pyrazin-2-yl)-2-(6-azaspiro[2.5]octan-6-yl)benzamide | 506.1/508.1 |

### Intermediate 38: 4-Iodo-2-(6-azaspiro[2.5]octan-6-yl)-N-(6-(3,3,3-trifluoropropoxy)pyridin-2-yl)benzamide

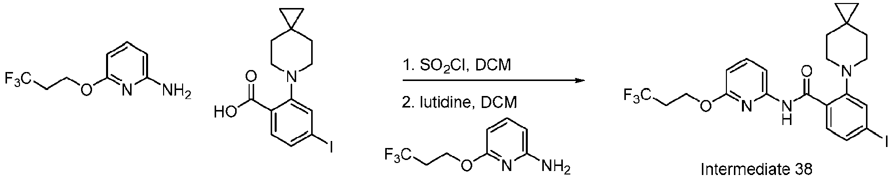

4-Iodo-2-(6-azaspiro[2.5]octan-6-yl)benzoic acid (3.0 g, 8.4 mmol, Intermediate 9) was suspended in DCM (10 mL) under argon. Thionyl chloride (1.463 g, 12.29 mmol, Sigma-Aldrich Corporation) was added and the mixture stirred at rt for 15 min. The mixture was evaporated to dryness under reduced pressure. The crude residue was azeotroped with toluene (2 x 100 mL) and suspended in dichloromethane (50 mL) under argon. A solution of 6-(3,3,3-trifluoropropoxy)pyridin-2-amine (2.0 g, 9.7 mmol, Intermediate 6) and 2,6-lutidine (3.68 g, 34.3 mmol, Sigma-Aldrich Corporation) in dichloromethane (10 mL) was added in one portion. The yellow mixture was stirred at rt for 20 min then evaporated to dryness under reduced pressure. The crude solids were triturated in methanol (30 mL) for 20 min then filtered through a sintered glass frit. The solids were dried under a stream of nitrogen to give 4-iodo-2-(6-azaspiro[2.5]octan-6-yl)-*N*-(6-(3,3,3-trifluoropropoxy)pyridin-2-yl)benzamide (3.8 g, 7.0 mmol, 83 % yield). ¹H NMR (400 MHz, DMSO-*d*₆) δ ppm 0.36 (s, 4 H) 1.58-180 (m, 4 H) 2.83 (dt, *J*=10.94, 5.42 Hz, 2 H) 2.91 - 3.13 (m, 4 H) 4.49 (t, *J*=5.29 Hz, 2 H) 6.58 (d, *J*=7.88 Hz, 1 H) 7.60 - 8.00 (m, 5 H) 13.20 (br s, 1 H)

**Table 5: Intermediates 38-1 to 38-3 were prepared following a similar procedure as described for intermediate 38**

| Int. # | Chemical Structure | Name | LRMS: (ESI + ve ion) *m*/*z* |
|---|---|---|---|
| 38-1 | 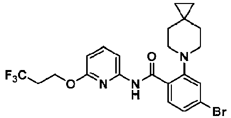 | 4-Bromo-2-(6-azaspiro[2.5]octan-6-yl)-*N-*(6-(3,3,3-trifluoropropoxy)pyridin-2-yl)benzamide | 498.1/500.1 |
| 38-2 | 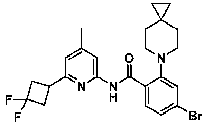 | 4-Bromo-*N*-(6-(3,3-difluorocyclobutyl)-4-methylpyridin-2-yl)-2-(6-azaspiro[2.5]octan-6-yl)benzamide | 490.1/492.1 |
| 38-3 | 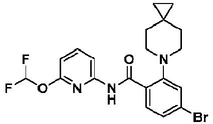 | 4-Bromo-*N*-(6-(difluoromethoxy)pyridin-2-yl)-2-(6-azaspiro[2.5]octan-6-yl)benzamide | 452.1/454.1 |

### Intermediate 39: 4-Bromo-N-(6-(3,3-difluoroazetidin-1-yl)-4-methylpyridin-2-yl)-2-(6-azaspiro[2.5]octan-6-yl)benzamide

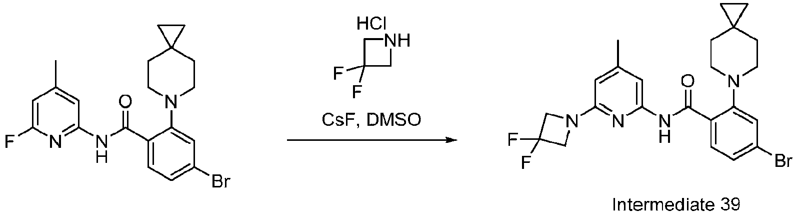

A mixture of 4-bromo-*N*-(6-bromo-4-methylpyridin-2-yl)-2-(6-azaspiro[2.5]octan-6-yl)benzamide (5.0 g, 10 mmol, Int. 22), 3,3-difluoroazetidine hydrochloride (2.027 g, 15.65 mmol, Combi-Blocks) and CsF (9.51 g, 62.6 mmol) in DMSO (50 mL) was stirred at 145 °C for 60 h. The reaction mixture was diluted with ice-water (200 mL) and was stirred for 30 min. The precipitate was filtered and was purified by flash column chromatography using a gradient of 0 % to 5 % EtOAc in petroleum ether to provide 4-bromo-*N*-(6-(3,3-diffuoroazetidin-1-yl)-4-methylpyridin-2-yl)-2-(6-azaspiro[2.5]octan-6-yl)benzamide (2.4 g, 4.8 mmol, 46% yield) as a white solid. ¹H NMR (300 MHz, DMSO-*d*₆): δ ppm 13.05 (s, 1 H), 8.03 (d, *J*=8.7 Hz, 1 H), 7.67 (s, 1 H), 7.53 - 7.56 (m, 2 H), 6.21 (s, 1 H), 4.32 - 4.41 (m, 4 H), 3.33 (t, *J*=5.1 Hz, 4 H), 2.28 (s, 3 H), 1.72 (br s, 4 H), 0.37 (s, 4 H). m/z (ESI): 491.1 (M+H)⁺.

### Intermediate 40: Ethyl 2-sulfamoylpropanoate

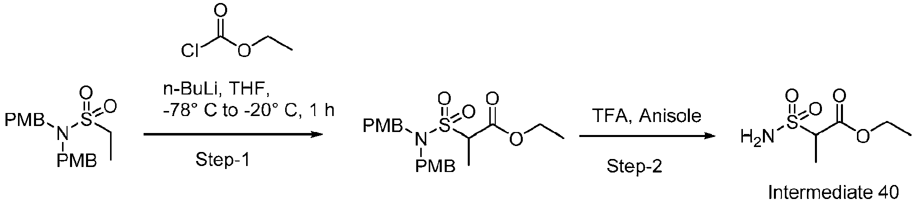

Step 1: To a solution of *N,N-*bis(4-methoxybenzyl)ethanesulfonamide (200.0 g, 572.0 mmol) in tetrahydrofuran (4000 mL) was added nBuLi (1.6 M in hexane, 608.0 mL, 973.0 mmol) at -78 °C slowly and stirred for 30 min. Ethyl carbonochloridate (92.0 mL, 973.0 mmol) in THF (50 mL) was added to the reaction mixture and stirred at -78 °C for 1 h. The reaction mixture was quenched with HCl (1.5 N, 3000 mL) and extracted with EtOAc (2 x 3000 mL). The organic extract was dried over sodium sulphate, filtered and concentrated in reduced pressure to give the crude material of ethyl 2-(N,N-bis(4-methoxybenzyl)sulfamoyl)propanoate (250.0 g, 60% pure) as yellow oil. The ¹H-NMR showed desired peaks and proceeded to next step without any purification.

Step 2: To solution of ethyl 2-(*N*,*N* bis(4-methoxybenzyl)sulfamoyl)propanoate (600.0 g, 1.4 mol) in trifluoroacetic acid (2.50 L, 32.45 mol) was added anisole (500.0 mL, 4.57 mol) and stirred at room temperature for 3 h. The reaction mixture was concentrated under reduced pressure, quenched with 10% cold aqueous NaHCO₃ solution (3 L) and extracted with EtOAc (2 x 3 L). The organic layer was dried over sodium sulfate, filtered and concentrated under reduced pressure. The crude residue was purified by column chromatography over silica gel using 25% ethyl acetate in hexanes to give a pale yellow solid (168 g) which was dissolved in DCM (1 L) and precipitated by the addition of hexanes (3000 mL). The solid was filtered, dried under vacuum to give the title compound (109.0 g, 42% yield) as a white solid. ¹H NMR (400 MHz, DMSO-*d*₆) *δ* ppm 7.14 (s, 2H), 4.15 (q, *J* = 7.1 Hz, 2H), 3.98 (q, *J* = 7.0 Hz, 1H), 1.45 (d, *J* = 7.0 Hz, 3H), 1.21 (t, *J* = 7.1 Hz, 3H). *m*/*z* (ESI): 180.1 (M-H)⁺.

### EXAMPLES

### General Methods and Representatives Examples

### Method A (Pd-catalyzed Br-S coupling)

### Example 1: 4-(N-(2-Hydroxyethyl)sulfamoyl)-2-(6-azaspiro[2.5]octan-6-yl)-N-(6-(3.3.3-trifluoropropoxy)pyridin-2-yl)benzamide

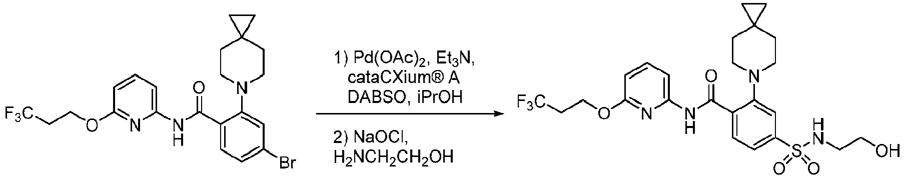

A glass vial was charged with 4-bromo-2-(6-azaspiro[2.5]octan-6-yl)-*N*-(6-(3,3,3-trifluoropropoxy)pyridin-2-yl)benzamide (348 mg, 0.70 mmol Intermediate 38-1), 1,4-diazabicyclo[2.2.2]octane bis(sulfur dioxide) adduct (DABSO) (101 mg, 0.42 mmol, Sigma-Aldrich), diacetoxypalladium (16 mg, 0.07 mmol, Strem), rac-((3R,5R,7R)-adamantan-1-yl)((3S,5S,7S)-adamantan-1-yl)(butyl)phosphane (cataCXium^{®} A) (38 mg, 0.11 mmol, Strem), triethylamine (194 uL, 1.40 mmol), and iPrOH (3 mL). The tube was degassed for 3 min, sealed, and heated at 80 °C in an oil bath for 3 h. The heterogeneous mixture was cooled to RT and treated with 2-aminoethan-1-ol (85 mg, 1.4 mmol, Sigma-Aldrich) followed by sodium hypochlorite solution (10 % wt., 1040 mg, 1.40 mmol, Sigma-Aldrich) and stirred at RT for 18 h. EtOAc (20 mL) and water (5 mL) were added to the heterogeneous mixture. The insoluble solid was filtered off. The filter cake was washed with 2 x 2 mL of water followed by 2 x 4 mL of EtOAc. The combined organic extracts were separated and concentrated. The residue was purified by silica gel chromatography (20% to 80% EtOAc in heptane) to give 4-(N-(2-hydroxyethyl)sulfamoyl)-2-(6-azaspiro[2.5]octan-6-yl)-N-(6-(3,3,3-trifluoropropoxy)pyridin-2-yl)benzamide (106 mg, 0.19 mmol, 28 % yield) as a light yellow solid. ¹H NMR (400 MHz, DMSO-*d*₆) δ 13.04 (s, 1H), 8.26 (d, *J*=8.09 Hz, 1H), 7.91 (d, *J*=7.67 Hz, 1H), 7.77-7.87 (m, 3H), 7.72 (d, *J*=7.26 Hz, 1H), 6.62 (d, *J*=8.09 Hz, 1H), 4.71 (t, *J*=5.08 Hz, 1H), 4.53 (t, *J*=6.01 Hz, 2H), 3.33-3.44 (m, 2H), 3.09 (m, 4H), 2.77-2.93 (m, 4H), 1.73 (s, 4H), 0.39 (s, 4H). *m*/*z* (ESI): (M+H)⁺ 543.2.

**Table 6: Examples 1-1 to 1-13 were prepared following a similar procedure as described for Example 1**

| Ex. # | Chemical Structure | Name | LRMS: (ESI + ve ion) *m*/*z* |
|---|---|---|---|
| 1-1 | 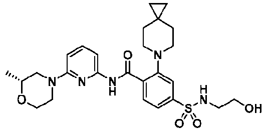 | (*R)*-4-(*N*-(2-Hydroxyethyl)sulfamoyl)-*N*-(6-(2-methylmorpholino)pyridin-2-yl)-2-(6-azaspiro[2.5]octan-6-yl)benzamide | 530.0 |
| 1-2 | 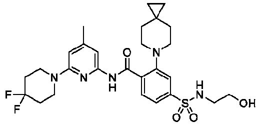 | *N*-(6-(4,4-Difluoropiperidin-1-yl)-4-methylpyridin-2-yl)-4-(*N*-(2-hydroxyethyl)sulfamoyl)-2-(6-azaspiro[2.5]octan-6-yl)benzamide | 564.2 |
| 1-3 | 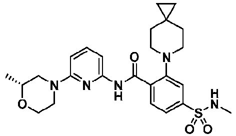 | (*R*)-*N*-(6-(2-Methylmorpholino)pyridin-2-yl)-4-(*N*-methylsulfamoyl)-2-(6-azaspiro[2.5]octan-6-yl)benzamide | 500.1 |
| 1-4 | 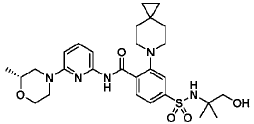 | (*R*)-4-(*N*-(1-Hydroxy-2-methylpropan-2-yl)sulfamoyl)-*N*-(6-(2-methylmorpholino)pyridin-2-yl)-2-(6-azaspiro[2.5]octan-6-yl)benzamide | 558.2 |
| 1-5 | 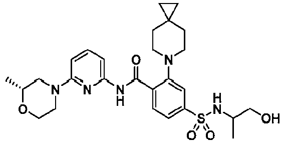 | 4-(*N*-(1-Hydroxypropan-2-yl)sulfamoyl)-*N-*(6-((R)-2-methylmorpholino)pyridin-2-yl)-2-(6-azaspiro[2.5]octan-6-yl)benzamide | 544.1 |
| 1-6 | 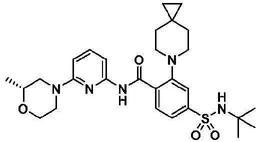 | (*R*)-4-(*N*-(*tert*-Butyl)sulfamoyl)-*N*-(6-(2-methylmorpholino)pyridin-2-yl)-2-(6-azaspiro[2.5]octan-6-yl)benzamide | 542.3 |
| 1-7 | 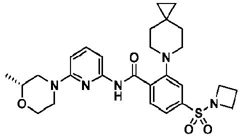 | (*R*)-4-(Azetidin-1-ylsulfonyl)-*N*-(6-(2-methylmorpholino)pyridin-2-yl)-2-(6-azaspiro[2.5]octan-6-yl)benzamide | 526.1 |
| 1-8 | 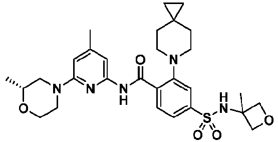 | (*R*)-*N*-(4-Methyl-6-(2-methylmorpholino)pyridin-2-yl)-4-(N-(3-methyloxetan-3-yl)sulfamoyl)-2-(6-azaspiro[2.5]octan-6-yl)benzamide | 570.4 |
| 1-9 | 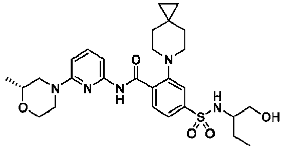 | 4-(*N*-(1-Hydroxybutan-2-yl)sulfamoyl)-*N*-(6-((R)-2-methylmorpholino)pyridin-2-yl)-2-(6-azaspiro[2.5]octan-6-yl)benzamide | 558.2 |
| 1-10 | 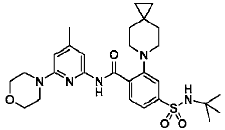 | 4-(*N-*(tert-Butyl)sulfamoyl)-*N*-(4-methyl-6-morpholinopyridin-2-yl)-2-(6-azaspiro[2.5]octan-6-yl)benzamide | 542.0 |
| 1-11 | 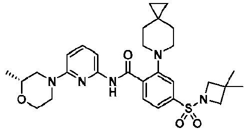 | (R)-4-((3,3-Dimethylazeridin-1-yl)sulfonyl)-*N*-(6-(2-methylmorpholino)pyridin-2-yl)-2-(6-azaspiro[2.5]octan-6-yl)benzamide | 554.2 |
| 1-12 | 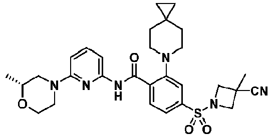 | (*R*)-4-((3-Cyano-3-methylazetidin-1-yl)sulfonyl)-N-(6-(2-methylmorpholino)pyridin-2-yl)-2-(6-azaspiro[2.5]octan-6-yl)benzamide | 565.3 |
| 1-13 | 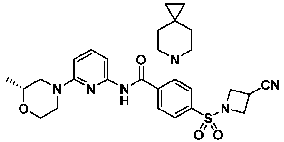 | (*R*)-4-((3-Cyanoazetidin-1-yl)sulfonyl)-N-(6-(2-methylmorpholino)pyridin-2-yl)-2-(6-azaspiro[2.5]octan-6-yl)benzamide | 551.2 |

### Method B (Sulfone formation)

### Example 2: (R)-4-(Isopropylsulfonyl)-N-(6-(2-methylmorpholino)pyridin-2-yl)-2-(6-azaspiro[2.5]octan-6-yl)benzamide

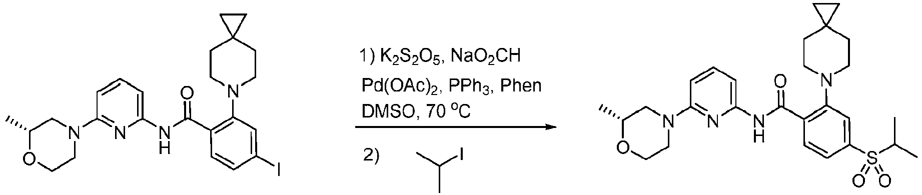

A mixture of (*R*)-4-iodo-*N*-(6-(2-methylmorpholino)pyridin-2-yl)-2-(6-azaspiro[2.5]octan-6-yl)benzamide (89 mg, 0.17 mmol, Intermediate 29), triphenylphosphine (6 mg, 0.025 mmol, Aldrich St. Louis, MO USA), 1,10-phenanthroline (24 mg, 0.132 mmol, Aldrich St. Louis, USA), palladium(ii) acetate (5 mg, 0.025 mmol, Strem Chemicals, Inc. Newburyport, MA USA), sodium formate (25 mg, 0.37 mmol, Thermo Fisher Scientific, Grand Island, NY USA), and tetrabutylammonium bromide (59 mg, 0.18 mmol, Aldrich St. Louis, MO USA ) in DMSO (2 mL) under N₂ was stirred at 70 °C for 3 h. Then, the mixture was cooled to room temperature and isopropyl iodide (0.025 mL, 0.25 mmol, Aldrich St. Louis, MO USA) was added. The mixture was then stirred at room temperature for 18 h. Then, the mixture was diluted with water (20 mL) and was then extracted with EtOAc (2 x 40 mL). The combined organic extracts were then dried over Na₂SO₄ and concentrated. Chromatographic purification of the residue (silica gel, 0%-30% EtOAc/heptane) provided (*R*)-4-(isopropylsulfonyl)-*N*-(6-(2-methylmorpholino)pyridin-2-yl)-2-(6-azaspiro[2.5]octan-6-yl)benzamide (46 mg, 0.09 mmol, 36% yield) as a light yellow solid. ¹H NMR (400 MHz, DMSO-*d*₆) δ 12.63 (s, 1H), 8.31 (d, *J* = 8.22 Hz, 1H), 7.82 (d, *J* = 1.37 Hz, 1H), 7.77 (dd, *J* = 1.56, 8.22 Hz, 1H), 7.57-7.66 (m, 2H), 6.64 (d, *J* = 7.83 Hz, 1H), 4.17 (br d, *J* = 11.93 Hz, 1H), 4.08 (br d, *J* = 12.52 Hz, 1H), 3.93 (dd, *J* = 3.33, 11.35 Hz, 1H), 3.49-3.67 (m, 3H), 3.37 (quin, *J* = 6.65 Hz, 1H), 3.10 (br t, *J* = 5.18 Hz, 4H), 2.85 (dt, *J* = 3.52, 12.32 Hz, 1H), 1.72 (br s, 4H), 1.13-1.28 (m, 9H), 0.39 (s, 4H). m/z (ESI): 513.2 (M+H)+.

**Table 7: Examples 2-1 to 2-7 were prepared following a similar procedure as described for Example 2**

| Ex. # | Chemical Structure | Name | LRMS: (ESI + ve ion) *m*/*z* |
|---|---|---|---|
| 2-1 | 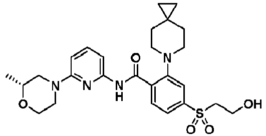 | (*R*)-4-((2-Hydroxyethyl)sulfonyl)-*N*-(6-(2-methylmorpholino)pyridin-2-yl)-2-(6-azaspiro[2.5]octan-6-yl)benzamide | 515.2 |
| 2-2 | 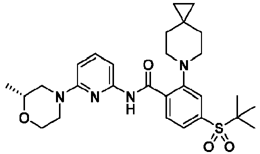 | (*R*)-4-(*tert-*Butylsulfonyl)-*N*-(6-(2-methylmorpholino)pyridin-2-yl)-2-(6-azaspiro[2.5]octan-6-yl)benzamide | 527.0 |
| 2-3 | 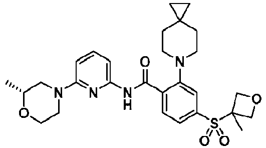 | (*R*)-*N*-(6-(2-Methylmorpholino)pyridin-2-yl)-4-((3-methyloxetan-3-yl)sulfonyl)-2-(6-azaspiro[2.5]octan-6-yl)benzamide | 541.3 |
| 2-4 | 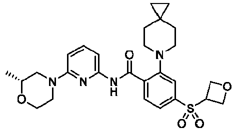 | (*R*)-*N*-(6-(2-Methylmorpholino)pyridin-2-yl)-4-(oxetan-3-ylsulfonyl)-2-(6-azaspiro[2.5]octan-6-yl)benzamide | 527.2 |
| 2-5 | 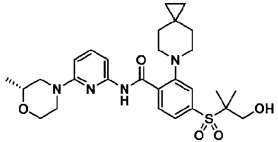 | (*R*)-4-((1-Hydroxy-2-methylpropan-2-yl)sulfonyl)-*N*-(6-(2-methylmorpholino)pyridin-2-yl)-2-(6-azaspiro[2.5]octan-6-yl)benzamide | 543.2 |
| 2-6 | 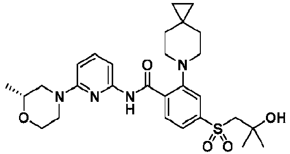 | (*R*)-4-((2-Hydroxy-2-methylpropyl)sulfonyl)-*N*-(6-(2-methylmorpholino)pyridin-2-yl)-2-(6-azaspiro[2.5]octan-6-yl)benzamide | 543.2 |
| 2-7 | 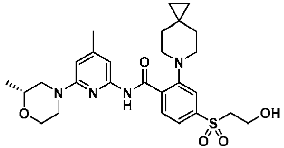 | (*R*)-4-((2-Hydroxyethyl)sulfonyl)-*N*-(4-methyl-6-(2-methylmorpholino)pyridin-2-yl)-2-(6-azaspiro[2.5]octan-6-yl)benzamide | 529.2 |

### Method C (SNAr)

### Example 3: (R)-N-(6-(3-Hydroxypiperidin-1-yl)pyridin-2-yl)-4-((1-methylcvclopropane)-1-sulfonamido)-2-(6-azaspiro[2.5]octan-6-yl)benzamide

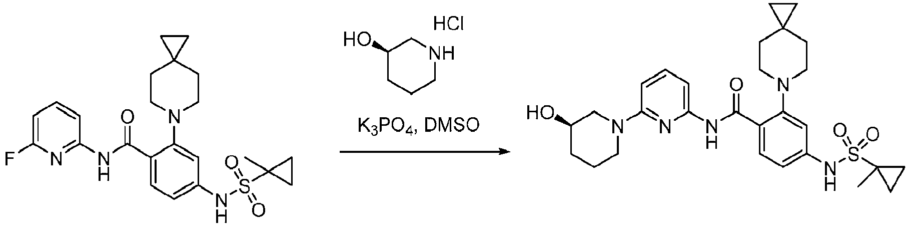

To a solution of *N*-(6-fluoropyridin-2-yl)-4-((1-methylcyclopropane)-1-sulfonamido)-2-(6-azaspiro[2.5]octan-6-yl)benzamide (0.20 g, 0.44 mmol, Intermediate 17) in DMSO (2 mL) was added (*R*)-piperidin-3-ol hydrochloride (0.060 g, 0.44 mmol, Essen Scientific) and potassium phosphate tribasic (0.278 g, 1.31mmol). The reaction mixture was stirred at 130 °C for 16 h, before cooled, quenched with water (5 mL), and extracted with ethyl acetate (2 x 10 mL). The combined organic extracts were washed with brine solution (10 mL), dried over Na₂SO₄, filtered, and concentrated under reduced pressure. The crude residue was purified by column chromatography over silica gel using 30-50% ethyl acetate in hexanes to give the title compound (0.1 g, 43% yield) as an off-white solid. ¹H NMR (400 MHz, DMSO-*d*₆) δ 12.85 (s, 1H), 10.23 (s, 1H), 8.06 (d, *J* = 8.6 Hz, 1H), 7.52 (d, *J* = 6.1 Hz, 2H), 7.34 (s, 1H), 7.14 (d, *J* = 8.6 Hz, 1H), 6.65 - 6.34 (m, 1H), 4.78 (d, *J* = 4.6 Hz, 1H), 4.13 (d, *J* = 11.7 Hz, 1H), 4.05 - 3.85 (m, 1H), 3.63 - 3.46 (m, 1H), 3.10 - 2.74 (m, 6H), 1.99 - 1.58 (m, 5H), 1.42 (d, *J* = 13.8 Hz, 5H), 1.20 (s, 3H), 0.84 (d, *J* = 5.6 Hz, 2H), 0.38 (s, 4H). m/z (ESI): 540.2 (M+H)+.

**Table 8: Examples 3-1 to 3-23 were prepared following a similar procedure as described for Example 3**

| Ex. # | Chemical Structure | Name | LRMS: (ESI + ve ion) *m*/*z* |
|---|---|---|---|
| 3-1 | 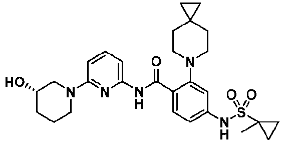 | (*S*)-*N*-(6-(3-Hydroxypiperidin-1-yl)pyridin-2-yl)-4-((1-methylcyclopropane)-1-sulfonamido)-2-(6-azaspiro[2.5]octan-6-yl)benzamide | 540.2 |
| 3-2 | 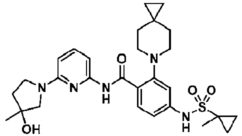 | *N*-(6-(3-Hydroay-3-methylpyrrolidin-1-yl)pyridin-2-yl)-4-((1-methylcyclopropane)-1-sulfonamido)-2-(6-azaspiro[2.5]octan-6-yl)benzamide | 540.2 |
| 3-3 | 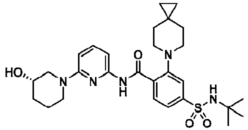 | (*S*)-4-(*N*-(*tert*-Butyl)sulfamoyl)-N-(6-(3-hydroxypiperidin-1-yl)pyridin-2-yl)-2-(6-azaspiro[2.5]octan-6-yl)benzamide | 542.3 |
| 3-4 | 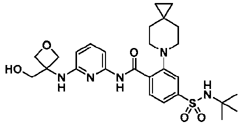 | 4-(*N*-(*tert*-Butyl)sulfamoyl)-*N*-(6-((3-(hydroxymethyl)oxetan-3-yl)amino)pyridin-2-yl)-2-(6-azaspiro[2.5]octan-6-yl)benzamide | 544.3 |
| 3-5 | 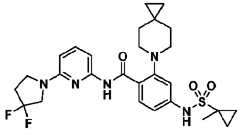 | *N*-(6-(3,3-Difluoropyrrolidin-1-yl)pyridin-2-yl)-4-((1-methylcyclopropane)-1-sulfonamido)-2-(6-azaspiro[2.5]octan-6-yl)benzamide | 546.2 |
| 3-6 | 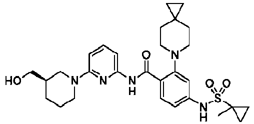 | (*R*)-*N-*(6-(3-(Hydroxymethyl)piperidin-1-yl)pyridin-2-yl)-4-((1-methylcyclopropane)-1-sulfonamido)-2-(6-azaspiro[2.5]octan-6-yl)benzamide | 554.2 |
| 3-7 | 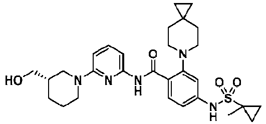 | (*S*)-*N*-(6-(3-(Hydroxymethyl)piperidin-1-yl)pyridin-2-yl)-4-((1-methylcyclopropane)-1-sulfonamido)-2-(6-azaspiro[2.5]octan-6-yl)benzamide | 554.2 |
| 3-8 | 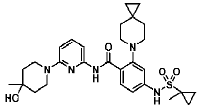 | *N*-(6-(4-Hydroxy-4-methylpiperidin-1-yl)pyridin-2-yl)-4-((1-methylcyclopropane)-1-sulfonamido)-2-(6-azaspiro[2.5]octan-6-yl)benzamide | 554.2 |
| 3-9 | 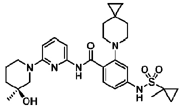 | (*S*)-*N*-(6-(3-Hydroxy-3-methylpiperidin-1-yl)pyridin-2-yl)-4-((1-methylcyclopropane)-1-sulfonamido)-2-(6-azaspiro[2.5]octan-6-yl)benzamide | 554.2 |
| 3-10 | 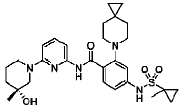 | (*R*)-*N*-(6-(3-Hydroxy-3-methylpiperidin-1-yl)pyridin-2-yl)-4-((1-methylcyclopropane)-1-sulfonamido)-2-(6-azaspiro[2.5]octan-6-yl)benzamide | 554.2 |
| 3-11 | 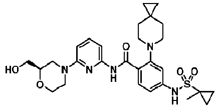 | (*R*)-*N*-(6-(2-(Hydroxymethyl)morpholino)pyridin-2-yl)-4-((1-methylcyclopropane)-1-sulfonamido)-2-(6-azaspiro[2.5]octan-6-yl)benzamide | 556.2 |
| 3-12 | 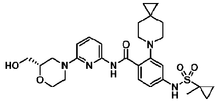 | (*S*)-*N*-(6-(2-(Hydroxymethyl)morpholino)pyridin-2-yl)-4-((1-methylcyclopropane)-1-sulfonamido)-2-(6-azaspiro[2.5]octan-6-yl)benzamide | 556.2 |
| 3-13 | 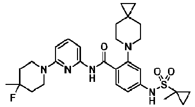 | *N*-(6-(4-Fluoro-4-methylpiperidin-1-yl)pyridin-2-yl)-4-((1-methylcyclopropane)-1-sulfonamido)-2-(6-azaspiro[2.5]octan-6-yl)benzamide | 556.3 |
| 3-14 | 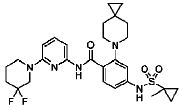 | *N*-(6-(3,3-Difluoropiperidin-1-yl)pyridin-2-yl)-4-((1-methylcyclopropane)-1-sulfonamido)-2-(6-azaspiro[2.5]octan-6-yl)benzamide | 560.2 |
| 3-15 | 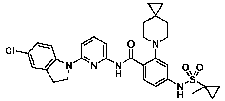 | *N*-(6-(5-Chloroindolin-1-yl)pyridin-2-yl)-4-((1-methylcyclopropane)-1-sulfonamido)-2-(6-azaspiro[2.5]octan-6-yl)benzamide | 592.2 |
| 3-16 | 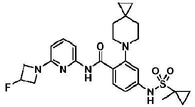 | *N*-(6-(3-Fluoroazetidin-1-yl)pyridin-2-yl)-4-((1-methylcyclopropane)-1-sulfonamido)-2-(6-azaspiro[2.5]octan-6-yl)benzamide | 514.2 |
| 3-17 | 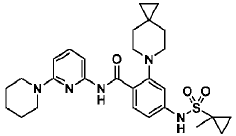 | 4-((1-Methylcyclopropane)-1-sulfonamido)-N-(6-(piperidin-1-yl)pyridin-2-yl)-2-(6-azaspiro[2.5]octan-6-yl)benzamide | 524.2 |
| 3-18 | 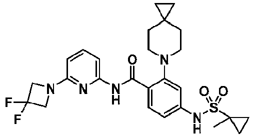 | *N*-(6-(3,3-Difluoroazetidin-1yl)pyridin-2-yl)-4-((1-methylcyclopropane)-1-sulfonamido)-2-(6-azaspiro[2.5]octan-6-yl)benzamide | 532.2 |
| 3-19 | 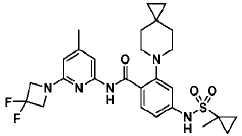 | *N*-(6-(3,3-Difluoroazetidin-1-yl)-4-methylpyridin-2-yl)-4-((1-methylcyclopropane)-1-sulfonamido)-2-(6-azaspiro[2.5]octan-6-yl)benzamide | 546.2 |
| 3-20 | 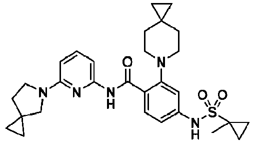 | N(6-(5-Azaspiro[2.4]heptan-5-yl)pyridin-2-yl)-4-((1-methylcyclopropane)-1-sulfonamido)-2-(6-azaspiro[2.5]octan-6-yl)benzamide | 536.2 |
| 3-21 | 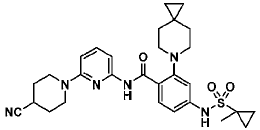 | *N*-(6-(4-Cyanopipeiidin-1-yl)pyridin-2-yl)-4-((1-methylcyclopropane)-1-sulfonamido)-2-(6-azaspiro[2.5]octan-6-yl)benzamide | 549.2 |
| 3-22 | 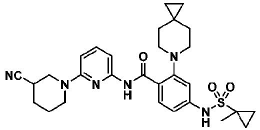 | *N*-(6-(3-Cyanopiperidin-l-yl)pyridin-2-y1)-4-((1-methylcyclopropane)-1-sulfonamido)-2-(6-azaspiro[2.5]octan-6-yl)benzamide | 549.2 |
| 3-23 | 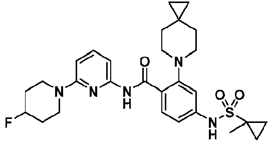 | *N*-(6-(4-Fluoropiperidin-1-yl)pyridin-2-yl)-4-((1-methylcyclopropane)-1-sulfonamido)-2-(6-azaspiro[2.5]octan-6-yl)benzamide | 542.2 |

### Method D (Amide Coupling)

### Example 4: (R)-N-(6-(3-Hydroxypiperidin-1-yl)pyridin-2-yl)-4-((methylsulfonyl)methyl)-2-(6-azaspiro[2.5]octan-6-yl)benzamide

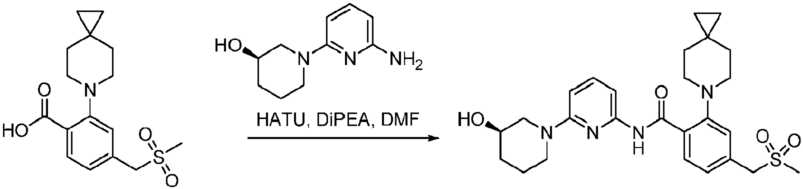

To a solution of 4-((methylsulfonyl)methyl)-2-(6-azaspiro[2.5]octan-6-yl)benzoic acid (200 mg, 0.618 mmol, Intermediate 14) in DMF (3 mL) were added (R)-1-(6-aminopyridin-2-yl)piperidin-3-ol (0.143 g, 0.742 mmol, Intermediate 4-5), HATU (353 mg, 0.928 mmol) and DIPEA (216 µL, 1.24 mmol) and stirred at room temperature for 12 h. The reaction mixture was quenched with water (20 mL) and extracted with ethyl acetate (3 × 20 mL). The combined organic extracts were washed with brine (20 mL), dried over Na₂SO₄, filtered, and concentrated under reduced pressure. The crude residue was purified by column chromatography over silica gel using 50 % ethyl acetate in hexanes to give the title compound (0.20 g, 65 % yield) as a yellow solid.¹H NMR (400 MHz, DMSO-*d*₆): *δ* 13.06 (s, 1H), 8.15 - 8.13 (m, 1H), 7.58 - 7.50 (m, 3H), 7.37 (d, *J=* 7.8 Hz, 1H), 6.55 (d, *J=* 8.2 Hz, 1H), 4.80 (dd, *J=* 7.0 , 3.6 Hz, 1H), 4.59 (s, 2H), 4.13 (d, *J=* 12.4 Hz, 1H), 3.94 (d, *J=* 12.4 Hz, 1H), 3.60 - 2.80 (m, 7 H), 1.73 (b s, 4H), 1.42 (b s, 4H), 1.42 (q, *J=* 6.4 Hz, 3H), 0.39 (s, 4H). m/z (ESI): 499.1 (M+H)+.

**Table 9: Examples 4-1 to 4-19 were prepared following a similar procedure as described for Example 4**

| Ex. # | Chemical Structure | Name | LRMS: (ESI + ve ion) *m*/*z* |
|---|---|---|---|
| 4-1 | 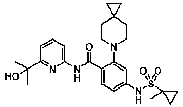 | *N*-(6-(2-Hydroxypropan-2-yl)pyridin-2-yl)-4-((1-methylcyclopropane)-1-sulfonamido)-2-(6-azaspiro[2.5]octan-6-yl)benzamide | 499.3 |
| 4-2 | 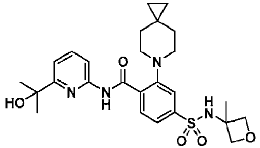 | *N*-(6-(2-Hydroxypropan-2-yl)pyridin-2-yl)-4-(*N*-(3-methyloxetan-3-yl)sulfamoyl)-2-(6-azaspiro[2.5]octan-6-yl)benzamide | 515.2 |
| 4-3 | 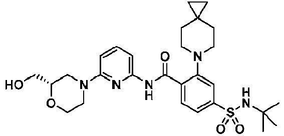 | (*S*)-4-(*N*-(*tert*-Butyl)sulfamoyl)-*N*-(6-(2-(hydroxymethyl)morpholino)pyridin-2-yl)-2-(6-azaspiro[2.5]octan-6-yl)benzamide | 558.0 |
| 4-4 | 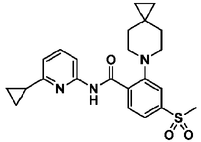 | *N*-(6-Cyclopropylpyridin-2-yl)-4-(methylsulfonyl)-2-(6-azaspiro[2.5]octan-6-yl)benzamide | 426.2 |
| 4-5 | 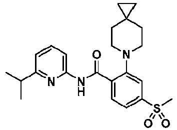 | *N*-(6-Isopropylpyridin-2-yl)-4-(methylsulfonyl)-2-(6-azaspiro[2.5]octan-6-yl)benzamide | 428.2 |
| 4-6 | 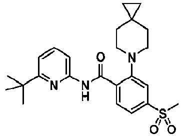 | *N*-(6-(*tert*-Butyl)pyridin-2-yl)-4-(methylsulfonyl)-2-(6-azaspiro[2.5]octan-6-yl)benzamide | 442.2 |
| 4-7 | 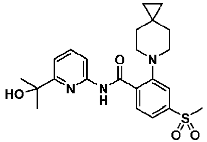 | *N*-(6-(2-Hydroxypropan-2-yl)pyridin-2-yl)-4-(methylsulfonyl)-2-(6-azaspiro[2.5]octan-6-yl)benzamide | 444.2 |
| 4-8 | 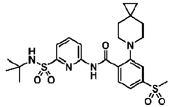 | *N*-(6-(*N*-(*tert*-Butyl)sulfamoyl)pyridin-2-yl)-4-(methylsulfonyl)-2-(6-azaspiro[2.5]octan-6-yl)benzamide | 521.2 |
| 4-9 | 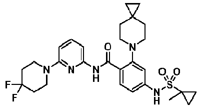 | *N*-(6-(4,4-Difluoropiperidin-1-yl)pyridin-2-yl)-4-((1-methylcyclopropane)-1-sulfonamido)-2-(6-azaspiro[2.5]octan-6-yl)benzamide | 560.2 |
| 4-10 | 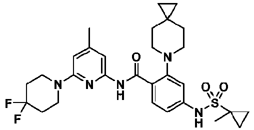 | *N*-(6-(4,4-Difluoropiperidin-1-yl)-4-methylpyridin-2-yl)-4-((1-methylcyclopropane)-1-sulfonamido)-2-(6-azaspiro[2.5]octan-6-yl)benzamide | 574.2 |
| 4-11 | 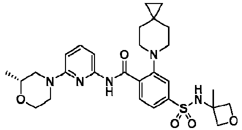 | (*R*)-*N*-(6-(2-Methylmorpholino)pyridin-2-yl)-4-(*N*-(3-methyloxetan-3-yl)sulfamoyl)-2-(6-azaspiro[2.5]octan-6-yl)benzamide | 556.2 |
| 4-12 | 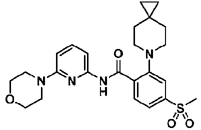 | 4-(Methylsulfonyl)-*N*-(6-morpholinopyridin-2-yl)-2-(6-azaspiro[2.5]octan-6-yl)benzamide | 471.2 |
| 4-13 | 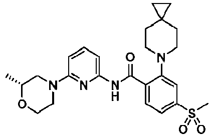 | (*R*)-*N-*(6-(2-Methylmorpholino)pyridin-2-yl)-4-(methylsulfonyl)-2-(6-azaspiro[2.5]octan-6-yl)benzamide | 485.1 |
| 4-14 | 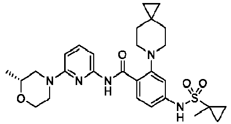 | (*R*)-4-((1-Methylcyclopropane)-1-sulfonamido)-*N*-(6-(2-methylmorpholino)pyridin-2-yl)-2-(6-azaspiro[2.5]octan-6-yl)benzamide | 540.2 |
| 4-15 | 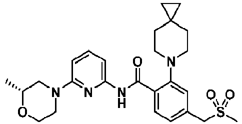 | (*R*)-*N-*(6-(2-Methylmorpholino)pyridin-2-yl)-4-((methylsulfonyl)methyl)-2-(6-azaspiro[2.5]octan-6-yl)benzamide | 499.2 |
| 4-16 | 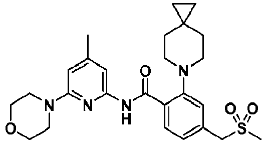 | *N*-(4-Methyl-6-morpholinopyridin-2-yl)-4-((methylsulfonyl)methyl)-2-(6-azaspiro[2.5]octan-6-yl)benzamide | 499.2 |
| 4-17 | 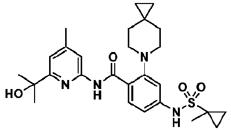 | *N*-(6-(2-Hydroxypropan-2-yl)-4-methylpyridin-2-yl)-4-((1-methylcyclopropane)-1-sulfonamido)-2-(6-azaspiro[2.5]octan-6-yl)benzamide | 513.2 |
| 4-18 | 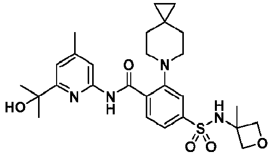 | *N*-(6-(2-Hydroxypropan-2-yl)-4-methylpyridin-2-yl)-4-(*N*-(3-methyloxetan-3 -yl)sulfamoyl)-2-(6-azaspiro [2.5] octan-6-yl)benzamide | 529.3 |
| 4-19 | 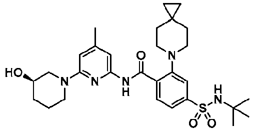 | (*R*)-4-(*N*-(*tert*-Butyl)sulfamoyl)-*N*-(6-(3-hydroaypiperidin-1-yl)-4-methylpyridin-2-yl)-2-(6-azaspiro[2.5]octan-6-yl)benzamide | 556.1 |

### Method E (Cu-catalyzed Sulfonamide Formation)

### Example 5: 4-((2-Hydroxyethyl)sulfonamido)-2-(6-azaspiro[2.5]octan-6-yl)-N-(6-(3,3,3-trifluoropropoxy)pyridin-2-yl)benzamide

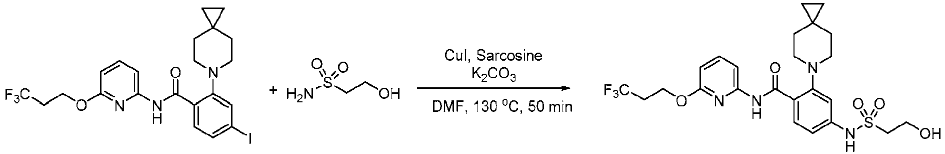

2-Hydroxyethane-1-sulfonamide (0.741 g, 5.92 mmol, Wuxi AppTec), sarcosine (0.172 g, 1.93 mmol, Ark Pharm, Inc.), copper(I) iodide (0.241 g, 1.26mmol, Sigma-Aldrich Corporation), potassium carbonate (2.78 g, 20.1 mmol, Thermo Fisher Scientific) and 4-iodo-2-(6-azaspiro[2.5]octan-6-yl)-N-(6-(3,3,3-trifluoropropoxy)pyridin-2-yl)benzamide (2.74 g, 5.02 mmol, Intermediate 38) were combined in degassed dry *N*,*N*-dimethylformamide (5 mL) under argon and heated to 130 °C for 50 min. The reaction was cooled to ambient temperature, water (100 mL) and ethyl acetate (150 mL) were added and the phases mixed and separated. The organic layer was washed with satd NH₄Cl: NH₄OH:H₂O (1:1:8, 2 × 75 mL) and evaporated to dryness under reduced pressure. The crude product was suspended in toluene (30 mL) and heated to 90 °C for 15 min. The mixture was cooled to ambient temperature and the solids were filtered off and dried under a stream of nitrogen. The white solids were suspended in water (100 mL) and heated to 90 °C for 20 minutes. The mixture was cooled to ambient temperature and the solids dried under a stream of nitrogen to give 4-((2-hydroxyethyl)sulfonamido)-2-(6-azaspiro[2.5]octan-6-yl)-*N*-(6-(3,3,3-trifluoropropoxy)pyridin-2-yl)benzamide (2.41 g, 4.44 mmol, 88 % yield). ¹H NMR (500 MHz, DMSO-d₆) δ 13.18 (s, 1H), 10.19 (br s, 1H), 8.08 (d, *J*=8.72 Hz, 1H), 7.91 (d, *J*=7.80 Hz, 1H), 7.76 (t, *J*=7.96 Hz, 1H), 7.29 (d, *J*=1.99 Hz, 1H), 7.14 (dd, *J*=2.07, 8.64 Hz, 1H), 6.57 (d, *J*=7.96 Hz, 1H), 4.93 (br s, 1H), 4.52 (t, *J*=6.12 Hz, 2H), 3.77 (t, *J*=6.43 Hz, 2H), 3.37 (t, *J*=6.43 Hz, 2H), 3.00 (br t, *J*=4.74 Hz, 4H), 2.80-2.87 (m, 2H), 1.74 (br s, 4H), 0.39 (s, 4H). *m*/*z* (ESI): 543.2.2 (M+H)⁺.

### Example 6: N-(6-(4,4-Difluoropiperidin-1-yl)-4-methylpyridin-2-yl)-4-((2-hydroxyethyl)sulfonamido)-2-(6-azaspiro[2.5]octan-6-yl)benzamide

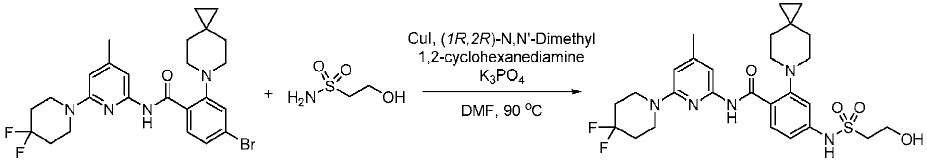

A mixture of 4-bromo-*N*-(6-(4,4-difluoropiperidin-1-yl)-4-methylpyridin-2-yl)-2-(6-azaspiro[2.5]octan-6-yl)benzamide (1.0 g, 1.9 mmol, Intermediate 27), methyl 2-sulfamoylacetate (0.361 g, 2.89 mmol, Wuxi AppTec), potassium phosphate (1.23 g, 5.78 mmol), (1*R,*2*R*)*-N1,N2-*dimethylcyclohexane-1,2-diamine (0.137 g, 0.963 mmol) and copper(I) iodide (0.183 g, 0.963 mmol) in DMF (20 mL) was heated at 90 °C for 16 h. Then the reaction mixture was filtered through a plug of celite. The filtrate was diluted with EtOAc, washed with water, brine, dried over Na₂SO₄, filtered, and concentrated. The residue was purified by flash column chromatography eluting with a gradient of 0% to 40% EtOAc in petroleum ether to provide *N-*(6-(4,4-difluoropiperidin-1-yl)-4-methylpyridin-2-yl)-4-((2-hydroxyethyl)sulfonamido)-2-(6-azaspiro[2.5]octan-6-yl)benzamide (0.580 g, 1.02 mmol, 53 % yield) as off-white solid. ¹H NMR (400 MHz, DMSO-*d*₆) δ ppm 12.85 (s, 1 H), 8.04 (d, *J*=8.6 Hz, 1 H), 7.51 (s, 1 H), 7.23 (d, *J*=2.2 Hz, 1 H), 7.09 (dd, *J*=8.7, 2.1 Hz, 1 H), 6.56 (s, 1 H), 3.74 (dt, *J*=12.5, 6.2 Hz, 6H), 2.97 (t, *J*=5.2 Hz, 4 H), 2.26 (s, 3 H), 1.99 (tt, *J*=13.6, 5.4 Hz, 3 H), 1.79 (s, 4 H), 1.60 (br s, 4H), 0.38 (s, 4 H). *m*/*z* (ESI): 564.2 (M+H)⁺.

**Table 10: Examples 7-1 to 7-44 were prepared following similar procedures as described for Examples 5 to 6**

| Ex. # | Chemical Structure | Name | LRMS: (ESI + ve ion) *m*/*z* |
|---|---|---|---|
| 7-1 | 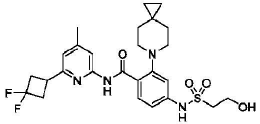 | *N-*(6-(3,3-Difluorocyclobutyl)-4-methylpyridin-2-yl)-4-((2-hydroxyethyl)sulfonamido)-2-(6-azaspiro[2.5]octan-6-yl)benzamide | 535.2 |
| 7-2 | 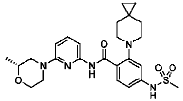 | (*R*)-*N-*(6-(2-Methylmorpholino)pyridin-2-yl)-4-(methylsulfonamido)-2-(6-azaspiro[2.5]octan-6-yl)benzamide | 500.2 |
| 7-3 | 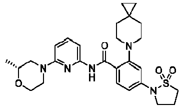 | (*R*)-4-(1,1-Dioxidoisothiazolidin-2-yl)-*N*-(6-(2-methylmorpholino)pyridin-2-yl)-2-(6-azaspiro[2.5]octan-6-yl)benzamide | 526.1 |
| 7-4 | 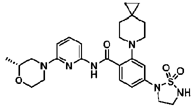 | (*R*)-4-(1,1-Dioxido-1,2,5-thiadiazolidin-2-yl)-*N*-(6-(2-methylmorpholino)pyridin-2-yl)-2-(6-azaspiro[2.5]octan-6-yl)benzamide | 527.2 |
| 7-5 | 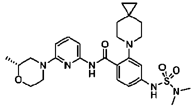 | (*R*)-4-((*N*,*N*-Dimethylsulfamoyl)amino)-*N*-(6-(2-methylmorpholino)pyridin-2-yl)-2-(6-azaspiro[2.5]octan-6-yl)benzamide | 529.0 |
| 7-6 | 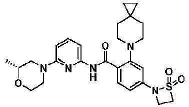 | (*R*)-4-(1,1-Dioxido-1,2-thiazetidin-2-yl)-*N*-(6-(2-methylmorpholino)pyridin-2-yl)-2-(6-azaspiro[2.5]octan-6-yl)benzamide | 512.3 |
| 7-7 | 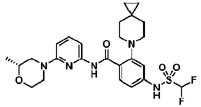 | (*R*)-4-((Difluoromethyl)sulfonamido)-*N-*(6-(2-methylmorpholino)pyridin-2-yl)-2-(6-azaspiro[2.5]octan-6-yl)benzamide | 536.2 |
| 7-8 | 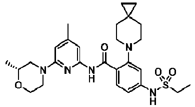 | (*R*)-4-(Ethylsulfonamido)-*N*-(4-methyl-6-(2-methylmorpholino)pyridin-2-yl)-2-(6-azaspiro[2.5]octan-6-yl)benzamide | 528.2 |
| 7-9 | 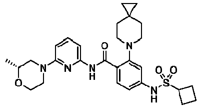 | (*R*)-4-(Cyclobutanesulfonamido)-*N-*(6-(2-methylmorpholino)pyridin-2-yl)-2-(6-azaspiro[2.5]octan-6-yl)benzamide | 540.2 |
| 7-10 | 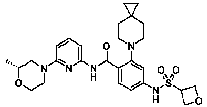 | (*R*)-*N*-(6-(2-Methylmorpholino)pyridin-2-yl)-4-(oxetane-3-sulfonamido)-2-(6-azaspiro[2.5]octan-6-yl)benzamide | 542.2 |
| 7-11 | 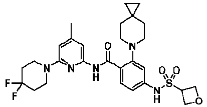 | *N*-(6-(4,4-Difluoropiperidin-1-yl)-4-methylpyridin-2-yl)-4-(oxetane-3-sulfonamido)-2-(6-azaspiro[2.5]octan-6-yl)benzamide | 576.2 |
| 7-12 | 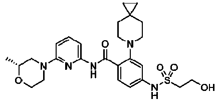 | (*R*)-4-((2-Hydroxyethyl)sulfonamido)-*N*-(6-(2-methylmorpholino)pyridin-2-yl)-2-(6-azaspiro[2.5]octan-6-yl)benzamide | 530.2 |
| 7-13 | 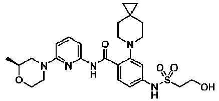 | (S)-4-((2-Hydroxyethyl)sulfonamido)-N-(6-(2-methylmorpholino)pyridin-2-yl)-2-(6-azaspiro [2.5]octan-6-yl)benzamide | 530.2 |
| 7-14 | 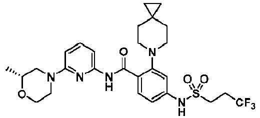 | (*R*)-*N-*(6-(2-Methylmorpholino)pyridin-2-yl)-2-(6-azaspiro[2.5]octan-6-yl)-4-((3,3,3-trifluoropropyl)sulfonamido)benzamide | 582.2 |
| 7-15 | 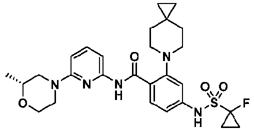 | (*R*)-4-((1-Fluorocyclopropane)-1-sulfonamido)-*N*-(6-(2-methylmorpholino)pyridin-2-yl)-2-(6-azaspiro[2.5]octan-6-yl)benzamide | 544.2 |
| 7-16 | 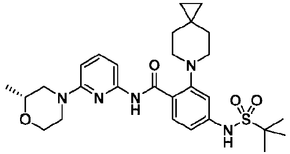 | (*R*)-4-((1,1-Dimethylethyl)sulfonamido)-*N*-(6-(2-methylmorpholino)pyridin-2-yl)-2-(6-azaspiro[2.5]octan-6-yl)benzamide | 542.2 |
| 7-17 | 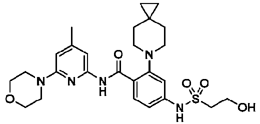 | 4-((2-Hydroxyethyl)sulfonamido)-*N*-(4-methyl-6-morpholinopyridin-2-yl)-2-(6-azaspiro[2.5]octan-6-yl)benzamide | 530.2 |
| 7-18 | 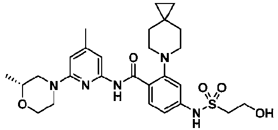 | (*R*)-4-((2-Hydroxyethyl)sulfonamido)-*N*-(4-methyl-6-(2-methylmorpholino)pyridin-2-yl)-2-(6-azaspiro[2.5]octan-6-yl)benzamide | 544.2 |
| 7-19 | 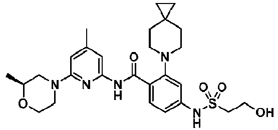 | (*S*)-4-((2-Hydroxyethyl)sulfonamido)-*N*-(4-methyl-6-(2-methylmorpholino)pyridin-2-yl)-2-(6-azaspiro[2.5]octan-6-yl)benzamide | 544.2 |
| 7-20 | 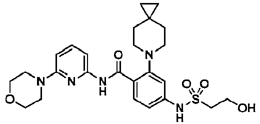 | 4-((2-Hydroxyethyl)sulfonamido)-*N*-(6-morpholinopyridin-2-yl)-2-(6-azaspiro[2.5]octan-6-yl)benzamide | 516.2 |
| 7-21 | 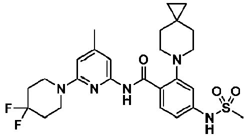 | *N*-(6-(4,4-Diffuoropiperidin-1-yl)-4-methylpyridin-2-yl)-4-(methylsulfonamido)-2-(6-azaspiro[2.5]octan-6-yl)benzamide | 534.2 |
| 7-22 | 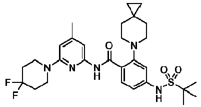 | *N*-(6-(4,4-Difluoropiperidin-1-yl)-4-methylpyridin-2-yl)-4-((1,1-dimethylethyl)sulfonamido)-2-(6-azaspiro[2.5]octan-6-yl)benzamide | 576.3 |
| 7-23 | 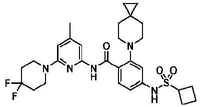 | 4-(Cyclobutanesulfonamido)-*N-*(6-(4,4-difluoropiperidin-1-yl)-4-methylpyridin-2-yl)-2-(6-azaspiro[2.5]octan-6-yl)benzamide | 574.2 |
| 7-24 | 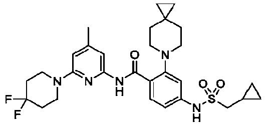 | 4-((Cyclopropylmethyl)sulfonamido)-*N*-(6-(4,4-difluoropiperidin-1-yl)-4-methylpyridin-2-yl)-2-(6-azaspiro[2.5]octan-6-yl)benzamide | 574.2 |
| 7-25 | 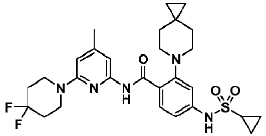 | 4-(Cyclopropanesulfonamido)-*N*-(6-(4,4-difluoropiperidin-1-yl)-4-methylpyridin-2-yl)-2-(6-azaspiro[2.5]octan-6-yl)benzamide | 560.2 |
| 7-26 | 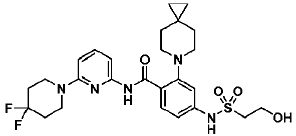 | *N*-(6-(4,4-Difluoropiperidin-1-yl)pyridin-2-yl)-4-((2-hydroxyethyl)sulfonamido)-2-(6-azaspiro[2.5]octan-6-yl)benzamide | 550.2 |
| 7-27 | 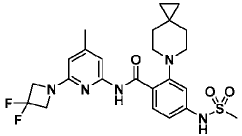 | *N*-(6-(3,3-Difluoroazetidin-1-yl)-4-methylpyridin-2-yl)-4-(methylsulfonamido)-2-(6-azaspiro[2.5]octan-6-yl)benzamide | 506.2 |
| 7-28 | 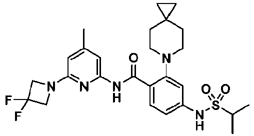 | *N*-(6-(3,3-Difluoroazetidin-1-yl)-4-methylpyridin-2-yl)-4-((1-methylethyl)sulfonamido)-2-(6-azaspiro[2.5]octan-6-yl)benzamide | 534.2 |
| 7-29 | 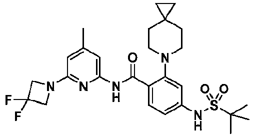 | *N*-(6-(3,3-Difluoroazetidin-1-yl)-4-methylpyridin-2-yl)-4-((1,1-dimethylethyl)sulfonamido)-2-(6-azaspiro[2.5]octan-6-yl)benzamide | 548.2 |
| 7-30 | 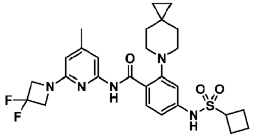 | 4-(Cyclobutanesulfonamido)-*N-*(6-(3,3-difluoroazetidin-1-yl)-4-methylpyridin-2-yl)-2-(6-azaspiro[2.5]octan-6-yl)benzamide | 546.2 |
| 7-31 | 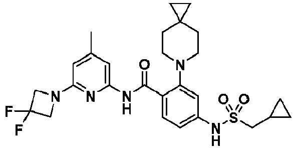 | 4-((Cyclopropylmethyl)sulfonamido)-*N*-(6-(3,3-difluoroazetidin-1-yl)-4-methylpyridin-2-yl)-2-(6-azaspiro[2.5]octan-6-yl)benzamide | 546.2 |
| 7-32 | 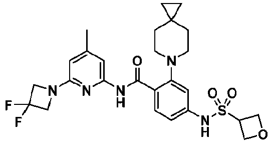 | *N*-(6-(3,3-Difluoroazetidin-1-yl)-4-methylpyridin-2-yl)-4-(oxetane-3-sulfonamido)-2-(6-azaspiro[2.5]octan-6-yl)benzamide | 548.2 |
| 7-33 | 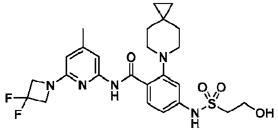 | *N*-(6-(3,3-Difluoroazetidin-1-yl)-4-methylpyridin-2-yl)-4-((2-hydroxyethyl)sulfonamido)-2-(6-azaspiro[2.5]octan-6-yl)benzamide | 522.1 |
| 7-34 | 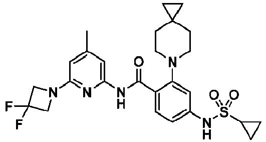 | 4-(Cyclopropanesulfonamido)-*N*-(6-(3,3-difluoroazetidin-1-yl)-4-methylpyridin-2-yl)-2-(6-azaspiro[2.5]octan-6-yl)benzamide | 532.2 |
| 7-35 | 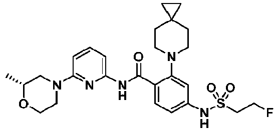 | (*R*)-4-((2-Fluoroethyl)sulfonamido)-*N*-(6-(2-methylmorpholino)pyridin-2-yl)-2-(6-azaspiro[2.5]octan-6-yl)benzamide | 532.3 |
| 7-36 | 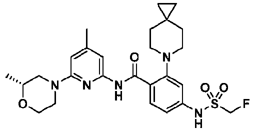 | (*R*)-4-((Fluoromethyl)sulfonamido)-*N*-(4-methyl-6-(2-methylmorpholino)pyridin-2-yl)-2-(6-azaspiro[2.5]octan-6-yl)benzamide | 532.3 |
| 7-37 | 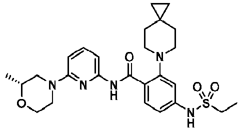 | (*R*)-4-(Ethylsulfonamido)-*N-*(6-(2-methylmorpholino)pyridin-2-yl)-2-(6-azaspiro[2.5]octan-6-yl)benzamide | 514.3 |
| 7-38 | 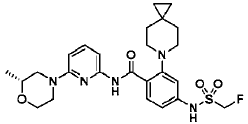 | (*R*)-4-((Fluoromethyl)sulfonamido)-N-(6-(2-methylmorpholino)pyridin-2-yl)-2-(6-azaspiro[2.5]octan-6-yl)benzamide | 518.2 |
| 7-39 | 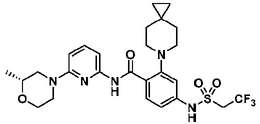 | (*R*)-*N*-(6-(2-methylmorpholino)pyridin-2-yl)-2-(6-azaspiro[2.5]octan-6-yl)-4-((2,2,2-trifluoroethyl)sulfonamido)benzamide | 568.3 |
| 7-40 | 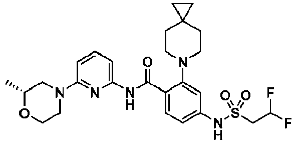 | (*R*)-4-((2,2-Difluoroethyl)sulfonamido)-*N*-(6-(2-methylmorpholino)pyridin-2-yl)-2-(6-azaspiro[2.5]octan-6-yl)benzamide | 551.2 |
| 7-41 | 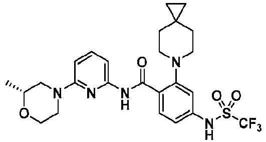 | (*R*)-*N*-(6-(2-Methylmorpholino)pyridin-2-yl)-2-(6-azaspiro[2.5]octan-6-yl)-4-((trifluoromethyl)sulfonamido)benzamide | 554.2 |
| 7-42 | 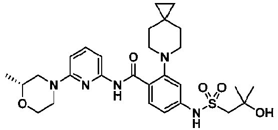 | (*R*)-4-((2-Hydroxy-2-methylpropyl)sulfonamido)-*N*-(6-(2-methylmorpholino)pyridin-2-yl)-2-(6-azaspiro[2.5]octan-6-yl)benzamide | 558.3 |
| 7-43 | 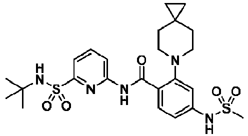 | *N*-(6-(*N*-(*tert*-Butyl)sulfamoyl)pyridin-2-yl)-4-(methylsulfonamido)-2-(6-azaspiro[2.5]octan-6-yl)benzamide | 536.2 |
| 7-44 | 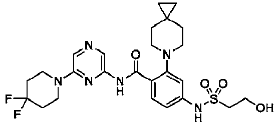 | *N*-(6-(4,4-Difluoropiperidin-1-yl)pyrazin-2-yl)-4-((2-hydroxyethyl)sulfonamido)-2-(6-azaspiro[2.5]octan-6-yl)benzamide | 551.2 |

### Example 8-1: (R)-4-((2-Hydroxy-1-methylethyl)sulfonamido)-2-(6-azaspiro[2.5]octan-6-yl)-N-(6-(3,3,3-trifluoropropoxy)pyridin-2-yl)benzamide and Example 8-2: (S)-4-((2-hydroxy-1-methylethyl)sulfonamido)-2-(6-azaspiro[2.5]octan-6-yl)-N-(6-(3,3,3-trifluoropropoxy)pyridin-2-yl)benzamide

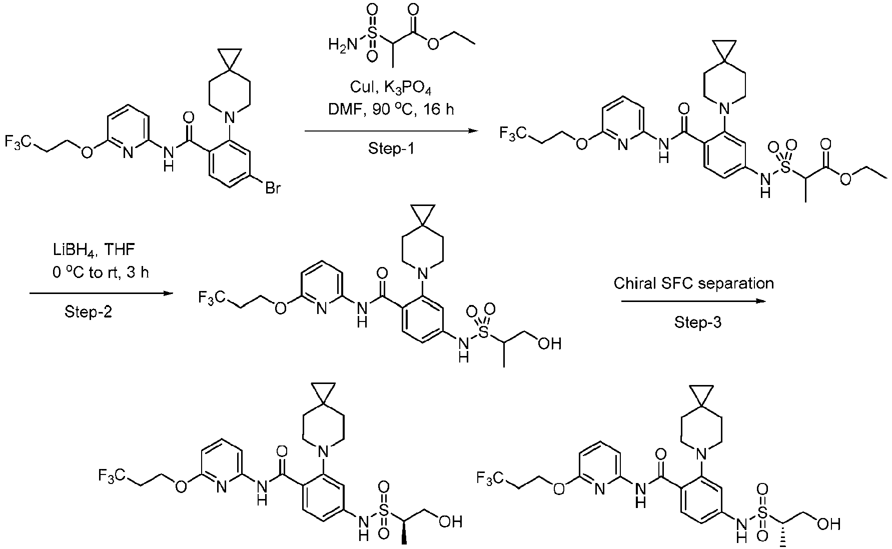

Step 1: A mixture of 4-bromo-2-(6-azaspiro[2.5]octan-6-yl)-*N*-(6-(3,3,3-trifluoropropoxy)pyridin-2-yl)benzamide (0.63g, 1.3 mmol, Intermediate 38-1), ethyl 2-sulfamoylpropanoate (0.275 g, 1.52 mmol, Intermediate 40), potassium phosphate tribasic (0.537 g, 2.53 mmol), (1*R*,2*R*)-*N1*,*N2*-dimethylcyclohexane-1,2-diamine (0.090 g, 0.63 mmol) and copper iodide (0.241 g, 1.26 mmol) in DMF (10 mL) was heated at 90 °C for 16 h. Then the reaction mixture was filtered, diluted with EtOAc, and washed with water, brine, dried over Na₂SO₄, filtered, and concentrated. The concentrate was purified by flash column chromatography eluting with a gradient of 0 % to 50 % EtOAc in petroleum ether to provide ethyl 2-(*N*(3-(6-azaspiro[2.5]octan-6-yl)-4-((6-(3,3,3-trifluoropropoxy)pyridin-2-yl)carbamoyl)phenyl)sulfamoyl)propanoate (0.56 g, 0.94 mmol, 74% yield) as white solid. ¹H NMR (400 MHz, DMSO-*d*₆) δ 13.19 (s, 1H), 10.68 (s, 1H), 8.09 (d, *J* = 8 7 Hz, 1H), 7.91 (d, *J*=7.9 Hz, 1H), 7.77 (t, *J*=7.9 Hz, 1 H), 7.32 (d, *J*=2.2 Hz, 1 H), 7.16 (dd, *J*=8.6, 2.2 Hz, 1 H), 6.58 (d, *J*=8.0 Hz, 1 H), 4.51 (t, *J*=6.1 Hz, 2 H), 4.32 (q, *J*=6.9 Hz, 1 H), 4.07 (qd, *J*=7.1, 5.2 Hz, 2 H), 3.00 (d, *J*=5.0 Hz, 4 H), 2.84 (qt, *J*=11.5, 6.0 Hz, 2 H), 1.74 (s, 4 H), 1.49 (d, *J*=6.9 Hz, 3 H), 1.14 (t, *J*=7.1 Hz, 3 H), 0.39 (s, 4 H). *m*/*z* (ESI): 598.8 (M+H)⁺.

Step-2: To a solution of ethyl 2-(*N*-(3-(6-azaspiro[2.5]octan-6-yl)-4-((6-(3,3,3-trifluoropropoxy)pyridin-2-yl)carbamoyl)phenyl)sulfamoyl)propanoate (0.56 g, 0.94 mmol) in THF(12 mL) was added LiBH₄ (1.871 mL, 3.74 mmol) at -78 °C. Then the reaction mixture was slowly warmed up to room temperature over 2 h before it was quenched with a saturated solution of NH₄Cl (20 mL) and was extracted with EtOAc (2 × 25 mL). The combined organic extracts were washed with brine, dried over Na₂SO₄, filtered, and concentrated. The concentrate was purified by flash column chromatography eluting with a gradient of 0 % to 50 % EtOAc in petroleum ether to provide 4-((2-hydroxy-1-methylethyl)sulfonamido)-2-(6-azaspiro[2.5]octan-6-yl)-*N*-(6-(3,3,3-trifluoropropoxy)pyridin-2-yl)benzamide (0.45 g, 0.81 mmol, 86 % yield) as white solid. *m*/*z* (ESI): 557.2 (M+H)⁺. The racemic mixture was subject to chiral separation by SFC, using a column- Lux A1 (250 × 21.2mm, 5µm), mobile phase: 80:20 (A: B), A = liquid CO₂, B = Methanol, flow rate: 80 mL/min to yield.

Example 8-1: (*R*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide. First eluting peak. ¹H NMR (400 MHz, DMSO-*d₆*) δ ppm 13.18 (s, 1 H), 1022 (s, 1 H), 8.07 (d, *J*=8.6 Hz, 1 H), 7.90 (d, *J*=7.8 Hz, 1 H), 7.76 (t, *J*=8.0 Hz, 1 H), 7.31 (d, *J=2.2* Hz, 1 H), 7.16 (dd, *J* = 8.6, 2.1 Hz, 1 H), 6.57 (dd, *J* = 8.1, 0.8 Hz, 1 H), 5.03 (s, 1 H), 4.51 (t, *J*=6.1 Hz, 2 H), 3.85 (dd, *J*=11.1, 4.4 Hz, 1 H), 3.49 (dd, *J*=11.1, 7.6 Hz, 1 H), 3.30 (m, 1 H), 2.87 - 3.24 (m, 4 H), 2.83 (tt, *J*=11.5, 6.0 Hz, 2 H), 1.74 (br s, 4 H), 1.30 (d, *J*=6.9 Hz, 3 H), 0.39 (s, 4 H). *m*/*z* (ESI): 557.1 (M+H)⁺.

Example 8-2: (*S*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide. Second eluting peak ¹H NMR (400 MHz, DMSO-*d*₆) δ ppm 13.18 (s, 1 H), 10.22 (s, 1 H), 8.07 (d, *J*=8.6 Hz, 1 H), 7.78 - 8.03 (m, 1 H), 7.75 (d, *J*=7.9 Hz, 1 H), 7.31 (d, *J*=2.2 Hz, 1 H), 7.16 (dd, *J*=8.7, 2.2 Hz, 1 H), 6.57 (d, *J*=8.0 Hz, 1 H), 5.03 (s, 1 H), 4.51 (t, *J*=6.0 Hz, 2 H), 3.85 (dd, *J*=11.1, 4.4 Hz, 1 H), 3.48 - 3.57 (m, 2 H), 3.30 (td, *J*=6.9, 4.3 Hz, 1 H), 2.99 (t, *J*=5.3 Hz, 4 H), 2.82 (tt, *J*=11.4, 5.9 Hz, 2 H), 1.74 (br s, 4 H), 1.29 (d, *J*=6.9 Hz, 3 H), 0.39 (s, 4 H). *m*/*z* (ESI): 557.1 (M+H)⁺.

The stereochemistry was arbitrary assigned.

**Table 11: Examples 9-1 to 9-18 were prepared following a similar procedure as described for Examples 8-1 and 8-2**

| Ex. # | Chemical Structure | Name | LRMS: (ESI + ve ion) *m*/*z* |
|---|---|---|---|
| 9-1 | 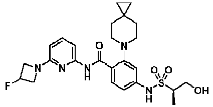 | (*R*)-*N*-(6-(3-Fluoroazetidin-1-yl)pyridin-2-yl)-4-((2-hydroxy-1-methylethyl)sulfonamido)-2-(6-azaspiro[2.5]octan-6-yl)benzamide | 518.2 |
| 9-2 | 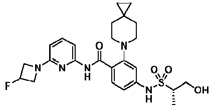 | (*S*)-*N*-(6-(3-Fluoroazetidin-1-yl)pyridin-2-yl)-4-((2-hydroxy-1-methylethyl)sulfonamido)-2-(6-azaspiro[2.5]octan-6-yl)benzamide | 518.2 |
| 9-3 | 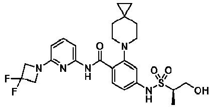 | (*R*)-*N*-(6-(3,3-Difluoroazetidin-1-yl)pyridin-2-yl)-4-((2-hydroxy-1-methylethyl)sulfonamido)-2-(6-azaspiro[2.5]octan-6-yl)benzamide | 536.2 |
| 9-4 | 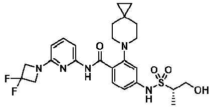 | (S)-*N*-(6-(3,3-Difluoroazefidin-1-yl)pyridin-2-yl)-4-((2-hydroxy-1-methylethyl)sulfonamido)-2-(6-azaspiro[2.5]octan-6-yl)benzamide | 536.2 |
| 9-5 | 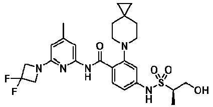 | (*R*)-*N*-(6-(3,3-Difluoroazetidin-1-yl)-4-methylpyridin-2-yl)-4-((2-hydroxy-1-methylethyl)sulfonamido)-2-(6-azaspiro[2.5]octan-6-yl)benzamide | 550.2 |
| 9-6 | 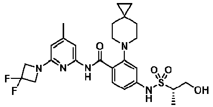 | (*S*)-*N*-(6-(3,3-Difluoroazetidin-1-yl)-4-methylpyridin-2-yl)-4-((2-hydroxy-1-methylethyl)sulfonamido)-2-(6-azaspiro[2.5]octan-6-yl)benzamide | 550.2 |
| 9-7 | 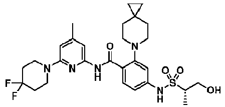 | (*S*)-*N*-(6-(4,4-Difluoropiperidin-1-yl)-4-methylpyridin-2-yl)-4-((2-hydroxy-1-methylethyl)sulfonamido)-2-(6-azaspiro[2.5]octan-6-yl)benzamide | 578.2 |
| 9-8 | 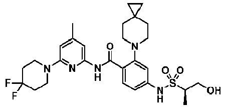 | (*R*)-*N*-(6-(4,4-Difluoropiperidin-1-yl)-4-methylpyridin-2-yl)-4-((2-hydroxy-1-methylethyl)sulfonamido)-2-(6-azaspiro[2.5]octan-6-yl)benzamide | 578.2 |
| 9-9 | 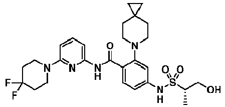 | (*S*)-*N*-(6-(4,4-Difluoropiperidin-1-yl)pyridin-2-yl)-4-((2-hydroxy-1-methylethyl)sulfonamido)-2-(6-azaspiro[2.5]octan-6-yl)benzamide | 564.2 |
| 9-10 | 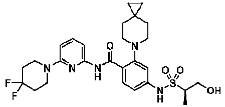 | (*R*)-*N*-(6-(4,4-Difluoropiperidin-1-yl)pyridin-2-yl)-4-((2-hydroxy-1-methylethyl)sulfonamido)-2-(6-azaspiro[2.5]octan-6-yl)benzamide | 564.2 |
| 9-11 | 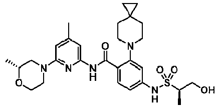 | 4-(((*R*)-2-Hydroxy-1-methylethyl)sulfonamido)-*N* (4-methyl-6-((*R*)-2-methylmorpholino)pyridin-2-yl)-2-(6-azaspiro[2.5]octan-6-yl)benzamide | 558.2 |
| 9-12 | 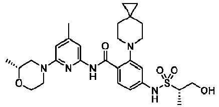 | 4-(((*S*)-2-Hydroxy-1-methylethyl)sulfonamido)-*N-*(4-methyl-6-((*R*)-2-methylmorpholino)pyridin-2-yl)-2-(6-azaspiro[2.5]octan-6-yl)benzamide | 558.2 |
| 9-13 | 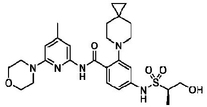 | (*R*)-4-((2-Hydroxy-1-methylethyl)sulfonamido)-N-(4-methyl-6-morpholinopyridin-2-yl)-2-(6-azaspiro[2.5]octan-6-yl)benzamide | 544.2 |
| 9-14 | 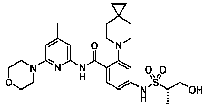 | (*S*)-4-((2-Hydroxy-1-methylethyl)sulfonamido)-N-(4-methyl-6-morpholinopyridin-2-yl)-2-(6-azaspiro[2.5]octan-6-yl)benzamide | 544.2 |
| 9-15 | 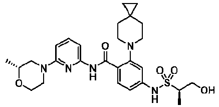 | 4-(((*R*)-2-Hydroxy-1-methylethyl)sulfonamido)-*N-*(6-((*R*)-2-methylmorpholino)pyridin-2-yl)-2-(6-azaspiro[2.5]octan-6-yl)benzamide | 544.2 |
| 9-16 | 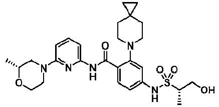 | 4-(((*S*)-2-Hydroxy-1-methylethyl)sulfonamido)-*N-*(6-((*R*)-2-methylmorpholino)pyridin-2-yl)-2-(6-azaspiro[2.5]octan-6-yl)benzamide | 544.2 |
| 9-17 | 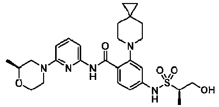 | 4-(((*R*)-2-Hydroxy-1-methylethyl)sulfonamido)-*N-*(6-((*S*)-2-methylmorpholino)pyridin-2-yl)-2-(6-azaspiro[2.5]octan-6-yl)benzamide | 544.2 |
| 9-18 | 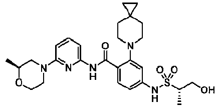 | 4-(((*S*)-2-Hydroxy-1-methylethyl)sulfonamido)-*N-*(6-((*S*)-2-methylmorpholino)pyridin-2-yl)-2-(6-azaspiro[2.5]octan-6-yl)benzamide | 544.2 |

### Example 10: N-(6-(4,4-Difluoropiperidin-1-yl)-4-methylpyridin-2-yl)-4-((1-(hydroxymethyl)cyclopropane)-1-sulfonatnido)-2-(6-azaspiro[2.5]octan-6-yl)benzamide

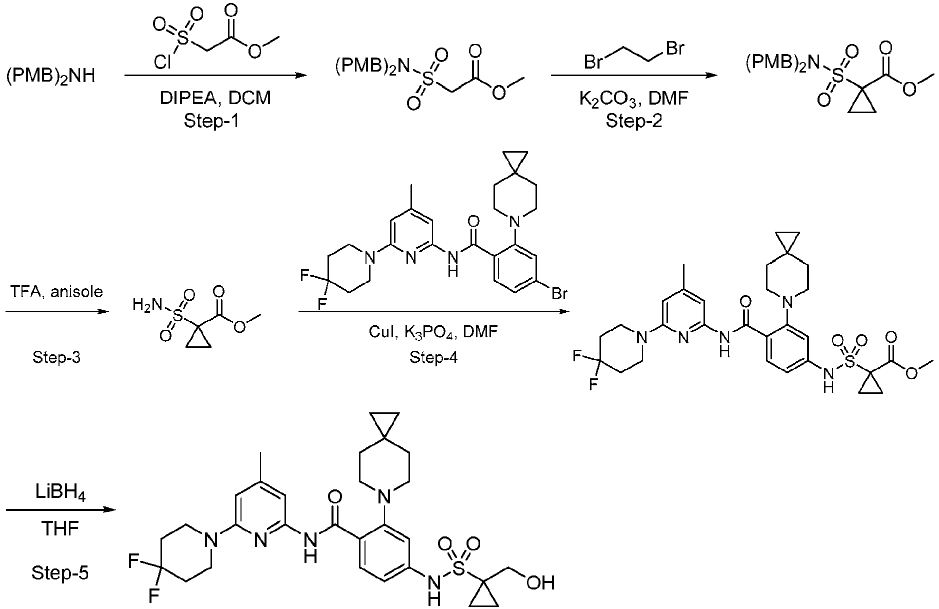

Step 1: To a solution of bis(4-methoxybenzyl)amine (8.95 g, 34.8 mmol) in DCM (50 mL) were added DIPEA (6.06 mL, 43.5 mmol) and methyl 2-(chlorosulfonyl)acetate (5.0 g, 29 mmol) at 0 °C. The reaction mixture was stirred at room temperature for 16 h before it was quenched with ice water and extracted with DCM. The organic layer was washed with a saturated aqueous solution of sodium bicarbonate, water, and brine. The organic extract was dried over Na₂SO₄, filtered, and concentrated. The concentrate was purified by flash column chromatography using a gradient of 15% ethyl acetate in petroleum ether to provide methyl 2-(*N*,*N-*bis(4-methoxybenzyl)sulfamoyl)acetate (5.5 g, 14 mmol, 48% yield) as pale yellow gum. ¹H NMR (400 MHz, DMSO-*d*₆): δ ppm 7.14 (d, *J*=8.4 Hz, 4 H), 6.86 (d, *J*=8.4 Hz, 4 H), 4.31 (s, 2 H), 4.25 (s, 4 H), 3.73 (s, 6 H), 3.70 (s, 3 H). *m*/*z* (ESI): 392.1 (M-H)⁻.

Step 2: A mixture of methyl 2-(*N,N* bis(4-methoxybenzyl)sulfamoyl)acetate (1.0 g, 2.5 mmol), K₂CO₃ (1.054 g, 7.62 mmol) and 1,2-dibromoethane (0.716 g, 3.81 mmol) in DMF (5 mL) was stirred at 65 °C for 16 h. The reaction mixture was quenched with ice water and was extracted with EtOAc. The organic extract was washed with water and brine, dried over Na₂SO₄, filtered, and concentrated. The concentrate was purified by flash column chromatography using a gradient of 5% ethyl acetate in petroleum ether to provide methyl 1-(*N*,*N-*bis(4-methoxybenzyl)sulfamoyl)cyclopropane-1-carboxylate (750 mg, 1.79 mmol, 70 % yield) as pale yellow gum. ¹H NMR (300 MHz, DMSO-d6): δ ppm 7.04 - 7.21 (m, 4 H), 6.83 - 6.94 (m, 4 H), 4.32 (s, 4 H), 3.72 (s, 6 H), 3.69 (s, 3 H), 1.67 (q, *J*=4.8, 3.6 Hz, 2 H), 1.56 (q, *J*=5.7, 4.8 Hz, 2 H).

Step 3: A mixture of methyl 1-(*N*,*N-*bis(4-methoxybenzyl)sulfamoyl)cyclopropane-1-carboxylate (2.33 g, 5.55 mmol), TFA (23 mL) and anisole (3.03 mL, 27.8 mmol) was stirred at room temperature for 16 h. The reaction mixture was concentrated, and the concentrate was treated with an aqueous saturated solution of sodium bicarbonate solution and was extracted with dichloromethane. The organic extract was washed with water and brine, dried over Na₂SO₄, filtered, and concentrated. The concentrate was purified by flash column chromatography using a gradient of 50% ethyl acetate in petroleum ether to provide methyl 1- sulfamoylcyclopropane-1-carboxylate (0.59 g, 3.3 mmol, 59% yield) as white solid. ¹H NMR (400 MHz, DMSO-*d*₆) δ ppm 7.08 (br s, 2 H), 3.70 (s, 3 H), 1.48 - 1.52 (m, 4 H). *m*/*z* (ESI): 180.0 (M+H)⁺.

Step 4: A mixture of 4-bromo-*N*-(2-(4,4-difluoropiperidin-1-yl)-6-methylpyridin-4-yl)-2-(6-azaspiro[2.5]octan-6-yl)benzamide (0.20 g, 0.38 mmol, Intermediate 27), methyl 1-sulfamoylcyclopropane-1-carboxylate (0.103 g, 0.578 mmol), copper (I) iodide (0.073 g, 0.38mmol), potassium phosphate tribasic (0.163 g, 0.770 mmol), (*1R*,*2R*)-*N1*,*N2*-dimethylcyclohexane-1,2-diamine (0.027 g, 0.19 mmol) in DMF (3 mL) was heated at 90 °C for 16 h. Then the reaction was filtered through a plug of celite and the filtrate was diluted with EtOAc and was washed with water and brine, dried over Na₂SO₄, filtered, and concentrated. The concentrate was purified by flash column chromatography using a gradient of 28% to 35 % EtOAc in petroleum ether to provide methyl 1-(*N-*(4-((6-(4,4-difluoropiperidin-1-yl)-4-methylpyridin-2-yl)carbamoyl)-3-(6-azaspiro[2.5]octan-6-yl)phenyl)sulfamoyl)cyclopropane-1-carboxylate (0.15 g, 0.24 mmol, 63.1 % yield) as white solid. *m*/*z* (ESI): 618.2 (M+H)⁺.

Step 5: To a solution of methyl 1-(*N*-(4-((6-(4,4-difluoropiperidin-1-yl)-4-methylpyridin-2-yl)carbamoyl)-3-(6-azaspiro[2.5]octan-6-yl)phenyl)sulfamoyl)cyclopropane-1-carboxylate (0.150 g, 0.243 mmol) in THF (8 mL) was added lithium borohydride (0.486 mL, 0.971 mmol) at -78 °C. The reaction mixture was slowly warmed up to room temperature over 2 h before it was quenched with an aqueous saturated solution of ammonium chloride and was extracted with ethyl acetate. The organic layer washed with water and brine, dried over Na₂SO₄, filtered, and concentrated. The concentrate was purified by flash column chromatography using a gradient of 40 % to 45% EtOAc in petroleum ether to afford *N-*(6-(4,4-diffuoropiperidin-1-yl)-4-methylpyridin-2-yl)-4-((1-(hydroxymethyl)cyclopropane)-1-sulfonamido)-2-(6-azaspiro[2.5]octan-6-yl)benzamide (0.090 g, 0.15mmol, 63% yield) as white solid. ¹H NMR (400 MHz, DMSO-*d*₆): δ ppm 12.89 (s, 1 H), 10.13 (s, 1 H), 8.04 (d, *J*=8.6 Hz, 1 H), 7.51 (s, 1 H), 7.32 (d, *J*=2.2 Hz, 1 H), 7.13 (dd, *J*=8.6, 2.1 Hz, 1 H), 6.57 (s, 1 H), 4.98 (t, *J*=6.2 Hz, 1 H), 3.55 - 3.86 (m, 6 H), 2.96 (s, 4 H), 2.26 (s, 3 H), 1.98 (q, *J*=8.7, 7.5 Hz, 4 H), 1.65 - 1.75 (m, 4 H), 1.06 - 1.19 (m, 2 H), 0.93 - 1.06 (m, 2 H), 0.39 (s, 4 H). *m*/*z* (ESI): 590.2 (M+H)⁺.

**Table 12: Examples 10-1 to 10-2 were prepared following a similar procedure as described for Example 10**

| Ex. # | Chemical Structure | Name | LRMS: (ESI + ve ion) *m*/*z* |
|---|---|---|---|
| 10-1 | 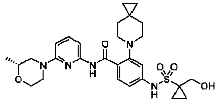 | (*R*)-4-((1-(Hydroxymethyl)cyclopropane)-1-sulfonamido)-*N*-(6-(2-methylmorpholino)pyridin-2-yl)-2-(6-azaspiro[2.5]octan-6-yl)benzamide | 556.2 |
| 10-2 | 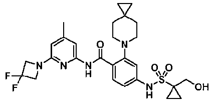 | *N*-(6-(3,3-Difluoroazetidin-1-yl)-4-methylpyridin-2-yl)-4-((1-(hydroxymethyl)cyclopropane)-1-sulfonamido)-2-(6-azaspiro[2.5]octan-6-yl)benzamide | 562.2 |

### Method E (Cu-catalyzed Sulfonamide Formation followed by reduction)

### Example 11: N-(6-(3,3-difluoroazetidin-1-yl)-4-methylpvridin-2-yl)-4-((2-hydroxyethyl)sulfonamido)-2-(6-azaspiro[2.5]octan-6-yl)benzamide

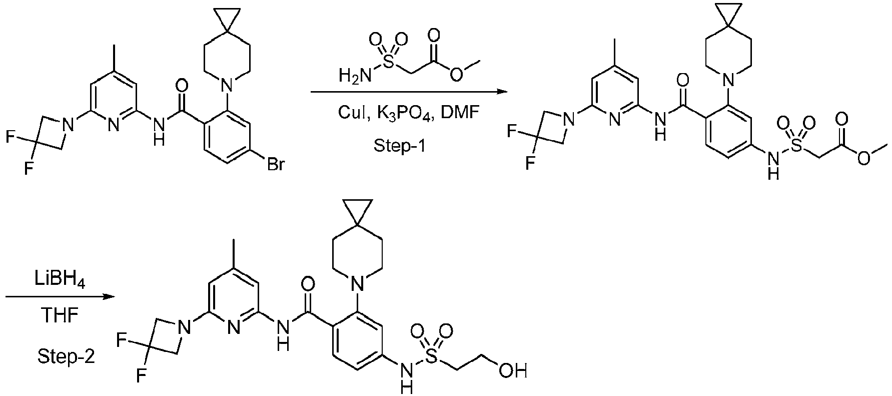

Step 1: A mixture of 4-bromo-*N-*(6-(3,3-difluoroazetidin-1-yl)-4-methylpyridin-2-yl)-2-(6-azaspiro[2.5]octan-6-yl)benzamide (0.17 g, 0.35 mmol, Intermediate 39), methyl 2-sulfamoylacetate (0.079 g, 0.52 mmol), copper (I) iodide (0.066 g, 0.35 mmol), potassium phosphate tribasic (0.147 g, 0.692 mmol), (*1R*,*2R*)-*N1*,*N2*-dimethylcyclohexane-1,2-diamine (0.025 g, 0.17 mmol) in DMF (4 mL) was heated at 90 °C for 16 h. Then the reaction mixture was filtered through a plug of celite. The filtrate was diluted with EtOAc and washed with water and brine, dried over Na₂SO₄, filtered, and concentrated. The concentrate was purified by flash column chromatography using a gradient of 0 % - 30 % EtOAc in petroleum ether to provide methyl 2-(N (4-((6-(3,3-difluoroazetidin-1-yl)-4-methylpyridin-2-yl)carbamoyl)-3-(6-azaspiro[2.5]octan-6-yl)phenyl)sulfamoyl)acetate (0.16 g, 0.28 mmol, 82 % yield) as semi solid. ¹H NMR (400 MHz, DMSO-d6): δ ppm 13.12 (br s, 1 H), 10.65 (s, 1 H), 8.07 (dd, *J*=8.6, 1.8 Hz, 1 H), 7.57 (s, 1 H), 7.28 (t, *J*=2.0 Hz, 1 H), 7.15 (dt, *J*=8.6, 2.0 Hz, 1 H), 6.19 (s, 1 H), 4.32 - 4.46 (m, 6 H), 3.64 (s, 3 H), 2.98 (t, *J*=5.0 Hz, 4 H), 2.28 (s, 3 H), 1.76 (br s, 4 H), 0.39 (s, 4 H). *m*/*z* (ESI): 564.2 (M+H)⁺.

Step 2: To a solution of methyl 2-(*N*-(4-((6-(3,3-difluoroazetidin-1-yl)-4-methylpyridin-2-yl)carbamoyl)-3-(6-azaspiro[2.5]octan-6-yl)phenyl)sulfamoyl)acetate (0.16 g, 0.28 mmol) in THF (3.2 mL) was added a 2M solution of lithium borohydride in THF (0.284 mL, 0.568 mmol) dropwise at 0 °C. The reaction mixture was slowly warmed to room temperature over 1 h before it was quenched with an aqueous saturated solution of ammonium chloride and was extracted with ethyl acetate. The organic layer washed with water and brine, dried over Na₂SO₄, filtered, and concentrated. The concentrate was purified by reverse phase column chromatography using a gradient of 0 - 60 % acetonitrile in water to afford *N-*(6-(3,3-difluoroazetidin-1-yl)-4- methylpyridin-2-yl)-4-((2-hydroxyethyl)sulfonamido)-2-(6-azaspiro[2.5]octan-6-yl)benzamide (0.095 g, 0.18 mmol, 62% yield) as white solid. ¹H NMR (400 MHz, DMSO-d₆): δ ppm 13.08 (s, 1 H), 10.20 (s, 1 H), 8.05 (d, *J*=8.6 Hz, 1 H), 7.56 (s, 1 H), 7.26 (d, *J*=2.2 Hz, 1 H), 7.12 (dd, *J*=8.6, 2.2 Hz, 1 H), 6.18 (s, 1 H), 4.93 (br s, 1 H), 4.36 (t, *J*=12.4 Hz, 4 H), 3.75 (q, *J*=6.0 Hz, 2 H), 3.35 (t, *J*=6.5 Hz, 2 H), 2.96 (t, *J*=5.3 Hz, 4 H), 2.27 (s, 3 H), 1.74 (br s, 4 H), 0.38 (s, 4 H). *m*/*z* (ESI): 564.2 (M+H)⁺.

### Examples 12-1 and 12-2: 2-(6-Azaspiro[2.5]octan-6-yl)-4-(S-cyclopropylsulfonimidoyl)-N-(6-((2R)-2-methyl-4-morpholinyl)-2-pyridinyl)benzamide and 2-(6-azaspiro[2.5]octan-6-yl)-4-(R-cyclopropylsulfonimidoyl)-N-(6-((2R)-2-methyl-4-morpholinyl)-2-pyridinyl)benzamide

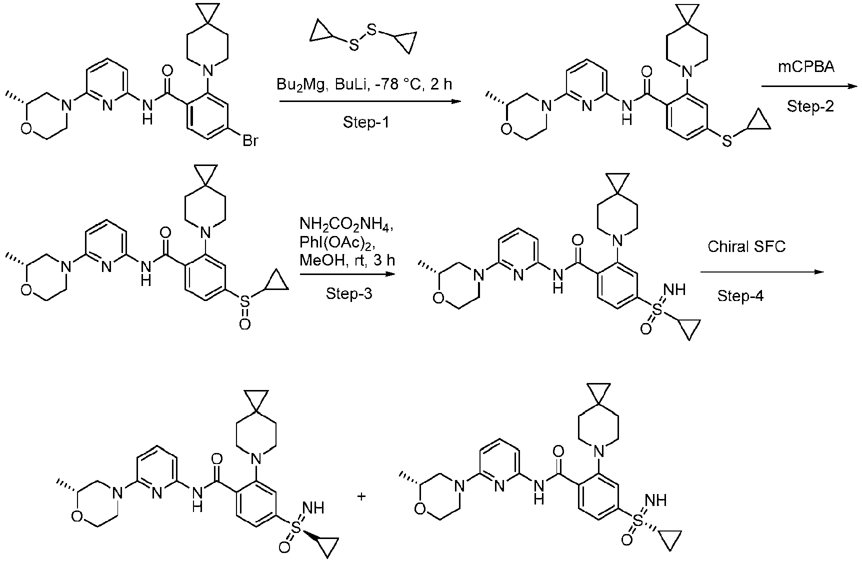

Step 1: To a solution of (*R*)-4-bromo-*N*-(6-(2-methylmorpholino)pyridin-2-yl)-2-(6-azaspiro[2.5]octan-6-yl)benzamide (1.0 g, 2.1 mmol, Intermediate 30) in THF (20 mL) was added dibutyl magnesium (1.0 M solution in THF, 1.24 mL, 1.24 mmol) dropwise at 0 °C and stirred for 10 min. The reaction mixture was cooled to -78 °C and was added n-butyl lithium (2.5 M in solution in hexanes, 0.989 mL, 2.47 mmol) and stirred for 10 min. 1,2-dicyclopropyldisulfane (0.301 g, 2.06 mmol, Wuxi AppTec) was added dropwise and stirred at room temperature for 2 h. The reaction mixture was quenched with 2N HCl (20 mL) and extracted with ethyl acetate (2 × 20 mL). The combined organic extracts were washed with brine (20 mL), dried over sodium sulfate, filtered, and concentrated under reduced pressure. The crude residue was purified by flash chromatography through a Redi-Sep pre-packed silica gel column (40 g), eluting with a gradient of 30% ethyl acetate in hexanes to give (*R*)-4-(cyclopropylthio)-*N*-(6-(2-methylmorpholino)pyridin-2-yl)-2-(6-azaspiro[2.5]octan-6-yl)benzamide (0.90 g, 91% yield) as a pale yellow solid.¹H NMR (400 MHz, DMSO-*d*₆): *δ* 12.91 (s, 1H), 8.06 (d, *J* = 8.4 Hz, 1H), 7.70 - 7.59 (m, 2H), 7.40 - 7.26 (m, 2H), 6.61 - 6.58 (m, 1H), 4.11 (dd, *J* = 25.9, 12.9 Hz, 2H), 3.93 (dd, *J* = 11.5, 3.2 Hz, 1H), 3.62 - 3.55 (m, 2H), 3.03 (t, *J=* 5.5 Hz, 4H), 2.83 (td, *J=* 12.2, 3.4 Hz, 1H), 2.38 (dq, *J* = 7.3, 3.8, 3.2 Hz, 1H), 1.72 (s, 4H), 1.20 - 1.16 (m, 6H), 0.66 - 0.62 (m, 2H), 0.38 (d, *J* = 1.6 Hz, 4H). *m*/*z* (ESI): 479.2 (M+H)⁺.

Step 2: To a solution of (*R*)-4-(cyclopropylthio)-N-(6-(2-methylmorpholino)pyridin-2-yl)-2-(6-azaspiro[2.5]octan-6-yl)benzamide (0.90 g, 1.9 mmol) in dichloromethane (10 mL) was added m-CPBA (43.3 mg, 0.188 mmol) at 0 °C and stirred at same temperature for 3 h. The reaction mixture was quenched with 10% aqueous sodium bicarbonate solution (15 mL) and extracted with DCM (2 × 20 mL). The combined organic extracts were washed with brine solution (20 mL), dried over sodium sulphate, filtered, and concentrated under reduced pressure. The crude residue was purified by flash chromatography through a Redi-Sep pre-packed silica gel column (40 g), eluting with a gradient of 1% to 50% ethyl acetate in hexanes to give 4-(cyclopropylsulfinyl)-*N*-(6-((*R*)-2-methylmorpholino)pyridin-2-yl)-2-(6-azaspiro[2.5]octan-6-yl)benzamide (650 mg, 70% yield) as a pale yellow solid. ¹H NMR (400 MHz, Chloroform-*d*): *δ* 12.89 (s, 1H), 8.23 (d, *J* = 8.3 Hz, 1H), 7.81 (dd, *J* = 7.9, 4.6 Hz, 1H), 7.57 (td, *J* = 8.0, 3.3 Hz, 1H), 7.30 (dd, *J* = 8.3, 1.8 Hz, 1H), 7.25 (d, *J* = 1.8 Hz, 1H), 6.42 (dd, *J* = 8.2, 3.7 Hz, 1H), 4.18 - 3.95 (m, 3H), 3.81 - 3.65 (m, 2H), 3.09 (q, *J =* 6.3, 5.6 Hz, 4H), 2.97 (tt, *J* = 12.8, 11.7, 2.7 Hz, 1H), 2.63 (ddd, *J* = 12.7, 10.4, 1.9 Hz, 1H), 2.23 (tt, *J =* 7.3, 4.4 Hz, 1H), 1.31 - 1.27 (m, 4H), 1.19 - 1.15 (m, 5H), 0.77 - 0.73 (m, 2H), 0.40 (d, *J* = 1.7 Hz, 4H). *m*/*z* (ESI): 495.2 (M+H)⁺.

Step 3: To a solution of 4-(cyclopropylsulfmyl)-A-(6-((R)-2-methylmorpholino)pyridin-2-yl)-2-(6-azaspiro[2.5]octan-6-yl)benzamide ( 0.65 g, 1.30 mmol) in MeOH (20 mL) were added iodobenzene diacetate (1.27 g, 3.94 mmol) and ammonium carbamate (410 mg, 5.26 mmol) at 0 °C and for 10 min and warmed to room temperature and stirred for 12 h. The reaction mixture was quenched with water (10 mL) and extracted with ethyl acetate (2 × 10 mL). The combined organic extracts were washed with brine solution (10 mL), dried over sodium sulfate, filtered, and concentrated under reduced pressure. The crude residue was purified by flash chromatography through a Redi-Sep pre-packed silica gel column (40 g), eluting with a gradient of 40% ethyl acetate in hexanes to give 4-(cyclopropanesulfonimidoyl)-*N*-(6-((*R*)-2-methylmorpholino)pyridin-2-yl)-2-(6-azaspiro[2.5]octan-6-yl)benzamide (150 mg, racemic) as a pale yellow solid. ¹H NMR (400 MHz, Chloroform-*d*): *δ* 12.67 (s, 1H), 8.45 (dd, *J =* 8.3, 1.2 Hz, 1H), 7.93 (t*, J* = 1.5 Hz, 1H), 7.84 (dt, *J* = 8.3, 1.5 Hz, 1H), 7.82 - 7.72 (m, 1H), 7.66 - 7.56 (m, 1H), 6.46 (d, *J* = 8.2 Hz, 1H), 4.22 - 3.92 (m, 3H), 3.92 - 3.62 (m, 2H), 3.33 - 3.11 (m, 4H), 2.99 (td, *J* = 12.2, 3.5 Hz, 1H), 2.77 - 2.55 (m, 2H), 2.01 -1.69 (m, 4H), 1.47 (ddt, *J* = 9.9, 7.0, 5.0 Hz, 1H), 1.30 (dd, *J* = 6.2, 1.0 Hz, 3H), 1.28 - 1.20 (m, 2H), 1.18 - 1.08 (m, 1H), 1.00 (qd, *J* = 8.0, 6.0 Hz, 1H), 0.43 (d, *J* = 1.1 Hz, 4H). *m*/*z* (ESI): 510.2 (M+H)⁺. The racemic mixture was separated by chiral separation by SFC using a (S,S)Whelk-01 (250 × 30mm, 5µm) column, mobile phase: 70:30 (A: B), A = liquid CO₂, B = Methanol, flow rate: 70 mL/min to give:

Example 12-1: 2-(6-Azaspiro[2.5]octan-6-yl)-4-(*S*-cyclopropylsulfonimidoyl)-*N*-(6-((2*R*)-2-methyl-4-morpholinyl)-2-pyridinyl)benzamide. ¹H NMR (400 MHz, Chloroform-*d*) δ 12.64 (s, 1H), 8.44 (d, *J* = 8.1 Hz, 1H), 7.93 (d, *J* = 1.7 Hz, 1H), 7.83 (dd, *J* = 8.3, 1.8 Hz, 1H), 7.77 (d, *J* = 7.8 Hz, 1H), 7.60 (t, *J* = 8.0 Hz, 1H), 6.46 (d, *J* = 8.1 Hz, 1H), 4.13 - 4.01 (m, 3H), 3.80 - 3.64 (m, 2H), 3.19 - 3.13 (m, 4H), 2.98 (td, J= 12.2, 3.6 Hz, 1H), 2.67 - 2.60 (m, 2H), 2.01- 1.50 (m, 3H), 1.47 (ddt, *J* = 9.7, 6.8, 4.8 Hz, 1H), 1.32 - 1.26 (m, 6H), 1.15 (qd, *J* = 7.9, 4.9 Hz, 1H), 1.05 - 0.95 (m, 1H), 0.42 (s, 4H). *m*/*z* (ESI): 510.2 (M+H)⁺.

Example 12-2: 2-(6-Azaspiro[2.5]octan-6-yl)-4-(*R*-cyclopropylsulfonimidoyl)-*N*-(6-((2*R*)-2-methyl-4-morpholinyl)-2-pyridinyl)benzamide.¹H NMR (400 MHz, Chloroform-d): δ 12.65 (s, 1H), 8.44 (d, *J* = 8.2 Hz, 1H), 7.93 (d, *J* = 1.7 Hz, 1H), 7.83 (dd, *J* = 8.2, 1.8 Hz, 1H), 7.77 (d, *J* = 7.8 Hz, 1H), 7.59 (t, *J* = 8.1 Hz, 1H), 6.45 (d, *J* = 8.3 Hz, 1H), 4.13 - 3.95 (m, 3H), 3.73 (dddd, *J* = 17.1, 9.9, 8.1, 5.2 Hz, 2H), 3.19 - 3.13 (m, 4H), 2.98 (td, *J=* 12.3, 3.6 Hz, 1H), 2.63 (dq, *J* = 10.8, 3.9 Hz, 2H), 2.01 - 1.50 (m, 3H), 1.47 (dtd, *J* = 10.2, 4.8, 2.4 Hz, 1H), 1.32 - 1.29 (m, 2H), 1.29 (d, *J* = 6.2 Hz, 4H), 1.14 (ddd, *J* = 10.1, 7.9, 3.8 Hz, 1H), 0.99 (dtd, *J* = 9.0, 7.4, 4.9 Hz, 1H), 0.42 (s, 4H). *m*/*z* (ESI): 510.2 (M+H)⁺.

The stereochemistry was arbitrary assigned.

**Table 13: Examples 13-1 to 13-4 were prepared following a similar procedure for examples 12-1 and 12-2**

| Ex. # | Chemical Structure | Name | LRMS: (ESI + ve ion) *m*/*z* |
|---|---|---|---|
| 13-1 | 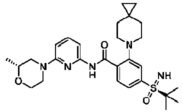 | *N-*(6-((*R*)-2-Methylmorpholino)pyridin-2-yl)-4-((*R*)-2-methylpropan-2-ylsulfonimidoyl)-2-(6-azaspiro[2.5]octan-6-yl)benzamide | 526.2 |
| 13-2 | 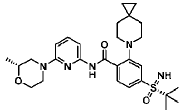 | *N-*(6-((*R*)-2-Methylmorpholino)pyridin-2-yl)-4-((*S*)-2-methylpropan-2-ylsulfonimidoyl)-2-(6-azaspiro[2.5]octan-6-yl)benzamide | 526.2 |
| 13-3 | 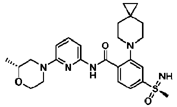 | 2-(6-Azaspiro[2.5]octan-6-yl)-*N*-(6-((2*R*)-2-methyl-4-morpholinyl)-2-pyridinyl)-4-(*S-*methylsulfonimidoyl)benzamide | 484.5 |
| 13-4 | 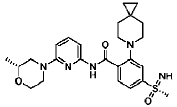 | 2-(6-Azaspiro[2.5]octan-6-yl)-*N*-(6-((2*R*)-2-methyl-4-morpholinyl)-2-pyridinyl)-4-(*R-*methylsulfonimidoyl)benzamide | 484.5 |

### Example 14: N-(6-(cyclopropylmethoxy)pyridin-2-yl)-4-((1-methylaclopropane)-1-sulfonamido)-2-(6-azaspiro[2.5]octan-6-yl)benzamide

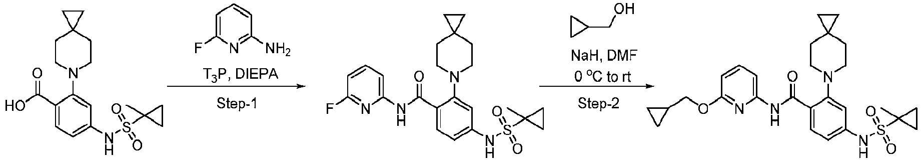

Step 1: A solution of 4-((1-methylcyclopropane)-1-sulfonamido)-2-(6-azaspiro[2.5]octan-6-yl)benzoic acid (5.0 g, 14 mmol, Intermediate 11) in DCM (50 mL) were treated with DIPEA (7.19 mL, 41.2 mmol), 6-fluoropyridin-2-amine(1.84 g, 16.5 mmol) followed by T₃P (17.46 g, 27.4 mmol, 50 % in EtOAc, Spectrochem) at rt. Then the reaction mixture was stirred for 16 h before it was quenched with water and was extracted with DCM. The organic extract was washed with brine, dried over Na₂SO₄, filtered, and concentrated. The concentrate was purified by flash column chromatography by eluting with a gradient of 15% to 20% EtOAc in petroleum ether to provide *N*-(6-fluoropyridin-2-yl)-4-((1-methylcyclopropane)-1-sulfonamido)-2-(6-azaspiro[2.5]octan-6-yl)benzamide (3.0 g, 6.5 mmol, 48 % yield) as off-white solid. ¹H NMR (400 MHz, DMSO-*d*₆): δ ppm 13.09 (s, 1 H), 10.28 (s, 1 H), 8.20 (d, *J*=7.7 Hz, 1 H), 8.03 (t, *J*=9.0 Hz, 2 H), 7.36 (s, 1 H), 7.17 (d, *J*=8.5 Hz, 1 H), 6.89 (d, *J*=8.0 Hz, 1 H), 2.97 (t, *J*=5.3 Hz, 4 H), 1.67 (s, 4 H), 1.41 (s, 3 H), 1.21 (s, 2 H), 0.81 - 0.89 (m, 2 H), 0.41 (s, 4 H). *m*/*z* (ESI): 459.1 (M+H)⁺.

Step 2: A solution of cyclopropylmethanol (23.59 mg, 0.327 mmol) in DMF (1.5 mL) was treated with sodium hydride (13.08 mg, 0.327 mmol, 60% in mineral oil) at 0 °C under N₂ atmosphere and was stirred for 10 min. Then a solution of *N*-(6-fluoropyridin-2-yl)-4-((1-methylcyclopropane)-1-sulfonamido)-2-(6-azaspiro[2.5]octan-6-yl)benzamide (100 mg, 0.218 mmol) in DMF (0.5 mL) was added to the reaction mixture. The resulting solution was stirred at rt for 2 h before it was quenched with an aqueous saturated solution of NH₄Cl and extracted with EtOAc. The organic extract was washed with brine, dried over Na₂SO₄, filtered, and concentrated. The concentrate was purified by reverse phase HPLC to provide *N*-(6-(cyclopropylmethoxy)pyridin-2-yl)-4-((1-methylcyclopropane)-1-sulfonamido)-2-(6-azaspiro[2.5]octan-6-yl)benzamide as white solid. ¹H NMR (400 MHz, DMSO-*d*₆): δ ppm 13.20 (s, 1 H), 10.22 (br s, 1 H), 8.08 (d, *J*=8.6 Hz, 1 H), 7.85 (d, *J*=7.8 Hz, 1 H), 7.72 (t, *J*=7.9 Hz, 1 H), 7.36 (d, *J*=2.2 Hz, 1 H), 7.17 (dd, *J*=8.7, 2.2 Hz, 1 H), 6.56 (d, *J*=8.0 Hz, 1 H), 4.12 (d, *J*=7.2 Hz, 2 H), 2.98 (d, *J*=5.3 Hz, 4H), 1.71 (br s, 4H), 1.41 (s, 3 H), 1.24 - 1.35 (m, 1 H), 1.17 - 1.27 (m, 2 H), 0.80 - 0.88 (m, 2 H), 0.55 - 0.64 (m, 2 H), 0.39 (s, 4 H), 0.28 - 0.36 (m, 2 H). *m*/*z* (ESI): 511.2 (M+H)⁺.

### Example 15: N-(6-(cyclopropylmethoxy)pyridin-2-yl)-4-((1-methylgyclopropane)-1-sulfonamido)-2-(6-azaspiro [2.5]octan-6-yl)benzamide

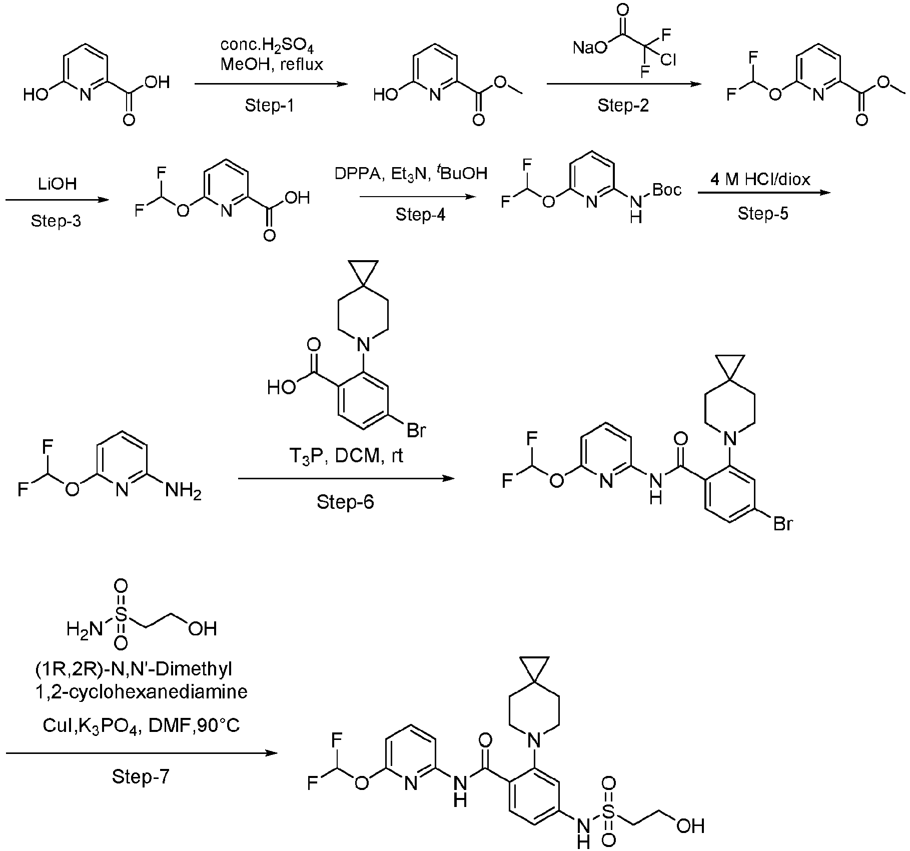

Step 1: To a solution of 6-hydroxypicolinic acid (5.0 g, 36 mmol) in MeOH (83 mL) was dropwise added H₂SO₄ (4.79 mL, 90 mmol) at rt. The reaction mixture was then heated at reflux for 18 h before volatiles were evaporated and the concentrate was neutralized with NaHCO₃ (100 mL) solution slowly to maintain pH of 7-8. Then the reaction mixture was extracted with CH₂Cl₂. The organic layer was washed with water and brine, dried over Na₂SO₄, filtered, and concentrated. The concentrate was triturated with petroleum ether to provide methyl 6-hydroxypicolinate (4.2 g, 27 mmol, 76 % yield) as white solid. ¹H NMR (400 MHz, DMSO-*d*₆) δ ppm 11.54 (s, 1 H), 7.64 (dd, *J*=8.9, 7.0 Hz, 1 H), 7.16 (dd, *J*=7.0, 1.0 Hz, 1 H), 6.73 (dd, *J*=8.9, 1.0 Hz, 1 H), 3.84 (s, 3 H). *m*/*z* (ESI): 154.1 (M+H)⁺.

Step 2: A solution of methyl 6-hydroxypicolinate (1.0 g, 6.4 mmol) and sodium 2-chloro-2,2-difluoroacetate (1.971 g, 12.93 mmol, TCI India) in acetonitrile (25 mL) was heated at 85°C for 72 h. Then the reaction mixture was cooled to rt and was evaporated to dryness to afford methyl 6-(difluoromethoxy)picolinate (2.1 g, 6.3 mmol, 98 % yield) as yellow oil which was used in next step without further purification. ¹H NMR (400 MHz, DMSO-*d*₆) δ ppm 8.12 (ddd, *J*=11.3, 6.7, 2.7 Hz, 1 H), 7.92 - 7.97 (m, 1 H), 7.50 - 7.91 (m, 1 H), 7.34 - 7.42 (m, 1 H), 3.89 (s, 3 H). *m*/*z* (ESI): 204.1 (M+H)⁺.

Step 3: To a solution of methyl 6-(difluoromethoxy)picolinate (2.0 g, 5.9 mmol) in THF (90 mL) was added LiOH (11.81 mL, 23.63 mmol, 2 M solution in H₂O). The reaction mixture was stirred at rt for 16 h before it was quenched with 1N HCl (pH=2-3) and was extracted with CH₂Cl₂. The organic layer was washed with brine, dried over Na₂SO₄, filtered, and concentrated to provide 6-(difluoromethoxy)picolinic acid as a light-yellow solid which was used in next step without purification. ¹H NMR (300 MHz, DMSO-*d*₆) δ ppm 13.34 (s, 1 H), 8.02 - 8.19 (m, 1 H), 7.91 (d, *J*=7.4 Hz, 1 H), 7.79 (t, *J*=72.6 Hz, 1 H), 7.34 (d, *J* = 8.2 Hz, 1 H). *m*/*z* (ESI): 190.0 (M+H)⁺.

Step 4: To a solution of 6-(difluoromethoxy)picolinic acid (0.80 g, 4.1 mmol) in a mixture of *tert-*butanol (2.5 mL) and toluene (28 mL) were added Et₃N (2.30 mL, 16.51 mmol) followed by diphenyl phosphorazidate (1.31 mL, 6.19 mmol) at rt under N₂ atmosphere. The reaction mixture was heated at 100 °C for 16 h before it was neutralized aqueous NaHCO₃ solution (100 mL) and was extracted with EtOAc (2 × 50 mL). The combined organic layers were dried over Na₂SO₄, filtered, and concentrated. The concentrate was purified by flash column chromatography using a gradient of 2 % to 5 % EtOAc in petroleum ether to provide *tert*-butyl (6-(difluoromethoxy)pyridin-2-yl)carbamate (0.51 g, 1.9 mmol, 47 % yield) as light-yellow oil. ¹H NMR (400 MHz, DMSO-*d*₆) δ ppm 9.85 (s, 1 H), 7.83 (t, *J*=8 Hz, 1 H), 7.73 - 7.36 (m, 2 H), 6.66 (d, *J*=12 Hz, 1 H), 1.47 (s, 9 H). *m*/*z* (ESI): 204.1 (M-Bu)⁺, 161.1 (M-Boc)⁺.

Step 5: To a solution of *tert*-butyl (6-(difluoromethoxy)pyridin-2-yl)carbamate (0.50 g, 1.9 mmol) in 1,4-dioxane (3.0 mL) was added 4.0 M HCl in Dioxane (9.98 mL, 39.9 mmol) at rt over 5 min. The reaction mixture was stirred for 6 h before it was quenched with a saturated aqueous solution of NaHCO₃ solution (100 mL) and was extracted with CH₂Cl₂. The organic extracts were washed with brine, dried over Na₂SO₄, filtered, and concentrated to provide 6-(difluoromethoxy)pyridin-2-amine (0.31 g, 1.8 mmol, 95 % yield) as orange oil which was used in next step without purification. ¹H NMR (300 MHz, DMSO-*d*₆) δ ppm 7.21 - 7.79 (m, 2 H), 6.23 (d, *J*=7.7 Hz, 3 H), 6.05 (d, *J*=7.7 Hz, 1 H). *m*/*z* (ESI): 161.1 (M+H)⁺.

Step 6: To a solution of 4-bromo-2-(6-azaspiro[2.5]octan-6-yl)benzoic acid (0.40 g, 1.3 mmol, Intermediate 9-1) in DCM (5.0 mL) were added DIPEA (0.676 mL, 3.87 mmol) followed by T₃P (2.28 mL, 3.87 mmol, 50% in Ethyl acetate) at rt and stirred for 15 min. Then a solution of 6-(difluoromethoxy)pyridin-2-amine (0.327 g, 1.93 mmol) in dichloromethane (2.0 mL) was added to the reaction mixture. The resulting mixture was stirred at rt for 16 h before it was quenched with water (50 mL) and was extracted with CH₂Cl₂. The combined organic layers were washed with brine, dried over Na₂SO₄, filtered, and concentrated. The concentrate was purified by flash column chromatography using a gradient of 0 % to 17 % EtOAc in petroleum ether to provide 4-bromo-N-(6-(difluoromethoxy)pyridin-2-yl)-2-(6-azaspiro[2.5]octan-6-yl)benzamide (0.30 g, 0.62 mmol, 48% yield) as light-yellow gummy liquid. ¹H NMR (300 MHz, DMSO-*d*₆) δ ppm 13.45 (s, 1 H), 7.92 - 8.17 (m, 3 H), 7.70 - 7.80 (m, 1 H), 7.57 (d, *J*=6.7 Hz, 2 H), 6.84 (d, *J*=7.9 Hz, 1 H), 3.05 (t, *J*=5.3 Hz, 4 H), 1.69 (s, 4 H), 0.38 (s, 4 H). *m*/*z* (ESI): 452.0, 454.0 (M+H)⁺.

Step 7: A mixture of 4-bromo-*N*-(6-(difluoromethoxy)pyridin-2-yl)-2-(6-azaspiro[2.5]octan-6-yl)benzamide (0.30 g, 0.66 mmol), 2-hydroxyethane-1-sulfonamide (0.125 g, 0.995 mmol), potassium phosphate (0.422 g, 1.99 mmol), (*1R*,*2R*)-*N1,N2*-dimethylcyclohexane-1,2-diamine (0.094 g, 0.66 mmol) and copper(I) iodide (0.126 g, 0.663 mmol) in DMF (3.0 mL) was heated at 90 °C for 16 h. The reaction mixture was filtered through a plug of celite and the filtrate was diluted with EtOAc. The organic layer was washed with water, brine, dried over Na₂SO₄, filtered, and concentrated. The concentrate was purified by flash column chromatography eluting with 5% to 44 % EtOAc in petroleum ether to provide *N-*(6-(difluoromethoxy)pyridin-2-yl)-4-((2-hydroxyethyl)sulfonamido)-2-(6-azaspiro[2.5]octan-6-yl)benzamide (0.18 g, 0.36 mmol, 54% yield) as white solid. ¹H NMR (400 MHz, DMSO-*d*₆) δ ppm 13.50 (s, 1 H), 10.26 (s, 1 H), 8.11 (t, *J*=8.0 Hz, 2 H), 7.96 (t, *J*=8.0 Hz, 1 H), 7.30 (d, *J*= 2.2 Hz, 1 H), 7.16 (dd, *J*=8.7, 2.2 Hz, 1 H), 6.81 (d, *J*=8.0 Hz, 1 H), 4.96 (t, *J*=5.6 Hz, 1 H), 3.77 (q, *J*=60 Hz, 2 H), 3.38 (d, *J*=6.4 Hz, 2 H), 2.99 (t, *J*=5.5 Hz, 4 H), 1.72 (br s, 4 H), 0.40 (s, 4 H). *m*/*z* (ESI): 497.1 (M+H)⁺.

### Example 16: N-(6-(2-hydroxy-2-methylpropoxy)pyridin-2-yl)-4-((2-hydroxyethyl)sulfonamido)-2-(6-azaspiro[2.5]octan-6-yl)benzamide

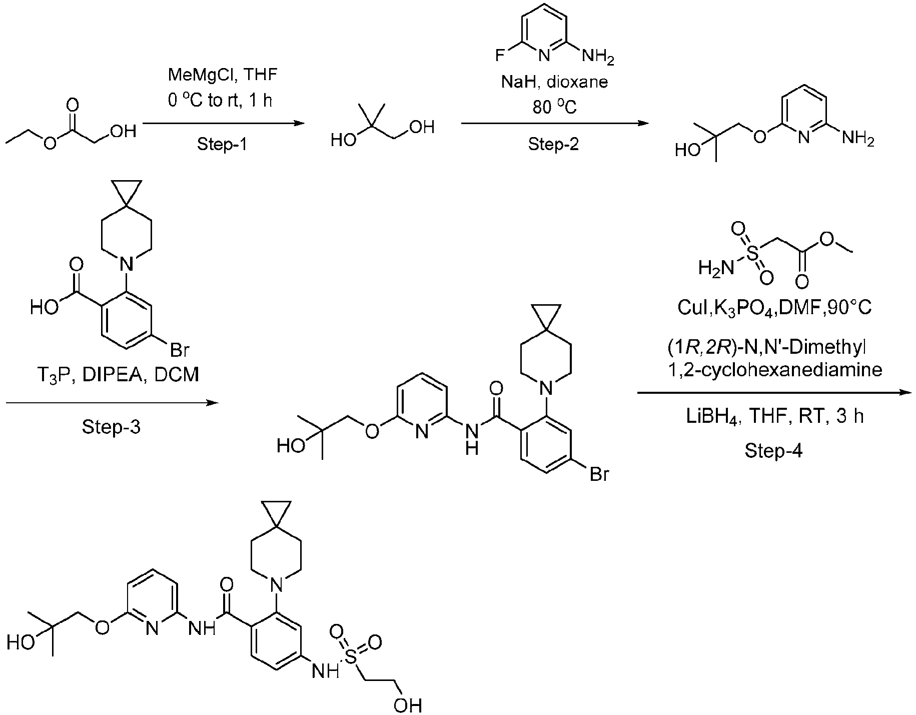

Step 1: To a solution of ethyl 2-hydroxyacetate (5.0 g, 48 mmol, Combi-Blocks) in THF (100 mL) was added methyl magnesium chloride (48.0 mL, 3.0 M in THF, 144 mmol) and the reaction mixture was stirred at 0 °C for 30 min. The reaction mixture was quenched with a saturated aqueous NH₄Cl solution and extracted with EtOAc. The organic extract was washed with brine, dried over Na₂SO₄, filtered, and concentrated to provide 2-methylpropane-1,2-diol (2.3 g, 26 mmol 53% yield) as light-yellow oil. ¹H NMR (300 MHz, DMSO-*d*₆) δ ppm 4.49 (t, *J*=5.8 Hz, 1 H), 4.10 (br s, 1 H), 3.12 - 3.16 (m, 2 H), 1.03 (s, 6 H).

Step 2: To a solution of 6-fluoropyridin-2-amine (0.700 g, 6.24 mmol) and 2-methylpropane-1,2-diol (0.844 g, 9.37 mmol) in dioxane (7 mL) at rt was added and NaH (60% in mineral oil, 0.749 g, 18.7 mmol). The reaction mixture was stirred at 80 °C for 4 h, cooled to rt, quenched with saturated aqueous NH₄Cl solution, and was extracted with EtOAc. The organic extract was washed with brine, dried over Na₂SO₄, filtered, concentrated and purified by flash column chromatography, eluting with a gradient of 10 % to 80 % ethyl acetate in petroleum ether to provide 1-((6-aminopyridin-2-yl)oxy)-2-methylpropan-2-ol (0.60 g, 3.3 mmol, 53 % yield) as orange oil. ¹H NMR (400 MHz, DMSO-*d*₆) δ ppm 7.25 - 7.29 (m, 1 H), 5.97 (d, *J*=7.8 Hz, 1 H), 5.87 (d, *J*=7.8 Hz, 1 H), 5.82 (s, 2 H), 4.56 (s, 1 H), 3.88 (s, 2 H), 1.15 (s, 6 H). *m*/*z* (ESI): 183.2 (M+H)⁺.

Step 3: A mixture of 4-bromo-2-(6-azaspiro[2.5]octan-6-yl)benzoic acid (0.30 g, 0.97 mmol, Intermediate 9-1), DIPEA (0.203 mL, 1.16 mmol), T₃P (50% in ethyl acetate, 0.691 mL, 1.16 mmol) and 1-((6-aminopyridin-2-yl)oxy)-2-methylpropan-2-ol (0.211 g, 1.16 mmol) in DCM (3 mL) was stirred at room temperature for 4 h. The reaction mixture was diluted with water and was extracted with ethyl acetate. The organic extract was washed with brine, dried over Na₂SO₄, filtered, concentrated and purified by flash column chromatography, eluting with a gradient of 5 % to 30 % ethyl acetate in petroleum ether to provide 4-bromo-*N*-(6-(2-hydroxy-2-methylpropoxy)pyridin-2-yl)-2-(6-azaspiro[2.5]octan-6-yl)benzamide (0.20 g, 0.42 mmol, 44% yield) as off white solid. ¹H NMR (400 MHz, DMSO-*d*₆) δ ppm 13.08 (s, 1 H), 8.05 (d, *J*=8.4 Hz, 1 H), 7.85 (d, *J*=7.8 Hz, 1 H), 7.69 - 7.76 (m, 2 H), 7.55 - 7.59 (m, 1 H), 6.59 (d, *J*=8.0 Hz, 1 H), 4.60 (s, 1 H), 4.06 (s, 2 H), 3.06 (t, *J*=5.3 Hz, 4 H), 1.73 (br s, 4 H), 1.22 (s, 6 H), 0.39 (s, 4 H). *m*/*z* (ESI): 476.1 (M+H)⁺.

Step 4: A mixture of 4-bromo-*N*-(6-(2-hydroxy-2-methylpropoxy)pyridin-2-yl)-2-(6-azaspiro[2.5]octan-6-yl)benzamide (0.27 g, 0.57 mmol), methyl 2-sulfamoylacetate (0.131 g, 0.854 mmol), potassium phosphate (0.302 g, 1.42 mmol), (1*R*,2*R*)-*N*1,*N*2-dimethylcyclohexane-1,2-diamine (0.040 g, 0.28 mmol) and copper(I) iodide (0.108 g, 0.569 mmol) in DMF (4 mL) was stirred at 90 °C for 16 h. The reaction mixture was diluted with water and extracted with ethyl acetate. The organic extract was washed with brine, dried over Na₂SO₄, filtered, concentrated and purified by flash column chromatography, eluting with a gradient of 10 % to 80 % ethyl acetate in petroleum ether to provide methyl 2-(*N*-(4-((6-(2-hydroxy-2-methylpropoxy)pyridin-2-yl)carbamoyl)-3-(6-azaspiro[2.5]octan-6-yl)phenyl)sulfamoyl)acetate (0.25 g, 0.46 mmol, 80 % yield) as light yellow oil. *m*/*z* (ESI): 547.2 (M+H)⁺.

To a solution of methyl 2-(*N*-(4-((6-(2-hydroxy-2-methylpropoxy)pyridin-2-yl)carbamoyl)-3-(6-azaspiro[2.5]octan-6-yl)phenyl)sulfamoyl)acetate (0.24 g, 0.44 mmol) in THF (3.75 mL) was added LiBH₄ (0.439 mL, 0.878 mmol) at -30°C and the resulting mixture was stirred at room temperature for 2 h. The reaction mixture was quenched with a saturated aqueous NH₄Cl solution and was extracted with EtOAc. The organic extract was washed with brine, dried over Na₂SO₄, filtered, concentrated, and purified by flash column chromatography, eluting with a gradient of 30 % to 90 % ethyl acetate in petroleum ether to provide *N*-(6-(2-hydroxy-2-methylpropoxy)pyridin-2-yl)-4-((2-hydroxyethyl)sulfonamido)-2-(6-azaspiro[2.5]octan-6-yl)benzamide (0.091 g, 0.18 mmol, 40% yield) as off white solid. ¹H NMR (400 MHz, DMSO-*d*₆): δ ppm 13.10 (br s, 1 H), 8.07 (d, *J*=8.7 Hz, 1 H), 7.84 (d, *J*=7.6 Hz, 1 H), 7.72 (t, *J*=7.9 Hz, 1 H), 7.28 (d, *J*=2.2 Hz, 1 H), 7.12 (dd, *J*=8.6, 2.1 Hz, 1 H), 6.55 (d, J=8.0 Hz, 1 H), 4.58 (s, 1 H), 4.05 (s, 2 H), 3.76 (t, *J*=6.5 Hz, 2 H), 3.35 (t, *J*=6.5 Hz, 2 H), 2.99 (t, *J*=5.2 Hz, 4 H), 1.75 (br s, 4 H), 1.21 (s, 6 H), 0.39 (s, 4 H). *m*/*z* (ESI): 519.2 (M+H)⁺.

**Table 14: Examples 16-1 to 16-9 were prepared following a similar procedure for Example 16**

| Ex. # | Chemical Structure | Name | LRMS: (ESI + ve ion) *m*/*z* |
|---|---|---|---|
| 16-1 | 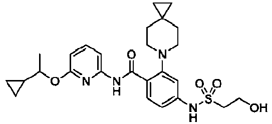 | *N*-(6-(1-Cyclopropylethoxy)pyridin-2-yl)-4-((2-hydroxyethyl)sulfonamido)-2-(6-azaspiro[2.5]octan-6-yl)benzamide | 515.2 |
| 16-2 | 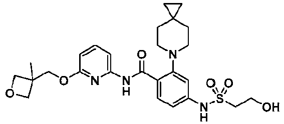 | 4-((2-Hydroxyethyl)sulfonamido)-*N*-(6-((3-methyloxetan-3-yl)methoxy)pyridin-2-yl)-2-(6-azaspiro[2.5]octan-6-yl)benzamide | 531.2 |
| 16-3 | 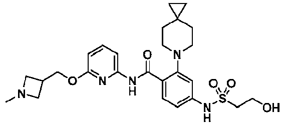 | 4-((2-Hydroxyethyl)sulfonamido)-*N*-(6-((1-methylazetidin-3-yl)methoxy)pyridin-2-yl)-2-(6-azaspiro[2.5]octan-6-yl)benzamide | 530.2 |
| 16-4 | 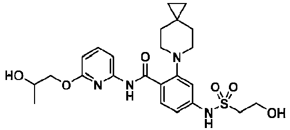 | 4-((2-Hydroxyethyl)sulfonamido)-*N*-(6-(2-hydroxypropoxy)pyridin-2-yl)-2-(6-azaspiro[2.5]octan-6-yl)benzamide | 505.2 |
| 16-5 | 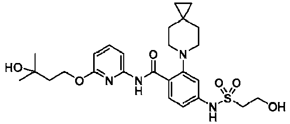 | *N*-(6-(3-Hydroxy-3-methylbutoxy)pyridin-2-yl)-4-((2-hydroxyethyl)sulfonamido)-2-(6-azaspiro[2.5]octan-6-yl)benzamide | 533.2 |
| 16-6 | 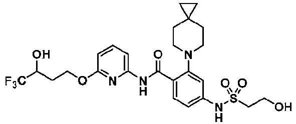 | 4-((2-Hydroxyethyl)sulfonamido)-2-(6-azaspiro[2.5]octan-6-yl)-N-(6-(4,4,4-trifluoro-3-hydroxybutoxy)pyridin-2-yl)benzamide | 573.2 |
| 16-7 | 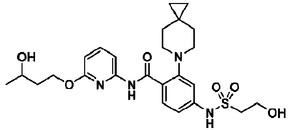 | *N-*(6-(3-Hydroxybutoxy)pyridin-2-yl)-4-((2-hydroxyethyl)sulfonamido)-2-(6-azaspiro[2.5]octan-6-yl)benzamide | 519.2 |
| 16-8 | 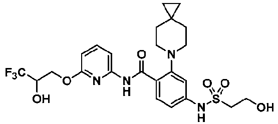 | 4-((2-Hydroxyethyl)sulfonamido)-2-(6-azaspiro[2.5]octan-6-yl)-*N*-(6-(3,3,3-trifluoro-2-hydroxypropoxy)pyridin-2-yl)benzamide | 559.2 |
| 16-9 | 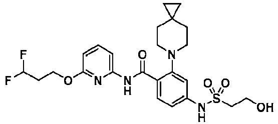 | *N*-(6-(3,3-Difluoropropoxy)pyridin-2-yl)-4-((2-hydroxyethyl)sulfonamido)-2-(6-azaspiro[2.5]octan-6-yl)benzamide | 525.2 |

### Example 17: 4-((2-Hydroxyethyl)sulfonamido)-N-(4-methyl-6-(3,3,3-trifluoropropoxy)pyridin-2-yl)-2-(6-azaspiro[2.5]octan-6-yl)benzamide

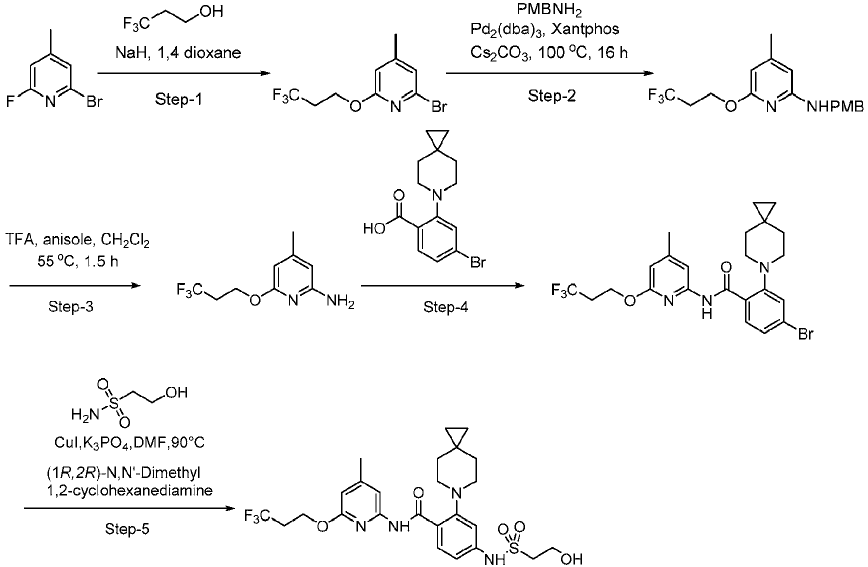

Step 1: To a solution of 3,3,3-trifluoropropan-1-ol (0.550 g, 4.83 mmol) in 1,4-dioxane (10 mL) was added sodium hydride (0.386 g, 9.64 mmol, 60% in oil) at 10 °C and stirred for 30 min at room temperature. A solution of 2-bromo-6-fluoro-4-methylpyridine (0.61 g, 3.2 mmol, Combi-Blocks) in dioxane (4 mL) was added to the reaction mixture. The resulting solution was stirred at room temperature for 2 h before it was quenched with ice cold water and was extracted with ethyl acetate. The organic extract was washed with brine, dried over Na₂SO₄, filtered, concentrated and purified by flash column chromatography, eluting with a gradient of 0 % to 4 % ethyl acetate in petroleum ether to provide 2-bromo-4-methyl-6-(3,3,3-trifluoropropoxy)pyridine (0.76 g, 85 % yield) as clear oil. ¹H NMR (400 MHz, Chloroform-*d*): δ ppm 6.96 (s, 1 H), 6.53 (s, 1 H), 4.51 - 4.56 (m, 2 H), 2.56 - 2 66 (m, 2 H), 2.29 (s, 3 H). *m*/*z* (ESI): 284.0 (M+H)⁺.

Step 2: A mixture of 2-bromo-4-methyl-6-(3,3,3-trifluoropropoxy)pyridine (0.71 g, 2.5 mmol), (4-methoxyphenyl)methanamine (0.514 g, 3.75 mmol), cesium carbonate (2.443 g, 7.50 mmol), xantphos (0.289 g, 0.500 mmol) and Pd₂(dba)₃ (0.023 g, 0.025 mmol) in 1,4-dioxane (14 mL) was stirred at 100°C for 16 h. The reaction mixture was filtered through a celite bed and the filtrate was washed with water, brine, dried over Na₂SO₄ and concentrated. The concentrate was purified by flash column chromatography using 6 % ethyl acetate in petroleum ether to afford *N*-(4-methoxybenzyl)-4-methyl-6-(3,3,3-trifluoropropoxy)pyridin-2-amine (0.65 g, 1.91 mmol, 76 % yield) as yellow solid. ¹H NMR (300 MHz, DMSO-*d*₆): δ ppm 7.23 (d, *J*=8.7 Hz, 2 H), 6.94 (t, *J*=5.9 Hz, 1 H), 6.82 - 6.90 (m, 2 H), 5.89 (s, 1 H), 5.71 (s, 1 H), 4.33 (t, *J*=6.1 Hz, 4 H), 3.71 (s, 3 H), 2.56 - 2.66 (m, 2 H), 2.07 (s, 3 H). *m*/*z* (ESI): 341.1 (M+H)⁺.

Step 3: A solution of *N*-(4-methoxybenzyl)-4-methyl-6-(3,3,3-trifluoropropoxy)pyridin-2-amine (0.30 g, 0.88 mmol), anisole (0.193 mL, 1.76 mmol), TFA (1.5 mL, 19 mmol) in dichloromethane (3 mL) was stirred at 55 °C for 1.5 h. Then the reaction mixture was concentrated, and the residue was dissolved in water and basified to pH~ 8 with 10 % sodium bicarbonate and was extracted with ethyl acetate. The organic extract was washed with brine, dried over Na₂SO₄, filtered, concentrated and purified by flash column chromatography, eluting with a gradient of 10 % to 20 % ethyl acetate in petroleum ether to provide 4-methyl-6-(3,3,3-trifluoropropoxy)pyridin-2-amine (0.16 g, 0.73mmol, 82 % yield) as a yellow oil. ¹H NMR (300 MHz, DMSO-*d*₆): δ ppm 5.88 (s, 1 H), 5.74 (s, 1 H), 4.30 (t, *J*=6.1 Hz, 2H), 2.60 - 2.68 (m, 2 H), 2.05 (s, 3 H). *m*/*z* (ESI): 221.1 (M+H)⁺.

Step 4: A mixture of 4-methyl-6-(3,3,3-trifluoropropoxy)pyridin-2-amine (0.16 g, 0.73 mmol), 4-bromo-2-(6-azaspiro[2.5]octan-6-yl)benzoic acid (0.270 g, 0.872 mmol, Intermediate 9-1), Et₃N (0.304 mL, 2.18 mmol) and T₃P (50 % solution in ethyl acetate, 1.386 g, 2.180 mmol) in 1,2-dimethoxyethane (4 mL) was heated at 80 °C for 16 h. The reaction mixture was quenched with ice cold water and was extracted with ethyl acetate. The organic extract was washed with brine, dried over Na₂SO₄, filtered, concentrated and purified by flash column chromatography, eluting with a gradient of 0 % to 8 % ethyl acetate in petroleum ether to provide 4-bromo-*N-*(4-methyl-6-(3,3,3-trifluoropropoxy)pyridin-2-yl)-2-(6-azaspiro[2.5]octan-6-yl)benzamide (0.20 g, 0.39 mmol, 54% yield) as white solid. ¹H NMR (300 MHz, DMSO-*d*₆): δ ppm 13.04 (s, 1 H), 8.02 (d, *J*=8.5 Hz, 1 H), 7.77 (s, 1 H), 7.68 (d, *J*=2.0 Hz, 1 H), 7.56 (dd, *J*=8.4, 1.9 Hz, 1 H), 6.45 (s, 1 H), 4.49 (t, *J*=6.1 Hz, 2 H), 3.05 (t, *J*=5.3 Hz, 4 H), 2.82 (dt, *J*= 11.5, 5.8 Hz, 2 H), 2.31 (s, 3 H), 1.66 - 1.78 (m, 4 H), 0.37 (s, 4 H). *m*/*z* (ESI): 512.1 (M+H)⁺.

Step 5: A mixture of 4-bromo-*N*-(4-methyl-6-(3,3,3-trifluoropropoxy)pyridin-2-yl)-2-(6-azaspiro[2.5]octan-6-yl)benzamide (0.20 g, 0.39 mmol), 2-hydroxyethane-1-sulfonamide (0.073 g, 0.59 mmol), potassium phosphate tribasic (0.166 g, 0.781 mmol), copper(I) iodide (0.074 g, 0.39mmol) and (1*R*,2*R*)-*N*,*N'*-dimethyl-1,2-cyclohexanediamine (0.028 g, 0.20 mmol) in DMF (3 mL) was stirred at 95 °C for 16 h. The reaction mixture was quenched with ice cold water and was extracted with ethyl acetate. The organic extract was washed with brine, dried over Na₂SO₄, filtered, concentrated and purified by reverse phase column chromatography using a gradient of 65 % acetonitrile in water to provide 4-((2-hydroxyethyl)sulfonamido)-*N-*(4-methyl-6-(3,3,3-trifluoropropoxy)pyridin-2-yl)-2-(6-azaspiro[2.5]octan-6-yl)benzamide (0.166 g, 0.298 mmol, 76 % yield) as a white solid. ¹H NMR (400 MHz, DMSO-*d*₆): δ ppm 13.09 (s, 1 H), 10.21 (br s, 1 H), 8.06 (d, *J*=8.6 Hz, 1 H), 7.78 (s, 1 H), 7.27 (d, *J*=2.1 Hz, 1 H), 7.13 (dd, *J*=8.6, 2.1 Hz, 1 H), 6.42 (s, 1 H), 4.96 (br s, 1 H), 4.49 (t, *J*=6.1 Hz, 2 H), 3.76 (t, *J*=6.4 Hz, 2 H), 3.38 - 3.40 (m, 2 H), 2.96 - 3.20 (m, 4 H), 2.76 - 2.88 (m, 2 H), 2.31 (s, 3 H), 1.60 - 1.80 (m, 4 H), 0.38 (s, 4 H). *m*/*z* (ESI): 557.2 (M+H)⁺.

**Table 15: Examples 17-1 to 17-3 were prepared following a similar procedure as described for example 17**

| Ex. # | Chemical Structure | Name | LRMS: (ESI + ve ion) *m*/*z* |
|---|---|---|---|
| 17-1 | 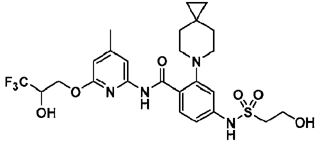 | 4-((2-Hydroxyethyl)sulfonamido)-*N*-(4-methyl-6-(3,3,3-trifluoro-2-hydroxypropoxy)pyridin-2-yl)-2-(6-azaspiro[2.5]octan-6-yl)benzamide | 573.2 |
| 17-2 | 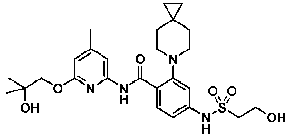 | *N-*(6-(2-Hydroxy-2-methylpropoxy)-4-methylpyridin-2-yl)-4-((2-hydroxyethyl)sulfonamido)-2-(6-azaspiro[2.5]octan-6-yl)benzamide | 533.2 |
| 17-3 | 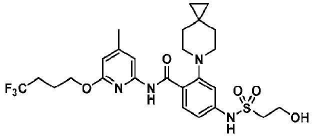 | 4-((2-Hydroxyethyl)sulfonamido)-*N*-(4-methyl-6-(4,4,4-trifluorobutoxy)pyridin-2-yl)-2-(6-azaspiro[2.5]octan-6-yl)benzamide | 571.2 |

### Example 18: 4-((2-Hydroxyethyl)sulfonamido)-N-(5-methyl-6-morpholinopyridin-2-yl)-2-(6-azaspiro [2.5]octan-6-yl)benzamide

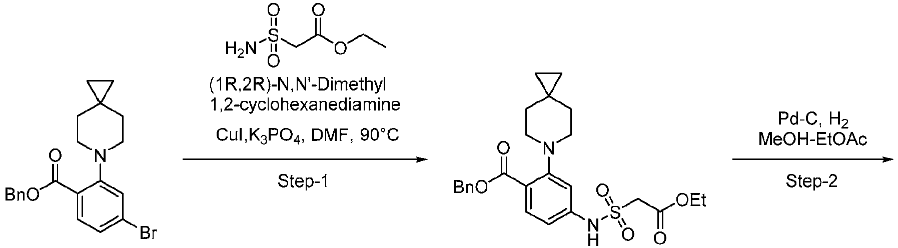

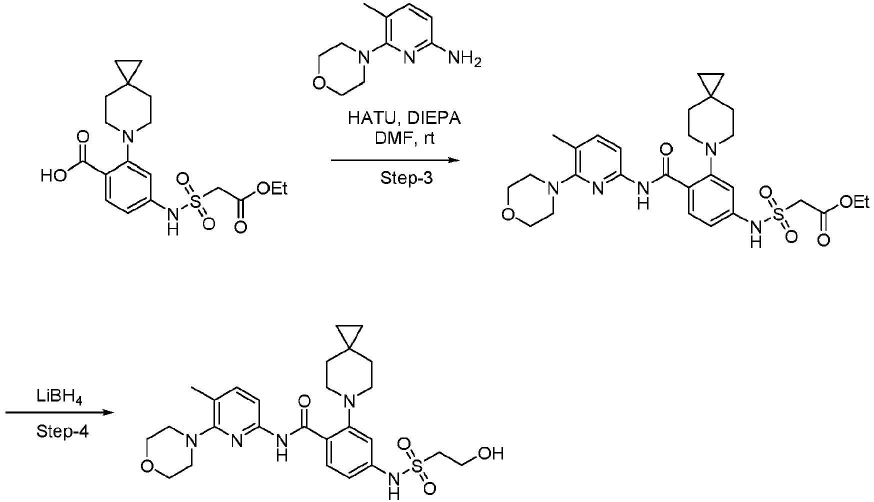

Step 1: A mixture of benzyl 4-bromo-2-(6-azaspiro[2.5]octan-6-yl)benzoate (1.0 g, 2.5 mmol, Intermediate 9-4), ethyl 2-sulfamoylacetate (0.626 g, 3.75 mmol), potassium phosphate (1.06 g, 5.00 mmol), copper(I) iodide (0.476 g, 2.50 mmol), (*1R*,*2R*)-*N1*,*N2*-dimethylcyclohexane-1,2-diamine (0.178 g, 1.25 mmol) in DMF (15 mL) was heated at 90 °C for 16 h. Then the reaction mixture was filtered through a plug of celite and the filtrate was diluted with EtOAc, washed with water, brine, dried over Na₂SO₄, filtered, and concentrated. The concentrate was purified by flash column chromatography eluting with a gradient of 0% to 25% EtOAc in petroleum ether to provide benzyl 4-((2-ethoxy-2-oxoethyl)sulfonamido)-2-(6-azaspiro[2.5]octan-6-yl)benzoate (0.75 g, 1.5 mmol, 53 % yield) as orange oil. ¹H NMR (400 MHz, DMSO-*d*₆): δ ppm 10.45 (s, 1 H), 7.64 (d, *J*=8.5 Hz, 1 H), 7.29 - 7.48 (m, 5 H), 6.93 (d, *J*=2.1 Hz, 1 H), 6.79 (dd, *J*=8.5, 2.0 Hz, 1 H), 5.28 (s, 2 H), 4.31 (s, 2 H), 4.05 (dq, *J*=19.5, 7.1 Hz, 2 H), 2.94 (t, *J*=5.3 Hz, 4 H), 1.37 (t, *J*=5.3 Hz, 4H), 1.16 (dt, *J*=11.2, 7.1 Hz, 3 H), 0.28 (s, 4H). *m*/*z* (ESI): 487.2 (M+H)⁺.

Step 2: To a solution of benzyl 4-((2-ethoxy-2-oxoethyl)sulfonamido)-2-(6-azaspiro[2.5]octan-6-yl)benzoate (0.55 g, 1.1 mmol) in ethyl acetate (3 mL) and methanol (6 mL) was added palladium on carbon (10%, 0.28 g, 0.26 mmol) and stirring was continued under hydrogen atmosphere (1 atm) for 2 h. Then the reaction mixture was filtered through celite bed, washed with methanol (150 mL). The filtrate was concentrate and triturated with Et₂O to provide 4-((2-ethoxy-2-oxoethyl)sulfonamido)-2-(6-azaspiro[2.5]octan-6-yl)benzoic acid (0.55 g, 1.4mmol, 90 % yield) as off white solid. ¹H NMR (400 MHz, DMSO-*d*₆): δ ppm 10.69 (s, 1 H), 7.93 (d, *J*=8.6 Hz, 1 H), 7.31 (d, *J*=2.1 Hz, 1 H), 7.12 (dd, *J*=8.7, 2.1 Hz, 1 H), 4.31 (s, 2 H), 4.07 (q, *J*=7.1 Hz, 2 H), 3.00 (t, *J*=5.4 Hz, 4 H), 1.57 (s, 4 H), 1.14 (t, *J*=7.1 Hz, 3 H), 0.42 (s, 4 H). *m*/*z* (ESI): 397.1 (M+H)⁺.

Step 3: To a solution of 5-methyl-6-morpholinopyridin-2-amine (45.0 mg, 0.232 mmol, Intermediate 8) in DMF (3 mL) were added 4-((2-ethoxy-2-oxoethyl)sulfonamido)-2-(6-azaspiro[2.5]octan-6-yl)benzoic acid (92 mg, 0.23 mmol), DIPEA (48.8 µL, 0.279 mmol) and HATU (106 mg, 0.279 mmol) at room temperature. Then the reaction mixture was stirred for 12 h before it was quenched with water and was extracted with EtOAc. The organic layer was washed with brine, dried over Na₂SO₄, filtered, and concentrated. The concentrate was purified by flash column chromatography eluting with 15 % ethyl acetate in petroleum ether to provide ethyl 2-(N-(4-((5-methyl-6-morpholinopyridin-2-yl)carbamoyl)-3-(6-azaspiro[2.5]octan-6-yl)phenyl)sulfamoyl)acetate (50 mg, 0.087 mmol, 38% yield) as pale yellow solid *m*/*z* (ESI): 572.2 (M+H)⁺.

Step 4: To a solution of ethyl 2-(A-(4-((5-methyl-6-morpholinopyridin-2-yl)carbamoyl)-3-(6-azaspiro[2.5]octan-6-yl)phenyl)sulfamoyl)acetate (50 mg, 0.087 mmol) in THF (10 mL) was added lithium borohydride (65.6 µL, 0.131 mmol, 2M in THF) at 0 °C. Then the reaction mixture was stirred at rt for 3 h before it was quenched with an aqueous saturated solution of ammonium chloride and was extracted with EtOAc. The combined organic layers were washed with brine, dried over Na₂SO₄, filtered, and concentrated. The concentrate was purified by flash column chromatography eluting with 30 % ethyl acetate in petroleum ether to provide 4-((2-hydroxyethyl)sulfonamido)-*N-*(5-methyl-6-morpholinopyridin-2-yl)-2-(6-azaspiro[2.5]octan-6-yl)benzamide (30 mg , 0.057 mmol, 64.8 % yield) as white solid. ¹H NMR (400 MHz, DMSO-*d*₆): δ ppm 13.10 (d, *J*=5.4 Hz, 1 H), 10.21 (s, 1 H), 8.08 (dd, *J*=9.6, 3.7 Hz, 1 H), 7.83 (t, *J*=6.8 Hz, 1 H), 7.55 (dd, *J*=9.2, 4.2 Hz, 1 H), 7.26 (d, *J*=5.4 Hz, 1 H), 7.13 (dd, *J*=8.7, 2.3 Hz, 1 H), 4.95 (s, 1 H), 3.75 (m, 6 H), 3.11 (q, *J*=4.4 Hz, 4 H), 2.97 (d, *J*=6.6 Hz, 4 H), 2.69 (d, *J*=2.9 Hz, 2 H), 2.22 (s, 3 H), 1.77 (br s, 4 H), 0.38 (d, *J*=5.7 Hz, 4 H). *m*/*z* (ESI): 530.2 (M+H)⁺.

### Example 19: (R)-N-(4-cyclopropyl-6-(2-methylmorpholino)pyridin-2-yl)-4-((2 hydroxyethyl)sulfonamido)-2-(6-azaspiro[2.5]octan-6-yl)benzamide

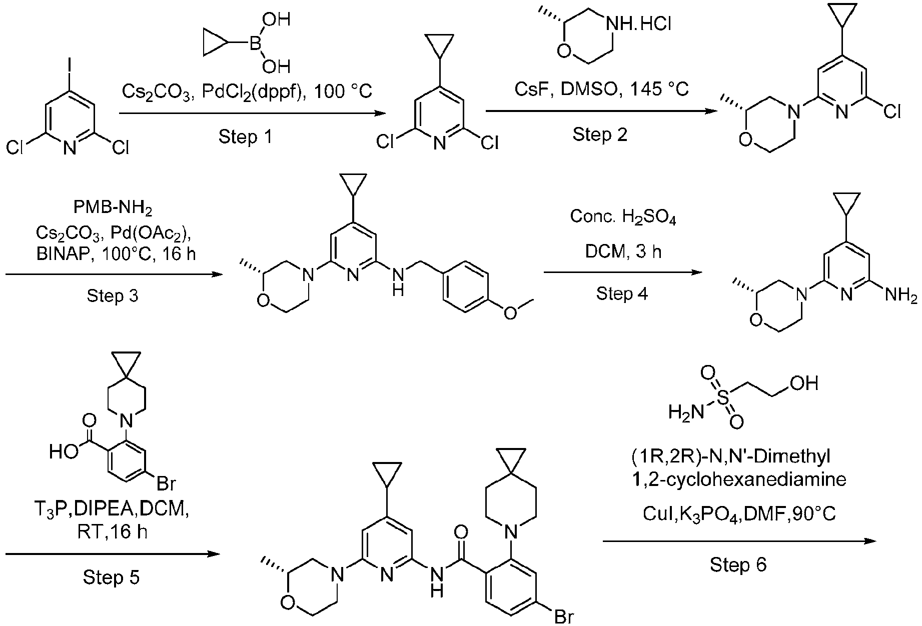

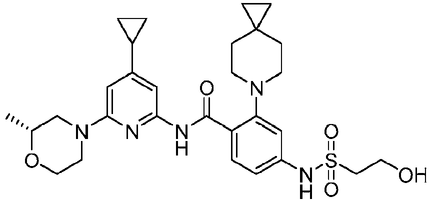

Step 1: A mixture of 2,6-dichloro-4-iodopyridine (1.8 g, 6.6 mmol, Combi-Blocks), cyclopropylboronic acid (0.565 g, 6.57 mmol, Combi-Blocks), potassium carbonate (0.908 g, 6.57 mmol) and Xphos-Pd-G3 (0.278 g, 0.329 mmol, Strem chemicals) in 1,4-dioxane (10 mL) was stirred at 100 °C for 3 h. The reaction mixture was filtered through a celite bed. The filtrate was concentrated and the residue was triturated with diethyl ether to get 2,6-dichloro-4-cyclopropylpyridine (0.95 g, 5.0 mmol, 77 % yield) as brown semi solid.

Step 2: A mixture of 2,6-dichloro-4-cyclopropylpyridine (0.90 g, 4.8 mmol) and (R)-2-methylmorpholine hydrochloride (0.988 g, 7.18 mmol, Suzhou chemicals) and CsF (2.91 g, 19.1mmol) in DMSO (9 mL) was stirred at 145 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 5 % to 50 % ethyl acetate in petroleum ether to provide (*R*)-4-(6-chloro-4-cyclopropylpyridin-2-yl)-2-methylmorpholine (1.1 g, 4.4 mmol, 91 % yield) as orange oil. ¹H NMR (300 MHz, DMSO-*d*₆): δ ppm 6.50 (s, 1 H), 6.39 (s, 1 H), 3.84 - 4.11 (m, 4H), 3.50 (dt, *J* = 11.7, 2.6 Hz, 2 H), 2.78 (td, *J* = 12.3, 3.5 Hz, 1 H), 1.87 (td, *J* = 8.5, 4.3 Hz, 1 H), 1.15 (d, *J* = 6.2 Hz, 3 H), 0.98 (dt, *J* = 8.4, 3.3 Hz, 2 H), 0.78 - 0.85 (m, 2 H). *m*/*z* (ESI): 253.1 (M+H)⁺.

Step 3: A mixture of (*R*)-4-(6-chloro-4-cyclopropylpyridin-2-yl)-2-methylmorpholine (1.0 g, 4.0 mmol), (4-methoxyphenyl)methanamine (0.775 mL, 5.93 mmol), Cs₂CO₃ (2.58 g, 7.91 mmol), BINAP (0.246 g, 0.396 mmol) and palladium (II) acetate (0.089 g, 0.40 mmol) in dioxane (15 mL) was stirred at 100 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 5 % to 40 % ethyl acetate in petroleum ether to provide (*R*)-4-cyclopropyl-*N*-(4-methoxybenzyl)-6-(2-methylmorpholino)pyridin-2-amine (1.2 g, 3.4 mmol, 86 % yield) as orange oil. ¹H NMR (300 MHz, DMSO-*d*₆): δ ppm 7.15 - 7.28 (m, 2 H), 6.74 - 6.92 (m, 2 H), 6.48 (t, *J*=6.0 Hz, 1 H), 5.61 (s, 1 H), 5.54 (s, 1 H), 4.29 (d, *J*=6.0 Hz, 2 H), 3.79 - 4.06 (m, 4 H), 3.71 (s, 3 H), 3.40 - 3.52 (m, 2 H), 2.26 - 2.36 (m, 1 H), 1.63 (td, *J*=8.0, 4.0 Hz, 1 H), 1.11 (d, *J*=6.2 Hz, 3 H), 0.80 - 0.89 (m, 2 H), 0.58 - 0.66 (m, 2 H). *m*/*z* (ESI): 354.2 (M+H)⁺.

Step 4: A mixture of (*R*)-4-cyclopropyl-*N*-(4-methoxybenzyl)-6-(2-methylmorpholino)pyridin-2-amine (1.0 g, 2.8 mmol) and H₂SO₄ (1.51 mL, 28.3 mmol) in dichloromethane (20 mL) was stirred at room temperature for 2 h. The reaction mixture was basified with 1 N NaOH (pH=9) and was extracted with EtOAc. The organic extract was washed with brine, dried over Na₂SO₄, filtered, concentrated to provide (*R*)-4-cyclopropyl-6-(2-methylmorpholino)pyridin-2-amine as brown sticky liquid. ¹H NMR (400 MHz, DMSO-*d*₆): δ ppm 5.64 - 5.78 (m, 1 H), 5.52 (d, *J*=2.4 Hz, 1 H), 5.39 (br s, 2 H), 4.00 (d, *J*=12.6 Hz, 1 H), 3.81 - 3.94 (m, 2 H), 3.46 - 3.52 (m, 2 H), 2.62 (td, *J*=12.3, 3.3 Hz, 1 H), 2.29 (td, *J*=12.7, 2.2 Hz, 1H), 1.61 - 1.69 (m, 1 H), 1.13 (d, *J*=6.2 Hz, 3 H), 0.84 - 0.88 (m, 2 H), 0.62 - 0.66 (m, 2 H). *m*/*z* (ESI): 234.2 (M+H)⁺.

Step 5: A mixture of 4-bromo-2-(6-azaspiro[2.5]octan-6-yl)benzoic acid (0.50 g, 1.6 mmol, Intermediate 9-1) and DIPEA (0.845 mL, 4.84 mmol), 2,4,6-tripropyl-1,3,5,2,4,6-trioxatriphosphinane 2,4,6-trioxide (50% in ethyl acetate, 1.452 mL, 4.84 mmol) and (R)-4-cyclopropyl-6-(2-methylmorpholino)pyridin-2-amine (0.40 g, 1.7 mmol) in dichloromethane (15 mL) was stirred at room temperature for 16 h. The reaction mixture was diluted with water and was extracted with ethyl acetate. The organic extract was washed with brine, dried over Na₂SO₄, filtered, concentrated and purified by flash column chromatography, eluting with a gradient of 10 % to 80 % ethyl acetate in petroleum ether to provide (R)-4-bromo-N-(4-cyclopropyl-6-(2-methylmorpholino)pyridin-2-yl)-2-(6-azaspiro[2.5]octan-6-yl)benzamide (0.50 g, 0.94 mmol, 58% yield) as white sticky solid. ¹H NMR (400 MHz, DMSO-*d*₆): δ ppm 12.67 (s, 1H), 8.00 (d, *J*=8.5 Hz, 1 H), 7.63 (s, 1 H), 7.53 (d, *J*=8.5 Hz, 1 H), 7.34 (s, 1 H), 6.35 (s, 1 H), 4.01 - 4.18 (m, 3 H), 3.90 (dd, *J*=11.1, 3.1 Hz, 1 H), 3.51 - 3.63 (m, 2 H), 3.01 (t, *J*=5.5 Hz, 4H), 2.78 (td, *J*=12.3, 3.5 Hz, 1 H), 1.87 (td, *J*=8.5, 4.3 Hz, 1 H), 1.69 (br s, 4 H), 1.16 (d, *J*=6.3 Hz, 3 H), 0.98 - 1.04 (m, 2 H), 0.72 - 0.80 (m, 2 H), 0.35 (s, 4 H). *m*/*z* (ESI): 525.1 (M+H)⁺.

Step 6: A mixture of (*R*)-4-bromo-N-(4-cyclopropyl-6-(2-methylmorpholino)pyridin-2-yl)-2-(6-azaspiro[2.5]octan-6-yl)benzamide (0.30 g, 0.57 mmol), 2-hydroxyethane-1-sulfonamide (0.107 g, 0.856 mmol), tripotassium phosphate (0.303 g, 1.43mmol), (1*R*,2*R*)-*N*1,*N*2-dimethylcyclohexane-1,2-diamine (0.041 g, 0.28 mmol) and copper(I) iodide (0.109 g, 0.571 mmol) in N, N-dimethylformamide (9 mL) was stirred at 90 °C for 16 h. The reaction mixture was quenched with water and was extracted with ethyl acetate. The organic extract was washed with brine, dried over Na₂SO₄, filtered, concentrated, and purified by flash column chromatography, eluting with a gradient of 20 % to 80 % ethyl acetate in petroleum ether to provide (R)-N-(4-cyclopropyl-6-(2-methylmorpholino)pyridin-2-yl)-4-((2-hydroxyethyl)sulfonamido)-2-(6-azaspiro[2.5]octan-6-yl)benzamide (0.155 g, 0.272 mmol, 48% yield) as white solid. ¹H NMR (400 MHz, DMSO-*d*₆): δ ppm 12.73 (s, 1 H), 8.04 (dd, *J*=8.7, 2.9 Hz, 1 H), 7.37 (d, *J*=2.8 Hz, 1 H), 7.23 (t, *J*=2.6 Hz, 1 H), 7.10 (dt, *J*=8.7, 2.6 Hz, 1 H), 6.34 (d, *J*=2.7 Hz, 1 H), 4.14 (d, *J*=12.9 Hz, 1 H), 4.10 (d, J--12.9 Hz, 1 H), 3.91 (d, *J*=11.3 Hz, 1 H), 3.76 (t, .7=6.5 Hz, 2 H), 3.53 - 3.61 (m, 2 H), 3.01 (t, *J*=5.5 Hz, 4 H), 2.79 (td, *J*=9.2, 4.6 Hz, 1 H), 2.42 - 2.48 (m, 2 H), 1.82 - 1.92 (m, 2 H), 1.68 (br s, 4 H), 1.18 (d, *J*=6.2 Hz, 3 H), 1.00 (d, *J*=8.2 Hz, 2 H), 0.77 (d, *J*=5.2 Hz, 2 H), 0.37 (s, 4 H). *m*/*z* (ESI): 570.2 (M+H)⁺.

### Example 20: (R)-4-((2-Hydroxyethyl)sulfonamido)-N-(6-(2-methylmorpholino)-4-(trifluoromethyl)pyridin-2-yl)-2-(6-azaspiro[2.5]octan-6-yl)benzamide

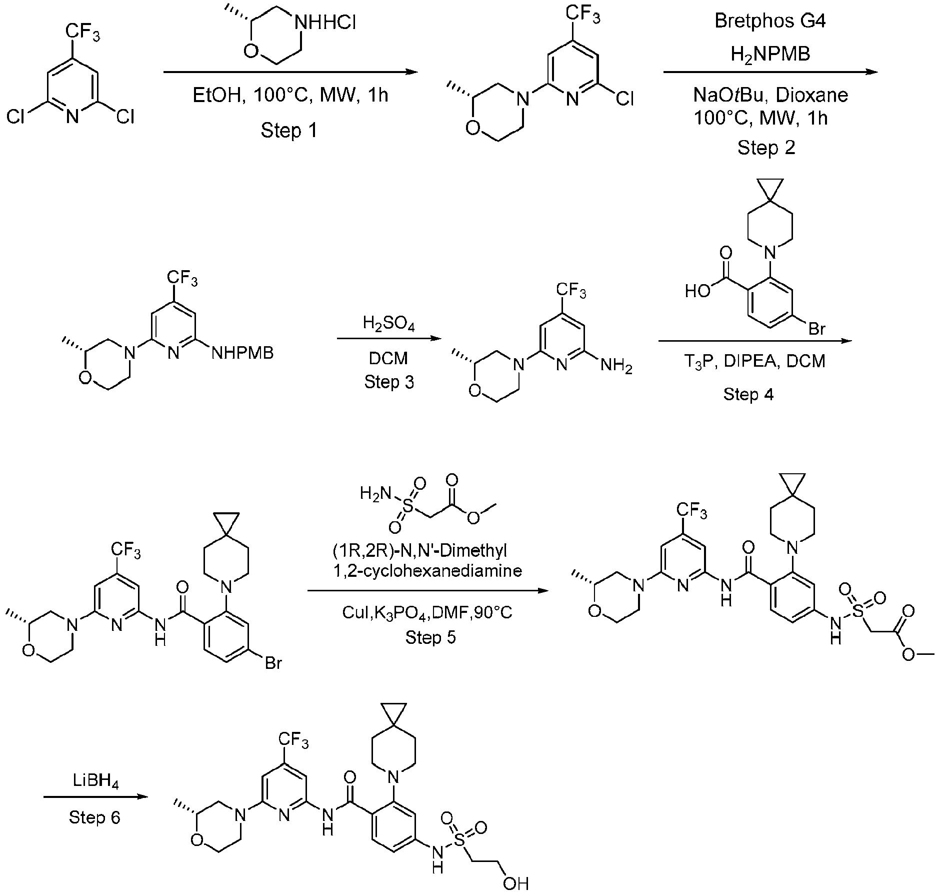

Step 1: A mixture of 2,6-dichloro-4-(trifluoromethyl)pyridine (6.0 g, 28 mmol, Combi-Blocks), (*R*)-2-methylmorpholine hydrochloride (4.59 g, 33.3 mmol), DIPEA (9.70 mL, 55.6 mmol) in ethanol (30 mL) was heated at 100 °C in a microwave for 1 h. The reaction mixture was concentrated and purified by flash column chromatography eluting with a gradient of 0 % to 20 % ethyl acetate in petroleum ether to provide (*R*)-4-(6-chloro-4-(trifluoromethyl)pyridin-2-yl)-2-methylmorpholine (6.0 g, 21 mmol, 77 % yield) as off white solid. ¹H NMR (400 MHz, DMSO-*d*₆): δ ppm 7.11 (s, 1 H), 6.97 (s, 1 H), 4.02 - 4.25 (m, 2 H), 3.76 - 3.97 (m, 1 H), 3.39 - 3.63 (m, 2 H), 2.92 (td, *J*=3.5, 12.5 Hz, 1 H), 2.60 (dd, *J*=10.4, 13.0 Hz, 1 H), 1.15 (d, *J*=6.2 Hz, 3 H). *m*/*z* (ESI): 281.1 (M+H)⁺.

Step 2: A mixture of (R)-4-(6-chloro-4-(trifluoromethyl)pyridin-2-yl)-2-methylmorpholine (2.0 g, 7.1 mmol), (4-methoxyphenyl)methanamine (1.173 g, 8.55 mmol) and DIPEA (2.489 mL, 14.25 mmol) in NMP (10 mL) was stirred at 180° C in a microwave for 1 h. The reaction mixture was diluted with water and extracted with ethyl acetate. The organic extract was washed with brine, dried over Na₂SO₄, filtered, concentrated and purified by flash column chromatography, eluting with a gradient of 0 % to 25 % ethyl acetate in petroleum ether to provide (R)-N-(4-methoxybenzyl)-6-(2-methylmorpholino)-4-(trifluoromethyl)pyridin-2-amine (1.5 g, 2.8 mmol, 39 % yield) as colorless oil. ¹H NMR (400 MHz, DMSO-*d*₆): δ ppm 7.25 (dd, *J*=4.7, 7.1 Hz, 2 H), 6.76 - 6.98 (m, 2 H), 6.05 (d, *J*=6.4 Hz, 2 H), 4.37 (d, *J*=5.8 Hz, 2 H), 3.89 - 4.15 (m, 2 H), 3.75 - 3.95 (m, 1 H), 3.73 (s, 3 H), 3.38 - 3.60 (m, 2 H), 2.63 - 2.89 (m, 1 H), 2.41 (dd, *J*=10.3, 12.7 Hz, 1 H), 1.22 (d, *J*=6.2 Hz 3 H). *m*/*z* (ESI): 382.1 (M+H)⁺.

Step 3: A mixture of (R)-N-(4-methoxybenzyl)-6-(2-methylmorpholino)-4-(trifluoromethyl)pyridin-2-amine (1.5 g, 3.9 mmol) and H₂SO₄ (2.096 mL, 39.3 mmol) in dichloromethane (50 mL) was stirred at room temperature for 5 h. The reaction mixture was concentrated, and the residue was dissolved in ice cold water, basified with 10% NaOH (pH 10) and extracted with EtOAc. The organic extract was washed with brine, dried over Na₂SO₄, filtered, concentrated, and purified by reverse phase column chromatography, eluting with a gradient of 0 % to 80 % acetonitrile in water to provide (*R*)-6-(2-methylmorpholino)-4-(trifluoromethyl)pyridin-2-amine (0.30 g, 1.1mmol, 29% yield) as light yellow solid. ¹H NMR (400 MHz, DMSO-*d*₆): δ ppm 6.13 (br s, 2 H), 6.08 (s, 1 H), 5.98 (d, *J*=1.1 Hz, 1 H), 4.05 - 4.16 (m, 1 H), 3.94 - 4.05 (m, 1 H), 3.87 (ddd, *J*=1.4, 3.6, 11.5 Hz, 1 H), 3.41-3.59 (m, 2 H), 2.74 (ddd, *J*=3.5, 11.8, 12.8 Hz, 1 H), 2.42 (dd, *J*=10.4, 12.8 Hz, 1 H), 1.13 (d, *J*=6.2 Hz, 3 H). *m*/*z* (ESI): 262.1 (M+H)⁺.

Step 4: A mixture of 4-bromo-2-(6-azaspiro[2.5]octan-6-yl)benzoic acid (0.20 g, 0.64 mmol, Intermediate 9-1), (*R*)-6-(2-methylmorphofino)-4-(trifluoromethyl)pyridin-2-amine (0.253 g, 0.967 mmol), DIPEA (0.338 mL, 1.93 mmol) and T₃P (50% in EtOAc, 1.231 g, 1.934 mmol) in dichloromethane (5 mL) was stirred at room temperature for 16 h. The reaction mixture was diluted with water and was extracted with ethyl acetate. The organic extract was washed with brine, dried over Na₂SO₄, filtered, concentrated and purified by flash column chromatography eluting with a gradient of 0 % to 30 % ethyl acetate in petroleum ether to provide (*R*)-4-bromo-*N*-(6-(2-methylmorpholino)-4-(trifluoromethyl)pyridin-2-yl)-2-(6-azaspiro[2.5]octan-6-yl)benzamide (0.30 g, 0.54 mmol, 84 % yield) as white solid. ¹H NMR (400 MHz, DMSO-*d*₆): δ ppm 13.20 (s, 1 H), 8.04 (d, *J*=8.4 Hz, 1 H), 7.82 (s, 1 H), 7.71 (d, *J*=2.0 Hz, 1 H), 7.57 (dd, *J*=1.9, 8.5 Hz, 1 H), 6.92 (s, 1 H), 4.24 (dd, *J*=12.9, 21.6 Hz, 2H), 3.94 (d, *J*=11.4 Hz, 1H), 3.33 - 3.72 (m, 2 H), 3.06 (s, 4 H), 2.52 (m, 1 H), 2.93 (t, *J*=11.6 Hz, 1 H), 1.70 (s, 4 H), 1.38 (d, *J*=5.1 Hz, 3 H), 0.38 (s, 4H). *m*/*z* (ESI): 553.1, 555.1 (M+H)⁺.

Step 5: A mixture of (R)-4-bromo-A-(6-(2-methylmorpholino)-4-(trifluoromethyl)pyridin-2-yl)-2-(6-azaspiro[2.5]octan-6-yl)benzamide (0.30 g, 0.54 mmol), methyl 2-sulfamoylacetate (0.125 g, 0.813 mmol), potassium phosphate (0.230 g, 1.08 mmol), (1*R*,2*R*)-*N*1,*N*2-dimethylcyclohexane-1,2-diamine (0.039 g, 0.27 mmol) and copper(I) iodide (0.103 g, 0.542 mmol) in N, N-dimethylformamide (5 mL) was stirred at 90 °C for 16 h. The reaction mixture was diluted with water and was extracted with ethyl acetate. The organic extract was washed with brine, dried over Na₂SO₄, filtered, concentrated and purified by flash column chromatography eluting with a gradient of 0 % to 40 % ethyl acetate in petroleum ether to provide methyl (*R*)-2-(*N*-(4-((6-(2-methylmorpholino)-4-(trifluoromethyl)pyridin-2-yl)carbamoyl)-3-(6-azaspiro[2.5]octan-6-yl)phenyl)sulfamoyl)acetate (0.25 g, 0.40 mmol, 74% yield) as off white solid. ¹H NMR (400 MHz, DMSO-*d*₆): δ ppm 13.21 (s, 1 H), 10.69 (s, 1 H), 8.04 (d, *J*=86 Hz, 1 H), 7.77 (s, 1 H), 7.25 (d, *J*=2.2 Hz, 1 H), 7.12 (dd, *J*=2.1, 8.7 Hz, 1 H), 6.82 (s, 1 H), 4.45 (s, 2 H), 4.02 - 4.27 (m, 2 H), 3.90 (dd, *J*=3.4, 11.5 Hz, 1 H), 3.51 - 3.65 (m, 4 H), 2.91- 3.02 (m, 4 H), 2.55 - 2.64 (m, 1 H), 1.96 (s, 2 H), 1.66 (s, 4 H), 1.15 (dd, *J*=4.3, 6.7 Hz, 3 H), 0.34 (s, 4 H). *m*/*z* (ESI): 626.1 (M+H)⁺.

Step 6: To a solution of methyl (*R*)-2-(*N-*(4-((6-(2-methylmorpholino)-4-(trifluoromethyl)pyridin-2-yl)carbamoyl)-3-(6-azaspiro[2.5]octan-6-yl)phenyl)sulfamoyl)acetate (0.25 g, 0.40 mmol) in THF (5 mL) was added LiBH₄ (2.0M in THF, 0.599 mL, 1.20 mmol) at -78 °C and stirred at room temperature for 1 h. The reaction mixture was quenched with a saturated aqueous solution of NH₄Cl and was extracted with EtOAc. The organic extract was washed with brine, dried over Na₂SO₄, filtered, concentrated and purified by reverse phase column chromatography, eluting with a gradient of 0 % to 60 % acetonitrile and water to provide (*R*)-4-((2-hydroxyethyl)sulfonamido)-*N-*(6-(2-methylmorpholino)-4-(trifluoromethyl)pyridin-2-yl)-2-(6-azaspiro[2.5]octan-6-yl)benzamide (0.10 g, 0.17 mmol, 42% yield) as white solid. ¹H NMR(400 MHz, DMSO-*d*₆): δ ppm 13.22 (s, 1 H), 10.23 (s, 1 H), 8.07 (d, *J*=86 Hz, 1 H), 7.83 (s, 1 H), 7.27 (d, *J*=2.2 Hz, 1 H), 7.13 (dd, *J*=2.1, 8.7 Hz, 1 H), 6.88 (s, 1 H), 4.94 (s, 1 H), 4.14 - 4.34 (m, 2 H), 3.93 (d, *J*=10.7 Hz, 1 H), 3.76 (t, *J*=6.5 Hz, 2 H), 3.57 (t, *J*=11.4 Hz, 2 H), 3.36 (t, *J*=6.4 Hz, 2 H), 2.86 - 3.06 (m, 5 H), 2.60 (m, 1 H), 1.71 (s, 4 H), 1.18 (d, *J*=6.2 Hz, 3 H), 0.38 (s, 4 H). m/z (ESI): 598.2 (M+H)⁺.

### Example 21: N-(5-Cyano-6-(4,4-difluoropiperidin-1-yl)pyridin-2-yl)-4-((2-hydroxyethyl)sulfonamido)-2-(6-azaspiro[2.5]octan-6-yl)benzamide

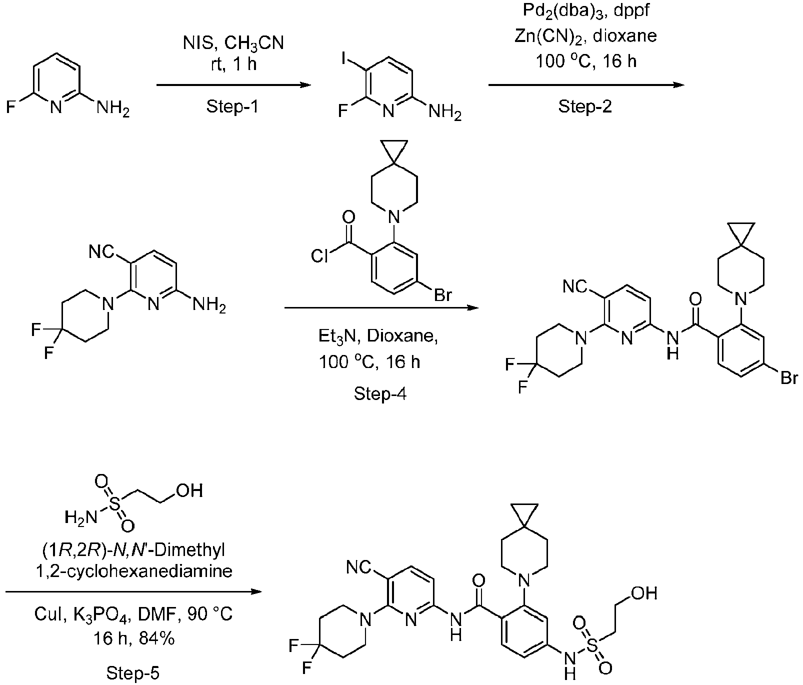

Step 1: A mixture of 6-fluoropyridin-2-amine (5.0 g, 45 mmol, Combi-Blocks) and N-iodosuccinimide (10.5 g, 46.8 mmol) in acetonitrile (50 mL) was stirred at 10 °C for 30 min and then at room temperature for 1 h. The reaction mixture was concentrated, and the residue was diluted with water. The precipitated solid was filtered and purified by flash column chromatography eluting with 0 % to 30 % EtOAc in petroleum ether to provide 6-fluoro-5-iodopyridin-2-amine (8.9 g, 37mmol, 84 % yield) as brown solid. ¹H NMR (300 MHz, DMSO-*d*₆): δ ppm 7.74 (t, *J*=8.8 Hz, 1 H), 6.53 (s, 2 H), 6.19 (dd, *J*=8.3, 2.3 Hz, 1 H). *m*/*z* (ESI): 238.9 (M+H)⁺.

Step 2: A mixture of 6-fluoro-5-iodopyridin-2-amine (1.0 g, 4.2 mmol), Zn(CN)₂ (0.296 g, 2.52 mmol), Pd₂(dba)₃ (0.192 g, 0.210 mmol) and dppf (0.233 g, 0.420 mmol) in dioxane (9 mL) and water (1 mL) was stirred at 100°C for 16 h. The reaction mixture was filtered through a celite bed and the filtrate was washed with water, brine, dried over anhydrous Na₂SO₄, filtered, and concentrated. The concentrate was purified by flash column chromatography using 0 % to 50 % ethyl acetate in petroleum ether to provide 6-amino-2-fluoronicotinonitrile (0.51 g, 3.7 mmol, 89 % yield) as brown solid. ¹H NMR (300 MHz, DMSO-*d*₆): δ ppm 7.81 (t, *J*=9.1 Hz, 1 H), 7.64 (s, 1 H), 7.51 (s, 2 H). *m*/*z* (ESI): 138.1 (M+H)⁺.

Step 3: A mixture of 6-amino-2-fluoronicotinonitrile (0.50 g, 3.6 mmol), 4,4-difluoropiperidine hydrochloride (1.322 g, 8.39 mmol) and DIPEA (5.10 mL, 29.2 mmol) in DMSO (5 mL) was stirred at 145 °C for 16 h. The reaction mixture was concentrated and purified by flash column chromatography, eluting with 15 % to 25 % EtOAc in petroleum ether to provide 6-amino-2-(4,4-difluoropiperidin-1-yl)nicotinonitrile (0.60 g, 2.5 mmol, 69.1 % yield) as yellow solid. ¹H NMR (400 MHz, DMSO-*d*₆): δ ppm 7.52 (d, *J*=8.4 Hz, 1 H), 6.82 (s, 2 H), 5.99 (d, *J*=8.5 Hz, 1 H), 3.60 - 3.68 (m, 4 H), 2.00 - 2.10 (m, 4 H). *m*/*z* (ESI): 239.1 (M+H)⁺.

Step 4: A mixture of 4-bromo-2-(6-azaspiro[2.5]octan-6-yl)benzoic acid (0.3 g, 0.967 mmol, Intermediate 9-1), oxalyl chloride (0.127 mL, 1.451 mmol) and DMF (a drop) in dichloromethane (5 mL) was stirred at 0 °C for 2 h. The reaction mixture was concentrated, and the residue was dissolved in dioxane (10 mL). A solution of 6-amino-2-(4,4-difluoropiperidin-1-yl)nicotinonitrile (0.230 g, 0.967 mmol) and triethylamine (0.404 mL, 2.90 mmol) in 1,4-dioxane (3 mL) was added to the above solution and the resulting mixture was stirred at 100 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 5 % to 15 % ethyl acetate in petroleum ether to provide 4-bromo-N (5-cyano-6-(4,4-difluoropiperidin-1-yl)pyridin-2-yl)-2-(6-azaspiro[2.5]octan-6-yl)benzamide (0.19 g, 0.36 mmol, 37% yield) as off white solid. ¹H NMR (400 MHz, DMSO-*d*₆): δ ppm 13.40 (s, 1 H), 8.14 (d, *J*=8.4 Hz, 1 H), 8.03 (d, *J*=8.5 Hz, 1 H), 7.84 (d, *J*=8.5 Hz, 1 H), 7.73 (d, *J*=2.0 Hz, 1 H), 7.58 (dd, *J*=8.5, 1.9 Hz, 1 H), 3.76 - 3.83 (m, 4 H), 3.06 (t, *J*=5.3 Hz, 4 H), 2.08 - 2.20 (m, 4 H), 1.68 (br s, 4 H), 0.39 (s, 4 H). *m*/*z* (ESI): 532.1 (M+H)⁺.

Step 5: A mixture of 4-bromo-*N*-(5-cyano-6-(4,4-difluoropiperidin-1-yl)pyridin-2-yl)-2-(6-azaspiro[2.5]octan-6-yl)benzamide (0.19 g, 0.36 mmol), 2-hydroxyethane-1-sulfonamide (0.067 g, 0.54 mmol), potassium phosphate tribasic (0.152 g, 0.716 mmol), (1*R*,2*R*)-*N*1,*N*2-dimethylcyclohexane-1,2-diamine (0.025 g, 0.18 mmol) and copper(I) iodide (0.068 g, 0.36 mmol) in N, N-dimethylformamide (3 mL) was stirred at 90 °C for 16 h. The reaction mixture was diluted with water and was extracted with ethyl acetate. The organic extract was washed with brine, dried over Na₂SO₄, filtered, concentrated, and purified by preparative HPLC using 0.1 % TFA in acetonitrile and water to provide *N*-(5-cyano-6-(4,4-diffuoropiperidin-1-yl)pyridin-2-yl)-4-((2-hydroxyethyl)sulfonamido)-2-(6-azaspiro[2.5]octan-6-yl)benzamide 2,2,2-trifluoroacetate (0.040 g, 0.058 mmol, 16% yield) as white solid. ¹H NMR (400 MHz, DMSO-*d*₆): δ ppm 13.45 (s, 1 H), 10.28 (s, 1 H), 8.09 (dd, *J*=12.7, 8.5 Hz, 2 H), 7.84 (d, *J*=8.4 Hz, 1 H), 7.28 (d, *J*=2.2 Hz, 1 H), 7.14 (dd, *J*=8.7, 2.1 Hz, 1 H), 4.95 (br s, 1 H), 3.72 - 3.82 (m, 6 H), 3.37 (t, *J*=6.4 Hz, 2 H), 2.92 - 3.00 (m, 4 H), 2.08 - 2.18 (m, 4 H), 1.70 (br s, 4 H), 0.39 (s, 4 H). *m*/*z* (ESI): 575.2 (M+H)⁺.

### Example 22: N-(6-(4,4-Difluoropiperidin-1-yl)-5-methylpyridin-2-yl)-4-((2-hydroxyethyl)sulfonamido)-2-(6-azaspiro[2.5]octan-6-yl)benzamide

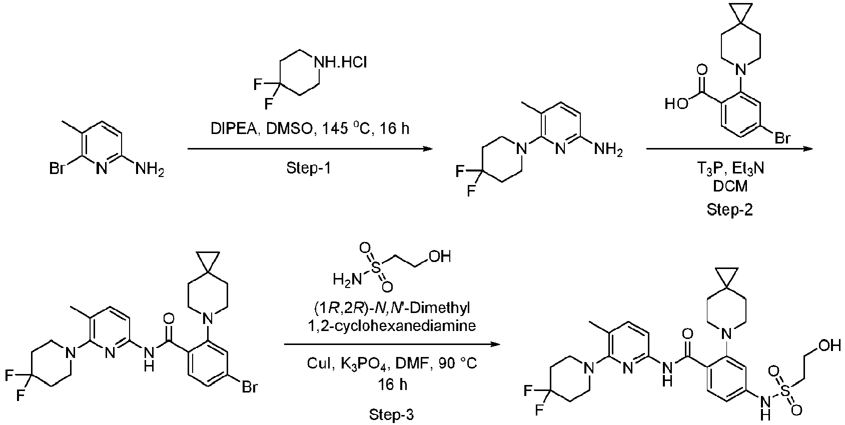

Step 1: A mixture of 6-bromo-5-methylpyridin-2-amine (650 mg, 3.48 mmol, Sibian chemicals), 4,4-difluoropiperidine hydrochloride (1643 mg, 10.43 mmol, Combi-Blocks) and DIPEA (3035 µL, 17.38 mmol) in NMP (6.5 mL) was heated at 180 °C for 8 h. The reaction mixture was diluted with water and was extracted with ethyl acetate. The organic extract was washed with brine, dried over Na₂SO₄, filtered, concentrated and purified by flash column chromatography, eluting with a gradient of 0 % to 30 % ethyl acetate in petroleum ether to provide 6-(4,4-difluoropiperidin-1-yl)-5-methylpyridin-2-amine (350 mg, 1.54 mmol, 44.3 % yield) as pale yellow gum. ¹H NMR (300 MHz, DMSO-*d*₆): δ ppm 7.12 (dd, *J*=7.9, 2.3 Hz, 1 H), 6.05 (dd, *J*=8.0, 2.2 Hz, 1 H), 5.47 (s, 2 H), 3.08 (q, *J*=3.9, 2.5 Hz, 4 H), 1.26 - 2.48 (m, 7 H). *m*/*z* (ESI): 228.1 (M+H)⁺.

Step 2: A mixture of 4-bromo-2-(6-azaspiro[2.5]octan-6-yl)benzoic acid (573 mg, 1.85 mmol, Intermediate 9-1), triethylamine (644 µL, 4.62 mmol), T₃P (50% in ethyl acetate, 1.47 g, 2.31 mmol) and 6-(4,4-difluoropiperidin-1-yl)-5-methylpyridin-2-amine (350 mg, 1.54 mmol) in DCM (5 mL) was stirred at room temperature for 16 h. The reaction mixture was diluted with water and was extracted with ethyl acetate. The organic extract was washed with brine, dried over Na₂SO₄, filtered, concentrated and purified by flash column chromatography, eluting with a gradient of 0 % to 15 % ethyl acetate in petroleum ether to provide 4-bromo-*N*-(6-(4,4-difluoropiperidin-1-yl)-5-methylpyridin-2-yl)-2-(6-azaspiro[2.5]octan-6-yl)benzamide (500 mg, 0.96 mmol, 62.5 % yield) as pale yellow solid.

¹H NMR (400 MHz, DMSO-*d*₆): δ ppm 13.01 (s, 1 H), 8.04 (d, *J*=86 Hz, 1 H), 7.85 (d, *J*=8.0 Hz, 1 H), 7.67 (d, *J*=2.0 Hz, 1 H), 7.15 - 7.66 (m, 2 H), 3.09 - 3.32 (m, 4 H), 3.04 (t, *J*=5.3 Hz, 4 H), 2.24 (s, 3H), 1.91 - 2.23 (m, 4 H), 1.21- 1.99 (m, 4 H), 0.38 (s, 4 H). *m*/*z* (ESI): 519.1 (M+H)⁺.

Step 3: A mixture of 4-bromo-*N*-(6-(4,4-difluoropiperidin-1-yl)-5-methylpyridin-2-yl)-2-(6-azaspiro[2.5]octan-6-yl)benzamide (200 mg, 0.385 mmol), 2-hydroxyethane-1-sulfonamide (72.3 mg, 0.578 mmol), potassium phosphate tribasic (163 mg, 0.770 mmol), (1*R*,2*R*)-*N*,*N*-dimethyl-1,2-cyclohexanediamine (27.4 mg, 0.193 mmol) and copper(I) iodide (73.3 mg, 0.385 mmol) in *N,N-*dimethylformamide (2 mL) was stirred at 100 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 0 % to 30 % ethyl acetate in petroleum ether to provide *N*-(6-(4,4-difluoropiperidin-1-yl)-5-methylpyridin-2-yl)-4-((2-hydroxyethyl)sulfonamido)-2-(6-azaspiro[2.5]octan-6-yl)benzamide ( 100 mg, 0.177 mmol, 46.1 % yield) as off white solid. ¹H NMR (400 MHz, DMSO-*d*₆): δ ppm 13.03 (s, 1 H), 10.21 (br s, 1 H), 8.07 (d, *J* = 8.6 Hz, 1 H), 7.86 (d, *J* = 8.0 Hz, 1 H), 7.57 (d, *J*=8.1 Hz, 1 H), 7.26 (d, *J*=2.2 Hz, 1 H), 7.12 (dd, *J*=8.7, 2.1 Hz, 1 H), 4.95 (br s, 1 H), 3.74 - 4.80 (m, 2 H), 3.36 - 3.40 (m, 2 H), 3.20 - 3.28 (m, 4 H), 2.92 - 3.02 (m, 4 H), 2.24 (s, 3 H), 2.09 - 2.16 (m, 4 H), 1.70 - 1.90 (m, 4 H), 0.38 (s, 4 H). *m*/*z* (ESI): 564.2 (M+H)⁺.

### Example 23: N-(6-(4,4-difluorocyclohexyl)-4-methylpyridin-2-yl)-4-((2-hydroxyethyl)sulfonamido)-2-(6-azaspiro[2.5]octan-6-yl)benzamide

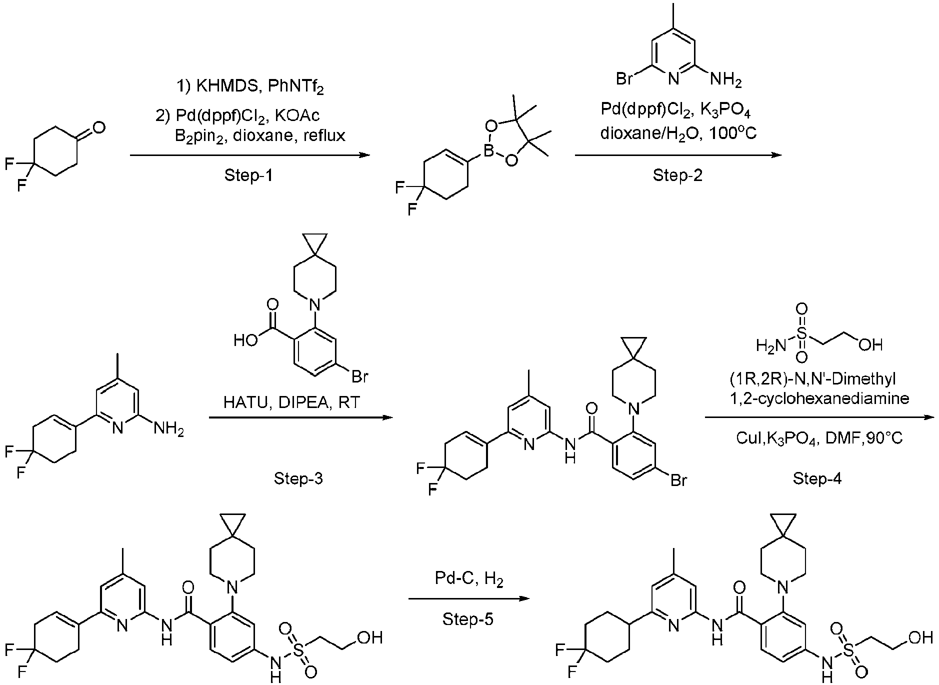

Step 1: To a solution of 4,4-difluorocyclohexan-1-one (1.0 g, 7.5 mmol, Combi-Blocks) in THF (10 mL) was added LiHMDS (1. 0 M in THF, 8.95 mL, 8.95 mmol) at -78°C. The reaction mixture was stirred for 1 h before 1,1,1-trifluoro-*N*-phenyl-*N*-((trifluoromethyl)sulfonyl)methanesulfonamide (2.93 g, 8.20 mmol) was added at -78°C and the resulting solution was slowly warmed to room temperature and stirred for 16 h. The reaction mixture was quenched with water and extracted with ethyl acetate. The organic extract was washed with brine, dried over Na₂SO₄, filtered, and concentrated. To a solution of the residue in 1,4-dioxane (20 mL) were added potassium acetate (1.46 g, 14.91 mmol), bispinacolato diboron (2.272 g, 8.95 mmol) and PdCl₂(dppf) (0.546 g, 0.746 mmol). The reaction mixture was stirred at 100°C for 16 h before it was diluted with water and was extracted with ethyl acetate. The organic extract was washed with brine, dried over Na₂SO₄, filtered, concentrated and purified by flash column chromatography, eluting with a gradient of 10 % to 20 % ethyl acetate in petroleum ether to provide 2-(4,4-difluorocyclohex-1-en-1-yl)-4,4,5,5-tetramethyl-1,3,2-dioxaborolane (900 mg, 3.69 mmol, 49.5 % yield) as yellow oil. ¹H NMR (400 MHz, Chloroform-*d*): δ ppm 6.41 (t, *J*=3.7 Hz, 1 H), 2.53 - 2.68 (m, 2 H), 2.43 (t, *J*=6.7 Hz, 2 H), 1.91 - 2.06 (m, 2 H), 1.29 (s, 12 H).

Step 2: A mixture of 6-bromo-4-methylpyridin-2-amine (800 mg, 4.28 mmol), 2-(4,4-diffuorocyclohex-1-en-1-yl)-4,4,5,5-tetramethyl-1,3,2-dioxaborolane (1.148 g, 4.70 mmol), potassium phosphate tribasic (1907 mg, 8.98 mmol) and PdCl₂(dppf)-CH₂Cl₂ adduct (349 mg, 0.428 mmol) in 1,4-dioxane (9 mL) and water (3 mL) was stirred at 100 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 20 % to 30 % ethyl acetate in petroleum ether to provide 6-(4,4-difluorocyclohex-1-en-1-yl)-4-methylpyridin-2-amine (800 mg, 3.57 mmol, 83 % yield) as brown gummy solid. ¹H NMR (400 MHz, DMSO-*d*₆): δ ppm 6.49 (d, *J*=2.4 Hz, 1 H), 6.41 (dt, *J*=5.0, 3.0 Hz, 1 H), 6.18 (s, 1 H), 5.73 (s, 2 H), 2.59 - 2.77 (m, 4 H), 2.12 (s, 3 H), 2.0 - 2.10 (m, 2 H). *m*/*z* (ESI): 225.1 (M+H)⁺.

Step 3: A mixture of 4-bromo-2-(6-azaspiro[2.5]octan-6-yl)benzoic acid (200 mg, 0.645 mmol, Intermediate 9-1), DIPEA (338 µL, 1.93 mmol), HATU (368 mg, 0.967 mmol) and 6-(4,4-difluorocyclohex-1-en-1-yl)-4-methylpyridin-2-amine (217 mg, 0.967 mmol) in DMF (5 mL) was stirred at 100 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 10 % to 20 % ethyl acetate in petroleum ether to provide 4-bromo-*N*-(6-(4,4-difluorocyclohex-1-en-1-yl)-4-methylpyridin-2-yl)-2-(6-azaspiro[2.5]octan-6-yl)benzamide (150 mg, 0.290 mmol, 45.0 % yield) as grey solid. ¹H NMR (300 MHz, Chloroform-*d*): δ ppm 13.21 (s, 1 H), 8.09 - 8.26 (m, 2 H), 7.37 - 7.55 (m, 2 H), 7.04 (t, *J*=1.0 Hz, 1 H), 6.52 - 6.60 (m, 1 H), 3.10 (t, *J*=5.4 Hz, 4 H), 2.74 - 2.90 (Wm, 4 H), 2.40 (s, 3 H), 2.14 - 2.28 (m, 2 H), 1.70 - 1.90 (m, 4 H), 0.43 (s, 4 H). *m*/*z* (ESI): 516.1 (M+H)⁺.

Step 4: A mixture of 4-bromo-*N*-(6-(4,4-difluorocyclohex-1-en-1-yl)-4-methylpyridin-2-yl)-2-(6-azaspiro[2.5]octan-6-yl)benzamide (150 mg, 0.290 mmol), 2-hydroxyethane-1-sulfonamide (54 mg, 0.436 mmol), potassium phosphate tribasic (154 mg, 0.726 mmol), (1*R*,2*R*)-*N*1,*N*2-dimethylcyclohexane-1,2-diamine (21 mg, 0.145 mmol) and copper(I) iodide (55.3 mg, 0.290 mmol) in DMF (5 mL) was stirred at 100 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 30 % to 40 % ethyl acetate in petroleum ether to provide *N*-(6-(4,4-difluorocyclohex-1-en-1-yl)-4-methylpyridin-2-yl)-4-((2-hydroxyethyl)sulfonamido)-2-(6-azaspiro[2.5]octan-6-yl)benzamide (150 mg, 0.268 mmol, 92 % yield) as yellow oil. ¹H NMR (400 MHz, DMSO-*d*₆): δ ppm 13.35 (s, 1 H), 8.02 - 8.12 (m, 2 H), 7.96 (s, 1 H), 7.27 (s, 1 H), 7.22 (s, 1 H), 7.13 (d, *J*=8.9 Hz, 1 H), 663 (m, 1 H), 3.76 (d, *J*=6.6 Hz, 2 H), 2.99 (s, 2 H), 2.90 (s, 4 H), 2.81 (s, 1 H), 2.74 (s, 1 H), 2.47 (d, *J*=2.4 Hz, 2 H), 2.37 (s, 2 H), 2.19 (s, 3 H), 1.70 - 1.90 (m, 4 H), 0.40 (s, 4 H). *m*/*z* (ESI): 561.2 (M+H)⁺.

Step 5: A mixture of *N*-(6-(4,4-difluorocyclohex-1-en-1-yl)-4-methylpyridin-2-yl)-4-((2-hydroxyethyl)sulfonamido)-2-(6-azaspiro[2.5]octan-6-yl)benzamide (100 mg, 0.178 mmol) and 10% Pd/C (50 mg, 0.047 mmol) in ethanol (5 mL) was stirred at room temperature under hydrogen atmosphere (14 psi) for 6 h. The reaction mixture was filtered through a celite bed. The filtrate was concentrated and the residue was triturated with diethyl ether and hexanes to provide *N*-(6-(4,4-difluorocyclohexyl)-4-methylpyridin-2-yl)-4-((2-hydroxyethyl)sulfonamido)-2-(6-azaspiro[2.5]octan-6-yl)benzamide (10.6 mg, 0.019 mmol, 1011 % yield) as off white solid. ¹H NMR (400 MHz, DMSO-*d*₆): δ ppm 13.28 (s, 1 H), 7.98 (t, *J*=6.0 Hz, 2 H), 7.15 (d, *J*=2.2 Hz, 1 H), 6.98 - 7.05 (m, 1 H), 6.86 (s, 1 H), 3.73 (t, *J*=6.6 Hz, 2 H), 3.24 (t, *J*=6.6 Hz, 2 H), 2.95 (d, *J*=5.5 Hz, 4 H), 2.72 - 2.80 (m, 1 H), 2.31 (s, 3 H), 1.90 - 2.12 (m, 8 H), 1.70 - 1.84 (m, 4 H), 0.36 (s, 4 H). *m*/*z* (ESI): 563.2 (M+H)⁺.

### Example 24: (R)-N-(5-fluoro-6-(2-methylmorpholino)pyridin-2-yl)-4-((2-hydroxyethyl)sulfonamido)-2-16-azaspiro[2,5]octan-6-yl)benzamide

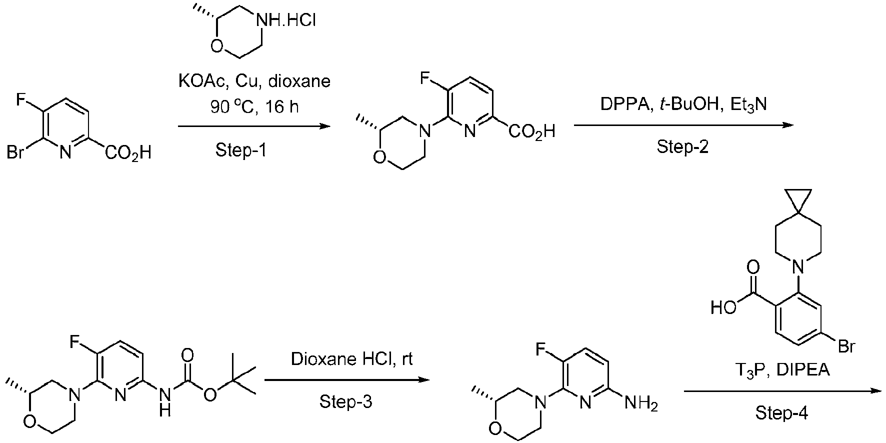

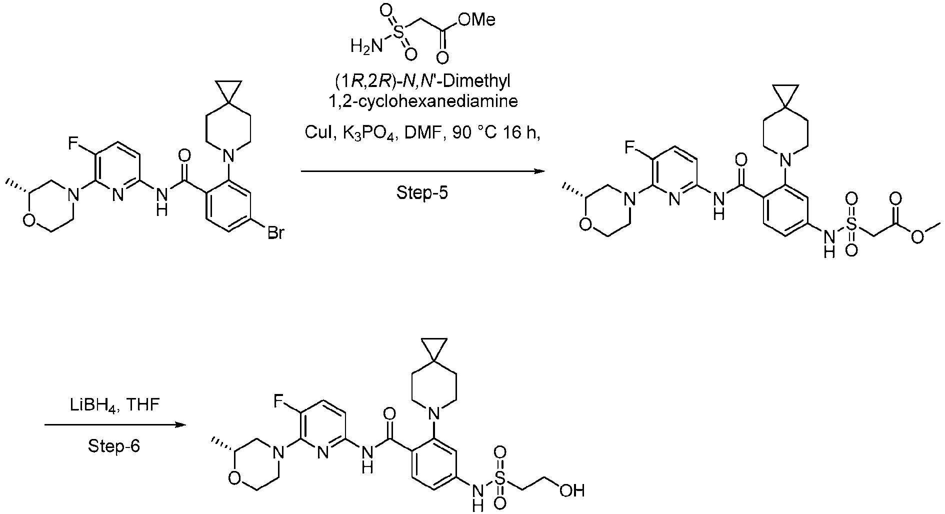

Step 1: A mixture of 6-bromo-5-fluoropicolinic acid (3.0 g, 14 mmol, Combi-Blocks), (*R*)-2-methylmorpholine hydrochloride (2.25 g, 16.36 mmol, Combi-Blocks), potassium acetate (2.94 g, 30.0 mmol) and copper powder (0.867 g, 13.6 mmol) in 1,4-dioxane (15 mL) was stirred at 90 °C for 16 h. The reaction mixture was filtered through a celite bed, diluted with water and was extracted with ethyl acetate. The organic extract was washed with brine, dried over Na₂SO₄, filtered, concentrated and purified by flash column chromatography, eluting with a gradient of 40 % to 75 % ethyl acetate in petroleum ether to provide (*R*)-5-fluoro-6-(2-methylmorpholino)picolinic acid (1.3 g, 5.4 mmol, 40% yield) as light yellow solid.¹H NMR (400 MHz, DMSO-*d*₆) δ ppm 12.91 (b s, 1 H), 7.65 (dd, *J*=13.1, 8.1 Hz, 1 H), 7.54 (dd, *J*=8.0, 3.0 Hz, 1 H), 3.94 (t, *J*=*2.2* Hz, 1 H), 3.80 - 3.92 (m, 2 H), 3.64 (tdd, *J*=11.9*,* 5.7, 2.6 Hz, 2 H), 2.96 (ddd, *J*=12.9, 11.7, 3.4 Hz, 1 H), 2.65 (dd, *J*=12.8, 10.2 Hz, 1 H), 1.15 (d, *J*=6.2 Hz, 3 H). *m*/*z* (ESI): 241.1 (M+H)⁺.

Step 2: A mixture of (*R*)-5-fluoro-6-(2-methylmorpholino)picolinic acid (1.3 g, 5.4 mmol), triethylamine (1.095 g, 10.82 mmol) and diphenyl phosphorazidate (1.787 g, 6.49 mmol) in tert-butanol (13 mL) was stirred at 90 °C for 16 h. The reaction mixture was concentrated and purified by flash column chromatography, eluting with a gradient of 0 % to 30 % ethyl acetate in petroleum ether to provide *tert*-butyl (*R*)-(5-fluoro-6-(2-methylmorpholino)pyridin-2-yl)carbamate (1.2 g, 3.8 mmol, 71% yield) as pale yellow solid. ¹H NMR (300 MHz, DMSO-*d*₆) δ ppm 9.91 (s, 1 H), 7.40 - 7.58 (m, 1 H), 7.20 (d, *J*=19.6 Hz, 1 H), 3.82 - 3.94 (m, 1 H), 3.67 - 3.80 (m, 2 H), 3.58 - 3.69 (m, 2 H), 2.83 - 3.09 (m, 1 H), 2.63 (dt, .7=12.2, 9.5 Hz, 1 H), 1.20 (d, *J*=18.9 Hz, 9 H), 1.12 (d, *J*=6.2 Hz, 3 H). *m*/*z* (ESI): 312.1 (M+H)⁺.

Step 3: To a solution of *tert*-butyl (R)-(5-fluoro-6-(2-methylmorpholino)pyridin-2-yl)carbamate (1.2 g, 3.8 mmol) in dichloromethane (12 mL) was added hydrochloric acid in 1,4-dioxane (10 mL, 20mmol) at 0° C and stirred at room temperature for 4 h. The reaction mixture was concentrated, and the residue was diluted with ice water (50 mL), basified (pH 9) with an aqueous 10% sodium bicarbonate solution, and extracted with ethyl acetate. The organic extract was washed with brine, dried over Na₂SO₄, filtered, concentrated, and purified by flash column chromatography, eluting with a gradient of 40 % to 60 % ethyl acetate in petroleum ether to provide (*R*)-5-fluoro-6-(2-methylmorpholino)pyridin-2-amine (0.63 g, 3.0 mmol, 77 % yield) as brown solid. ¹H NMR (400 MHz, DMSO-*d*₆) δ ppm 7.28 - 7.43 (m, 1 H), 7.18 (t, *J*=7.7 Hz, 1 H), 6.07 (dd, *J*=8.5, 2.1 Hz, 2H), 3.85 (dd, *J*=11.5, 3.1 Hz, 1 H), 3.60 - 3.83 (m, 2 H), 3.58 (dd, *J*=11.7, 2.7 Hz, 2 H), 2.92 (td, *J*=12.2, 3.3 Hz, 1 H), 2.61 (dd, *J*=12.7, 10.2 Hz, 1 H), 1.11 (d, *J*=6.1 Hz, 3 H). *m*/*z* (ESI): 212.1 (M+H)⁺.

Step 4: To a solution of 4-bromo-2-(6-azaspiro[2.5]octan-6-yl)benzoic acid (1.057 g, 3.41 mmol, Intermediate 9-1) in CH₂Cl₂(10 mL) were added DIPEA (1.101 g, 8.52 mmol), T₃P (2.71 g, 4.26 mmol, 50% in EtOAc) and (R)-5-fluoro-6-(2-methylmorpholino)pyridin-2-amine (0.60 g, 2.8 mmol) at room temperature and stirred for 16 h. The reaction mixture was diluted with water and was extracted with CH₂Cl₂. The organic extract was washed with brine, dried over Na₂SO₄, filtered, and concentrated. The concentrate was purified by flash column chromatography using a gradient of 60 % to 80% ethyl acetate in petroleum ether to afford (*R*)-4-bromo-*N*-(5-fluoro-6-(2-methylmorpholino)pyridin-2-yl)-2-(6-azaspiro[2.5]octan-6-yl)benzamide (0.38 g, 0.76 mmol, 27 % yield) as a pale brown solid. ¹H NMR (400 MHz, DMSO-*d*₆) δ ppm 13.01 (s, 1 H), 8.04 (d, *J*=8.4 Hz, 1 H), 7.75 (dd, *J*=8.5, 2.5 Hz, 1 H), 7.69 (d, *J*=1.9 Hz, 1 H), 7.44 - 7.66 (m, 2 H), 3.89 (d, *J*=13.1 Hz, 1 H), 3.83 (d, *J*=11.7 Hz, 2 H), 3.67 (t, *J*=10.9 Hz, 2 H), 3.05 (d, *J*=6.1 Hz, 4 H), 2.95 (t, *J*=10.8 Hz, 1 H), 2.60 - 2.68 (m, 1 H), 1.70 (s, 4 H), 1.16 (d, *J*=6.2 Hz, 3 H), 0.39 (s, 4 H). *m*/*z* (ESI): 504.1 (M+H)⁺.

Step 5: A mixture of (*R*)-4-bromo-N-(5-fluoro-6-(2-methylmorpholino)pyridin-2-yl)-2-(6-azaspiro[2.5]octan-6-yl)benzamide (380 mg, 0.755 mmol), methyl 2-sulfamoylacetate (139 mg, 0.906 mmol), potassium phosphate (320 mg, 1.51 mmol), (1*R*,2*R*)-*N*1,*N*2-dimethylcyclohexane-1,2-diamine (129 mg, 0.906 mmol) and copper(I) iodide (28.8 mg, 0.151 mmol) in N, N-dimethylformamide (3.8 mL) was stirred at 90 °C for 6 h. The reaction mixture was filtered through a celite bed, diluted with water and was extracted with ethyl acetate. The organic extract was washed with brine, dried over Na₂SO₄, filtered, concentrated and purified by flash column chromatography, eluting with a gradient of 40 % to 70 % ethyl acetate in petroleum ether to provide methyl (*R*)-2-(*N*-(4-((5-fluoro-6-(2-methylmorpholino)pyridin-2-yl)carbamoyl)-3-(6-azaspiro[2.5]octan-6-yl)phenyl)sulfamoyl)acetate (200 mg, 0.347 mmol, 46.0 % yield) pale yellow gummy liquid. ¹H NMR (400 MHz, DMSO-*d*₆) δ ppm 13.03 (d, *J*=4.4 Hz, 1 H), 10.66 (s, 1H), 8.09 (d, *J*=86 Hz, 1 H), 7.71 - 7.95 (m, 1 H), 7.58 (dd, *J*=12.8, 8.6 Hz, 1 H), 7.28 (dd, *J*=11.2, 2.2 Hz, 1 H), 7.14 (ddd, *J*=7.3, 5.5, 1.9 Hz, 1 H), 4.41 (s, 1 H), 4.03 (q, *J*=7.1 Hz, 2 H), 3.80 - 3.97 (m, 3 H), 3.65-3.67 (m, 2 H), 3.60 (s, 3 H), 2.93 - 2.99 (m, 3 H), 1.99 (s, 2 H), 1.73 (s, 4 H), 1.16 (d, *J*=5.8 Hz, 3 H), 0.40 (s, 4 H). *m*/*z* (ESI): 576.2 (M+H)⁺.

Step 6: To a solution of methyl (*R*)-2-(N-(4-((5-fluoro-6-(2-methylmorpholino)pyridin-2-yl)carbamoyl)-3-(6-azaspiro[2.5]octan-6-yl)phenyl)sulfamoyl)acetate (200 mg, 0.347 mmol) in THF (4 mL) was added lithium borohydride (2.0 M in THF, 0.521 mL, 1.04 mmol) at 0 °C and stirred at room temperature for 2 h. The reaction mixture was diluted with ice cold water and was extracted with ethyl acetate. The organic extract was washed with brine, dried over Na₂SO₄, filtered, concentrated and purified by flash column chromatography, eluting with a gradient of 40 % to 80 % ethyl acetate in petroleum ether to provide (*R*)-*N*-(5-fluoro-6-(2-methylmorpholino)pyridin-2-yl)-4-((2-hydroxyethyl)sulfonamido)-2-(6-azaspiro[2.5]octan-6-yl)benzamide (70 mg, 0.13 mmol, 37% yield) as off white solid. ¹H NMR (400 MHz, DMSO-*d*₆) δ ppm 12.76 - 13.48 (m, 1 H), 10.22 (s, 1 H), 7.88 - 8.42 (m, 1 H), 7.76 (dt, *J*=9.3, 4.6 Hz, 1 H), 7.57 (ddt, *J*=12.9, 9.1, 3.9 Hz, 1 H), 7.27 (d, *J*=6.1 Hz, 1 H), 7.13 (q, *J*=7.8, 5.2 Hz, 1 H), 4.95 (s, 1 H), 3.91 (s, 1 H), 3.79-3.81 (m, 4 H), 3.68 (s, 4 H), 2.91 - 2.97 (m, 5 H), 2.64 (d, *J*=9.0 Hz, 1 H), 1.86 (br s, 4 H), 0.95 - 1.31 (m, 3 H), 0.38 (br s, 4 H). *m*/*z* (ESI): 548.2 (M+H)⁺.

### Example 25: (R)-N-(4-Cyano-6-(2-methylmorpholino)pyridin-2-yl)-4-((2-hydroxyethyl)sulfonamido)-2-(6-azaspiro[2.5]octan-6-yl)benzamide

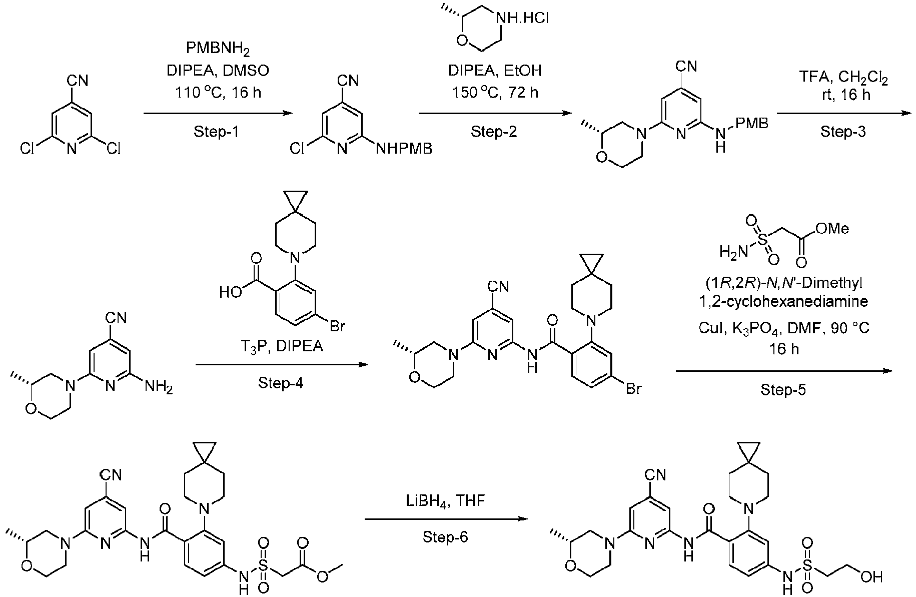

Step 1: A mixture of 2,6-dichloroisonicotinonitrile (3.0 g, 17mmol, Combi-Blocks), (4-methoxyphenyl)methanamine (2.379 g, 17.34 mmol) and DIPEA (3.03 mL, 17.34 mmol) in dimethyl sulfoxide (30 mL) was stirred at 110 °C for 16 h. The reaction mixture was diluted with water and was extracted with ethyl acetate. The organic extract was washed with brine, dried over Na₂SO₄, filtered, concentrated and purified by flash column chromatography, eluting with a gradient of 0 % to 20 % ethyl acetate in petroleum ether to provide 2-chloro-6-((4-methoxybenzyl)amino)isonicotinonitrile (3.8 g, 14 mmol, 80 % yield) as yellow solid. ¹H NMR (400 MHz, DMSO-*d*₆): δ ppm 7.96 - 8.02 (m, 1 H), 7.22 - 7.30 (m, 2 H), 6.94, (s, 1 H), 6.86 - 6.92 (m, 2 H), 6.84 (s, 1 H), 4.36 (d, *J*=5.8 Hz, 2 H), 3.72 (s, 3 H). *m*/*z* (ESI): 274.1 (M+H)⁺.

Step 2: A mixture of 2-chloro-6-((4-methoxybenzyl) amino) isonicotinonitrile (1.85 g, 6.76 mmol), (*R*)-2-methylmorpholine hydrochloride (2.139 g, 15.55 mmol, Combi-Blocks) and DIPEA (9.44 mL, 54.1 mmol) in ethanol (20 mL) was stirred at 150 °C for 76 h. The reaction mixture was concentrated and was purified by flash column chromatography, eluting with a gradient of 0 % to 10 % ethyl acetate in petroleum ether to provide (*R*)-2-((4-methoxybenzyl)amino)-6-(2-methylmorpholino)isonicotinonitrile (0.59 g, 1.7mmol, 26 % yield) as yellow oil. ¹H NMR (400 MHz, DMSO-*d*₆): δ ppm 7.28 (br s, 1 H), 7.22 - 7.24 (m, 2 H), 6.83 - 6.91 (m, 2 H), 6.21 (s, 1 H), 6.06 (s, 1 H), 4.35 (d, *J*=5.9 Hz, 2 H), 3.97 - 4.10 (m, 2 H), 3.80 - 3.88 (m, 1 H), 3.72 (s, 3 H), 3.39 - 3.52 (m, 2 H), 2.75 (td, *J*=12.3, 3.5 Hz, 1 H), 2.42 (dd, *J*=12.8, 10.4 Hz, 1 H), 1.12 (d, *J*=6.2 Hz, 3 H). *m*/*z* (ESI): 339.2 (M+H)⁺.

Step 3: A mixture of (*R*)-2-((4-methoxybenzyl)amino)-6-(2-methylmorpholino)isonicotinonitrile (0.59 g, 1.7 mmol) and TFA (2.821 mL, 36.61 mmol) in dichloromethane (6 mL) was stirred at room temperature for 16 h. The reaction mixture was concentrated and was neutralized (pH 7) with 10% NaHCO₃ solution and was extracted with dichloromethane. The organic extract was washed with brine, dried over Na₂SO₄, filtered, concentrated and purified by flash column chromatography, eluting with a gradient of 0 % to 20 % ethyl acetate in petroleum ether to provide (*R*)-2-amino-6-(2-methylmorpholino)isonicotinonitrile (0.325 g, 1.49 mmol, 85 % yield) as white solid. ¹H NMR (300 MHz, DMSO-*d*₆): δ ppm 6.23 (s, 1 H), 6.15 (s, 2 H), 5.97 (s, 1 H), 4.07 (dt, *J*=12.7, 2.1 Hz, 1 H), 3.92 - 4.02 (m, 2 H), 3.38 - 3.53 (m, 2 H), 2.72 (td, *J*=12.2, 3.6 Hz, 1 H), 2.34 - 2.42 (m, 1 H), 1.10 (d, *J*=6.2 Hz, 3 H). *m*/*z* (ESI): 219.1 (M+H)⁺.

Step 4: A mixture of 4-bromo-2-(6-azaspiro[2.5]octan-6-yl)benzoic acid (0.25 g, 0.81 mmol, Intermediate 9-1), DIPEA (0.422 mL, 2.42mmol), T₃P (50 % in ethyl acetate, 1.539 g, 2.418 mmol) and (*R*)-2-amino-6-(2-methylmorpholino)isonicotinonitrile (0.211 g, 0.967 mmol) in dichloromethane (4 mL) was stirred at room temperature for 18 h. The reaction mixture was diluted with water and was extracted with dichloromethane. The organic extract was washed with brine, dried over Na₂SO₄, filtered, concentrated and purified by flash column chromatography, eluting with a gradient of 0 % to 20 % ethyl acetate in petroleum ether to provide (*R*)-4-bromo-*N*-(4-cyano-6-(2-methylmorpholino)pyridin-2-yl)-2-(6-azaspiro[2.5]octan-6-yl)benzamide (0.263 g, 0.515 mmol, 63.9 % yield) as white solid. ¹H NMR (400 MHz, DMSO-*d*₆): δ ppm 13.21 (s, 1 H), 8.00 - 8.10 (m, 1 H), 7.76 (s, 1 H), 7.71 (d, *J*=1.9 Hz, 1 H), 7.57 (dd, *J*=8.5, 1.9 Hz, 1 H), 7.13 (s, 1 H), 4.23 (d, *J*=12.8 Hz, 1 H), 4.17 (d, *J*=12.9 Hz, 1 H), 3.93 (d, *J*=9.9 Hz, 1H), 3.50 - 3.61 (m, 2 H), 3.0 - 3.08 (m, 4 H), 2.89 - 2.99 (m, 1 H), 2.58 - 2.64 (m, 1 H), 1.68 (br s, 4 H), 1.17 (d, *J*=6.2 Hz, 3 H), 0.37 (s, 4 H). *m*/*z* (ESI): 512.2 (M+H)⁺.

Step 5: A mixture of (*R*)-4-bromo-*N*-(4-cyano-6-(2-methylmorpholino)pyridin-2-yl)-2-(6-azaspiro[2.5]octan-6-yl)benzamide (260 mg, 0.509 mmol), methyl 2-sulfamoylacetate (156 mg, 1.02 mmol), potassium phosphate tribasic (216 mg, 1.02mmol), copper(I) iodide (194 mg, 1.02 mmol) and (1*R*,2*R*)-*N*,*N*-dimethyl-1,2-cyclohexanediamine (72.4 mg, 0.509 mmol) in DMF (2 mL) was stirred at 90 °C for 16 h. The reaction mixture was filtered through a celite pad. The filtrate was washed with water (15 mL), brine (15 mL), dried over sodium sulfate, filtered, and concentrated. The concentrate was purified by flash column chromatography using a gradient of 40 % EtOAc in petroleum ether to afford methyl (R)-2-(N-(4-((4-cyano-6-(2-methylmorpholino)pyridin-2-yl)carbamoyl)-3-(6-azaspiro[2.5]octan-6-yl)phenyl)sulfamoyl)acetate (250 mg, 0.429 mmol, 84 % yield) as pale yellow gum. *m*/*z* (ESI): 583.2 (M+H)⁺.

Step 6: To a solution of methyl (*R*)-2-(*N*-(4-((4-cyano-6-(2-methylmorpholino)pyridin-2-yl)carbamoyl)-3-(6-azaspiro[2.5]octan-6-yl)phenyl)sulfamoyl)acetate (250 mg, 0.429 mmol) in THF (2 mL) was added LiBH₄ (2.0 M in THF, 429 µl, 0.858 mmol) at -30°C. The mixture as then stirred at 0° C for 30 min. The reaction mixture was quenched with a saturated aqueous solution of NH₄Cl and was extracted with EtOAc. The organic extract was washed with brine, dried over Na₂SO₄, filtered, concentrated and purified by flash column chromatography, eluting with a gradient of 20 % to 50 % ethyl acetate in petroleum ether to provide (R)-N (4-cyano-6-(2-methylmorpholino)pyridin-2-yl)-4-((2-hydroxyethyl)sulfonamido)-2-(6-azaspiro[2.5]octan-6-yl)benzamide (120 mg, 0.216 mmol, 50.4 % yield) as white solid. ¹H NMR (400 MHz, DMSO-*d*₆): δ ppm 13.25 (s, 1 H), 10.23 (br s, 1 H), 8.07 (dd, *J*=8.7, 2.9 Hz, 1 H), 7.78 (d, *J*=2.8 Hz, 1 H), 7.28 (d, *J*=3.1 Hz, 1 H), 7.07 - 7.17 (m, 2 H), 4.96 (br s, 1 H), 4.13 - 4.26 (m, 2 H), 3.93 (d, *J*=11.4 Hz, 1 H), 3.76 (td, *J*=6.6, 2.5 Hz, 2 H), 3.57 (d, *J*=11.7 Hz, 2 H), 3.37 (dd, *J*=6.7, 2.7 Hz, 2 H), 2.88 - 3.02 (m, 5 H), 2.54 - 2.62 (m, 1 H), 1.71 (br s, 4 H), 1.17 (d, *J*=6.3 Hz, 3 H), 0.39 (s, 4 H). *m*/*z* (ESI): 555.2 (M+H)⁺.

### Example 26: 4-((2-Hydroxyethyl)sulfonamido)-N-(6-(2-methylpropan-2-ylsulfonimidoyl)pyridin-2-yl)-2-(6-azaspiro [2.5]octan-6-yl)benzamide

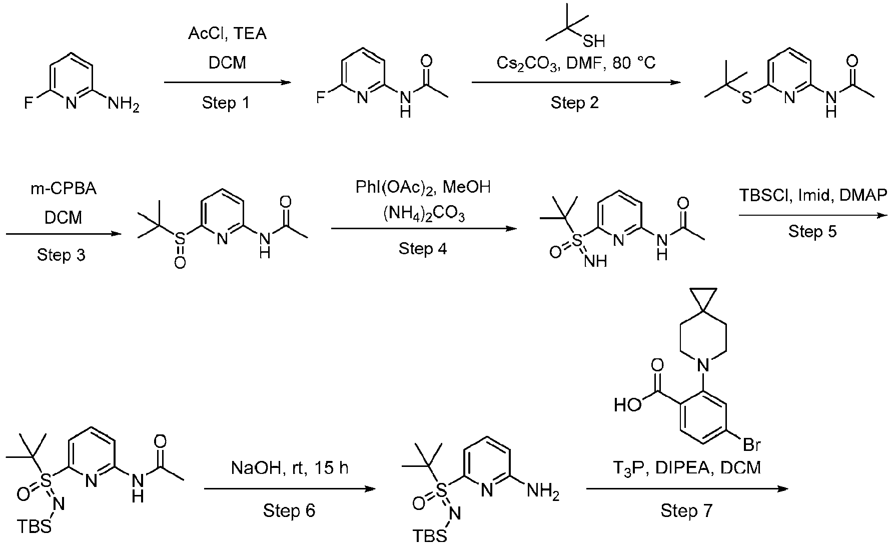

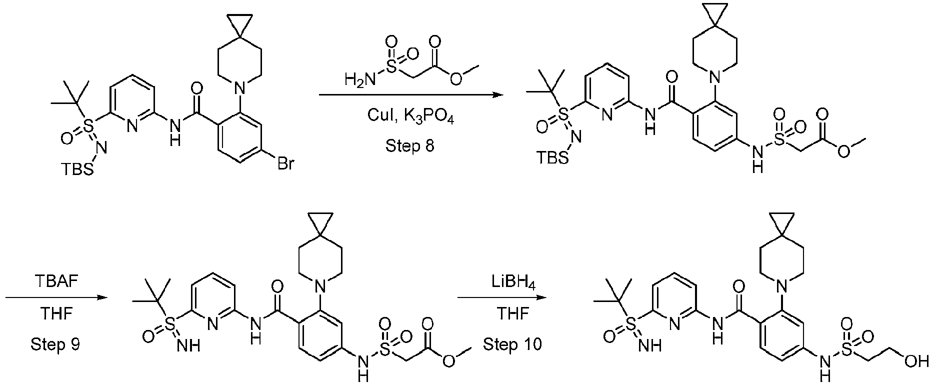

Step 1: To a solution of 6-fluoropyridin-2-amine (5.0 g, 45 mmol, Apollo scientific) in dichloromethane (100 mL) were added Et₃N (15.54 mL, 112 mmol) and acetyl chloride (4.76 mL, 66.9 mmol) at 0 °C. The reaction mixture was stirred at room temperature for 16 h before it was diluted with water and was extracted with dichloromethane. The organic extract was washed with brine, dried over Na₂SO₄, filtered, concentrated and purified by flash column chromatography, eluting with a gradient of 0 % to 20 % ethyl acetate in petroleum ether to provide *N*-(6-fluoropyridin-2-yl)acetamide (4.0 g, 26 mmol, 58% yield) as pale yellow solid. ¹H NMR (400 MHz, DMSO-*d*₆): δ ppm 10.65 (s, 1 H), 7.93 - 8.01 (m, 2 H), 6.29 (d, *J*=2.6 Hz, 1 H), 2.09 (s, 3 H). *m*/*z* (ESI): 155.1 (M+H)⁺.

Step 2: A mixture of *N*-(6-fluoropyridin-2-yl)acetamide (4.0 g, 26 mmol), cesium carbonate (16.91 g, 51.9 mmol) and 2-methylpropane-2-thiol (3.51 g, 38.9 mmol) in DMF (60 mL) was stirred at 80 °C for 24 h. The reaction mixture was quenched with ice cold water and the precipitated solid was filtered, and dried to provide *N*-(6-(*tert*-butylthio)pyridin-2-yl)acetamide (4.0 g, 18 mmol, 69 % yield) as off white solid. ¹H NMR (400 MHz, DMSO-*d*₆): δ ppm 10.34 (s, 1 H), 7.86 (d, *J*=8.5 Hz, 1 H), 7.61 (t, *J*=8.0 Hz, 1 H), 7.02 (d, *J*=7.6 Hz, 1 H), 2.11 (s, 3 H), 1.48 (s, 9 H). *m*/*z* (ESI): 225.1 (M+H)⁺.

Step 3: To a solution of *N*-(6-(*tert*-butylthio)pyridin-2-yl)acetamide (3.5 g, 16 mmol) in dichloromethane (75 mL) was added m-CPBA (75 %, 3.59 g, 15.6 mmol) at 0 °C and stirred at room temperature for 16 h. The reaction mixture was quenched with 10 % sodium bicarbonate and was extracted with dichloromethane. The organic extract was washed with brine, dried over Na₂SO₄, filtered, concentrated and triturated with diethyl ether to provide *N*-(6-(*tert*-butylsulfinyl)pyridin-2-yl)acetamide (3.3 g, 13mmol, 88 % yield) as white solid. ¹H NMR (300 MHz, DMSO-*d*₆): δ ppm 10.68 (s, 1 H), 8.18 (d, *J*=8.3 Hz, 1 H), 8.03 (t, *J*=8.0 Hz, 1 H), 7.52 (d, *J*=7.5 Hz, 1 H), 2.12 (s, 3 H), 1.15 (s, 9 H). *m*/*z* (ESI): 241.1 (M+H)⁺.

Step 4: A mixture of *N*-(6-(*tert*-butylsulfinyl)pyridin-2-yl)acetamide (3.3 g, 13mmol), iodobenzene diacetate (17.69 g, 54.9 mmol) and ammonium carbonate (5.36 g, 68.7 mmol) in methanol (75 mL) was stirred at room temperature for 24 h. The reaction mixture was concentrated and the residue treated with water and extracted with ethyl acetate. The organic extract was washed with brine, dried over Na₂SO₄, filtered, concentrated, and triturated with diethyl ether to provide *N*-(6-(2-methylpropan-2-ylsulfonimidoyl)pyridin-2-yl)acetamide (2.1 g, 8.2 mmol, 60 % yield) as off white solid. ¹H NMR (400 MHz, DMSO-*d*₆): δ ppm 10.82 (s, 1 H), 8.29 (d, *J*=8.4 Hz, 1 H), 8.03 (t, *J*=8.0 Hz, 1 H), 7.75 (dd, *J*=7.6, 0.9 Hz, 1H), 4.11 (s, 1 H), 2.14 (s, 3 H), 1.29 (s, 9 H).

Step 5: A solution ofN (6-(2-methylpropan-2-ylsulfonimidoyl)pyridin-2-yl)acetamide (2.24 g, 8.77 mmol) in CH₂Cl₂ (50 mL), imidazole (1.194 g, 17.55 mmol), DMAP (0.536 g, 4.39 mmol) and TBS-Cl (1.587 g, 10.53 mmol) was stirred at room temperature for 2.5 h. The reaction mixture was quenched with cold water and was extracted with CH₂Cl₂. The organic extract was washed with water, brine, dried over Na₂SO₄, filtered, and concentrated. The concentrate was purified by flash column chromatography using a gradient of 15 % EtOAc in petroleum ether to afford N-(6-(N-(tert-butyldimethylsilyl)-2-methylpropan-2-ylsulfonimidoyl)pyridin-2-yl)acetamide (2.7 g, 7.3 mmol, 83 % yield) as white solid. ¹H NMR (400 MHz, DMSO-*d*₆): δ ppm 10.70 (s, 1 H), 8.27 (d, *J*=8.4 Hz, 1 H), 8.01 - 8.07 (m, 1 H), 7.68 (d, *J*=7.6 Hz, 1 H), 2.15 (s, 3 H), 1.28 (s, 9 H), 0.86 (s, 9 H), -0.07 (d, 3 H), -0.11 (d, 3 H). *m*/*z* (ESI): 256 (M-2^{t}Bu)⁺.

Step 6: A mixture of *N*-(6-(*N*-(*tert*-butyldimethylsilyl)-2-methylpropan-2-ylsulfonimidoyl)pyridin-2-yl)acetamide (1.5 g, 4.1 mmol) and aqueous sodium hydroxide (2.5 M, 32.5 mL, 81 mmol) solution in methanol (30 mL) was stirred at room temperature for 15 h. The reaction mixture was concentrated, and the residue was treated with water. The precipitated solid was filtered, and dried to provide (6-aminopyridin-2-yl)(*tert*-butyl)((*tert*-butyldimethylsilyl)imino)-λ⁶-sulfanone (1.2 g, 3.7 mmol, 90 % yield) as white solid. ¹H NMR(400 MHz, DMSO-*d*₆): δ ppm 7.58 (t, *J*=7.8 Hz, 1 H), 7.09 (d, *J*=7.3 Hz, 1 H), 6.60 (d, *J*=8.3 Hz, 1 H), 6.35 (s, 2H), 1.26 (s, 9 H), 0.85 (s, 9 H), -0.07 (s, 3 H), -0.13 (s, 3 H). *m*/*z* (ESI): 214.1 (M-2^{t}Bu)⁺.

Step 7: A mixture of 4-bromo-2-(6-azaspiro[2.5]octan-6-yl)benzoic acid (0.50 g, 1.6 mmol, Intermediate 9-1), DIPEA (0.845 mL, 4.84 mmol), T₃P (50 % solution in ethyl acetate, 3.08 g, 4.84 mmol) and (6-aminopyridin-2-yl)(*tert*-butyl)((*tert*-butyldimethylsilyl)imino)- λ⁶-sulfanone (0.634 g, 1.93 mmol) in dichloromethane (10 mL) was stirred at room temperature for 48 h. The reaction mixture was diluted with water and was extracted with dichloromethane. The organic extract was washed with brine, dried over Na₂SO₄, filtered, concentrated and purified by flash column chromatography, eluting with a gradient of 0 % to 15 % ethyl acetate in petroleum ether to provide 4-bromo-*N*-(6-(*N*-(*tert*-butyldimethylsilyl)-2-methylpropan-2-ylsulfonimidoyl)pyridin-2-yl)-2-(6-azaspiro[2.5]octan-6-yl)benzamide (0.20 g, 0.32 mmol, 20% yield) as pale yellow gum. ¹H NMR (400 MHz, DMSO-*d*₆): δ ppm 12.86 (s, 1 H), 8.51 (d, *J*=8.4 Hz, 1 H), 8.13 (t, *J*=8.0 Hz, 1 H), 8.00 (d, *J*=8.4 Hz, 1 H), 7.77 (d, *J*=7.6 Hz, 1 H), 7.68 (d, *J*=1.8 Hz, 1H), 7.55 (dd, *J*=8.5, 1.8 Hz, 1 H), 2.97 - 3.10 (m, 4 H), 1.60 (br s, 4 H), 1.33 (s, 9 H), 0.82 (s, 9 H), 0.33 (s, 4 H), -0.12 (s, 3 H). -0.14 (s, 3 H). *m*/*z* (ESI): 507.0 (M-2^{t}Bu)⁺.

Step 8: A mixture of 4-bromo-*N*-(6-(*N*-(*tert-*butyldimethylsilyl)-2-methylpropan-2-ylsulfonimidoyl)pyridin-2-yl)-2-(6-azaspiro[2.5]octan-6-yl)benzamide (0.20 g, 0.32 mmol), methyl 2-sulfamoylacetate (0.074 g, 0.48 mmol), potassium phosphate tribasic (0.137 g, 0.645 mmol), copper(I) iodide (0.061 g, 0.32 mmol) and (1*R*,2*R*)-*N*,*N*-dimethyl-1,2-cyclohexanediamine (0.023 g, 0.16 mmol) in DMF (4 mL) was stirred at 90 °C for 16 h. The reaction mixture was diluted with water and was extracted with ethyl acetate. The organic extract was washed with brine, dried over Na₂SO₄, filtered, and concentrated to provide methyl 2-(*N-*(4-((6-(*N-*(*tert*-butyldimethylsilyl)-2-methylpropan-2-ylsulfonimidoyl)pyridin-2-yl)carbamoyl)-3-(6-azaspiro[2.5]octan-6-yl)phenyl)sulfamoyl)acetate (0.18 g, 0.26 mmol, 81 % yield) as pale yellow gum. *m*/*z* (ESI): 692.2 (M+H)⁺.

Step 9: A mixture of methyl 2-(*N*-(4-((6-(*N*-(tert-butyldimethylsilyl)-2-methylpropan-2-ylsulfonimidoyl)pyridin-2-yl)carbamoyl)-3-(6-azaspiro[2.5]octan-6-yl)phenyl)sulfamoyl)acetate (0.18 g, 0.260 mmol) and tetrabutylammonium fluoride (1 M solution in THF, 0.390 mL, 0.390 mmol) in THF (4 mL) was stirred at room temperature for 3 h. The reaction mixture was quenched with a saturated aqueous ammonium chloride solution and was extracted with ethyl acetate. The organic extract was washed with brine, dried over Na₂SO₄, filtered, and concentrated to provide methyl 2-(*N*-(4-((6-(2-methylpropan-2-ylsulfonimidoyl)pyridin-2-yl)carbamoyl)-3-(6-azaspiro[2.5]octan-6-yl)phenyl)sulfamoyl)acetate (0.10 g, 0.17 mmol, 66% yield) as pale yellow gum. *m*/*z* (ESI): 578.1 (M+H)⁺.

Step 10: To a solution of methyl 2-(N-(4-((6-(2-methylpropan-2-ylsulfonimidoyl)pyridin-2-yl)carbamoyl)-3-(6-azaspiro[2.5]octan-6-yl)phenyl)sulfamoyl)acetate (0.10 g, 0.17 mmol) in THF (3 mL) was added LiBH₄ (2.0 M solution in THF, 0.173 mL, 0.346 mmol) at 0 °C and stirred at room temperature for 1.5 h. The reaction mixture was quenched with a saturated aqueous ammonium chloride solution and was extracted with ethyl acetate. The organic extract was washed with brine, dried over Na₂SO₄, filtered, concentrated and purified by preparative HPLC to provide 4-((2-hydroxyethyl)sulfonamido)-*N*-(6-(2-methylpropan-2-ylsulfonimidoyl)pyridin-2-yl)-2-(6-azaspiro[2.5]octan-6-yl)benzamide (0.016 g, 0.029 mmol, 16 % yield) as white solid. ¹H NMR (400 MHz, DMSO-*d*₆): δ ppm 13.16 (s, 1 H), 10.26 (s, 1 H), 8.53 (d, *J* = 8.4 Hz, 1 H), 8.17 - 8.05 (m, 2 H), 7.82 (dd, *J* = 7.5, 0.9 Hz, 1 H), 7.29 (d, *J* = 2.2 Hz, 1 H), 7.14 (dd, *J* = 8.6, 2.1 Hz, 1 H), 4.95 (br s, 1 H), 4.04 (s, 1 H), 3.76 - 3.80 (m, 2 H), 3.37 (t, *J* = 6.5 Hz, 2 H), 2.99 (t, *J* = 5.4 Hz, 4 H), 1.60 - 1.70 (m, 3 H), 1.35 (s, 9 H), 0.36 (s, 4 H). *m*/*z* (ESI): 550.2 (M+H)⁺.

### Example 27-1 and 27-2: 2-(6-Azaspiro[2.5]octan-6-yl)-4-(S-cyclopropylsulfonimidoyl)-N-(6-(3,3,3-trifluoropropoxy)-2-pyridinyl)benzamide and 2-(6-Azaspiro[2.5]octan-6-yl)-4-(R-cyclopropylsulfonimidoyl)-N-(6-(3,3,3-trifluoropropoxy)-2-pyridinyl)

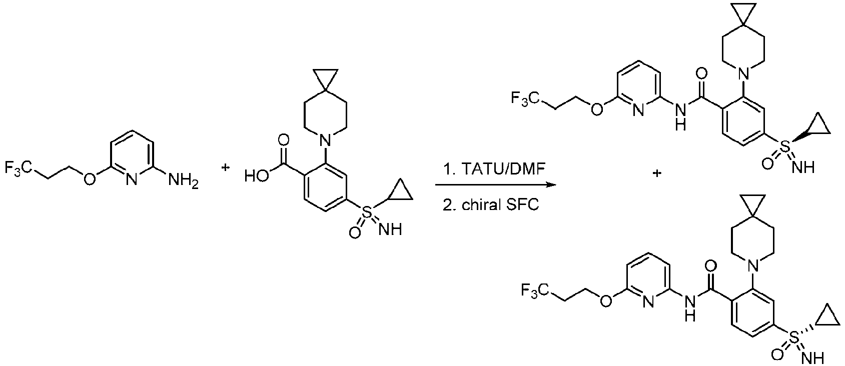

A mixture of 4-(cyclopropanesulfonimidoyl)-2-(6-azaspiro[2.5]octan-6-yl)benzoic acid (0.200 g, 0.598 mmol, Intermediate 16), 6-(3,3,3-trifluoropropoxy)pyridin-2-amine (0.160 g, 0.78 mmol, Intermediate 6), TATU (0.250 g, 0.776 mmol, Combi-Blocks), DMF (6 mL), and DIPEA (0.309 g, 0.426 mL, 2.39 mmol, Aldrich) was stirred at room temperature for 18 h. The mixture was diluted with saturated Na₂CO₃ and EtOAc. The organic phase was washed with Na₂CO₃, water and brine, dried over Na₂SO₄ and concentrated in vacuo. The crude was purified by silica gel chromatography: 0-100% EtOAc in heptane. The product was obtained as off-white solid. *m*/*z* (ESI): 523.1 (M+H)⁺. The racemic mixture was purified by chiral SFC using an Regis (S,S) Whelk-01 (250 X 21 mm, 5mm) with a mobile phase of 65% Liquid CO₂ and 35% MeOH using a flowrate of 80 mL/min to generate:

Example 27-1: 2-(6-Azaspiro[2.5]octan-6-yl)-4-(*S*-cyclopropylsulfonimidoyl)-*N*-(6-(3,3,3-trifluoropropoxy)-2-pyridinyl)benzamide. First eluting peak (68 mg, ee>99%). ¹H NMR (400 MHz, CHLOROFORM-d) δ 12.92 (s, 1H), 8.45 (d, *J*=8.29 Hz, 1H), 8.00 (d, *J*=7.67 Hz, 1H), 7.95 (d, *J*=1.66 Hz, 1H), 7.85 (dd, *J*=1.76, 8.19 Hz, 1H), 7.66 (t, *J*=7.88 Hz, 1H), 6.55 (d, *J*=8.09 Hz, 1H), 4.54 (t, *J*=6.63 Hz, 2H), 3.15 (t, *J*=5.29 Hz, 4H), 2.55-2.72 (m, 3H), 1.66-1.98 (m, 5H), 1.46 (tdd, *J*=5.05, 6.87, 10.18 Hz, 1H), 1.24 (tdd, *J*=4.90, 6.95, 10.21 Hz, 1H), 1.13 (dq, *J*=5.18, 7.95 Hz, 1H), 0.94-1.04 (m, 1H), 0.42 (s, 4H). ¹⁹F NMR (377 MHz, CHLOROFORM-d) δ -64.71 (s, 3F).

Example 27-2:2-(6-Azaspiro[2.5]octan-6-yl)-4-(*R*-cyclopropylsulfonimidoyl)-*N*-(6-(3,3,3-trifluoropropoxy)-2-pyridinyl). Second eluting peak (67 mg, ee 99.3%). ¹H NMR (400 MHz, CHLOROFORM-d) δ 12.92 (s, 1H), 8.45 (d, *J*=8.29 Hz, 1H), 8.00 (d, *J*=7.88 Hz, 1H), 7.95 (d, *J*=1.66 Hz, 1H), 7.85 (dd, *J*=1.66, 8.29 Hz, 1H), 7.66 (t, *J*=7.98 Hz, 1H), 6.55 (d, *J*=8.09 Hz, 1H), 4.54 (t, *J*=6.74 Hz, 2H), 3.15 (t, *J*=5.29 Hz, 4H), 2.54-2.73 (m, 3H), 1.69-1.80 (m, 4H), 1.41-1.49 (m, 1H), 1.19-1.29 (m, 2H), 1.08-1.17 (m, 1H), 0.98 (dq, *J*=5.29, 7.98 Hz, 1H), 0.42 (s, 4H). ¹⁹F NMR (376 MHz, CHLOROFORM-d) δ -64.70 (s, 3F).

The stereochemistry was arbitrarily assigned.

**Table 16: Examples 18-1 to 18-3 were prepared following a similar procedure as described for Example 27-1 and 27-2**

| Ex. # | Chemical Structure | Name | LRMS: (ESI + ve ion) *m*/*z* |
|---|---|---|---|
| 28-1 | 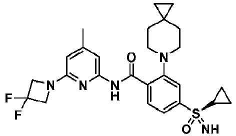 | 2-(6-Azaspiro[2.5]octan-6-yl)-4-(*S-*cyclopropylsulfonimidoyl)-*N*-(6-(3,3-difluoro-1-azetidinyl)-4-methyl-2-pyridinyl)benzamide | 516.2 |
| 28-2 | 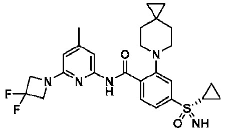 | 2-(6-Azaspiro[2.5]octan-6-yl)-4-(R-cyclopropylsulfonimidoyl)-*N*-(6-(3,3-difluoro-1-azetidinyl)-4-methyl-2-pyridinyl)benzamide | 516.2 |
| 29-1 | 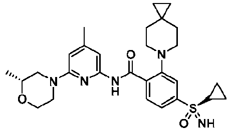 | 2-(6-Azaspiro[2.5]octan-6-yl)-4-(*S-*cyclopropylsulfonimidoyl)-*N*-(4-methyl-6-((2*R*)-2-methyl-4-morpholinyl)-2-pyridinyl)benzamide | 524.3 |
| 29-2 | 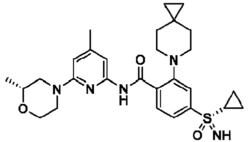 | 2-(6-Azaspiro[2.5]octan-6-yl)-4-(*R-*cyclopropylsulfonimidoyl)-*N*-(4-methyl-6-((2*R*)-2-methyl-4-morpholinyl)-2-pyridinyl)benzamide | 524.3 |
| 30-1 | 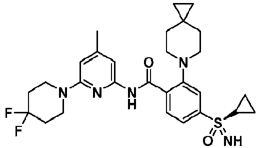 | 2-(6-Azaspiro[2.5]octan-6-yl)-4-(*S-*cyclopropylsulfonimidoyl)-*N*-(6-(4,4-difluoro-1-piperidinyl)-4-methyl-2-pyridinyl)benzamide | 544.3 |
| 30-2 | 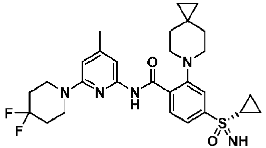 | 2-(6-Azaspiro[2.5]octan-6-yl)-4-(*R-*cyclopropylsulfonimidoyl)-*N*-(6-(4,4-difluoro-1-piperidinyl)-4-methyl-2-pyridinyl)benzamide | 544.3 |

### Example 31: 4-(Azetidin-3-ylsulfonyl)-2-(6-azaspiro[2.5]octan-6-yl)-N-(6-(3,3,3-trifluoropropoxy)pyridin-2-yl)benzamide trifluoroacetate

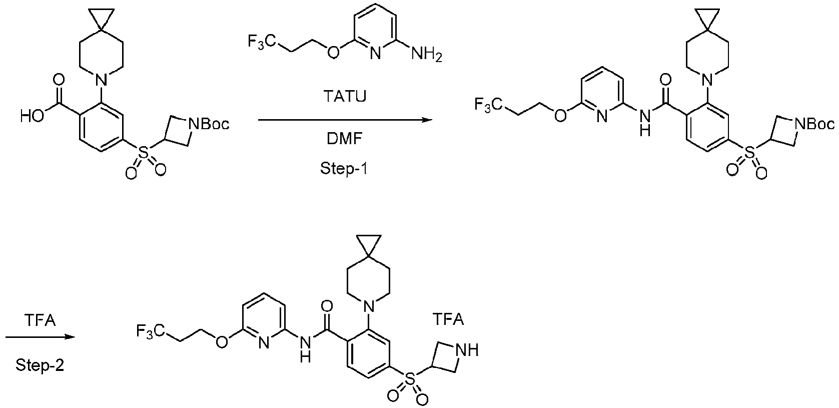

Step-1: A mixture of 4-((1-(tert-butoxycarbonyl)azetidin-3-yl)sulfonyl)-2-(6-azaspiro[2.5]octan-6-yl)benzoic acid (0.200 g, 0.444 mmol, Intermediate 15), 6-(3,3,3-trifluoropropoxy)pyridin-2-amine (0.119 g, 0.577 mmol, Intermediate 6), DMF (4 mL), Hunig's base (0.229 g, 0.316 mL, 1.78 mmol, Aldrich) and TATU (0.250 g, 0.776 mmol, Combi-Blocks) was stirred at room temperature overnight. The mixture was diluted with saturated Na₂CO₃ and EtOAc. The organic phase was taken and washed with Na₂CO₃, water and brine, dried over Na₂SO₄ and concentrated in vacuo. Purification by silica gel chromatography (0-100% EtOAc-heptane) gave *tert*-butyl 3-((3-(6-azaspiro[2.5]octan-6-yl)-4-((6-(3,3,3-trifluoropropoxy)pyridin-2-yl)carbamoyl)phenyl)sulfonyl)azetidine-1-carboxylate as an off-white solid. *m*/*z* (ESI): 639.2 (M+H)⁺.

Step-2: *tert*-Butyl 3-((3-(6-azaspiro[2.5]octan-6-yl)-4-((6-(3,3,3-trifluoropropoxy)pyridin-2-yl)carbamoyl)phenyl)sulfonyl)azetidine-1-carboxylate (0.118 g, 0.184 mmol) was dissolved in DCM (8 mL) and TFA (4 mL). The reaction mixture was stirred at RT for 30 min and concentrated in vacuo. EtOAc was added to the TFA salt and evaporated. The product was obtained as off-white solid. ¹H NMR (400 MHz, METHANOL-d₄) δ 8.38 (d, *J*=8.09 Hz, 1H), 7.90-7.96 (m, 2H), 7.85 (dd, *J*=1.66, 8.29 Hz, 1H), 7.74 (t, *J*=7.98 Hz, 1H), 6.60 (d, *J*=8.09 Hz, 1H), 4.57 (t, *J*=6.32 Hz, 2H), 4.42 (d, *J*=7.46 Hz, 4H), 3.14-3.23 (m, 4H), 2.66-2.80 (m, 2H), 1.81 (br s, 4H), 0.44 (s, 4H). ¹⁹F NMR (376 MHz, METHANOL-d₄) δ -66.16 (s, 3F), -76.96 (s, 3F). *m*/*z* (ESI): 539.2 (M+H)⁺.

### BIOLOGICAL EXAMPLES

The following assays were used in testing the exemplary compounds of the invention. Data for those examples tested in accordance with the procedures described below are presented in Table A below.

KIF18A Enzyme Assay: Microtubule-stimulated ATPase activity assay is used to measure KIF18A enzyme activity after treatment with compound. Compounds were 2-fold serially diluted in DMSO (Sigma Inc) over 22-point concentration range. Recombinant human KIF18A (1-467 His-tagged) protein was expressed using a baculovirus system and purified by affinity chromatography by Amgen Inc. Concentrations of KIF18A protein, microtubules (MT), and ATP in the reaction were optimized for standardized homogenous enzyme assay using ADP-Glo^{™} Kinase/ATPase Assay Kit (Promega Inc). The assay measures ADP formed from the ATPase reaction. Prepare reaction buffer [(15 mM Tris, pH 7.5 (Teknova Inc), 10 mM MgCl2 (JT Baker Inc), 0.01% Pluronic F-68 (Life Technologies Inc), 1 µM Taxol (Cytoskeleton Inc), and 30 µg/mL pig microtubules (Cytoskeleton Inc)]. Add compound and KIF18A protein (30 nM) to prepared reaction buffer and incubated for 15 minutes at room temperature, next add ATP (at Kₘ, 75 µM) to the reaction mixture and incubated for an additional 15 minutes at room temperature. Mix 5 µl of ADP-Glo^{™} Reagent and 2.5 µl of the reaction mixture and incubate for 40 minutes at room temperature. Add 10 µl ADP-Glo^{™} Detection Reagent and incubate for 40 minutes at room temperature. Read luminescence using EnVision microplate reader with ultra-luminescence module (Perkin Elmer Inc). Concentration-response curve-fitting and IC₅₀ determination was performed using Genedata Screener Software (Standard 15.0.1, Genedata Inc) with a four-parameter logistic regression fit model.

Table A provides data for compounds exemplified in the present application and priority document thereof, as representative compounds of the present invention, as follows: compound name and biological data. (IC₅₀ in uM, where available. Ex. # refers to Example No.)

**TABLE A: BIOLOGICAL DATA**

| Ex. # | Compound Name | KIF18A ATPase IC₅₀ (µM) |
|---|---|---|
| 1 | 4-(*N*-(2-Hydroxyethyl)sulfamoyl)-2-(6-azaspiro[2.5]octan-6-yl)-*N*-(6-(3,3,3-trifluoropropoxy)pyridin-2-yl)benzamide | 0.012 |
| 1-1 | (*R*)-4-(*N*-(2-Hydroxyethyl)sulfamoyl)-*N*-(6-(2-methylmorpholino)pyridin-2-yl)-2-(6-azaspiro[2.5]octan-6-yl)benzamide | 0.016 |
| 1-2 | *N-*(6-(4,4-Difluoropiperidin-1-yl)-4-methylpyridin-2-yl)-4-(N-(2-hydroxyethyl)sulfamoyl)-2-(6-azaspiro[2.5]octan-6-yl)benzamide | 0.032 |
| 1-3 | (*R*)-*N*-(6-(2-methylmorpholino)pyridin-2-yl)-4-(N-methylsulfamoyl)-2-(6-azaspiro[2.5]octan-6-yl)benzamide | 0.044 |
| 1-4 | (*R*)-4-(*N-*(1-Hydroxy-2-methylpropan-2-yl)sulfamoyl)-*N*-(6-(2-methylmorpholino)pyridin-2-yl)-2-(6-azaspiro[2.5]octan-6-yl)benzamide | 0.046 |
| 1-5 | 4-(*N-*(1-Hydroxypropan-2-yl)sulfamoyl)-*N*-(6-((*R*)-2-methylmorpholino)pyridin-2-yl)-2-(6-azaspiro[2.5]octan-6-yl)benzamide | 0.047 |
| 1-6 | (*R*)-4-(*N*-(*tert*-Butyl)sulfamoyl)-*N*-(6-(2-methylmorpholino)pyridin-2-yl)-2-(6-azaspiro[2.5]octan-6-yl)benzamide | 0.051 |
| 1-7 | (*R*)-4-(Azetidin-1-ylsulfonyl)-*N-*(6-(2-methylmorpholino)pyridin-2-yl)-2-(6-azaspiro[2.5]octan-6-yl)benzamide | 0.067 |
| 1-8 | (*R*)-*N*-(4-Methyl-6-(2-methylmorpholino)pyridin-2-yl)-4-(N-(3-methyloxetan-3-yl)sulfamoyl)-2-(6-azaspiro[2.5]octan-6-yl)benzamide | 0.119 |
| 1-9 | 4-(*N*-(1-Hydroxybutan-2-yl)sulfamoyl)-*N*-(6-((*R*)-2-methylmorpholino)pyridin-2-yl)-2-(6-azaspiro[2.5]octan-6-yl)benzamide | 0.120 |
| 1-10 | 4-(*N-*(tert-Butyl)sulfamoyl)-*N-*(4-methyl-6-morpholinopyridin-2-yl)-2-(6-azaspiro[2.5]octan-6-yl)benzamide | 0.222 |
| 1-11 | (*R*)-4-((3,3-Dimethylazetidin-1-yl)sulfonyl)-*N*-(6-(2-methylmorpholino)pyridin-2-yl)-2-(6-azaspiro[2.5]octan-6-yl)benzamide | 0.566 |
| 1-12 | (*R*)-4-((3-Cyano-3-methylazetidin-1-yl)sulfonyl)-*N*-(6-(2-methylmorpholino)pyridin-2-yl)-2-(6-azaspiro[2.5]octan-6-yl)benzamide | 0.458 |
| 1-13 | (*R*)-4-((3-cyanoazetidin-1-yl)sulfonyl)-N-(6-(2-methylmorpholino)pyridin-2-yl)-2-(6-azaspiro[2.5]octan-6-yl)benzamide | 0.209 |
| 2 | (*R*)-4-(Isopropylsulfonyl)-*N*-(6-(2-methylmorpholino)pyridin-2-yl)-2-(6-azaspiro[2.5]octan-6-yl)benzamide | 0.034 |
| 2-1 | (*R*)-4-((2-Hydroxyethyl)sulfonyl)-*N-*(6-(2-methylmorpholino)pyridin-2-yl)-2-(6-azaspiro[2.5]octan-6-yl)benzamide | 0.032 |
| 2-2 | (*R*)-4-(*tert*-Butylsulfonyl)-*N-*(6-(2-methylmorpholino)pyridin-2-yl)-2-(6-azaspiro[2.5]octan-6-yl)benzamide | 0.078 |
| 2-3 | (*R*)-*N-*(6-(2-Methylmorpholino)pyridin-2-yl)-4-((3-methyloxetan-3-yl)sulfonyl)-2-(6-azaspiro[2.5]octan-6-yl)benzamide | 0.141 |
| **2-4** | (*R*)-*N*-(6-(2-methylmorpholino)pyridin-2-yl)-4-(oxetan-3-ylsulfonyl)-2-(6-azaspiro[2.5]octan-6-yl)benzamide | 0.038 |
| **2-5** | (*R*)-4-((1-hydroxy-2-methylpropan-2-yl)sulfonyl)-*N*-(6-(2-methylmorpholino)pyridin-2-yl)-2-(6-azaspiro[2.5]octan-6-yl)benzamide | 0.051 |
| **2-6** | (*R*)-4-((2-Hydroxy-2-methylpropyl)sulfonyl)-*N-*(6-(2-methylmorpholino)pyridin-2-yl)-2-(6-azaspiro[2.5]octan-6-yl)benzamide | 0.110 |
| **2-7** | (*R*)-4-((2-Hydroxyethyl)sulfonyl)-*N*-(4-methyl-6-(2-methylmorpholino)pyridin-2-yl)-2-(6-azaspiro[2.5]octan-6-yl)benzamide | 0.103 |
| **3** | (*R*)-*N*-(6-(3-Hydroxypiperidin-1-yl)pyridin-2-yl)-4-((1-methylcyclopropane)-1-sulfonamido)-2-(6-azaspiro[2.5]octan-6-yl)benzamide | 0.046 |
| **3-1** | (*S*)-*N*-(6-(3-Hydroxypiperidin-1-yl)pyridin-2-yl)-4-((1-methylcyclopropane)-1-sulfonamido)-2-(6-azaspiro[2.5]octan-6-yl)benzamide | 0.056 |
| **3-2** | *N*-(6-(3-Hydroxy-3-methylpyrrolidin-1-yl)pyridin-2-yl)-4-((1-methylcyclopropane)-1-sulfonamido)-2-(6-azaspiro[2.5]octan-6-yl)benzamide | 0.359 |
| **3-3** | (*S*)-4-(*N*-(*tert*-Butyl)sulfamoyl)-N-(6-(3-hydroxypiperidin-1-yl)pyridin-2-yl)-2-(6-azaspiro[2.5]octan-6-yl)benzamide | 0.096 |
| **3-4** | 4-(*N*-(*tert*-Butyl)sulfamoyl)-*N*-(6-((3-(hydroxymethyl)oxetan-3-yl)amino)pyridin-2-yl)-2-(6-azaspiro[2.5]octan-6-yl)benzamide | 0.149 |
| **3-5** | *N*-(6-(3,3-Difluoropyrrolidin-1-yl)pyridin-2-yl)-4-((1-methylcyclopropane)-1-sulfonamido)-2-(6-azaspiro[2.5]octan-6-yl)benzamide | 0.045 |
| **3-6** | (*R*)-*N*-(6-(3-(Hydroxymethyl)piperidin-1-yl)pyridin-2-yl)-4-((1-methylcyclopropane)-1-sulfonamido)-2-(6-azaspiro [2.5]octan-6-yl)benzamide | 0.086 |
| **3-7** | (*S*)-*N*-(6-(3-(Hydroxymethyl)piperidin-1-yl)pyridin-2-yl)-4-((1-methylcyclopropane)-1-sulfonamido)-2-(6-azaspiro[2.5]octan-6 - yl)benzamide | 0.065 |
| **3-8** | *N*-(6-(4-Hydroxy-4-methylpiperidin-1-yl)pyridin-2-yl)-4-((1-methylcyclopropane)-1-sulfonamido)-2-(6-azaspiro[2.5]octan-6-yl)benzamide | 0.243 |
| **3-9** | (*S*)-*N*-(6-(3-Hydroxy-3-methylpiperidin-1-yl)pyridin-2-yl)-4-((1-methylcyclopropane)-1-sulfonamido)-2-(6-azaspiro[2.5]octan-6-yl)benzamide | 0.175 |
| **3-10** | *(R*)*-N-*(6-(3-Hydroxy-3-methylpiperidin-1 - yl)pyridin-2-yl)-4-((1-methylcyclopropane)-1-sulfonamido)-2-(6-azaspiro[2.5]octan-6-yl)benzamide | 0.143 |
| **3-11** | (*R*)-*N-*(6-(2-(Hydroxymethyl)morpholino)pyridin-2-yl)-4-((1-methylcyclopropane)-1-sulfonamido)-2-(6-azaspiro[2.5]octan-6-yl)benzamide | 0.073 |
| **3-12** | (*S*)-*N*-(6-(2-(Hydroxymethyl)morpholino)pyridin-2-yl)-4-((1-methylcyclopropane)-1-sulfonamido)-2-(6-azaspiro[2.5]octan-6-yl)benzamide | 0.081 |
| **3-13** | *N*-(6-(4-Fluoro-4-methylpiperidin-1-yl)pyridin-2-yl)-4-((1-methylcyclopropane)-1-sulfonamido)-2-(6-azaspiro[2.5]octan-6-yl)benzamide | 0.373 |
| **3-14** | *N*-(6-(3,3-Difluoropiperidin-1-yl)pyridin-2-yl)-4-((1-methylcyclopropane)-1-sulfonamido)-2-(6-azaspiro[2.5]octan-6-yl)benzamide | 0.038 |
| **3-15** | *N*-(6-(5-Chloroindolin-1-yl)pyridin-2-yl)-4-((1-methylcyclopropane)-1-sulfonamido)-2-(6-azaspiro[2.5]octan-6-yl)benzamide | 0.033 |
| **3-16** | *N-*(6-(3-Fluoroazetidin-1-yl)pyridin-2-yl)-4-((1-methylcyclopropane)-1-sulfonamido)-2-(6-azaspiro[2.5]octan-6-yl)benzamide | 0.058 |
| **3-17** | 4-((1-Methylcyclopropane)-1-sulfonamido)-N-(6-(piperidin-1-yl)pyridin-2-yl)-2-(6-azaspiro[2.5]octan-6-yl)benzamide | 0.034 |
| **3-18** | *N*-(6-(3,3-Difluoroazetidin-1-yl)pyridin-2-yl)-4-((1-methylcyclopropane)-1-sulfonamido)-2-(6-azaspiro[2.5]octan-6-yl)benzamide | 0.048 |
| **3-19** | *N*-(6-(3,3-Difluoroazetidin-1-yl)-4-methylpyridin-2-yl)-4-((1-methylcyclopropane)-1-sulfonamido)-2-(6-azaspiro[2.5]octan-6-yl)benzamide | 0.103 |
| **3-20** | *N-*(6-(5-Azaspiro[2.4]heptan-5-yl)pyridin-2-yl)-4-((1-methylcyclopropane)-1-sulfonamido)-2-(6-azaspiro[2.5]octan-6-yl)benzamide | 0.050 |
| **3-21** | *N*-(6-(4-Cyanopiperidin-1-yl)pyridin-2-yl)-4-((1-methylcyclopropane)-1-sulfonamido)-2-(6-azaspiro[2.5]octan-6-yl)benzamide | 0.091 |
| **3-22** | *N*-(6-(3-Cyanopiperidin-1-yl)pyridin-2-yl)-4-((1-methylcyclopropane)-1-sulfonamido)-2-(6-azaspiro[2.5]octan-6-yl)benzamide | 0.086 |
| **3-23** | *N*-(6-(4-Fluoropiperidin-1-yl)pyridin-2-yl)-4-(1-methylcyclopropane)-1-sulfonamido)-2-(6-azaspiro[2.5]octan-6-yl)benzamide | 0.044 |
| **4** | (*R*)-*N*-(6-(3-hydroxypiperidin-1-yl)pyridin-2-yl)-4-((methylsulfonyl)methyl)-2-(6-azaspiro[2.5]octan-6-yl)benzamide | 0.094 |
| **4-1** | *N*-(6-(2-Hydroxypropan-2-yi)pyridin-2-yl)-4-((1-methylcyclopropane)-1-sulfonamido)-2-(6-azaspiro[2.5]octan-6-yl)benzamide | 0.128 |
| **4-2** | *N*-(6-(2-Hydroxypropan-2-yl)pyridin-2-yl)-4-(N-(3-methyloxetan-3-yl)sulfamoyl)-2-(6-azaspiro[2.5]octan-6-yl)benzamide | 0.080 |
| **4-3** | (*S*)-4-(*N*-(*tert*-Butyl)sulfamoyl)-*N*-(6-(2-(hydroxymethyl)morpholino)pyridin-2-yl)-2-(6-azaspiro[2.5]octan-6-yl)benzamide | 0.086 |
| **4-4** | *N*-(6-Cyclopropylpyridin-2-yl)-4-(methylsulfonyl)-2-(6-azaspiro[2.5]octan-6-yl)benzamide | 0.180 |
| **4-5** | *N*-(6-Isopropylpyridin-2-yl)-4-(methylsulfonyl)-2-(6-azaspiro[2.5]octan-6-yl)benzamide | 0.179 |
| **4-6** | *N*-(6-(*tert*-Butyl)pyridin-2-yl)-4-(methylsulfonyl)-2-(6-azaspiro[2.5]octan-6-yl)benzamide | 0.166 |
| **4-7** | *N*-(6-(2-Hydroxypropan-2-yl)pyridin-2-yl)-4-(methylsulfonyl)-2-(6-azaspiro[2.5]octan-6-yl)benzamide | 0.315 |
| **4-8** | *N*-(6-(*N*-(*tert*-Butyl)sulfamoyl)pyridin-2-yl)-4-(methylsulfonyl)-2-(6-azaspiro[2.5]octan-6-yl)benzamide | 0.076 |
| **4-9** | *N*-(6-(4,4-Difluoropiperidin-1-yl)pyridin-2-yl)-4-((1-methylcyclopropane)-1-sulfonamido)-2-(6-azaspiro[2.5]octan-6-yl)benzamide | 0.046 |
| **4-10** | *N*-(6-(4,4-Difluoropiperidin-1-yl)-4-methylpyridin-2-yl)-4-((1-methylcyclopropane)-1-sulfonamido)-2-(6-azaspiro [2.5]octan-6-yl)benzamide | 0.095 |
| **4-11** | (*R*)-*N*-(6-(2-Methylmorpholino)pyridin-2-yl)-4-(*N*-(3-methyloxetan-3-yl)sulfamoyl)-2-(6-azaspiro[2.5]octan-6-yl)benzamide | 0.043 |
| **4-12** | 4-(Methylsulfonyl)-*N*-(6-morphofinopyridin-2-yl)-2-(6-azaspiro[2.5]octan-6-yl)benzamide | 0.118 |
| **4-13** | (*R*)-*N*-(6-(2-Methylmorpholino)pyridin-2-yl)-4-(methylsulfonyl)-2-(6-azaspiro[2.5]octan-6-vl)benzamide | 0.069 |
| **4-14** | (*R*)-4-((1-Methylcyclopropane)-1-sulfonamido)-*N*-(6-(2-methylmorpholino)pyridin-2-yl)-2-(6-azaspiro[2.5]octan-6-yl)benzamide | 0.066 |
| **4-15** | (*R*)-*N*-(6-(2-Methylmorpholino)pyridin-2-yl)-4-((methylsulfonyl)methyl)-2-(6-azaspiro[2.5]octan-6-yl)benzamide | 0.085 |
| **4-16** | *N*-(4-Methyl-6-morpholinopyridin-2-yl)-4-((methylsulfonyl)methyl)-2-(6-azaspiro[2.5]octan-6-yl)benzamide | 0.145 |
| **4-17** | *N*-(6-(2-Hydroxypropan-2-yl)-4-methylpyridin-2-yl)-4-((1-methylcyclopropane)-1-sulfonamido)-2-(6-azaspiro[2.5]octan-6-yl)benzamide | 0.163 |
| **4-18** | *N*-(6-(2-Hydroxypropan-2-yl)-4-methylpyridin-2-yl)-4-(N-(3-methyloxetan-3-yl)sulfamoyl)-2-(6-azaspiro[2.5]octan-6-yl)benzamide | 0.089 |
| **4-19** | (*R*)-4-(*N*-(*tert*-Butyl)sulfamoyl)-*N*-(6-(3-hydroxypiperidin-1-yl)-4-methylpyridin-2-yl)-2-(6-azaspiro[2.5]octan-6-yl)benzamide | 0.115 |
| **5** | 4-((2-Hydroxyethyl)sulfonamido)-2-(6-azaspiro[2.5]octan-6-yl)-N-(6-(3,3,3-trifluoropropoxy)pyridin-2-yl)benzamide | 0.025 |
| **6** | *N*-(6-(4,4-difluoropiperidin-1-yl)-4-methylpyridin-2-yl)-4-((2-hydroxyethyl)sulfonamido)-2-(6-azaspiro[2.5]octan-6-yl)benzamide | 0.047 |
| **7-1** | *N*-(6-(3,3-Difluorocyclobutyl)-4-methylpyridin-2-yl)-4-((2-hydroxyethyl)sulfonamido)-2-(6-azaspiro[2.5]octan-6-yl)benzamide | 0.045 |
| **7-2** | (*R*)-*N*-(6-(2-Methylmorpholino)pyridin-2-yl)-4-(methylsulfonamido)-2-(6-azaspiro[2.5]octan-6-yl)benzamide | 0.039 |
| **7-3** | (*R*)-4-(1,1-Dioxidoisothiazolidin-2-yl)-N-(6-(2-methylmorpholino)pyridin-2-yl)-2-(6-azaspiro[2.5]octan-6-yl)benzamide | 0.076 |
| **7-4** | (*R*)-4-(1,1-Dioxido-1,2,5-thiadiazolidin-2-yl)-N-(6-(2-methylmorpholino)pyridin-2-yl)-2-(6-azaspiro[2.5]octan-6-yl)benzamide | 0.287 |
| **7-5** | (*R*)-4-((*N,N*-Dimethylsulfamoyl)amino)-N-(6-(2-methylmorpholino)pyridin-2-yl)-2-(6-azaspiro[2.5]octan-6-yl)benzamide | 0.055 |
| **7-6** | (*R*)-4-(1,1-Dioxido-1,2-thiazetidin-2-yl)-N-(6-(2-methylmorpholino)pyridin-2-yl)-2-(6-azaspiro[2.5]octan-6-yl)benzamide | 0.098 |
| **7-7** | (*R*)-4-((Difluoromethyl)sulfonamido)-N-(6-(2-methylmorpholino)pyridin-2-yl)-2-(6-azaspiro[2.5]octan-6-yl)benzamide | 0.049 |
| **7-8** | (*R*)-4-(Ethylsulfonamido)-*N*-(4-methyl-6-(2-methylmorpholino)pyridin-2-yl)-2-(6-azaspiro[2.5]octan-6-yl)benzamide | 0.072 |
| **7-9** | (*R*)-4-(Cyclobutanesulfonamido)-N-(6-(2-methylmorpholino)pyridin-2-yl)-2-(6-azaspiro[2.5]octan-6-yl)benzamide | 0.062 |
| **7-10** | (*R*)-*N*-(6-(2-Methylmorpholino)pyridin-2-yl)-4-(oxetane-3-sulfonamido)-2-(6-azaspiro[2.5]octan-6-yl)benzamide | 0.063 |
| **7-11** | *N*-(6-(4,4-Difluoropiperidin-1-yl)-4-methylpyridin-2-yl)-4-(oxetane-3-sulfonamido)-2-(6-azaspiro[2.5]octan-6-yl)benzamide | 0.081 |
| **7-12** | (*R*)-4-((2-Hydroxyethyl)sulfonamido)-*N*-(6-(2-methylmorpholino)pyridin-2-yl)-2-(6-azaspiro[2.5]octan-6-yl)benzamide | 0.030 |
| **7-13** | (*S*)-4-((2-Hydroxyethyl)sulfonamido)-*N*-(6-(2-methylmorpholino)pyridin-2-yl)-2-(6-azaspiro[2.5]octan-6-yl)benzamide | 0.046 |
| **7-14** | (*R*)-*N*-(6-(2-Methylmorpholino)pyridin-2-yl)-2-(6-azaspiro[2.5]octan-6-yl)-4-((3,3,3-trifluoropropyl)sulfonamido)benzamide | 0.343 |
| **7-15** | (*R*)-4-((1-Fluorocyclopropane)-1-sulfonamido)-*N-*(6-(2-methylmorpholino)pyridin-2-yl)-2-(6-azaspiro[2.5]octan-6-yl)benzamide | 0.090 |
| **7-16** | (*R*)-4-((1,1-Dimethylethyl)sulfonamido)-N-(6-(2-methylmorpholino)pyridin-2-yl)-2-(6-azaspiro[2.5]octan-6-yl)benzamide | 0.067 |
| **7-17** | 4-((2-Hydroxyethyl)sulfonamido)-*N*-(4-methyl-6-morpholinopyridin-2-yl)-2-(6-azaspiro[2.5]octan-6-yl)benzamide | 0.0170 |
| **7-18** | (*R*)-4-((2-Hydroxyethyl)sulfonamido)-*N*-(4-methyl-6-(2-methylmorpholino)pyridin-2-yl)-2-(6-azaspiro[2.5]octan-6-yl)benzamide | 0.074 |
| **7-19** | (*S*)-4-((2-Hydroxyethyl)sulfonamido)-*N*-(4-methyl-6-(2-methylmorpholino)pyridin-2-yl)-2-(6-azaspiro[2.5]octan-6-yl)benzamide | 0.049 |
| **7-20** | 4-((2-Hydroxyethyl)sulfonamido)-N-(6-morpholinopyridin-2-yl)-2-(6-azaspiro[2.5]octan-6-yl)benzamide | 0.036 |
| **7-21** | *N*-(6-(4,4-Difluoropiperidin-1-yl)-4-methylpyridin-2-yl)-4-(methylsulfonamido)-2-(6-azaspiro[2.5]octan-6-yl)benzamide | 0.049 |
| **7-22** | *N*-(6-(4,4-Difluoropiperidin-1-yl)-4-methylpyridin-2-yl)-4-((1,1-dimethylethyl)sulfonamido)-2-(6-azaspiro[2.5]octan-6-yl)benzamide | 0.103 |
| **7-23** | 4-(Cyclobutanesulfonamido)-*N*-(6-(4,4-difluoropiperidin-1-yl)-4-methylpyridin-2-yl)-2-(6-azaspiro[2.5]octan-6-yl)benzamide | 0.110 |
| **7-24** | 4-((Cyclopropylmethyl)sulfonamido)-*N*-(6-(4,4-difluoropiperidin-1-yl)-4-methylpyridin-2-yl)-2-(6-azaspiro[2.5]octan-6-yl)benzamide | 0.186 |
| **7-25** | 4-(Cyclopropanesulfonamido)-*N*-(6-(4,4-difluoropiperidin-1-yl)-4-methylpyridin-2-yl)-2-(6-azaspiro[2.5]octan-6-yl)benzamide | 0.039 |
| **7-26** | *N*-(6-(4,4-Difluoropiperidin-1-yl)pyridin-2-yl)-4-((2-hydroxyethyl)sulfonamido)-2-(6-azaspiro[2.5]octan-6-yl)benzamide | 0.023 |
| **7-27** | *N*-(6-(3,3-Difluoroazetidin-1-yl)-4-methylpyridin-2-yl)-4-(methylsulfonamido)-2-(6-azaspiro[2.5]octan-6-yl)benzamide | 0.014 |
| **7-28** | *N*-(6-(3,3-Difluoroazetidin-1-yl)-4-methylpyridin-2-yl)-4-((1-methylethyl)sulfonamido)-2-(6-azaspiro[2.5]octan-6-yl)benzamide | 0.068 |
| **7-29** | *N*-(6-(3,3-Difluoroazetidin-1-yl)-4-methylpyridin-2-yl)-4-((1,1-dimethylethyl)sulfonamido)-2-(6-azaspiro[2.5]octan-6-yl)benzamide | 0.134 |
| **7-30** | 4-(Cyclobutanesulfonamido)-*N*-(6-(3,3-difluoroazetidin-1-yl)-4-methylpyridin-2-yl)-2-(6-azaspiro[2.5]octan-6-yl)benzamide | 0.078 |
| **7-31** | 4-((Cyclopropylmethyl)sulfonamido)-*N*-(6-(3,3-difluoroazetidin-1-yl)-4-methylpyridin-2-yl)-2-(6-azaspiro[2.5]octan-6-yl)benzamide | 0.059 |
| **7-32** | *N*-(6-(3,3-Difluoroazetidin-1-yl)-4-methylpyridin-2-yl)-4-(oxetane-3-sulfonamido)-2-(6-azaspiro[2.5]octan-6-yl)benzamide | 0.025 |
| **7-33** | *N*-(6-(3,3-Difluoroazetidin-1-yl)-4-methylpyridin-2-yl)-4-((2-hydroxyethyl)sulfonamido)-2-(6-azaspiro[2.5]octan-6-yl)benzamide | 0.024 |
| **7-34** | 4-(Cyclopropanesulfonamido)-N-(6-(3,3-difluoroazetidin-1-yl)-4-methylpyridin-2-yl)-2-(6-azaspiro[2.5]octan-6-yl)benzamide | 0.041 |
| **7-35** | (*R*)-4-((2-Fluoroethyl)sulfonamido)-*N*-(6-(2-methylmorpholino)pyridin-2-yl)-2-(6-azaspiro[2.5]octan-6-yl)benzamide | 0.024 |
| **7-36** | (*R*)-4-((Fluoromethyl)sulfonamido)-*N*-(4-methyl-6-(2-methylmorpholino)pyridin-2-yl)-2-(6-azaspiro[2.5]octan-6-yl)benzamide | 0.062 |
| **7-37** | (*R*)-4-(Ethylsulfonamido)-*N*-(6-(2-methylmorpholino)pyridin-2-yl)-2-(6-azaspiro[2.5]octan-6-yl)benzamide | 0.050 |
| **7-38** | (*R*)-4-((Fluoromethyl)sulfonamido)-N-(6-(2-methylmorpholino)pyridin-2-yl)-2-(6-azaspiro[2.5]octan-6-yl)benzamide | 0.048 |
| **7-39** | (*R*)-*N*-(6-(2-methylmorpholino)pyridin-2-yl)-2-(6-azaspiro[2.5]octan-6-yl)-4-((2,2,2-trifluoroethyl)sulfonamido)benzamide | 0.135 |
| **7-40** | (*R*)-4-((2,2-Difluoroethyl)sulfonamido)-*N*-(6-(2-methylmorpholino)pyridin-2-yl)-2-(6-azaspiro[2.5]octan-6-yl)benzamide | 0.067 |
| **7-41** | (*R*)-*N-*(6-(2-Methylmorpholino)pyridin-2-yl)-2-(6-azaspiro[2.5]octan-6-yl)-4-((trifluoromethyl)sulfonamido)benzamide | 0.207 |
| **7-42** | (*R*)-4-((2-Hydroxy-2-methylpropyl)sulfonamido)-*N*-(6-(2-methylmorpholino)pyridin-2-yl)-2-(6-azaspiro[2.5]octan-6-yl)benzamide | 0.111 |
| **7-43** | *N-*(6-(*N*-(*tert*-Butyl)sulfamoyl)pyridin-2-yl)-4-(methylsulfonamido)-2-(6-azaspiro[2.5]octan-6-yl)benzamide | 0.055 |
| **7-44** | *N*-(6-(4,4-Difluoropiperidin-1-yl)pyrazin-2-yl)-4-((2-hydroxyethyl)sulfonamido)-2-(6-azaspiro[2.5]octan-6-yl)benzamide | 0.016 |
| **8-1** | (*R*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide | 0.016 |
| **8-2** | (*S*)-4-((2-hydroxy-1-methylethyl)sulfonamido)-2-(6-azaspiro[2.5]octan-6-yl)-*N*-(6-(3,3,3-trifluoropropoxy)pyridin-2-yl)benzamide | 0.014 |
| **9-1** | (*R*)-*N*-(6-(3-Fluoroazetidin-1-yl)pyridin-2-yl)-4-((2-hydroxy-1-methylethyl)sulfonamido)-2-(6-azaspiro[2.5]octan-6-yl)benzamide | 0.033 |
| **9-2** | (*S*)-N-(6-(3-Fluoroazetidin-1-yl)pyridin-2-yl)-4-((2-hydroxy-1-methylethyl)sulfonamido)-2-(6-azaspiro[2.5]octan-6-yl)benzamide | 0.0472 |
| **9-3** | (*R*)-*N*-(6-(3,3-Difluoroazetidin-1-yl)pyridin-2-yl)-4-((2-hydroxy-1-methylethyl)sulfonamido)-2-(6-azaspiro[2.5]octan-6-yl)benzamide | 0.025 |
| **9-4** | (*S*)-*N*-(6-(3,3-Difluoroazetidin-1-yl)pyridin-2-yl)-4-((2-hydroxy-1-methylethyl)sulfonamido)-2-(6-azaspiro[2.5]octan-6-yl)benzamide | 0.068 |
| **9-5** | (*R*)-*N*-(6-(3,3-Difluoroazetidin-1-yl)-4-methylpyridin-2-yl)-4-((2-hydroxy-1- methylethyl)sulfonamido)-2-(6-azaspiro[2.5]octan-6-yl)benzamide | 0.017 |
| **9-6** | (*S*)-*N*-(6-(3,3-Difluoroazetidin-1-yl)-4-methylpyridin-2-yl)-4-((2-hydroxy-1-methylethyl)sulfonamido)-2-(6-azaspiro[2.5]octan-6-yl)benzamide | 0.057 |
| **9-7** | (*S*)-*N*-(6-(4,4-Difluoropiperidin-1-yl)-4-methylpyridin-2-yl)-4-((2-hydroxy-1-methylethyl)sulfonamido)-2-(6-azaspiro[2.5]octan-6-yl)benzamide | 0.027 |
| **9-8** | (*R*)-*N*-(6-(4,4-Difluoropiperidin-1-yl)-4-methylpyridin-2-yl)-4-((2-hydroxy-1-methylethyl)sulfonamido)-2-(6-azaspiro[2.5]octan-6-yl)benzamide | 0.038 |
| **9-9** | (*S*)-*N*-(6-(4,4-Difluoropiperidin-1-yl)pyridin-2-yl)-4-((2-hydroxy-1-methylethyl)sulfonamido)-2-(6-azaspiro[2.5]octan-6-yl)benzamide | 0.041 |
| **9-10** | (*R*)-*N*-(6-(4,4-Difluoropiperidin-1-yl)pyridin-2-yl)-4-((2-hydroxy-1-methylethyl)sulfonamido)-2-(6-azaspiro[2.5]octan-6-yl)benzamide | 0.037 |
| **9-11** | 4-(((*R*)-2-Hydroxy-1-methylethyl)sulfonamido)-N (4-methyl-6-((*R*)-2-methylmorpholino)pyridin-2-yl)-2-(6-azaspiro[2.5]octan-6-yl)benzamide | 0.038 |
| **9-12** | 4-(((*S*)-2-Hydroxy-1-methylethyl)sulfonamido)-*N-*(4-methyl-6-((*R*)-2-methylmorpholino)pyridin-2-yl)-2-(6-azaspiro[2.5]octan-6-yl)benzamide | 0.039 |
| **9-13** | (*R*)-4-((2-Hydroxy-1-methylethyl)sulfonamido)-N-(4-methyl-6-morpholinopyridin-2-yl)-2-(6-azaspiro[2.5]octan-6-yl)benzamide | 0.018 |
| **9-14** | (*S*)-4-((2-Hydroxy-1-methylethyl)sulfonamido)-N-(4-methyl-6-morpholinopyridin-2-yl)-2-(6-azaspiro[2.5]octan-6-yl)benzamide | 0.018 |
| **9-15** | 4-(((*R*)-2-Hydroxy-1-methylethyl)sulfonamido)-*N*-(6-((*R*)-2-methylmorpholino)pyridin-2-yl)-2-(6-azaspiro[2.5]octan-6-yl)benzamide | 0.037 |
| **9-16** | 4-(((*S*)-2-Hydroxy-1-methylethyl)sulfonamido)-*N-*(6-((*R*)-2-methylmorpholino)pyridin-2-yl)-2-(6-azaspiro[2.5]octan-6-yl)benzamide | 0.048 |
| **9-17** | 4-(((*R*)-2-Hydroxy-1-methylethyl)sulfonamido)-N-(6-((*S*)-2-methylmorpholino)pyridin-2-yl)-2-(6-azaspiro[2.5]octan-6-yl)benzamide | 0.024 |
| **9-18** | 4-(((*S*)-2-Hydroxy-1-methylethyl)sulfonamido)-N-(6-((*S*)-2-methylmorpholino)pyridin-2-yl)-2-(6-azaspiro[2.5]octan-6-yl)benzamide | 0.036 |
| **10** | *N*-(6-(4,4-Difluoropiperidin-1-yl)-4-methylpyridin-2-yl)-4-((1-(hydroxymethyl)cyclopropane)-1-sulfonamido)-2-(6-azaspiro[2.5]octan-6-yl)benzamide | 0.122 |
| **10-1** | (*R*)-4-(1-(Hydroxymethyl)cyclopropane)-1-sulfonamido)-*N*-(6-(2-methylmorpholino)pyridin-2-yl)-2-(6-azaspiro[2.5]octan-6-yl)benzamide | 0.057 |
| **10-2** | *N*-(6-(3,3-Difluoroazetidin-1-yl)-4-methylpyridin-2-yl)-4-((1-(hydroxymethyl)cyclopropane)-1-sulfonamido)-2-(6-azaspiro[2.5]octan-6-yl)benzamide | 0.076 |
| **11** | *N*-(6-(3,3-difluoroazetidin-1-yl)-4-methylpyridin-2-yl)-4-((2-hydroxyethyl)sulfonamido)-2-(6-azaspiro[2.5]octan-6-yl)benzamide | 0.031 |
| **12-1** | 2-(6-Azaspiro [2.5]octan-6-yl)-4-(*S-*cyclopropylsulfonimidoyl)-*N*-(6-((2*R*)-2-methyl-4-morpholinyl)-2-pyridinyl)benzamide | 0.085 |
| **12-2** | 2-(6-Azaspiro[2.5]octan-6-yl)-4-(*R-*cyclopropylsulfonimidoyl)-*N*-(6-((2*R*)-2-methyl-4-morpholinyl)-2-pyridinyl)benzamide | 0.043 |
| **13-1** | *N*-(6-((R)-2-Methylmorpholino)pyridin-2-yl)-4-((*R*)-2-methylpropan-2-ylsulfonimidoyl)-2-(6-azaspiro[2.5]octan-6-yl)benzamide | 0.199 |
| **13-2** | *N*-(6-((*R*)-2-Methylmorpholino)pyridin-2-yl)-4-((*S*)-2-methylpropan-2-ylsulfonimidoyl)-2-(6-azaspiro[2.5]octan-6-yl)benzamide | 0.182 |
| **13-3** | 2-(6-Azaspiro[2.5]octan-6-yl)-*N-*(6-((2*R*)-2-methyl-4-morpholinyl)-2-pyridinyl)-4-(*S-*methylsulfonimidoyl)benzamide | 0.418 |
| **13-4** | 2-(6-Azaspiro[2.5]octan-6-yl)-*N*-(6-((2*R*)-2-methyl-4-morpholinyl)-2-pyridinyl)-4-(*R-*methylsulfonimidoyl)benzamide | 0.154 |
| **14** | *N*-(6-(Cyclopropylmethoxy)pyridin-2-yl)-4-((1-methylcyclopropane)-1-sulfonamido)-2-(6-azaspiro[2.5]octan-6-yl)benzamide | 0.019 |
| **15** | *N*-(6-(Cyclopropylmethoxy)pyridin-2-yl)-4-((1-methylcyclopropane)-1-sulfonamido)-2-(6-azaspiro[2.5]octan-6-yl)benzamide | 0.045 |
| **16** | *N*-(6-(2-Hydroxy-2-methylpropoxy)pyridin-2-yl)-4-((2-hydroxyethyl)sulfonamido)-2-(6-azaspiro[2.5]octan-6-yl)benzamide | 0.080 |
| **16-1** | *N-*(6-(1-Cyclopropylethoxy)pyridin-2-yl)-4-((2-hydroxyethyl)sulfonamido)-2-(6-azaspiro[2.5]octan-6-yl)benzamide | 0.051 |
| **16-2** | 4-((2-Hydroxyethyl)sulfonamido)-*N*-(6-((3-methyloxetan-3-yl)methoxy)pyridin-2-yl)-2-(6-azaspiro[2.5]octan-6-yl)benzamide | 0.050 |
| **16-3** | 4-((2-Hydroxyethyl)sulfonamido)-*N*-(6-(1-methylazetidin-3-yl)methoxy)pyridin-2-yl)-2-(6-azaspiro[2.5]octan-6-yl)benzamide | 0.063 |
| **16-4** | 4-((2-Hydroxyethyl)sulfonamido)-*N*-(6-(2-hydroxypropoxy)pyridin-2-yl)-2-(6-azaspiro[2.5]octan-6-yl)benzamide | 0.115 |
| **16-5** | *N*-(6-(3-Hydroxy-3-methylbutoxy)pyridin-2-yl)-4-((2-hydroxyethyl)sulfonamido)-2-(6-azaspiro[2.5]octan-6-yl)benzamide | 0.084 |
| **16-6** | 4-((2-Hydroxyethyl)sulfonamido)-2-(6-azaspiro[2.5]octan-6-yl)-N-(6-(4,4,4-trifluoro-3-hydroxybutoxy)pyridin-2-yl)benzamide | 0.081 |
| **16-7** | *N*-(6-(3-Hydroxybutoxy)pyridin-2-yl)-4-((2-hydroxyethyl)sulfonamido)-2-(6-azaspiro[2.5]octan-6-yl)benzamide | 0.031 |
| **16-8** | 4-((2-Hydroxyethyl)sulfonamido)-2-(6-azaspiro[2.5]octan-6-yl)-*N*-(6-(3,3,3-trifluoro-2-hydroxypropoxy)pyridin-2-yl)benzamide | 0.018 |
| **16-9** | *N*-(6-(3,3-Diffuoropropoxy)pyridin-2-yl)-4-((2-hydroxyethyl)sulfonamido)-2-(6-azaspiro[2.5]octan-6-yl)benzamide | 0.051 |
| **17** | 4-((2-Hydroxyethyl)sulfonamido)-*N*-(4-methyl-6-(3,3,3-trifluoropropoxy)pyridin-2-yl)-2-(6-azaspiro[2.5]octan-6-yl)benzamide | 0.024 |
| **17-1** | 4-((2-Hydroxyethyl)sulfonamido)-*N*-(4-methyl-6-(3,3,3-trifluoro-2-hydroxypropoxy)pyridin-2-yl)-2-(6-azaspiro[2.5]octan-6-yl)benzamide | 0.068 |
| **17-2** | *N*-(6-(2-Hydroxy-2-methylpropoxy)-4-methylpyridin-2-yl)-4-((2-hydroxyethyl)sulfonamido)-2-(6-azaspiro[2.5]octan-6-yl)benzamide | 0.059 |
| **17-3** | 4-((2-Hydroxyethyl)sulfonamido)-*N*-(4-methyl-6-(4,4,4-trifluorobutoxy)pyridin-2-yl)-2-(6-azaspiro[2.5]octan-6-yl)benzamide | 0.184 |
| **18** | 4-((2-Hydroxyethyl)sulfonamido)-*N*-(5-methyl-6-morpholinopyridin-2-yl)-2-(6-azaspiro[2.5]octan-6-yl)benzamide | 0.012 |
| **19** | (*R*)-*N*-(4-cyclopropyl-6-(2-methylmorpholino)pyridin-2-yl)-4-((2-hydroxyethyl)sulfonamido)-2-(6-azaspiro[2.5]octan-6-yl)benzamide | 0.087 |
| **20** | (*R*)-4-((2-Hydroxyethyl)sulfonamido)-*N*-(6-(2-methylmorpholino)-4-(trifluoromethyl)pyridin-2-yl)-2-(6-azaspiro[2.5]octan-6-yl)benzamide | 0.093 |
| **21** | *N*-(5-Cyano-6-(4,4-difluoropiperidin-1-yl)pyridin-2-yl)-4-((2-hydroxyethyl)sulfonamido)-2-(6-azaspiro[2.5]octan-6-yl)benzamide | 0.025 |
| **22** | *N-*(6-(4,4-Difluoropiperidin-1-yl)-5-methylpyridin-2-yl)-4-((2-hydroxyethyl)sulfonamido)-2-(6-azaspiro[2.5]octan-6-yl)benzamide | 0.041 |
| **23** | *N*-(6-(4,4-difluorocyclohexyl)-4-methylpyridin-2-yl)-4-((2-hydroxyethyl)sulfonamido)-2-(6-azaspiro[2.5]octan-6-yl)benzamide | 0.041 |
| **24** | (*R*)-*N-*(5-fluoro-6-(2-methylmorpholino)pyridin-2-yl)-4-((2-hydroxyethyl)sulfonamido)-2-(6-azaspiro[2.5]octan-6-yl)benzamide | 0.033 |
| **25** | (*R*)-*N*-(4-Cyano-6-(2-methylmorpholino)pyridin-2-yl)-4-((2-hydroxyethyl)sulfonamido)-2-(6-azaspiro[2.5]octan-6-yl)benzamide | 0.041 |
| **26** | 4-((2-Hydroxyethyl)sulfonamido)-*N*-(6-(2-methylpropan-2-ylsulfonimidoyl)pyridin-2-yl)-2-(6-azaspiro[2.5]octan-6-yl)benzamide | 0.363 |
| **27-1** | 2-(6-Azaspiro [2.5] octan-6-yl)-4-(*S-*cyclopropylsulfonimidoyl)-*N*-(6-(3,3,3-trifluoropropoxy)-2-pyridinyl)benzamide | 0.022 |
| **27-2** | 2-(6-Azaspiro[2.5]octan-6-yl)-4-(*R-*cyclopropylsulfonimidoyl)-*N*-(6-(3,3,3-trifluoropropoxy)-2-pyridinyl)benzamide | 0.016 |
| **28-1** | 2-(6-Azaspiro[2.5]octan-6-yl)-4-(*S-*cyclopropylsulfonimidoyl)-*N*-(6-(3,3-difluoro-1- azetidinyl)-4-methyl-2-pyridinyl)benzamide | 0.071 |
| **28-2** | 2-(6-Azaspiro[2.5]octan-6-yl)-4-(*R-*cyclopropylsulfonimidoyl)-*N*-(6-(3,3-difluoro-1-azetidinyl)-4-methyl-2-pyridinyl)benzamide | 0.037 |
| **29-1** | 2-(6-Azaspiro[2.5]octan-6-yl)-4-(*S-*cyclopropylsulfonimidoyl)-*N*-(4-methyl-6-((2*R*)-2-methyl-4-morpholinyl)-2-pyridinyl)benzamide | 0.165 |
| **29-2** | 2-(6-Azaspiro[2.5]octan-6-yl)-4-(*R-*cyclopropylsulfonimidoyl)-*N*-(4-methyl-6-((2*R*)-2-methyl-4-morpholinyl)-2-pyridinyl)benzamide | 0.059 |
| **30-1** | 2-(6-Azaspiro[2.5]octan-6-yl)-4-(*S-*cyclopropylsulfonimidoyl)-*N*-(6-(4,4-difluoro-1- piperidinyl)-4-methyl-2-pyridinyl)benzamide | 0.081 |
| **30-2** | 2-(6-Azaspiro[2.5]octan-6-yl)-4-(*R-*cyclopropylsulfonimidoyl)-*N*-(6-(4,4-difluoro-1- piperidinyl)-4-methyl-2-pyridinyl)benzamide | 0.042 |
| **31** | 4-(Azetidin-3-ylsulfonyl)-2-(6-azaspiro[2.5]octan-6-yl)-*N*-(6-(3,3,3-trifluoropropoxy)pyridin-2-yl)benzamide trifluoroacetate | 0.055 |

The foregoing invention has been described in some detail by way of illustration and example, for purposes of clarity and understanding. Those skilled in the art understand that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims.

## Claims

1. A compound of formula I: 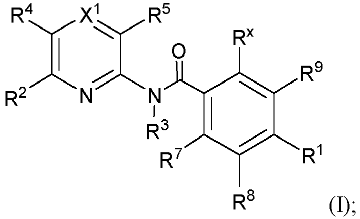 or any pharmaceutically-acceptable salt thereof, wherein:
X¹ is N or -CR⁶;
R¹ is a group -Z-R¹² wherein Z is -C₀₋₄alk-, -NR¹¹-, -NR¹¹SO₂-C₀₋₄alk-, -SO₂NR¹¹-C₀₋₄alk-, -NR¹¹SO₂NR¹¹-, -NR¹¹SO₂NR¹¹-C(=O)-O-, -C₀₋₄alk-S(=O)(=NH)-, C₀₋₄alk-NR¹¹-S(=O)(=NH), -C₀₋₄alk-S-, -C₀₋₄alk-S(=O)-, -C₀₋₄alk-SO₂-, C₀₋₄alk-O-, -P-, -P(=O), -P(=O)₂, -(C=O)-, -(C=O)NR¹¹-, -C=N(OH)-, or
-NR¹¹(C=O); or
the group -Z-R¹² is -N=S(=O)-(R¹²)₂, wherein the two R¹² pair can alternatively combine with the sulfur atom attached to each of them to form a saturated or partially-saturated 3-, 4-, 5-, or 6-membered monocyclic ring containing 0, 1, 2 or 3 N atoms and 0, 1, or 2 atoms selected from O and S;
R² is halo or a group -Y-R¹³, wherein Y is -C₀₋₄alk-, -N(C₀₋₁alk)-C₀₋₄alk-, -C(=O)NR^{a}(C₁₋₄alk)-, -O-C₀₋₄alk-, -S-, -S=O, -S(=O)₂-, -SO₂N(C₀₋₁alk)-C₀₋₄alk-, -N(C₀₋₁alk)-SO₂-C₀₋₄alk-, -C₀₋₄alk-S(=O)(=NH)-, -(C=O)-, -C₀₋₄alk-(C=O)-O-; or
the group -Y-R¹³ is -N=S(=O)-(R¹³)₂, wherein the two R¹³ pair can alternatively combine with the sulfur atom attached to each of them to form a saturated or partially-saturated 3-, 4-, 5-, or 6-membered monocyclic ring containing 0, 1, 2 or 3 N atoms and 0, 1, or 2 atoms selected from O and S;
R³ is H, methyl, or ethyl;
R⁴ is H, halo, CN, C₁₋₄alk, or C₁₋₄haloalk;
R⁵ is H, halo, C₁₋₈alk, or C₁₋₄haloalk;
R⁶ is H, halo, CN, C₁₋₈alk, C₁₋₄haloalk, -O-C₀₋₆alk-, or R^{6a};
R⁷ is H, halo, C₁₋₈alk, or C₁₋₄haloalk;
R⁸ is H, halo, C₁₋₈alk, or C₁₋₄haloalk;
R⁹ is H, halo, C₁₋₈alk, or C₁₋₄haloalk;
R^{x} is selected from the group consisting of 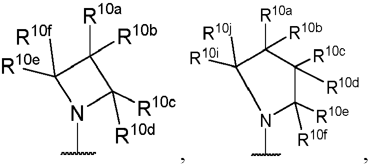 and 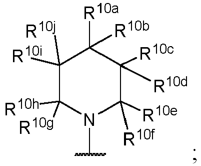
Each of R^{10a}, R^{10b}, R^{10c}, R^{10d}, R^{10e}, R^{10f}, R^{10g}, R^{10h}, R¹⁰ⁱ, and R^{10j} is H, halo, R^{10k}, or R^{10l};
or alternatively, each of R^{10a} and R^{10b} pair, R^{10c} and R^{10d} pair, R^{10e} and R^{10f} pair, R^{10g} and R^{10h} pair, or R¹⁰ⁱ and R^{10j} pair, independently, can combine with the carbon atom attached to each of them to form a saturated or partially-saturated 3-, 4-, 5-, 6-membered monocyclic ring spiro to the R^{x} ring; wherein said 3-, 4-, 5-, 6-membered monocyclic ring contains 0, 1, 2 or 3 N atoms and 0, 1, or 2 atoms selected from O and S, and further wherein said 3-, 4-, 5-, 6-membered monocyclic ring is substituted by 0, 1, 2 or 3 group(s) selected from F, Cl, Br, C₁₋₆alk, C₁₋₄haloalk, -OR^{a}, -OC₁₋₄haloalk, CN, -NR^{a}R^{a}, or oxo;
R¹¹ is H or C₁₋₈alk;
R¹² is H, R^{12a}, or R^{12b};
R¹³ is R^{13a} or R^{13b};
R^{6a}, R^{10k}, R^{12a}, and R^{13a} is independently, at each instance, selected from the group consisting of a saturated, partially-saturated or unsaturated 3-, 4-, 5-, 6-, or 7-membered monocyclic or 4-, 5-, 6-, 7-, 8-, 9-, 10-, 11-, or 12-membered bicyclic ring containing 0, 1, 2 or 3 N atoms and 0, 1, or 2 atoms selected from O and S, which is substituted by 0, 1, 2 or 3 group(s) selected from F, Cl, Br, C₁₋₆alk,
C₁₋₄haloalk, -OR^{a}, -OC₁₋₄haloalk, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆alkNR^{a}R^{a}, -OC₂₋₆alkOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkNR^{a}R^{a}, -NR^{a}C₂₋₆alkOR^{a}, -C₁₋₆alkNR^{a}R^{a}, -C₁₋₆alkOR^{a}, -C₁₋₆alkN(R^{a})C(=O)R^{b}, -C₁₋₆alkOC(=O)R^{b}, -C₁₋₆alkC(=O)NR^{a}R^{a}, -C₁₋₆alkC(=O)OR^{a}, R¹⁴, and oxo;
R^{10l}, R^{12b}, and R^{13b} is independently, at each instance, selected from the group consisting of C₁₋₆alk substituted by 0, 1, 2, 3, 4, or 5 group(s) selected from F, Cl, Br, -C(=O)OR^{a}, -OR^{a}, -C₁₋₂haloalk, -OC₁₋₄haloalk, CN, NH₂, NH(CH₃), or N(CH₃)₂;
R¹⁴ is independently, at each instance, selected from the group consisting of a saturated, partially-saturated or unsaturated 3-, 4-, 5-, 6-, or 7-membered monocyclic or 4-, 5-, 6-, 7-, 8-, 9-, 10-, 11-, or 12-membered bicyclic ring containing 0, 1, 2 or 3 N atoms and 0, 1, or 2 atoms selected from O and S, which is substituted by 0, 1, 2 or 3 group(s) selected from F, Cl, Br, C₁₋₆alk, C₁₋₄haloalk, -OR^{a}, -OC₁₋₄haloalk, CN, -C(=O)R^{b}, -C(=O)OR^{a}, -C(=O)NR^{a}R^{a}, -C(=NR^{a})NR^{a}R^{a},
-OC(=O)R^{b}, -OC(=O)NR^{a}R^{a}, -OC₂₋₆alkNR^{a}R^{a}, -OC₂₋₆alkOR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆alkNR^{a}R^{a}, -NR^{a}C₂₋₆alkOR^{a}, -C₁₋₆alkNR^{a}R^{a}, -C₁₋₆alkOR^{a}, -C₁₋₆alkN(R^{a})C(=O)R^{b}, -C₁₋₆alkOC(=O)R^{b}, -C₁₋₆alkC(=O)NR^{a}R^{a}, -C₁₋₆alkC(=O)OR^{a}, and oxo;
R^{a} is independently, at each instance, H or R^{b}; and
R^{b} is independently, at each instance, C₁₋₆alk, phenyl, or benzyl, wherein the C₁₋₆alk is being substituted by 0, 1, 2 or 3 substituents selected from halo, -OH, -OC₁₋₄alk, -NH₂, -NHC₁₋₄alk, -OC(=O)C₁₋₄alk, or -N(C₁₋₄alk)C₁₋₄alk; and the phenyl or benzyl is being substituted by 0, 1, 2 or 3 substituents selected from halo, C₁₋₄alk, C₁₋₃haloalk, -OH, -OC₁₋₄alk, -NH₂, -NHC₁₋₄alk, -OC(=O)C₁₋₄alk, or -N(C₁₋₄alk)C₁₋₄alk.

2. The compound of Claim 1 wherein R^{x} is

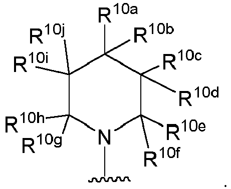

3. The compound of any one of Claims 1-2 wherein:
X' is -CR⁶; having the formula (Ia): 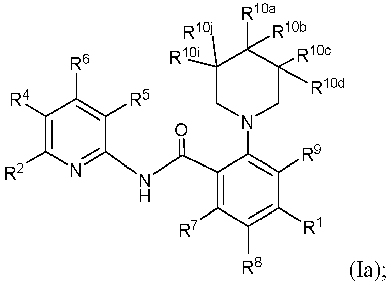 or
X' is N; having the formula (Ib):

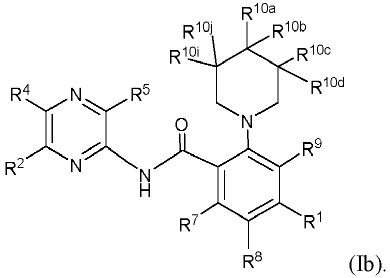

4. The compound of any one of Claims 1-3 wherein R³ is H or methyl.

5. The compound of any one of Claims 1-4 wherein each of R^{10c}, R^{10d}, R^{10e}, R^{10f}, R^{10g}, R^{10h}, R¹⁰ⁱ, and R^{10j} is H, halo, C₁₋₆alk, or C₁₋₄haloalk; and each of R^{10a} and R^{10b} pair combine with the carbon atom attached to each of them form a saturated 3-, 4-, or 5-membered monocyclic ring spiro to the R^{x} ring; wherein said ring contains 0, 1, 2 or 3 N atoms and 0 or 1 atoms selected from O and S; or
wherein each of R^{10c}, R^{10d}, R^{10e}, R^{10f}, R^{10g}, R^{10h}, R¹⁰ⁱ, and R^{10j} is H, methyl, or ethyl; and each of R^{10a} and R^{10b} pair combine with the carbon atom attached to each of them form a cyclopropyl, cyclobutyl, or cyclopentyl ring spiro to the R^{x} ring.

6. The compound of any one of Claims 1-5 wherein R^{x} is selected from:

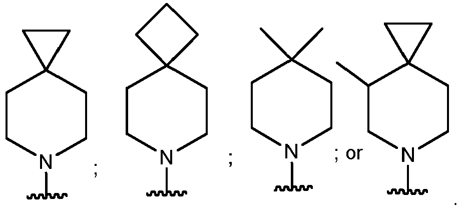

7. The compound of any one of Claims 1-6 wherein R^{x} is

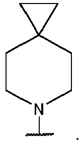

8. The compound of any one of Claims 1-7 wherein Z is absent, NH-, -NHSO₂-(CH₂)₀₋₄-, - N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-,-(CH₂)₀₋₂-S(=O)-, (CH₃CH)-S(=O)-, -(CH₂)₀₋₂-SO₂-, -O-, -P(=O), -(C=O)-, or -NH(C=O).

9. The compound of any one of Claims 1-8 wherein
R¹ is the group -Z-R¹² which is -N=S(=O)-(R¹²)₂, wherein the two R¹² pair can alternatively combine with the sulfur atom attached to each of them to form a saturated or partially-saturated 3-, 4-, 5-, or 6-membered monocyclic ring containing 0, 1, 2 or 3 N atoms and 0, 1, or 2 atoms selected from O and S; which is selected from: 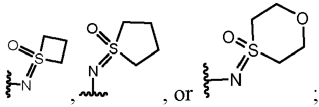 or
R¹ is -Z-R¹², wherein Z is absent, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-, -(CH₂)₀₋₂-S(=O)-, (CH₃CH)-S(=O)-, -(CH₂)₀₋₂-SO₂-, -O-, -P(=O), -(C=O)-, or -NH(C=O); and R¹² is selected from:
(a) H;
**(b)** cyclopropyl, cyclobutyl, cyclopentyl, cyclohexyl, oxiranyl, oxetanyl, tetrahydrofuranyl, azetidinyl, imidazolyl, morpholinyl, pyrrolidinyl, piperazinyl, 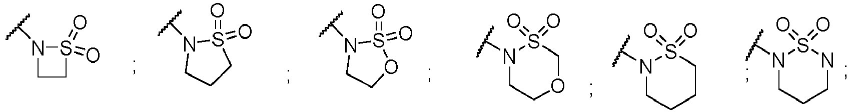 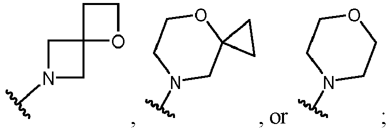 wherein each said ring is substituted by 0, 1, 2 or 3 group(s) selected from wherein each ring is substituted by 0, 1, 2 or 3 OH, F, methyl, -CH₂OH, -C(=O)OCH₃, - C(=O)OC(CH₃)₃, NH₂, CN, and oxo; or
(c) C₁₋₆alk substituted by 0, 1, 2 or 3 OH, F, -C(=O)OCH₃, -NH₂, -NH(CH₃), or
-N(CH₃)₂; or
R¹ is a group -Z-R¹², wherein Z is -NHSO₂- or -SO₂NH-; and R¹² is oxetanyl, cyclopropyl, or R¹² is C₁₋₆alk substituted by 0, 1, 2 or 3 OH group(s); or
R¹ is a group -Z-R¹², wherein Z is -NHSO₂- and R¹² is -CH₂-CH₂-OH.

10. The compound of any one of Claims 1-3, 4-5, or 6-9
wherein R² is halo or a group -Y-R¹³, wherein Y is absent, -SO₂NH-(CH₂)₀₋₄-, NH-, -NH-SO₂-(CH)₂)₀₋₄-, -O-(CH₂)₀₋₄, -O-(CH(CH₃))-, -(CH₂)₀₋₄-S(=O)(=NH)-, -(C=O)-, -(CH₂)₀₋₄-(C=O)-O-, or -(CH₂)₁₋₄;
R¹³ is a saturated, partially-saturated or unsaturated 3-, 4-, 5-, 6-, or 7-membered monocyclic or 8-, 9-, 10-, 11-, or 12-membered bicyclic ring containing 0, 1, 2 or 3 N atoms and 0 or 1 atoms selected from O and S, which is substituted by 0, 1, 2 or 3 group(s) selected from F, Cl, Br, C₁₋₆alk, C₁₋₄haloalk, -OH, -OC₁₋₄haloalk, CN, R¹⁴, and oxo; or;
R¹³ is C₁₋₆alk substituted by 0, 1, 2, 3, 4, or 5 group(s) selected from F, Cl, Br, -OH, -OC₁₋₄haloalk, or CN; or
wherein R² is a saturated 5- or 6-membered monocyclic ring wherein each said ring contains 0, 1, or 2 N atoms and 0 or 1 O atom, and wherein each said ring is substituted by 0, 1, 2 or 3 group(s) selected from F, Cl, Br, C₁₋₆alk, C₁₋₄haloalk, -OH, -OCH₃, -OC₁₋₄haloalk, CN, R¹⁴, and oxo; or
wherein R² is:
(a) F, Br;
(b) a group -Y-R¹³, wherein Y is absent; and R¹³ is morpholinyl, piperidinyl, azetidinyl, pyrrolidinyl, cyclopropyl, cyclobutyl, cyclopentyl, cyclohexyl, piperazinyl, tetrahydrofuranyl, tetrahydropyranyl, pyridinyl, pyrimidinyl, 3,6-dihydro-2H-pyranyl, 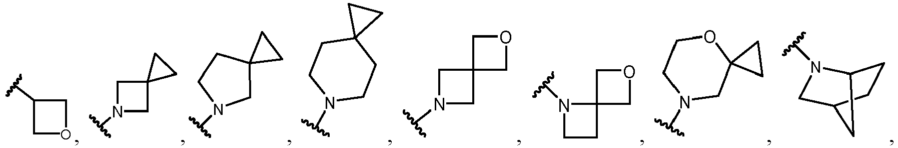 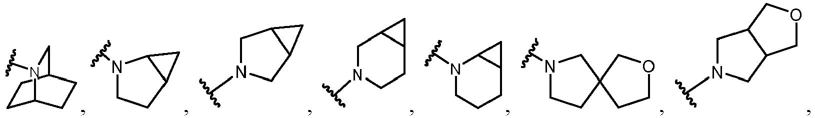 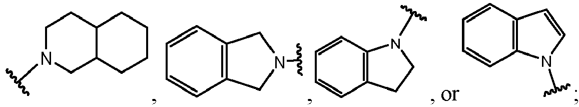 wherein each said ring is substituted by 0, 1, 2 or 3 group(s) selected from F, Cl, Br, methyl, CF₃, CH₂OH, CH(CH₃)OH, C(CH₃)₂OH, -OH, -OCHF₂, CN, oxo, or cyclopropyl; or
(c) a group -Y-R¹³, wherein Y is absent, -SO₂NH-, NH, -O-, S(=O)(=NH)- , -O-(CH₂), -O-(CH(CH₃))-, C(=O)-, C(=O)-O-, -CH₂C(=O)-O-, or -CH₂-; and wherein R¹³ is 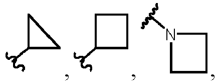 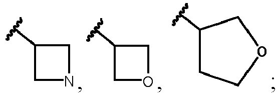 wherein each ring is substituted by 0, 1, 2, 3, 4, or 5 group(s) selected from F, Cl, Br, methyl, CF₃, -OH, or CN;
or R¹³ is H or C₁₋₆alk substituted by 0, 1, 2, 3, 4, or 5 group(s) selected from F, Cl, Br, methyl, CF₃, -OH, or CN; or
wherein R² is (a) halo; (b) a group -Y-R¹³, wherein Y is absent; and R¹³ is morpholinyl, piperidinyl, azetidinyl, pyrrolidinyl, cyclopropyl, cyclobutyl, cyclopentyl, cyclohexyl, piperazinyl, tetrahydrofuranyl, 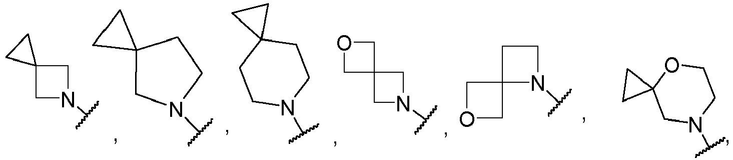 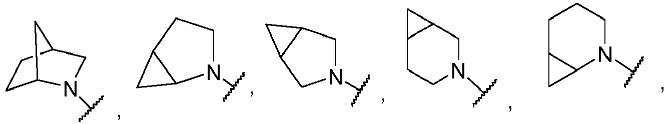 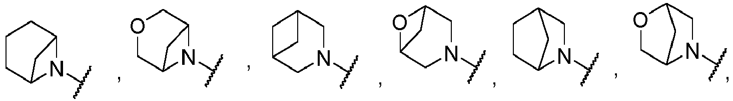 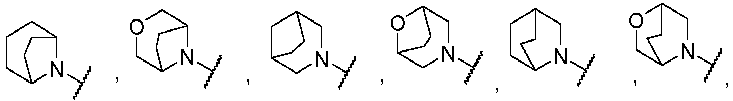 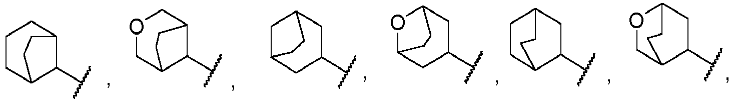 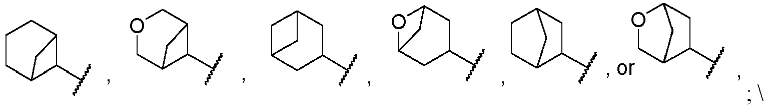 wherein each said ring is substituted by 0, 1, 2 or 3 group(s) selected from F, Cl, Br, methyl, CF₃, -OH, -OCHF₂, CN, and oxo; or (c) a group -Y-R¹³, wherein Y is NH, -O-, -O-(CH₂)-, -O-(CH₂)-(CH₂)-, or
-O-(CH₂)-(CH₂)-(CH₂)-, and wherein R¹³ is 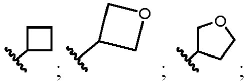 or R¹³ is C₁₋₆alk substituted by 0, 1, 2, 3, 4, or 5 group(s) selected from F, Cl, Br, methyl, CF₃, -OH, or CN; or
wherein R² is 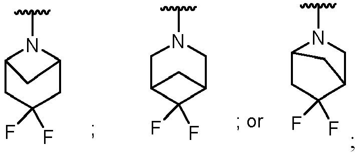 or
wherein R² is morpholinyl or piperidinyl substituted by 0, 1, 2 or 3 group(s) selected from F, Cl, Br, methyl, CF₃, -OH, -OCHF₂, CN, or oxo; or
wherein R² is morpholinyl substituted by 1, 2 or 3 methyl group(s).

11. The compound of any one of Claims 1-10
wherein R⁴ is selected from H, F, methyl, CN, or Br; or
wherein R⁴ is H.

12. The compound of any one of Claims 1-11
wherein R⁵ is H.

13. The compound of any one of Claims 1-12
wherein R⁶ is H, methyl, cyclopropyl, CN, CF₃, or azetidinyl.

14. The compound of any one of Claims 1-13
wherein R⁷ is H.

15. The compound of any one of Claims 1-14
wherein R⁸ is H or F.

16. The compound of any one of Claims 1-15
wherein R⁹ is H or F.

17. The compound of claim 1, selected from the group:
| Ex. # | Chemical Structure | Name |
|---|---|---|
| 1 | 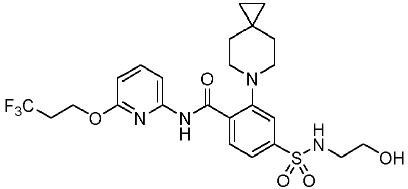 | 4-(*N*-(2-Hydroxyethyl)sulfamoyl)-2-(6-azaspiro[2.5]octan-6-yl)-N-(6-(3,3,3-trifluoropropoxy)pyridin-2-yl)benzamide |
| 1-1 | 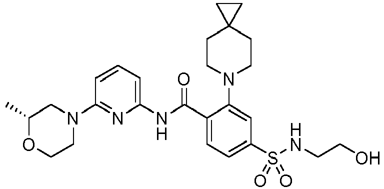 | (*R*)-4-(*N*-(2-Hydroxyethyl)sulfamoyl)-*N*-(6-(2-methylmorpholino)pyridin-2-yl)-2-(6-azaspiro[2.5]octan-6-yl)benzamide |
| 1-2 | 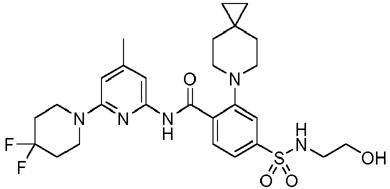 | *N*-(6-(4,4-Difluoropiperidin-1-yl)-4-methylpyridin-2-yl)-4-(*N*-(2-hydroxyethyl)sulfamoyl)-2-(6-azaspiro[2.5]octan-6-yl)benzamide |
| 2 | 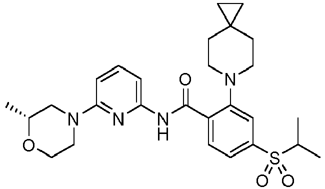 | (*R*)-4-(Isopropylsulfonyl)-*N*-(6-(2-methylmorpholino)pyridin-2-yl)-2-(6-azaspiro[2.5]octan-6-yl)benzamide |
| 2-7 | 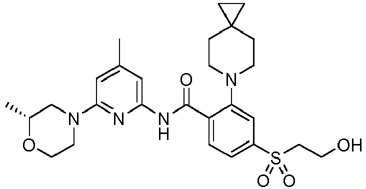 | (*R*)-4-((2-Hydroxyethyl)sulfonyl)-*N*-(4-methyl-6-(2-methylmorpholino)pyridin-2-yl)-2-(6-azaspiro[2.5]octan-6-yl)benzamide |
| 3 | 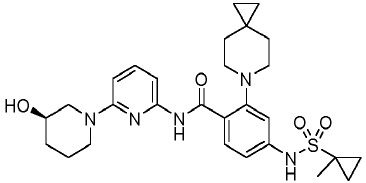 | (*R*)-*N*-(6-(3 -Hydroxypiperidin-1 - yl)pyridin-2-yl)-4-((1-methyl cyclopropane)-1 - sulfonamido)-2-(6-azaspiro[2.5]octan-6-yl)benzamide |
| 4 | 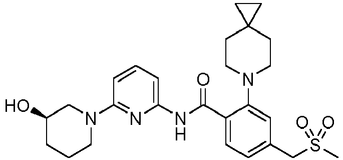 | (*R*)-*N*-(6-(3 -hydroxypiperidin-1-yl)pyridin-2-yl)-4-((methylsulfonyl)methyl)-2-(6-azaspiro[2.5]octan-6-yl)benzamide |
| 5 | 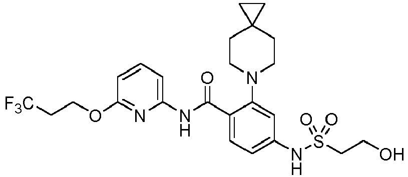 | 4-((2-Hydroxyethyl)sulfonamido)-2-(6-azaspiro[2.5]octan-6-yl)-N-(6-(3,3,3 -trifluoropropoxy)pyridin-2-yl)benzamide |
| 6 | 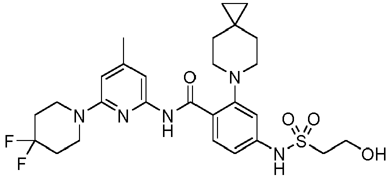 | *N*-(6-(4,4-Difluoropiperidin-1-yl)-4-methylpyridin-2-yl)-4-((2-hydroxyethyl)sulfonamido)-2-(6-azaspiro[2.5]octan-6-yl)benzamide |
| 7-1 | 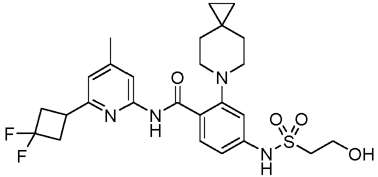 | *N*-(6-(3,3-Difluorocyclobutyl)-4-methylpyridin-2-yl)-4-((2-hydroxyethyl)sulfonamido)-2-(6-azaspiro[2.5]octan-6-yl)benzamide |
| 7-12 | 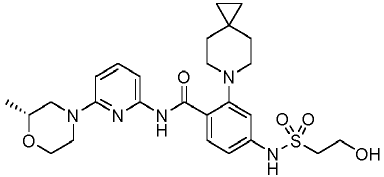 | (*R*)-4-((2-Hydroxyethyl)sulfonamido)-*N*-(6-(2-methylmorpholino)pyridin-2-yl)-2-(6-azaspiro[2.5]octan-6-yl)benzamide |
| 7-33 | 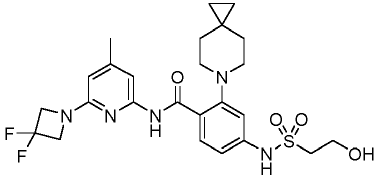 | *N*-(6-(3,3-Difluoroazetidin-1-yl)-4-methylpyridin-2-yl)-4-((2-hydroxyethyl)sulfonamido)-2-(6-azaspiro[2.5]octan-6-yl)benzamide |
| 7-44 | 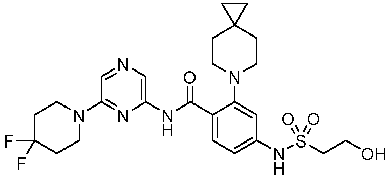 | *N*-(6-(4,4-Difluoropiperidin-1- yl)pyrazin-2-yl)-4-((2-hydroxyethyl)sulfonamido)-2-(6-azaspiro[2.5]octan-6-yl)benzamide |
| 8-1 | 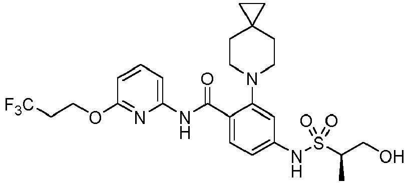 | (*R*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide |
| 8-2 | 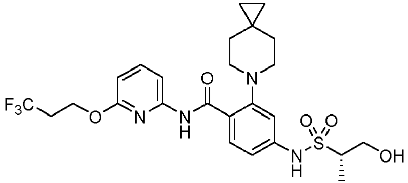 | (*S*)-4-((2-Hydroxy-1-methylethyl)sulfonamido)-2-(6-azaspiro[2.5]octan-6-yl)-*N*-(6-(3,3,3-trifluoropropoxy)pyridin-2-yl)benzamide |
| 9-7 | 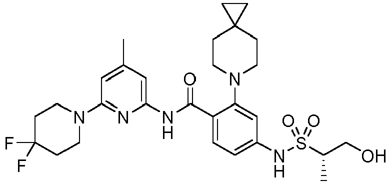 | (*S*)-*N*-(6-(4,4-Difluoropiperidin-1-yl)-4-methylpyridin-2-yl)-4-((2-hydroxy-1- methylethyl)sulfonamido)-2-(6-azaspiro[2.5]octan-6-yl)benzamide |
| 9-8 | 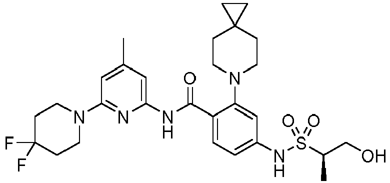 | (*R*)-*N*-(6-(4,4-Difluoropiperidin-1-yl)-4-methylpyridin-2-yl)-4-((2-hydroxy-1 - methylethyl)sulfonamido)-2-(6-azaspiro[2.5]octan-6-yl)benzamide |
| 10 | 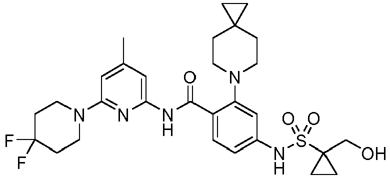 | *N*-(6-(4,4-Difluoropiperidin-1-yl)-4-methylpyridin-2-yl)-4-((1-(hydroxymethyl)cyclopropane)-1-sulfonamido)-2-(6-azaspiro[2.5]octan-6-yl)benzamide |
| 11 | 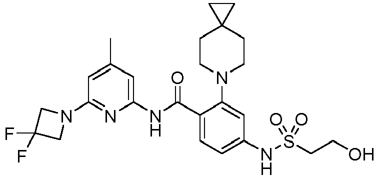 | *N*-(6-(3,3-Difluoroazetidin-1-yl)-4-methylpyridin-2-yl)-4-((2-hydroxyethyl)sulfonamido)-2-(6-azaspiro [2.5] octan-6-yl)benzamide |
| 14 | 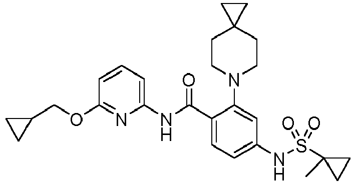 | *N*-(6-(Cyclopropylmethoxy)pyridin-2-yl)-4-((1-methylcyclopropane)-1-sulfonamido)-2-(6-azaspiro[2.5]octan-6-yl)benzamide |
| 16 | 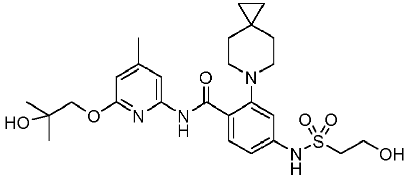 | *N*-(6-(2-Hydroxy-2-methylpropoxy)-4-methylpyridin-2-yl)-4-((2-hydroxyethyl)sulfonamido)-2-(6-azaspiro [2.5] octan-6-yl)benzamide |
| 17 | 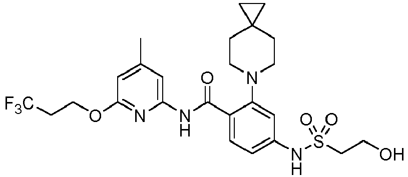 | 4-((2-Hydroxyethyl)sulfonamido)-*N*-(4-methyl-6-(3,3,3-trifluoropropoxy)pyridin-2-yl)-2-(6-azaspiro [2.5] octan-6-yl)benzamide |
| 21 | 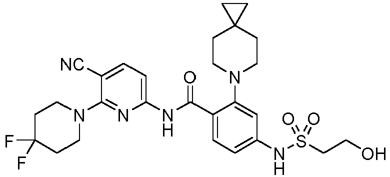 | *N*-(5-Cyano-6-(4,4-difluoropiperidin-1-yl)pyridin-2-yl)-4-((2-hydroxyethyl)sulfonamido)-2-(6-azaspiro [2.5] octan-6-yl)benzamide |
| 22 | 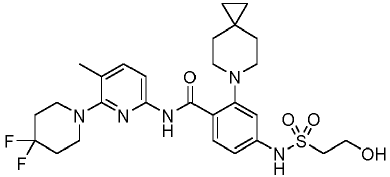 | *N*-(6-(4,4-Difluoropiperidin-1-yl)-5-methylpyridin-2-yl)-4-((2-hydroxyethyl)sulfonamido)-2-(6-azaspiro[2.5]octan-6-yl)benzamide |
| 23 | 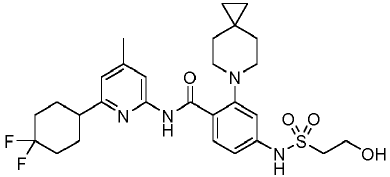 | *N*-(6-(4,4-difluorocyclohexyl)-4-methylpyridin-2-yl)-4-((2-hydroxyethyl)sulfonamido)-2-(6-azaspiro[2.5]octan-6-yl)benzamide |
| 24 | 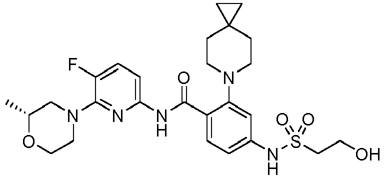 | (*R*)-N-(5-Fluoro-6-(2-methylmorpholino)pyridin-2-yl)-4-((2-hydroxyethyl)sulfonamido)-2-(6-azaspiro[2.5]octan-6-yl)benzamide |
| 25 | 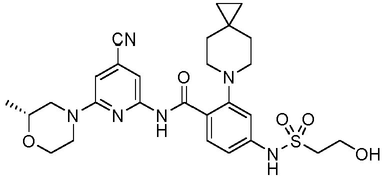 | (*R*)-*N*-(4-Cyano-6-(2-methylmorpholino)pyridin-2-yl)-4-((2-hydroxyethyl)sulfonamido)-2-(6-azaspiro[2.5]octan-6-yl)benzamide |
| 27-1 | 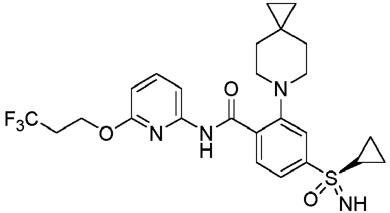 | 2-(6-Azaspiro[2.5]octan-6-yl)-4-(*S-*cyclopropylsulfonimidoyl)-*N*-(6-(3,3,3-trifluoropropoxy)-2-pyridinyl)benzamide |
| 27-2 | 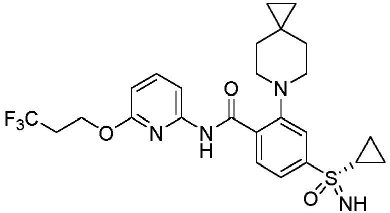 | 2-(6-Azaspiro[2.5]octan-6-yl)-4-(*R-*cyclopropylsulfonimidoyl)-*N*-(6-(3,3,3-trifluoropropoxy)-2-pyridinyl)benzamide |
; or any pharmaceutically-acceptable salt thereof.

18. The compound of anyone of Claims 1-17 for use as a medicament.

19. A pharmaceutical composition comprising the compound according to any one of Claims 1-17 or the pharmaceutically acceptable salt thereof, and a pharmaceutically-acceptable diluent or carrier.

20. The compound of anyone of Claims 1-17 or the composition comprising the compound of claim 19 for use in a method of treating a condition that may be treated with KIF18a inhibitors, the method comprising administering to a patient in need thereof a therapeutically effective amount of the compound;
wherein said condition is cancer selected from the group consisting of (a) a solid or hematologically derived tumor selected from cancer of the cancer of the bladder, endometrial, lung squamous cell, breast, colon, kidney, liver, lung, small cell lung cancer, esophagus, gall-bladder, brain, head and neck, ovary, pancreas, stomach, cervix, thyroid, prostate and skin, (b) a hematopoietic tumor of lymphoid lineage selected from leukemia, acute lymphocytic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma, (c) a hematopoietic tumor of myeloid lineage selected from acute and chronic myelogenous leukemias, myelodysplastic syndrome and promyelocytic leukemia (d) a tumor of mesenchymal origin selected from fibrosarcoma and rhabdomyosarcoma, (e) a tumor of the central and peripheral nervous system selected from astrocytoma, neuroblastoma, glioma and schwannoma, or (f) a melanoma, seminoma, teratocarcinoma, osteosarcoma, xeroderma pigmentosum, keratoacanthoma, thyroid follicular cancer or Kaposi's sarcoma.

21. The compound of anyone of Claims 1-17 or the composition comprising the compound of claim 19 for use in a method of reducing the size of a solid tumor in a subject; or
in a method of treating a cell proliferation disorder in a subject; or
in a method of inhibiting KIF18A in a cell.

## Patentansprüche

1. Eine Verbindung der Formel I: 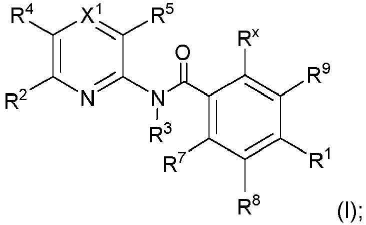 oder ein pharmazeutisch akzeptables Salz davon, wobei:
X¹ N oder-CR⁶ ist;
R¹ eine Gruppe -Z-R¹² ist, wobei Z -C₀₋₄-Alkyl-, -NR¹¹-, -NR¹¹SO₂-C₀₋₄-Alkyl-, -SO₂NR¹¹-C₀₋₄-Alkyl-, -NR¹¹SO₂NR¹¹-, -NR¹¹SO₂NR¹¹-C(=O)-O-, -C₀₋₄-Alkyl-S(=O)(=NH)-, C₀₋₄-Alkyl-NR¹¹-S(=O)(=NH), -C₀₋₄-Alkyl-S-, -C₀₋₄-Alkyl-S(=O)-, -C₀₋₄-Alkyl-SO₂-, C₀₋₄-Alkyl-O-, -P-, -P(=O), -P(=O)₂, -(C=O)-, -(C=O)NR¹¹-, -C=N(OH)- oder-NR¹¹(C=O) ist; oder
die Gruppe -Z-R¹² -N=S(=O)-(R¹²)₂ ist, wobei das Paar von zwei R¹² sich alternativ mit dem an jedem von ihnen befestigten Schwefelatom verbinden kann, sodass sich ein gesättigter oder teilweise gesättigter 3-, 4-, 5- oder 6-gliedriger monocyclischer Ring, enthaltend 0, 1, 2 oder 3 N-Atome und 0, 1 oder 2 aus O und S ausgewählte Atome, bildet;
R² Halogen oder eine Gruppe -Y-R¹³ ist, wobei Y -C₀₋₄-Alkyl-, -N(C₀₋₁-Alkyl)-C₀₋₄-Alkyl-, -C(=O)NR^{a}(C₁₋₄-Alkyl)-, -O-C₀₋₄-Alkyl-, -S-, -S=O, -S(=O)₂-, -SO₂N(C₀₋₁-Alkyl)-C₀₋₄-Alkyl-, -N(C₀₋₁-Alkyl)-SO₂-C₀₋₄-Alkyl-, -C₀₋₄-Alkyl-S(=O)(=NH)-, -(C=O)-, -C₀₋₄-Alkyl-(C=O)-O- ist; oder
die Gruppe -Y-R¹³ -N=S(=O)-(R¹³)₂ ist, wobei das Paar von zwei R¹³ sich alternativ mit dem an jedem von ihnen befestigten Schwefelatom verbinden kann, sodass sich ein gesättigter oder teilweise gesättigter 3-, 4-, 5- oder 6-gliedriger monocyclischer Ring,
enthaltend 0, 1, 2 oder 3 N-Atome und 0, 1 oder 2 aus O und S ausgewählte Atome, bildet;
R³ H, Methyl oder Ethyl ist;
R⁴ H, Halogen, CN, C₁₋₄-Alkyl oder C₁₋₄-Halogenalkyl ist;
R⁵ H, Halogen, C₁₋₈-Alkyl oder C₁₋₄-Halogenalkyl ist;
R⁶ H, Halogen, CN, C₁₋₈-Alkyl, C₁₋₄-Halogenalkyl, -O-C₀₋₆-Alkyl- oder R^{6a} ist;
R⁷ H, Halogen, C₁₋₈-Alkyl oder C₁₋₄-Halogenalkyl ist;
R⁸ H, Halogen, C₁₋₈-Alkyl oder C₁₋₄-Halogenalkyl ist;
R⁹ H, Halogen, C₁₋₈-Alkyl oder C₁₋₄-Halogenalkyl ist;
R^{x} aus der Gruppe ausgewählt ist, die aus 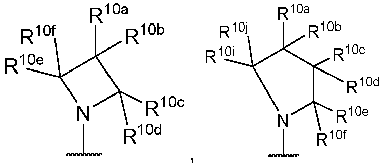 und 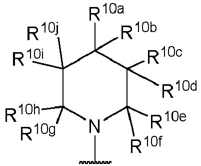 besteht;
jeder von R^{10a}, R^{10b}, R^{10c}, R^{10d}, R^{10e}, R^{10f}, R^{10g}, R^{10h}, R¹⁰ⁱ und R^{10j} H, Halogen, R^{10k} oder R^{10l} ist;
oder alternativ jeder von dem Paar R^{10a} und R^{10b}, dem Paar R^{10c} und R^{10d}, dem Paar R^{10e} und R^{10f}, dem Paar R^{10g} und R^{10h} oder dem Paar R¹⁰ⁱ und R^{10j} sich unabhängig mit dem an jedem von ihnen befestigten Kohlenstoffatom verbinden kann, sodass sich an dem R^{x}-Ring ein gesättigter oder teilweise gesättigter 3-, 4-, 5-, 6-gliedriger monocyclischer Spiroring bildet; wobei der 3-, 4-, 5-, 6-gliedrige monocyclische Ring 0, 1, 2 oder 3 N-Atome und 0, 1 oder 2 aus O und S ausgewählte Atome enthält, und wobei ferner der 3-, 4-, 5-, 6-gliedrige monocyclische Ring mit 0, 1, 2 oder 3 Gruppe(n), ausgewählt aus F, CI, Br, C₁₋₆-Alkyl, C₁₋₄-Halogenalkyl, -OR^{a}, -OC₁₋₄-Halogenalkyl, CN, -NR^{a}R^{a} oder Oxo, substituiert ist;
R¹¹ H oder C₁₋₈-Alkyl ist;
R¹² H, R^{12a} oder R^{12b} ist;
R¹³ R^{13a} oder R^{13b} ist;
R^{6a}, R^{10k}, R^{12a} und R^{13a} unabhängig in jedem Fall aus der Gruppe ausgewählt sind, die aus einem gesättigten, teilweise gesättigten oder ungesättigten 3-, 4-, 5-, 6- oder 7-gliedrigen monocyclischen oder 4-, 5-, 6-, 7-, 8-, 9-, 10-, 11- oder 12-gliedrigen bicyclischen Ring, enthaltend 0, 1, 2 oder 3 N-Atome und 0, 1 oder 2 aus O und S ausgewählte Atome, der mit 0, 1, 2 oder 3 Gruppe(n), ausgewählt aus F, Cl, Br, C₁₋₆-Alkyl, C₁₋₄-Halogenalkyl, -OR^{a}, -OC₁₋₄-Halogenalkyl, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆-Alkyl-NR^{a}R^{a}, -OC₂₋₆-Alkyl-OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-Alkyl-NR^{a}R^{a}, -NR^{a}C₂₋₆-Alkyl-OR^{a}, -C₁₋₆-Alkyl-NR^{a}R^{a}, -C₁₋₆-Alkyl-OR^{a}, -C₁₋₆-Alkyl-N(R^{a})C(=O)R^{b}, -C₁₋₆-Alkyl-OC(=O)R^{b}, -C₁₋₆-Alkyl-C(=O)NR^{a}R^{a}, -C₁₋₆-Alkyl-C(=O)OR^{a}, R¹⁴ und Oxo, substituiert ist, besteht;
R^{10l}, R^{12b} und R^{13b} unabhängig in jedem Fall aus der Gruppe ausgewählt sind, die aus C₁₋₆-Alkyl, substituiert mit 0, 1, 2, 3, 4 oder 5 Gruppe(n), ausgewählt aus F, Cl, Br, -C(=O)OR^{a}, -OR^{a}, -C₁₋₂-Halogenalkyl, -OC₁₋₄-Halogenalkyl, CN, NH₂, NH(CH₃) oder N(CH₃)₂, besteht;
R¹⁴ unabhängig in jedem Fall aus der Gruppe ausgewählt ist, die aus einem gesättigten, teilweise gesättigten oder ungesättigten 3-, 4-, 5-, 6- oder 7-gliedrigen monocyclischen oder 4-, 5-, 6-, 7-, 8-, 9-, 10-, 11- oder 12-gliedrigen bicyclischen Ring, enthaltend 0, 1, 2 oder 3 N-Atome und 0, 1 oder 2 aus O und S ausgewählte Atome, der mit 0, 1, 2 oder 3 Gruppe(n), ausgewählt aus F, Cl, Br, C₁₋₆-Alkyl, C₁₋₄-Halogenalkyl, -OR^{a}, -OC₁₋₄-Halogenalkyl, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆-Alkyl-NR^{a}R^{a}, -OC₂₋₆-Alkyl-OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆-Alkyl-NR^{a}R^{a}, -NR^{a}C₂₋₆-Alkyl-OR^{a}, -C₁₋₆-Alkyl-NR^{a}R^{a}, -C₁₋₆-Alkyl-OR^{a}, -C₁₋₆-Alkyl-N(R^{a})C(=O)R^{b}, -C₁₋₆-Alkyl-OC(=O)R^{b}, -C₁₋₆-Alkyl-C(=O)NR^{a}R^{a}, -C₁₋₆-Alkyl-C(=O)OR^{a} und Oxo, substituiert ist, besteht;
R^{a} unabhängig in jedem Fall H oder R^{b} ist; und
R^{b} unabhängig in jedem Fall C₁₋₆-Alkyl, Phenyl oder Benzyl ist, wobei das C₁₋₆-Alkyl mit 0, 1, 2 oder 3 Substituenten, ausgewählt aus Halogen, -OH, -OC₁₋₄-Alkyl, -NH₂, -NHC₁₋₄-Alkyl, -OC(=O)C₁₋₄-Alkyl oder-N(C₁₋₄-Alkyl)C₁₋₄-Alkyl, substituiert ist; und das Phenyl oder Benzyl mit 0, 1, 2 oder 3 Substituenten, ausgewählt aus Halogen, C₁₋₄-Alkyl, C₁₋₃-Halogenalkyl, -OH, -OC₁₋₄-Alkyl, -NH₂, -NHC₁₋₄-Alkyl, -OC(=O)C₁₋₄-Alkyl oder -N(C₁₋₄-Alkyl)C₁₋₄-Alkyl, substituiert ist.

2. Verbindung gemäß Anspruch 1, wobei R^{x} 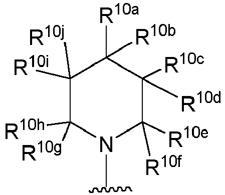 ist.

3. Verbindung gemäß einem der Ansprüche 1-2, wobei:
X¹ -CR⁶ ist; aufweisend die Formel (la): 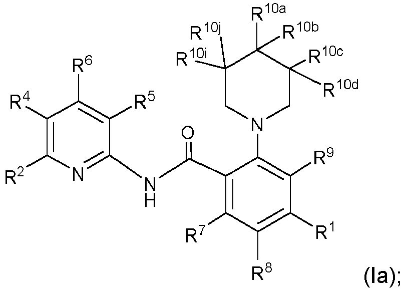 oder
X¹ N ist; aufweisend die Formel (Ib):

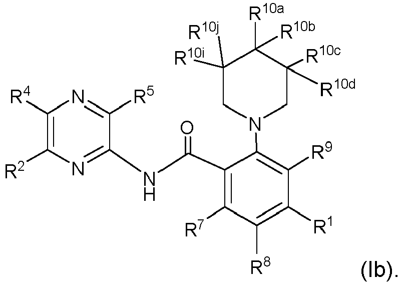

4. Verbindung gemäß einem der Ansprüche 1-3, wobei R³ H oder Methyl ist.

5. Verbindung gemäß einem der Ansprüche 1-4, wobei jeder von R^{10c}, R^{10d}, R^{10e}, R^{10f}, R^{10g}, R^{10h}, R¹⁰ⁱ und R^{10j} H, Halogen, C₁₋₆-Alkyl oder C₁₋₄-Halogenalkyl ist; und jeder von dem Paar R^{10a} und R^{10b} sich mit dem an jedem von ihnen befestigten Kohlenstoffatom verbindet, sodass sich an dem R^{x}-Ring ein gesättigter 3-, 4- oder 5-gliedriger monocyclischer Spiroring bildet; wobei der Ring 0, 1, 2 oder 3 N-Atome und 0 oder 1 aus O und S ausgewählte Atome enthält; oder
wobei jeder von R^{10c}, R^{10d}, R^{10e}, R^{10f}, R^{10g}, R^{10h}, R¹⁰ⁱ und R^{10j} H, Methyl oder Ethyl ist; und jeder von dem Paar R^{10a} und R^{10b} sich mit dem an jedem von ihnen befestigten Kohlenstoffatom verbindet, sodass sich an dem R^{x}-Ring ein Cyclopropyl-, Cyclobutyl- oder Cyclopentyl-Spiroring bildet.

6. Verbindung gemäß einem der Ansprüche 1-5, wobei R^{x} aus Folgendem ausgewählt ist:

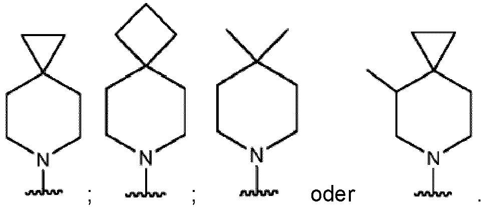

7. Verbindung gemäß einem der Ansprüche 1-6, wobei R^{x} 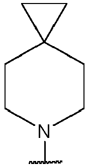 ist.

8. Verbindung gemäß einem der Ansprüche 1-7, wobei Z abwesend, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-,-(CH₂)₀₋₂-S(=O)-, (CH₃CH)-S(=O)-, -(CH₂)₀₋₂-SO₂-, -O-, -P(=O), -(C=O)- oder -NH(C=O) ist.

9. Verbindung gemäß einem der Ansprüche 1-8, wobei
R¹ die Gruppe -Z-R¹² ist, die -N=S(=O)-(R¹²)₂ ist, wobei das Paar von zwei R¹² sich alternativ mit dem an jedem von ihnen befestigten Schwefelatom verbinden kann, sodass sich ein gesättigter oder teilweise gesättigter 3-, 4-, 5- oder 6-gliedriger monocyclischer Ring, enthaltend 0, 1, 2 oder 3 N-Atome und 0, 1 oder 2 aus O und S ausgewählte Atome, bildet; der aus Folgendem ausgewählt ist: 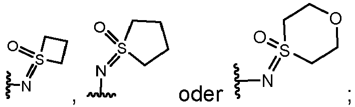 oder
R¹ -Z-R¹² ist, wobei Z abwesend, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-, -(CH₂)₀₋₂-S(=O)-, (CH₃CH)-S(=O)-, -(CH₂)₀₋₂-SO₂-, -O-, -P(=O), -(C=O)- oder -NH(C=O) ist; und R¹² aus Folgendem ausgewählt ist:
(a) H;
(b) Cyclopropyl, Cyclobutyl, Cyclopentyl, Cyclohexyl, Oxiranyl, Oxetanyl, Tetrahydrofuranyl, Azetidinyl, Imidazolyl, Morpholinyl, Pyrrolidinyl, Piperazinyl, 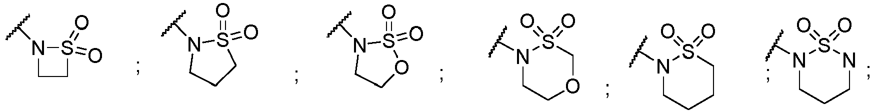 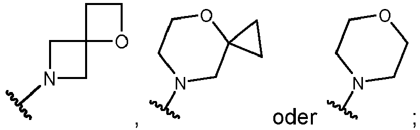 wobei jeder Ring mit 0, 1, 2 oder 3 Gruppe(n), ausgewählt aus, substituiert ist, wobei jeder Ring mit 0, 1, 2 oder 3 OH, F, Methyl, -CH₂OH, -C(=O)OCH₃, -C(=O)OC(CH₃)₃, NH₂, CN und Oxo substituiert ist; oder
(c) C₁₋₆-Alkyl, substituiert mit 0, 1, 2 oder 3 OH, F, -C(=O)OCH₃, -NH₂, -NH(CH₃) oder -N(CH₃)₂; oder
R¹ eine Gruppe -Z-R¹² ist, wobei Z -NHSO₂- oder-SO₂NH- ist; und R¹² Oxetanyl, Cyclopropyl ist oder R¹² C₁₋₆-Alkyl, substituiert mit 0, 1, 2 oder 3 OH-Gruppe(n) ist; oder R¹ eine Gruppe -Z-R¹² ist, wobei Z -NHSO₂- ist und R¹² -CH₂-CH₂-OH ist.

10. Verbindung gemäß einem der Ansprüche 1-3, 4-5 oder 6-9,
wobei R² Halogen oder eine Gruppe -Y-R¹³ ist, wobei Y abwesend, -SO₂NH-(CH₂)₀₋₄-, NH-, -NH-SO₂-(CH)₂)₀₋₄-, -O-(CH₂)₀₋₄, -O-(CH(CH₃))-, -(CH₂)₀₋₄-S(=O)(=NH)-, -(C=O)-, -(CH₂)₀₋₄-(C=O)-O- oder -(CH₂)₁₋₄ ist;
R¹³ ein ungesättigter, teilweise gesättigter oder ungesättigter 3-, 4-, 5-, 6- oder 7-gliedriger monocyclischer oder 8-, 9-, 10-, 11 - oder 12-gliedriger bicyclischer Ring, enthaltend 0, 1, 2 oder 3 N-Atome und 0 oder 1 aus O und S ausgewählte Atome, ist, der mit 0, 1, 2 oder 3 Gruppe(n), ausgewählt aus F, Cl, Br, C₁₋₆-Alkyl, C₁₋₄-Halogenalkyl, -OH, -OC₁₋₄-Halogenalkyl, CN, R¹⁴ und Oxo, substituiert ist; oder;
R¹³ C₁₋₆-Alkyl ist, das mit 0, 1, 2, 3, 4 oder 5 Gruppe(n), ausgewählt aus F, Cl, Br, -OH, -OC₁₋₄-Halogenalkyl oder CN, substituiert ist; oder
wobei R² ein gesättigter 5- oder 6-gliedriger monocyclischer Ring ist, wobei jeder Ring 0, 1 oder 2 N-Atome und 0 oder 1 O-Atom(e) enthält, und wobei jeder Ring mit 0, 1, 2 oder 3 Gruppe(n), ausgewählt aus F, Cl, Br, C₁₋₆-Alkyl, C₁₋₄-Halogenalkyl, -OH, -OCH₃, -OC₁₋₄-Halogenalkyl, CN, R¹⁴ und Oxo, substituiert ist; oder
wobei R² Folgendes ist:
(a) F, Br;
(b) eine Gruppe -Y-R¹³, wobei Y abwesend ist; und R¹³ Morpholinyl, Piperidinyl, Azetidinyl, Pyrrolidinyl, Cyclopropyl, Cyclobutyl, Cyclopentyl, Cyclohexyl, Piperazinyl, Tetrahydrofuranyl, Tetrahydropyranyl, Pyridinyl, Pyrimidinyl, 3,6-Dihydro-2H-pyranyl, 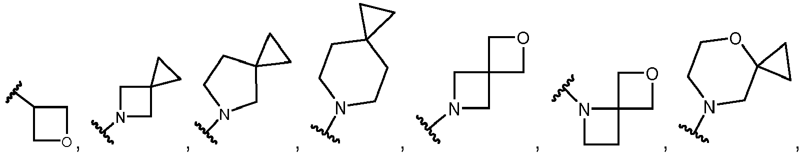 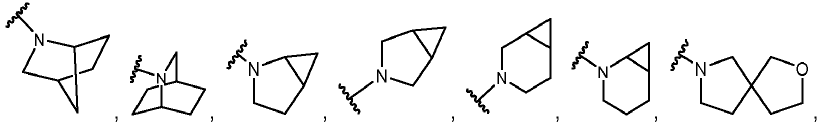 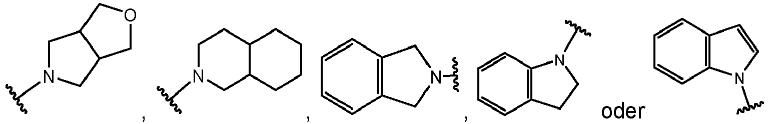 ist;
wobei jeder Ring mit 0, 1, 2 oder 3 Gruppe(n), ausgewählt aus F, Cl, Br, Methyl, CF₃, CH₂OH, CH(CH₃)OH, C(CH₃)₂OH, -OH, -OCHF₂, CN, Oxo oder Cyclopropyl, substituiert ist; oder
(c) eine Gruppe -Y-R¹³, wobei Y abwesend, -SO₂NH-, NH, -O-, S(=O)(=NH)-, -O-(CH₂), -O-(CH(CH₃))-, C(=O)-, C(=O)-O-, -CH₂C(=O)-O- oder-CH₂- ist; und wobei R¹³ 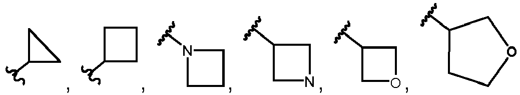 ist; wobei jeder Ring mit 0, 1, 2, 3, 4 oder 5 Gruppe(n), ausgewählt aus F, Cl, Br, Methyl, CF₃, -OH oder CN, substituiert ist;
oder R¹³ H oder C₁₋₆-Alkyl, das mit 0, 1, 2, 3, 4 oder 5 Gruppe(n), ausgewählt aus F, Cl, Br, Methyl, CF₃, -OH oder CN, substituiert ist, ist; oder
wobei R² Folgendes ist: (a) Halogen; (b) eine Gruppe -Y-R¹³, wobei Y abwesend ist; und R¹³ Morpholinyl, Piperidinyl, Azetidinyl, Pyrrolidinyl, Cyclopropyl, Cyclobutyl, Cyclopentyl, Cyclohexyl, Piperazinyl, Tetrahydrofuranyl, 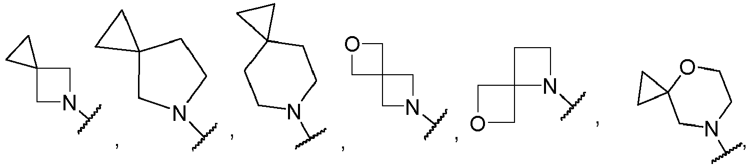 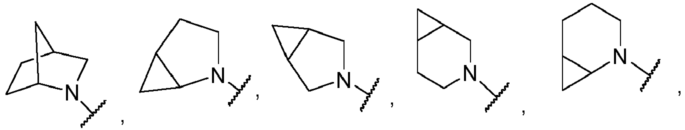 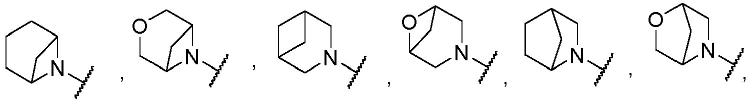 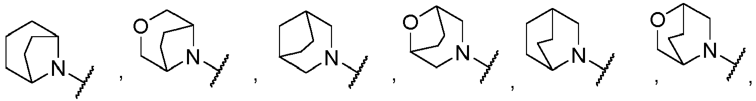 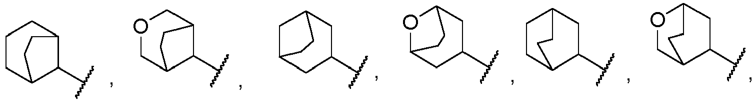 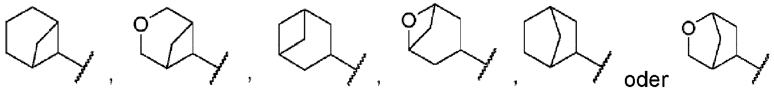 ist; \
wobei jeder Ring mit 0, 1, 2 oder 3 Gruppe(n), ausgewählt aus F, Cl, Br, Methyl, CF₃, -OH, -OCHF₂, CN und Oxo, substituiert ist; oder (c) eine Gruppe -Y-R¹³, wobei Y NH, -O-, -O-(CH₂)-, -O-(CH₂)-(CH₂)- oder -O-(CH₂)-(CH₂)-(CH₂)- ist, und wobei R¹³ 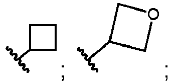 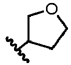 ist; oder R¹³ C₁₋₆-Alkyl ist, das mit 0, 1, 2, 3, 4 oder 5 Gruppe(n), ausgewählt aus F, Cl, Br, Methyl, CF₃, -OH oder CN, substituiert ist; oder
wobei R² 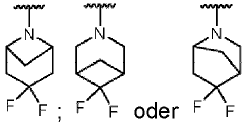 ist; oder
wobei R² Morpholinyl oder Piperidinyl ist, das mit 0, 1, 2 oder 3 Gruppe(n), ausgewählt aus F, Cl, Br, Methyl, CF₃, -OH, -OCHF₂, CN oder Oxo, substituiert ist; oder
wobei R² Morpholinyl ist, das mit 1, 2 oder 3 Methylgruppe(n) substituiert ist.

11. Verbindung gemäß einem der Ansprüche 1-10,
wobei R⁴ aus H, F, Methyl, CN oder Br ausgewählt ist; oder
wobei R⁴ H ist.

12. Verbindung gemäß einem der Ansprüche 1-11,
wobei R⁵ H ist.

13. Verbindung gemäß einem der Ansprüche 1-12,
wobei R⁶ H, Methyl, Cyclopropyl, CN, CF₃ oder Azetidinyl ist.

14. Verbindung gemäß einem der Ansprüche 1-13,
wobei R⁷ H ist.

15. Verbindung gemäß einem der Ansprüche 1-14,
wobei R⁸ H oder F ist.

16. Verbindung gemäß einem der Ansprüche 1-15,
wobei R⁹ H oder F ist.

17. Verbindung gemäß Anspruch 1, ausgewählt aus der Gruppe:
| Bsp. Nr. | chemische Struktur | Bezeichnung |
|---|---|---|
| 1 | 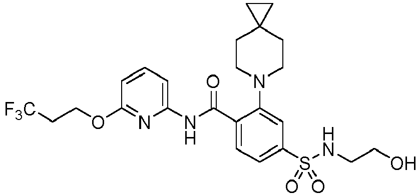 | 4-(*N*-(2-Hydroxyethyl)sulfamoyl)-2-(6-azaspiro[2.5]octan-6-yl)-N-(6-(3,3,3-trifluorpropoxy)pyridin-2-yl)benzamid |
| 1-1 | 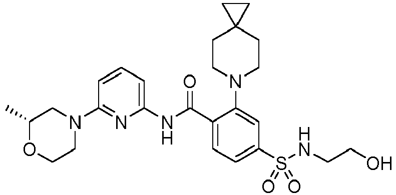 | (*R*)-4-(*N*-(2-Hydroxyethyl)sulfamoyl)-*N*-(6-(2-methylmorpholino)pyridin-2-yl)-2-(6-azaspiro[2.5]octan-6-yl)benzamid |
| 1-2 | 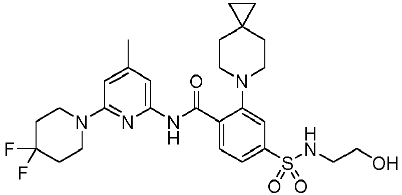 | *N*-(6-(4,4-Difluorpiperidin-1-yl)-4-methylpyridin-2-yl)-4-(*N*-(2-hydroxyethyl)sulfamoyl)-2-(6-azaspiro[2.5]octan-6-yl)benzamid |
| 2 | 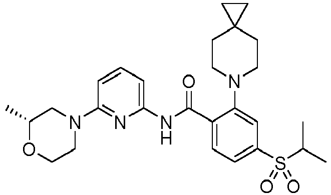 | (*R*)-4-(Isopropylsulfonyl)-*N*-(6-(2-methylmorpholino)pyridin-2-yl)-2-(6-azaspiro[2.5]octan-6-yl)benzamid |
| 2-7 | 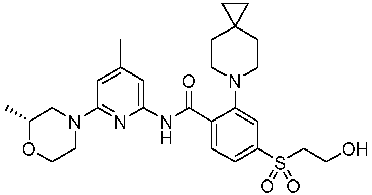 | (*R*)-4-((2-Hydroxyethyl)sulfonyl)-*N*-(4-methyl-6-(2-methylmorpholino)pyridin-2-yl)-2-(6-azaspiro[2.5]octan-6-yl)benzamid |
| 3 | 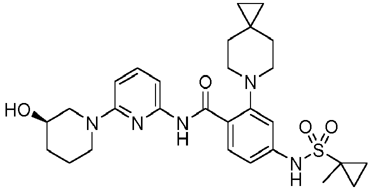 | (*R*)-*N*-(6-(3-Hydroxypiperidin-1-yl)pyridin-2-yl)-4-((1-methylcyclopropan)-1-sulfonamido)-2-(6-azaspiro[2.5]octan-6-yl)benzamid |
| 4 | 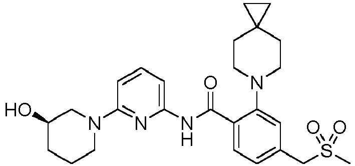 | (*R*)-*N*-(6-(3-Hydroxypiperidin-1-yl)pyridin-2-yl)-4-((methylsulfonyl)methyl)-2-(6-azaspiro[2.5]octan-6-yl)benzamid |
| 5 | 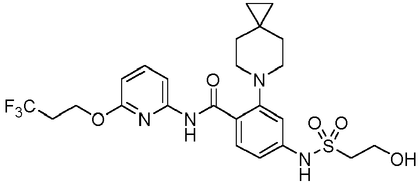 | 4-((2-Hydroxyethyl)sulfonamido)-2-(6-azaspiro[2.5]octan-6-yl)-N-(6-(3,3,3-trifluorpropoxy)pyridin-2-yl)benzamid |
| 6 | 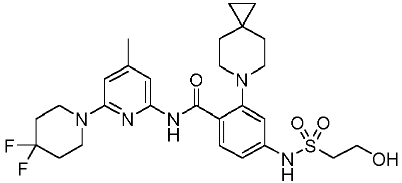 | *N*-(6-(4,4-Difluorpiperidin-1-yl)-4-methylpyridin-2-yl)-4-((2-hydroxyethyl)sulfonamido)-2-(6-azaspiro[2.5]octan-6-yl)benzamid |
| 7-1 | 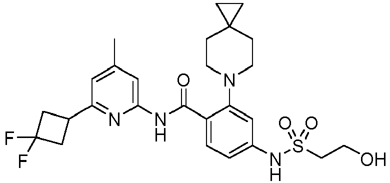 | *N*-(6-(3,3-Difluorcyclobutyl)-4-methylpyridin-2-yl)-4-((2-hydroxyethyl)sulfonamido)-2-(6-azaspiro[2.5]octan-6-yl)benzamid |
| 7-12 | 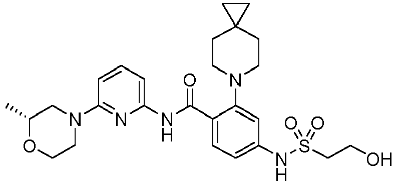 | (*R*)-4-((2-Hydroxyethyl)sulfonamido)-*N*-(6-(2-methylmorpholino)pyridin-2-yl)-2-(6-azaspiro[2.5]octan-6-yl)benzamid |
| 7-33 | 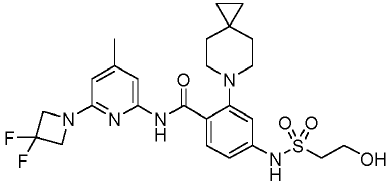 | *N*-(6-(3,3-Difluorazetidin-1-yl)-4-methylpyridin-2-yl)-4-((2-hydroxyethyl)sulfonamido)-2-(6-azaspiro[2.5]octan-6-yl)benzamid |
| 7-44 | 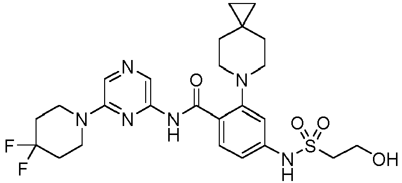 | *N*-(6-(4,4-Difluorpiperidin-1-yl)pyrazin-2-yl)-4-((2-hydroxyethyl)sulfonamido)-2-(6-azaspiro[2.5]octan-6-yl)benzamid |
| 8-1 | 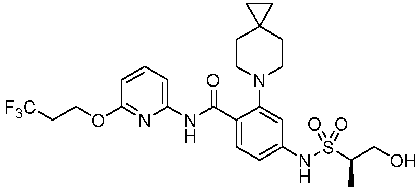 | (*R*)-4-((2-Hydroxy-1-methylethyl)sulfonamido)-2-(6-azaspiro[2.5]octan-6-yl)-*N*-(6-(3,3,3-trifluorpropoxy)pyridin-2-yl)benzamid |
| 8-2 | 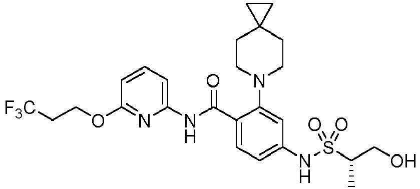 | (*S*)-4-((2-Hydroxy-1-methylethyl)sulfonamido)-2-(6-azaspiro[2.5]octan-6-yl)-*N*-(6-(3,3,3-trifluorpropoxy)pyridin-2-yl)benzamid |
| 9-7 | 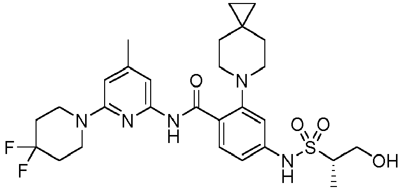 | (*S*)-*N*-(6-(4,4-Difluorpiperidin-1-yl)-4-methylpyridin-2-yl)-4-((2-hydroxy-1-methylethyl)sulfonamido)-2-(6-azaspiro[2.5]octan-6-yl)benzamid |
| 9-8 | 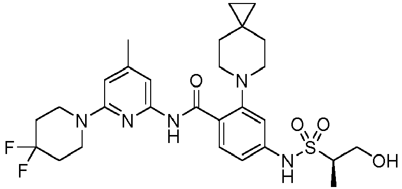 | (*R*)-*N*-(6-(4,4-Difluorpiperidin-1-yl)-4-methylpyridin-2-yl)-4-((2-hydroxy-1-methylethyl)sulfonamido)-2-(6-azaspiro[2.5]octan-6-yl)benzamid |
| 10 | 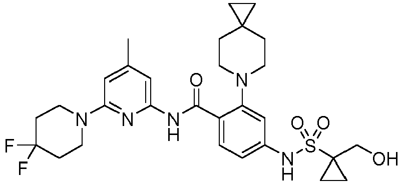 | *N*-(6-(4,4-Difluorpiperidin-1-yl)-4-methylpyridin-2-yl)-4-((1- (hydroxymethyl)cyclopropan)-1-sulfonamido)-2-(6-azaspiro[2.5]octan-6-yl)benzamid |
| 11 | 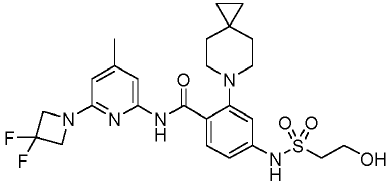 | *N*-(6-(3,3-Difluorazetidin-1-yl)-4-methylpyridin-2-yl)-4-((2-hydroxyethyl)sulfonamido)-2-(6-azaspiro[2.5]octan-6-yl)benzamid |
| 14 | 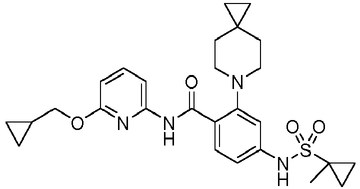 | *N*-(6-(Cyclopropylmethoxy)pyridin-2-yl)-4-((1-methylcyclopropan)-1-sulfonamido)-2-(6-azaspiro[2.5]octan-6-yl)benzamid |
| 16 | 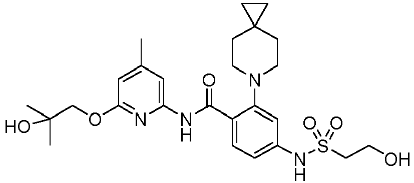 | *N*-(6-(2-Hydroxy-2-methylpropoxy)-4-methylpyridin-2-yl)-4-((2-hydroxyethyl)sulfonamido)-2-(6-azaspiro[2.5]octan-6-yl)benzamid |
| 17 | 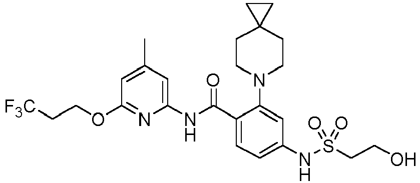 | 4-((2-Hydroxyethyl)sulfonamido)-*N*-(4-methyl-6-(3,3,3-trifluorpropoxy)pyridin-2-yl)-2-(6-azaspiro[2.5]octan-6-yl)benzamid |
| 21 | 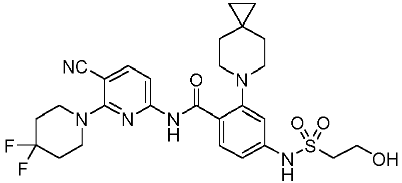 | *N*-(5-Cyan-6-(4,4-difluorpiperidin-1-yl)pyridin-2-yl)-4-((2-hydroxyethyl)sulfonamido)-2-(6-azaspiro[2.5]octan-6-yl)benzamid |
| 22 | 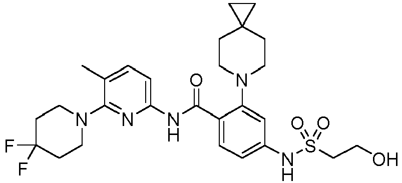 | *N*-(6-(4,4-Difluorpiperidin-1-yl)-5-methylpyridin-2-yl)-4-((2-hydroxyethyl)sulfonamido)-2-(6-azaspiro[2.5]octan-6-yl)benzamid |
| 23 | 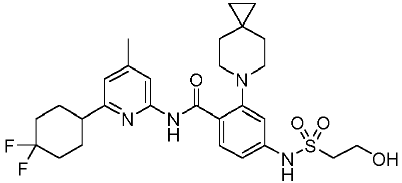 | *N*-(6-(4,4-Difluorcyclohexyl)-4-methylpyridin-2-yl)-4-((2-hydroxyethyl)sulfonamido)-2-(6-azaspiro[2.5]octan-6-yl)benzamid |
| 24 | 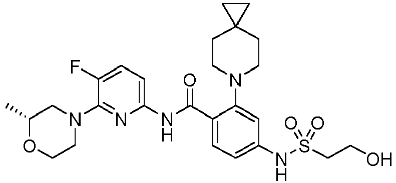 | (*R*)-*N*-(5-Fluor-6-(2-methylmorpholino)pyridin-2-yl)-4-((2-hydroxyethyl)sulfonamido)-2-(6-azaspiro[2.5]octan-6-yl)benzamid |
| 25 | 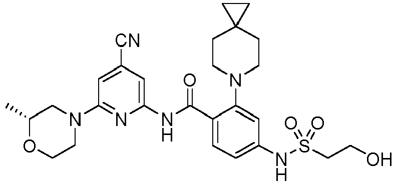 | (*R*)-*N*-(4-Cyan-6-(2-methylmorpholino)pyridin-2-yl)-4-((2-hydroxyethyl)sulfonamido)-2-(6-azaspiro[2.5]octan-6-yl)benzamid |
| 27-1 | 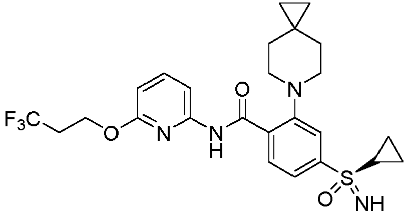 | 2-(6-Azaspiro[2.5]octan-6-yl)-4-(*S-*cyclopropylsulfonimidoyl)-*N*-(6-(3,3,3-trifluorpropoxy)-2-pyridinyl)benzamid |
| 27-2 | 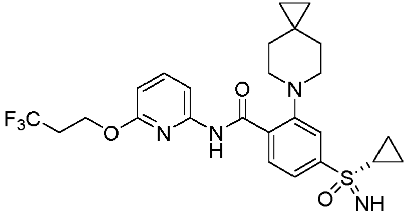 | 2-(6-Azaspiro[2.5]octan-6-yl)-4-(R-cyclopropylsulfonimidoyl)-*N*-(6-(3,3,3-trifluorpropoxy)-2-pyridinyl)benzamid |
; oder ein beliebiges pharmazeutisch akzeptables Salz davon.

18. Verbindung gemäß einem der Ansprüche 1-17 zur Verwendung als Medikament.

19. Eine pharmazeutische Zusammensetzung, beinhaltend die Verbindung gemäß einem der Ansprüche 1-17 oder das pharmazeutisch akzeptable Salz davon und ein pharmazeutisch akzeptables Verdünnungsmittel oder Trägermittel.

20. Verbindung gemäß einem der Ansprüche 1-17 oder die Verbindung beinhaltende Zusammensetzung gemäß Anspruch 19 zur Verwendung in einem Verfahren zum Behandeln einer Erkrankung, die mit KIF18a-Hemmern behandelt werden kann, wobei das Verfahren das Verabreichen einer therapeutisch wirksamen Menge der Verbindung an einen Patienten, der dessen bedarf, beinhaltet;
wobei die Erkrankung Krebs ist, ausgewählt aus der Gruppe, die aus Folgendem besteht: (a) einem soliden oder hämatologisch-stämmigen Tumor, ausgewählt aus Krebs der Blase, des Endometriums, des Lungenplattenepithels, der Brust, des Dickdarms, der Niere, der Leber, der Lunge, kleinzelligem Lungenkarzinom, der Speiseröhre, der Gallenblase, des Gehirns, des Kopfes und Halses, des Eierstocks, der Bauchspeicheldrüse, des Magens, des Gebärmutterhalses, der Schilddrüse, der Prostata und der Haut, (b) einem hämatopoetischen Tumor der lymphoiden Linie, ausgewählt aus Leukämie, akuter lymphozytischer Leukämie, akuter lymphoblastischer Leukämie, B-Zellen-Lymphom, T-Zellen-Lymphom, Hodgkin-Lymphom, Non-Hodgkin-Lymphom, Haarzelllymphom und Burkitt-Lymphom, (c) einem hämatopoetischen Tumor der myeloischen Linie, ausgewählt aus akuten und chronischen myelogenen Leukämien, myelodysplastischem Syndrom und Promyelozytenleukämie, (d) einem Tumor mesenchymalen Ursprungs, ausgewählt aus Fibrosarkom und Rhabdomyosarkom, (e) einem Tumor des zentralen und peripheren Nervensystems, ausgewählt aus Astrozytom, Neuroblastom, Gliom und Schwannom, oder (f) einem Melanom, Seminom, Teratokarzinom, Osteosarkom, Xeroderma pigmentosum, Keratoakanthom, Schilddrüsenfollikelkrebs oder Kaposi-Sarkom.

21. Verbindung gemäß einem der Ansprüche 1-17 oder die Verbindung beinhaltende Zusammensetzung gemäß Anspruch 19 zur Verwendung in einem Verfahren zum Reduzieren der Größe eines soliden Tumors bei einem Individuum; oder in einem Verfahren zum Behandeln einer Zellproliferationsstörung bei einem Individuum; oder
in einem Verfahren zum Hemmen von KIF18A in einer Zelle.

## Revendications

1. Un composé de formule I : 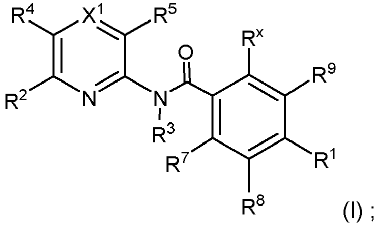 ou n'importe quel sel pharmaceutiquement acceptable de celui-ci, où :
X¹ est N ou -CR⁶ ;
R¹ est un groupe -Z-R¹² où Z est -alk en C₀₋₄-, -NR¹¹-, -NR¹¹SO₂-alk en C₀₋₄-, -SO₂NR¹¹-alk en C₀₋₄-, -NR¹¹SO₂NR¹¹-, -NR¹¹SO₂NR¹¹-C(=O)-O-, -alk en C₀₋₄-S(=O)(=NH)-, alk en C₀₋₄-NR¹¹-S(=O)(=NH), -alk en C₀₋₄-S-, -alk en C₀₋₄-S(=O)-, -alk en C₀₋₄-SO₂-, alk en C₀₋₄-O-, -P-, -P(=O), -P(=O)₂, -(C=O)-, -(C=O)NR¹¹-, -C=N(OH)-, ou -NR¹¹(C=O) ; ou
le groupe -Z-R¹² est -N=S(=O)-(R¹²)₂, où les deux R¹² de la paire peuvent se combiner autrement avec l'atome de soufre attaché à chacun d'eux pour former un anneau monocyclique saturé ou partiellement saturé à 3, 4, 5, ou 6 chaînons contenant 0, 1, 2 ou 3 atomes N et 0, 1, ou 2 atomes sélectionnés parmi O et S ;
R² est un halo ou un groupe -Y-R¹³, où Y est -alk en C₀₋₄-, -N(alk en C₀₋₁)-alk en C₀₋₄-, -C(=O)NR^{a}(alk en C₁₋₄)-, -O-alk en C₀₋₄-, -S-, -S=O, -S(=O)₂-, -SO₂N(alk en C₀₋₁)-alk en C₀₋₄-, -N(alk en C₀₋₁)-SO₂-alk en C₀₋₄-, -alk en C₀₋₄-S(=O)(=NH)-, -(C=O)-, -alk en C₀₋₄-(C=O)-O- ; ou
le groupe -Y-R¹³ est -N=S(=O)-(R¹³)₂, où les deux R¹³ de la paire peuvent se combiner autrement avec l'atome de soufre attaché à chacun d'eux pour former un anneau monocyclique saturé ou partiellement saturé à 3, 4, 5, ou 6 chaînons contenant 0, 1, 2 ou 3 atomes N et 0, 1, ou 2 atomes sélectionnés parmi O et S ;
R³ est H, un méthyle, ou un éthyle ;
R⁴ est H, un halo, CN, un alk en C₁₋₄, ou un haloalk en C₁₋₄ ;
R⁵ est H, un halo, un alk en C₁₋₈, ou un haloalk en C₁₋₄ ;
R⁶ est H, un halo, CN, un alk en C₁₋₈, un haloalk en C₁₋₄, -O-alk en C₀₋₆-, ou R^{6a} ;
R⁷ est H, un halo, un alk en C₁₋₈, ou un haloalk en C₁₋₄ ;
R⁸ est H, un halo, un alk en C₁₋₈, ou un haloalk en C₁₋₄ ;
R⁹ est H, un halo, un alk en C₁₋₈, ou un haloalk en C₁₋₄ ;
R^{x} est sélectionné dans le groupe constitué de 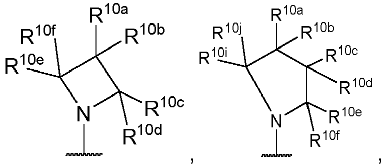 et 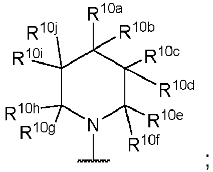
Chaque R de R^{10a}, R^{10b}, R^{10c}, R^{10d}, R^{10e}, R^{10f}, R^{10g}, R^{10h}, R¹⁰ⁱ, et R^{10j} est H, un halo, R^{10k},
ou R^{10l} ;
ou autrement, chaque R de la paire R^{10a} et R^{10b}, de la paire R^{10c} et R^{10d}, de la paire R^{10e} et
R^{10f}, de la paire R^{10g} et R^{10h}, ou de la paire R¹⁰ⁱ et R^{10j}, indépendamment, peut se combiner avec l'atome de carbone attaché à chacun d'eux pour former un anneau monocyclique saturé ou partiellement saturé à 3, 4, 5, 6 chaînons spiro à l'anneau R^{x} ; où ledit anneau monocyclique à 3, 4, 5, 6 chaînons contient 0, 1, 2 ou 3 atomes N et 0, 1, ou 2 atomes sélectionnés parmi O et S, et où en outre ledit anneau monocyclique à 3, 4, 5, 6 chaînons est substitué par 0, 1, 2 ou 3 groupe(s) sélectionné(s) parmi F, Cl, Br, un alk en C₁₋₆, un haloalk en C₁₋₄, -OR^{a}, -Ohaloalk en C₁₋₄, CN, -NR^{a}R^{a}, ou un oxo ;
R¹¹ est H ou un alk en C₁₋₈ ;
R¹² est H, R^{12a}, ou R^{12b} ;
R¹³ est R^{13a} ou R^{13b} ;
R^{6a}, R^{10k}, R^{12a}, et R^{13a} sont indépendamment, dans chaque cas, sélectionnés dans le groupe constitué d'un anneau monocyclique à 3, 4, 5, 6, ou 7 chaînons ou d'un anneau bicyclique à 4, 5, 6, 7, 8, 9, 10, 11, ou 12 chaînons saturé, partiellement saturé ou insaturé contenant 0, 1, 2 ou 3 atomes N et 0, 1, ou 2 atomes sélectionnés parmi O et S, lequel est substitué par 0, 1, 2 ou 3 groupe(s) sélectionné(s) parmi F, Cl, Br, un alk en C₁₋₆, un haloalk en C₁₋₄, -OR^{a}, -Ohaloalk en C₁₋₄, CN, -C(=O)R^{b}, -C(=O)OR^{a}, -C(=O)NR^{a}R^{a},
- C(=NR^{a})NR^{a}R^{a}, -OC(=O)R^{b}, -OC(=O)NR^{a}R^{a}, -Oalk en C₂₋₆NR^{a}R^{a}, -Oalk en C₂₋₆OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O) OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a},
- NR^{a}alk en C₂₋₆NR^{a}R^{a}, -NR^{a}alk en C₂₋₆OR^{a}, -alk en C₁₋₆NR^{a}R^{a}, -alk en C₁₋₆OR^{a}, -alk en C₁₋₆N(R^{a})C(=O)R^{b}, -alk en C₁₋₆OC(=O)R^{b}, -alk en C₁₋₆C(=O)NR^{a}R^{a}, -alk en C₁₋₆C(=O)OR^{a}, R¹⁴, et un oxo ;
R^{10l}, R^{12b}, et R^{13b} sont indépendamment, dans chaque cas, sélectionnés dans le groupe constitué d'un alk en C₁₋₆ substitué par 0, 1, 2, 3, 4, ou 5 groupe(s) sélectionné(s) parmi F, Cl, Br, -C(=O)OR^{a}, -OR^{a}, -haloalk en C₁₋₂, -Ohaloalk en C₁₋₄, CN, NH₂, NH(CH₃), ou N(CH₃)₂ ;
R¹⁴ est indépendamment, dans chaque cas, sélectionné dans le groupe constitué d'un anneau monocyclique à 3, 4, 5, 6, ou 7 chaînons ou d'un anneau bicyclique à 4, 5, 6, 7, 8, 9, 10, 11, ou 12 chaînons saturé, partiellement saturé ou insaturé contenant 0, 1, 2 ou 3 atomes N et 0, 1, ou 2 atomes sélectionnés parmi O et S, lequel est substitué par 0, 1, 2 ou 3 groupe(s) sélectionné(s) parmi F, Cl, Br, un alk en C₁₋₆, un haloalk en C₁₋₄, -OR^{a}, -Ohaloalk en C₁₋₄, CN, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -Oalk en C₂₋₆NR^{a}R^{a}, -Oalk en C₂₋₆OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}alk en C₂₋₆NR^{a}R^{a}, -NR^{a}alk en C₂₋₆OR^{a}, -alk en C₁₋₆NR^{a}R^{a}, -alk en C₁₋₆OR^{a}, -alk en C₁₋₆N(R^{a})C(=O)R^{b}, -alk en C₁₋₆OC(=O)R^{b}, -alk en C₁₋₆C(=O)NR^{a}R^{a}, -alk en C₁₋₆C(=O)OR^{a}, et un oxo ;
R^{a} est indépendamment, dans chaque cas, H ou R^{b} ; et
R^{b} est indépendamment, dans chaque cas, un alk en C₁₋₆, un phényle, ou un benzyle, où l'alk en C₁₋₆ est substitué par 0, 1, 2 ou 3 substituants sélectionnés parmi un halo, -OH, -Oalk en C₁₋₄, -NH₂, -NHalk en C₁₋₄, -OC(=O)alk en C₁₋₄, ou -N(alk en C₁₋₄)alk en C₁₋₄ ; et le phényle ou le benzyle est substitué par 0, 1, 2 ou 3 substituants sélectionnés parmi un halo, un alk en C₁₋₄, un haloalk en C₁₋₃, -OH, -Oalk en C₁₋₄, -NH₂, -NHalk en C₁₋₄, -OC(=O)alk en C₁₋₄, ou -N(alk en C₁₋₄)alk en C₁₋₄.

2. Le composé de la revendication 1 où R^{x} est

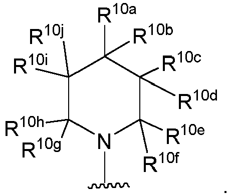

3. Le composé de n'importe laquelle des revendications 1 à 2 où :
X¹ est -CR⁶ ; ayant la formule (la) : 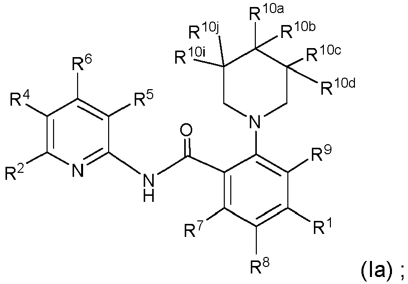 ou
X¹ est N ; ayant la formule (Ib) :

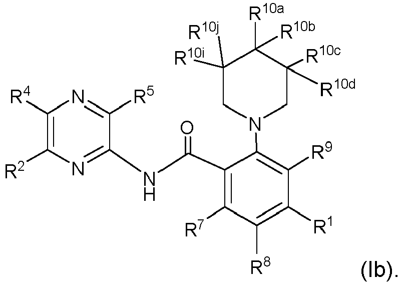

4. Le composé de n'importe laquelle des revendications 1 à 3 où R³ est H ou un méthyle.

5. Le composé de n'importe laquelle des revendications 1 à 4 où chaque R de R^{10c}, R^{10d}, R^{10e}, R^{10f} R^{10g}, R^{10h}, R¹⁰ⁱ, et R^{10j} est H, un halo, un alk en C₁₋₆, ou un haloalk en C₁₋₄ ; et chaque R de la paire R^{10a} et R^{10b} se combine avec l'atome de carbone attaché à chacun d'eux pour former un anneau monocyclique saturé à 3, 4, ou 5 chaînons spiro à l'anneau R^{x} ; où ledit anneau contient 0, 1, 2 ou 3 atomes N et 0 ou 1 atome sélectionné parmi O et S ; ou
où chaque R de R^{10c}, R^{10d}, R^{10e}, R^{10f}, R^{10g}, R^{10h}, R¹⁰ⁱ, et R^{10j} est H, un méthyle, ou un éthyle ; et chaque R de la paire R^{10a} et R^{10b} se combine avec l'atome de carbone attaché à chacun d'eux pour former un anneau cyclopropyle, cyclobutyle, ou cyclopentyle spiro à l'anneau R^{x}.

6. Le composé de n'importe laquelle des revendications 1 à 5 où R^{x} est sélectionné parmi :

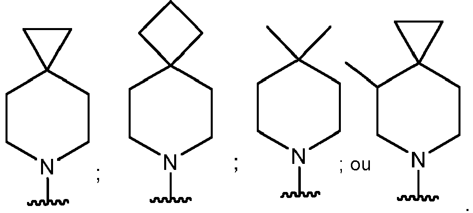

7. Le composé de n'importe laquelle des revendications 1 à 6 où R^{x} est

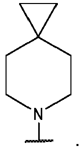

8. Le composé de n'importe laquelle des revendications 1 à 7 où Z est absent, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-, -(CH₂)₀₋₂-S(=O)-, (CH₃CH)-S(=O)-, -(CH₂)₀₋₂-SO₂-, -O-, -P(=O), -(C=O)-, ou -NH(C=O).

9. Le composé de n'importe laquelle des revendications 1 à 8 où R¹ est le groupe -Z-R¹², lequel est -N=S(=O)-(R¹²)₂, où les deux R¹² de la paire peuvent se combiner autrement avec l'atome de soufre attaché à chacun d'eux pour former un anneau monocyclique saturé ou partiellement saturé à 3, 4, 5, ou 6 chaînons contenant 0, 1, 2 ou 3 atomes N et 0, 1, ou 2 atomes sélectionnés parmi O et S ; lequel est sélectionné parmi : 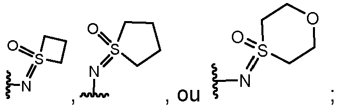 ou
R¹ est -Z-R¹², où Z est absent, NH-, -NHSO₂-(CH₂)₀₋₄-, -N(CH₃)-SO₂-(CH₂)₀₋₄-, -NCH₃SO₂NH, -NHSO₂NH-C(=O)-O-, -SO₂NH-(CH₂)₀₋₄-, -(CH₂)₀₋₂-S(=O)(=NH)-, -(CH₂)₀₋₂-S-, -(CH₂)₀₋₂-S(=O)-, (CH₃CH)-S(=O)-, -(CH₂)₀₋₂-SO₂-, -O-, -P(=O), -(C=O)-, ou -NH(C=O) ; et R¹² est sélectionné parmi :
(a) H ;
(b) un cyclopropyle, un cyclobutyle, un cyclopentyle, un cyclohexyle, un oxiranyle, un oxétanyle, un tétrahydrofuranyle, un azétidinyle, un imidazolyle, un morpholinyle, un pyrrolidinyle, un pipérazinyle, 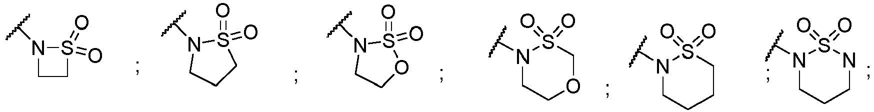 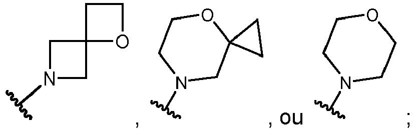 où chacun desdits anneaux est substitué par 0, 1, 2 ou 3 groupe(s) sélectionné(s) parmi où chacun des anneaux est substitué par 0, 1, 2 ou 3 OH, F, un méthyle, -CH₂OH, -C(=O)OCH₃, -C(=O)OC(CH₃)₃, NH₂, CN, et un oxo ; ou
(c) un alk en C₁₋₆ substitué par 0, 1, 2 ou 3 OH, F, -C(=O)OCH₃, -NH₂, -NH(CH₃), ou -N(CH₃)₂ ; ou
R¹ est un groupe -Z-R¹², où Z est -NHSO₂- ou -SO₂NH- ; et R¹² est un oxétanyle, un cyclopropyle, ou R¹² est un alk en C₁₋₆ substitué par 0, 1, 2 ou 3 groupe(s) OH ; ou
R¹ est un groupe -Z-R¹², où Z est -NHSO₂- et R¹² est -CH₂-CH₂-OH.

10. Le composé de n'importe laquelle des revendications 1 à 3, 4 à 5, ou 6 à 9
où R² est un halo ou un groupe -Y-R¹³, où Y est absent, -SO₂NH-(CH₂)₀₋₄-, NH-, -NH-SO₂-(CH)₂)₀₋₄-, -O-(CH₂)₀₋₄, -O-(CH(CH₃))-, -(CH₂)₀₋₄-S(=O)(=NH)-, -(C=O)-, -(CH₂)₀₋₄-(C=O)-O-, ou -(CH₂)₁₋₄ ;
R¹³ est un anneau monocyclique à 3, 4, 5, 6, ou 7 chaînons ou un anneau bicyclique à 8, 9, 10, 11, ou 12 chaînons saturé, partiellement saturé ou insaturé contenant 0, 1, 2 ou 3 atomes N et 0 ou 1 atome sélectionné parmi O et S, lequel est substitué par 0, 1, 2 ou 3 groupe(s) sélectionné(s) parmi F, Cl, Br, un alk en C₁₋₆, un haloalk en C₁₋₄, -OH, -Ohaloalk en C₁₋₄, CN, R¹⁴, et un oxo ; ou ;
R¹³ est un alk en C₁₋₆ substitué par 0, 1, 2, 3, 4, ou 5 groupe(s) sélectionné(s) parmi F, Cl, Br, -OH, -Ohaloalk en C₁₋₄, ou CN ; ou
où R² est un anneau monocyclique saturé à 5 ou 6 chaînons où chacun desdits anneaux contient 0, 1, ou 2 atomes N et 0 ou 1 atome O, et où chacun desdits anneaux est substitué par 0, 1, 2 ou 3 groupe(s) sélectionné(s) parmi F, Cl, Br, un alk en C₁₋₆, un haloalk en C₁₋₄, -OH, -OCH₃, -Ohaloalk en C₁₋₄, CN, R¹⁴, et un oxo ; ou
où R² est :
(a) F, Br ;
(b) un groupe -Y-R¹³, où Y est absent ; et R¹³ est un morpholinyle, un pipéridinyle, un azétidinyle, un pyrrolidinyle, un cyclopropyle, un cyclobutyle, un cyclopentyle, un cyclohexyle, un pipérazinyle, un tétrahydrofuranyle, un tétrahydropyranyle, un pyridinyle, un pyrimidinyle, un 3,6-dihydro-2H-pyranyle, 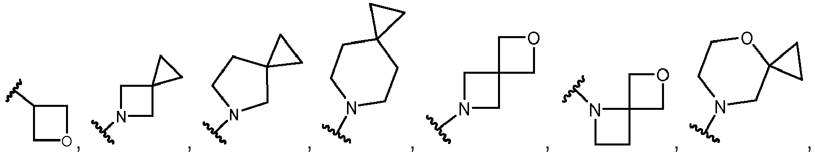 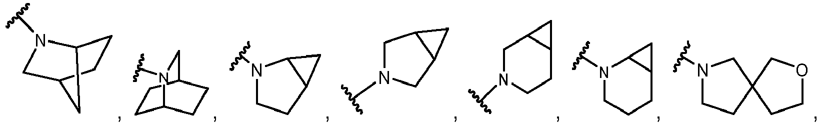 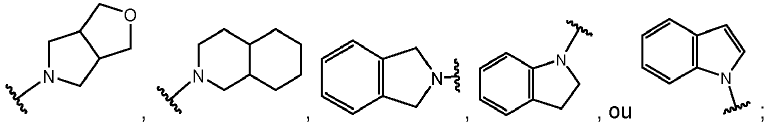 où chacun desdits anneaux est substitué par 0, 1, 2 ou 3 groupe(s) sélectionné(s) parmi F, Cl, Br, un méthyle, CF₃, CH₂OH, CH(CH₃)OH, C(CH₃)₂OH, -OH, -OCHF₂, CN, un oxo, ou un cyclopropyle ; ou
(c) un groupe -Y-R¹³, où Y est absent, -SO₂NH-, NH, -O-, S(=O)(=NH)-, -O-(CH₂), -O-(CH(CH₃))-, C(=O)-, C(=O)-O-, -CH₂C(=O)-O-, ou -CH₂- ; et où R¹³ est 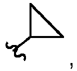 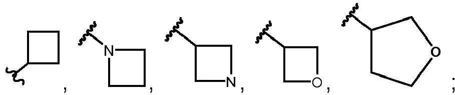 où chacun des anneaux est substitué par 0, 1, 2, 3, 4, ou 5 groupe(s) sélectionné(s) parmi F, Cl, Br, un méthyle, CF₃,-OH, ou CN ;
ou R¹³ est H ou un alk en C₁₋₆ substitué par 0, 1, 2, 3, 4, ou 5 groupe(s) sélectionné(s) parmi F, Cl, Br, un méthyle, CF₃, -OH, ou CN ; ou
où R² est (a) un halo ; (b) un groupe -Y-R¹³, où Y est absent ; et R¹³ est un morpholinyle, un pipéridinyle, un azétidinyle, un pyrrolidinyle, un cyclopropyle, un cyclobutyle, un cyclopentyle, un cyclohexyle, un pipérazinyle, un tétrahydrofuranyle, 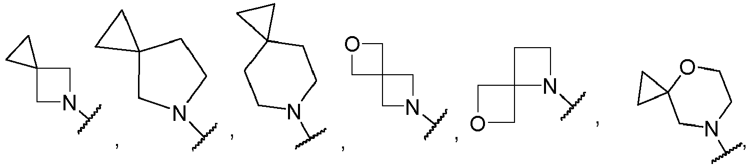 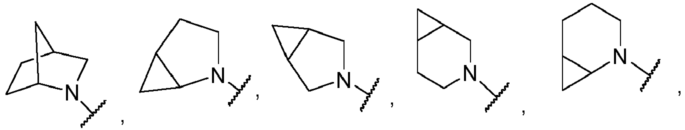 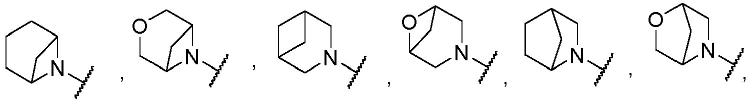 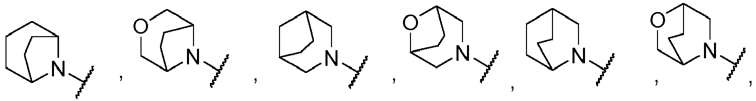 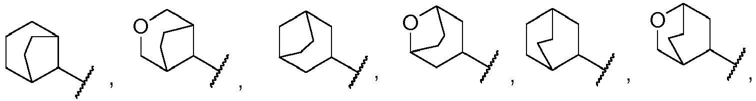 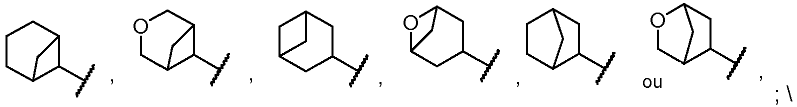
où chacun desdits anneaux est substitué par 0, 1, 2 ou 3 groupe(s) sélectionné(s) parmi F, Cl, Br, un méthyle, CF₃, -OH, -OCHF₂, CN, et un oxo ; ou (c) un groupe -Y-R¹³, où Y est NH, -O-, -O-(CH₂)-, -O-(CH₂)-(CH₂)-, ou -O-(CH₂)-(CH₂)-(CH₂)-, et où R¹³ est 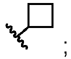 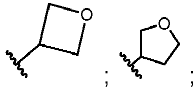 ou R¹³ est un alk en C₁₋₆ substitué par 0, 1, 2, 3, 4, ou 5 groupe(s) sélectionné(s) parmi F, Cl, Br, un méthyle, CF₃, -OH, ou CN ; ou
où R² est 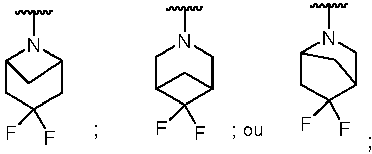 ou
où R² est un morpholinyle ou un pipéridinyle substitué par 0, 1, 2 ou 3 groupe(s) sélectionné(s) parmi F, Cl, Br, un méthyle, CF₃, -OH, -OCHF₂, CN, ou un oxo ; ou
où R² est un morpholinyle substitué par 1, 2 ou 3 groupe(s) méthyle.

11. Le composé de n'importe laquelle des revendications 1 à 10
où R⁴ est sélectionné parmi H, F, un méthyle, CN, ou Br ; ou
où R⁴ est H.

12. Le composé de n'importe laquelle des revendications 1 à 11
où R⁵ est H.

13. Le composé de n'importe laquelle des revendications 1 à 12
où R⁶ est H, un méthyle, un cyclopropyle, CN, CF₃, ou un azétidinyle.

14. Le composé de n'importe laquelle des revendications 1 à 13
où R⁷ est H.

15. Le composé de n'importe laquelle des revendications 1 à 14
où R⁸ est H ou F.

16. Le composé de n'importe laquelle des revendications 1 à 15
où R⁹ est H ou F.

17. Le composé de la revendication 1, sélectionné dans le groupe :
| Ex. # | Structure Chimique | Nom |
|---|---|---|
| 1 | 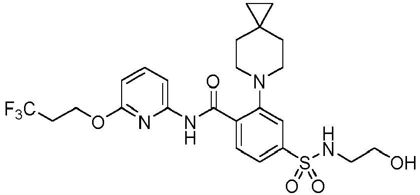 | 4-(*N*-(2-Hydroxyéthyl)sulfamoyl)-2-(6-azaspiro[2.5]octan-6-yl)-N-(6-(3,3,3-trifluoropropoxy)pyridin-2-yl)benzamide |
| 1-1 | 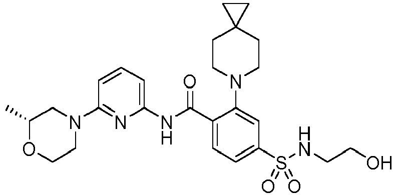 | (*R*)-4-(*N*-(2-Hydroxyéthyl)sulfamoyl)-*N*-(6-(2-méthylmorpholino)pyridin-2-yl)-2-(6-azaspiro[2.5]octan-6-yl)benzamide |
| 1-2 | 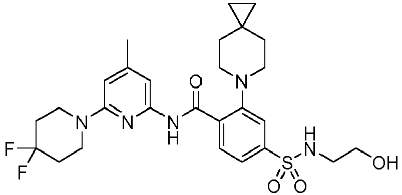 | *N*-(6-(4,4-Difluoropipéridin-1-yl)-4-méthylpyridin-2-yl)-4-(*N*-(2-hydroxyéthyl)sulfamoyl)-2-(6-azaspiro[2.5]octan-6-yl)benzamide |
| 2 | 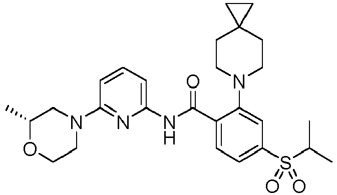 | (*R*)-4-(Isopropylsulfonyl)-*N*-(6-(2-méthylmorpholino)pyridin-2-yl)-2-(6-azaspiro[2.5]octan-6-yl)benzamide |
| 2-7 | 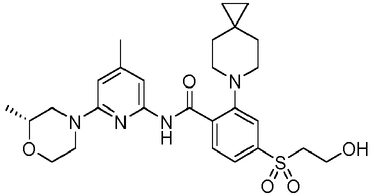 | (*R*)-4-((2-Hydroxyéthyl)sulfonyl)-*N*-(4-méthyl-6-(2-méthylmorpholino)pyridin-2-yl)-2-(6-azaspiro[2.5]octan-6-yl)benzamide |
| 3 | 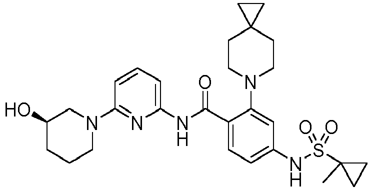 | (*R*)-*N*-(6-(3-Hydroxypipéridin-1-yl)pyridin-2-yl)-4-((1-méthylcyclopropane)-1-sulfonamido)-2-(6-azaspiro[2.5]octan-6-yl)benzamide |
| 4 | 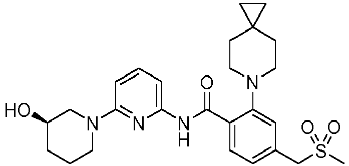 | (*R*)-*N*-(6-(3-hydroxypipéridin-1-yl)pyridin-2-yl)-4-((méthylsulfonyl)méthyl)-2-(6-azaspiro[2.5]octan-6-yl)benzamide |
| 5 | 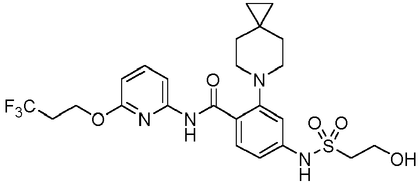 | 4-((2-Hydroxyéthyl)sulfonamido)-2-(6-azaspiro[2.5]octan-6-yl)-N-(6-(3,3,3-trifluoropropoxy)pyridin-2-yl)benzamide |
| 6 | 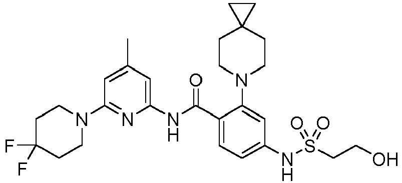 | *N*-(6-(4,4-Difluoropipéridin-1-yl)-4-méthylpyridin-2-yl)-4-((2-hydroxyéthyl)sulfonamido)-2-(6-azaspiro[2.5]octan-6-yl)benzamide |
| 7-1 | 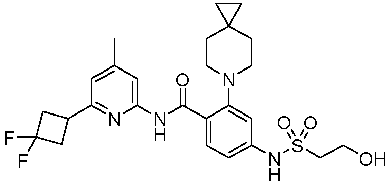 | *N*-(6-(3,3-Difluorocyclobutyl)-4-méthylpyridin-2-yl)-4-((2-hydroxyéthyl)sulfonamido)-2-(6-azaspiro[2.5]octan-6-yl)benzamide |
| 7-12 | 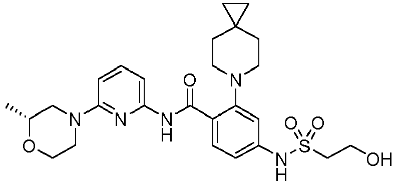 | (*R*)-4-((2-Hydroxyéthyl)sulfonamido)-*N*-(6-(2-méthylmorpholino)pyridin-2-yl)-2-(6-azaspiro[2.5]octan-6-yl)benzamide |
| 7-33 | 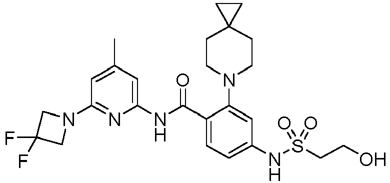 | *N*-(6-(3,3-Difluoroazétidin-1-yl)-4-méthylpyridin-2-yl)-4-((2-hydroxyéthyl)sulfonamido)-2-(6-azaspiro[2.5]octan-6-yl)benzamide |
| 7-44 | 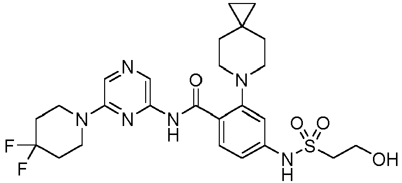 | *N*-(6-(4,4-Difluoropipéridin-1-yl)pyrazin-2-yl)-4-((2-hydroxyéthyl)sulfonamido)-2-(6-azaspiro[2.5]octan-6-yl)benzamide |
| 8-1 | 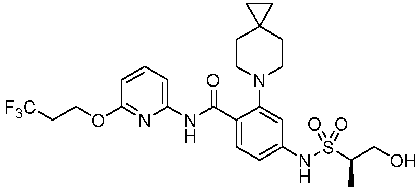 | (*R*)-4-((2-Hydroxy-1-méthyléthyl)sulfonamido)-2-(6-azaspiro[2.5]octan-6-yl)-*N*-(6-(3,3,3-trifluoropropoxy)pyridin-2-yl)benzamide |
| 8-2 | 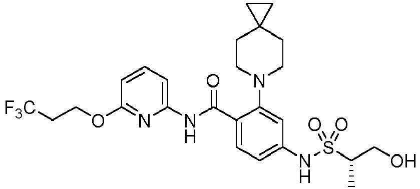 | (*S*)-4-((2-Hydroxy-1-méthyléthyl)sulfonamido)-2-(6-azaspiro[2.5]octan-6-yl)-*N*-(6-(3,3,3-trifluoropropoxy)pyridin-2-yl)benzamide |
| 9-7 | 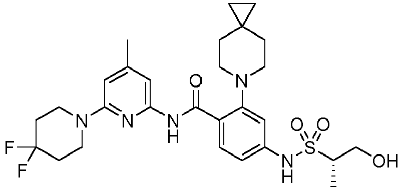 | (S)-*N*-(6-(4,4-Difluoropipéridin-1-yl)-4-méthylpyridin-2-yl)-4-((2-hydroxy-1-méthyléthyl)sulfonamido)-2-(6-azaspiro[2.5]octan-6-yl)benzamide |
| 9-8 | 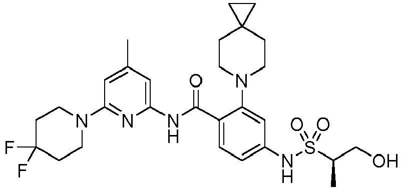 | (*R*)-*N*-(6-(4,4-Difluoropipéridin-1-yl)-4-méthylpyridin-2-yl)-4-((2-hydroxy-1-méthyléthyl)sulfonamido)-2-(6-azaspiro[2.5]octan-6-yl)benzamide |
| 10 | 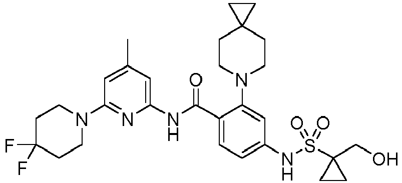 | *N*-(6-(4,4-Difluoropipéridin-1-yl)-4-méthylpyridin-2-yl)-4-((1-(hydroxyméthyl)cyclopropane)-1-sulfonamido)-2-(6-azaspiro[2.5]octan-6-yl)benzamide |
| 11 | 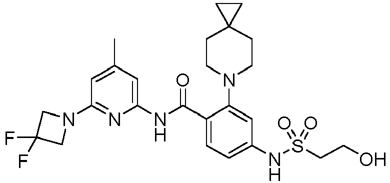 | *N*-(6-(3,3-Difluoroazétidin-1-yl)-4-méthylpyridin-2-yl)-4-((2-hydroxyéthyl)sulfonamido)-2-(6-azaspiro[2.5]octan-6-yl)benzamide |
| 14 | 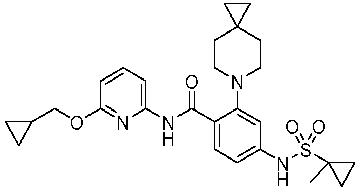 | *N*-(6-(Cyclopropylméthoxy)pyridin-2-yl)-4-((1-méthylcyclopropane)-1-sulfonamido)-2-(6-azaspiro[2.5]octan-6-yl)benzamide |
| 16 | 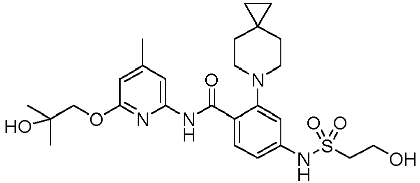 | *N*-(6-(2-Hydroxy-2-méthylpropoxy)-4-méthylpyridin-2-yl)-4-((2-hydroxyéthyl)sulfonamido)-2-(6-azaspiro[2.5]octan-6-yl)benzamide |
| 17 | 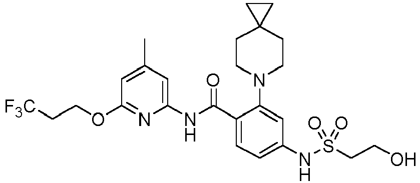 | 4-((2-Hydroxyéthyl)sulfonamido)-*N*-(4-méthyl-6-(3,3,3-trifluoropropoxy)pyridin-2-yl)-2-(6-azaspiro[2.5]octan-6-yl)benzamide |
| 21 | 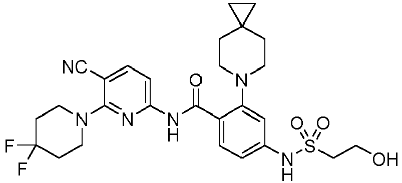 | *N*-(5-Cyano-6-(4,4-difluoropipéridin-1-yl)pyridin-2-yl)-4-((2-hydroxyéthyl)sulfonamido)-2-(6-azaspiro[2.5]octan-6-yl)benzamide |
| 22 | 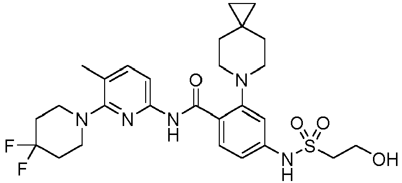 | *N*-(6-(4,4-Difluoropipéridin-1-yl)-5-méthylpyridin-2-yl)-4-((2-hydroxyéthyl)sulfonamido)-2-(6-azaspiro[2.5]octan-6-yl)benzamide |
| 23 | 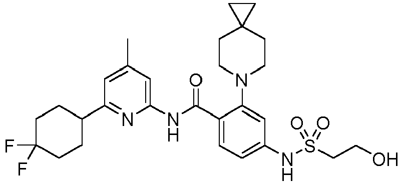 | *N*-(6-(4,4-difluorocyclohexyl)-4-méthylpyridin-2-yl)-4-((2-hydroxyéthyl)sulfonamido)-2-(6-azaspiro[2.5]octan-6-yl)benzamide |
| 24 | 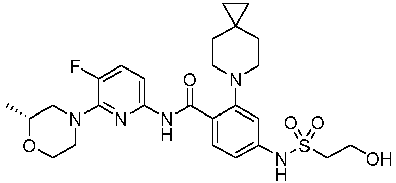 | (*R*)-*N*-(5-Fluoro-6-(2-méthylmorpholino)pyridin-2-yl)-4-((2-hydroxyéthyl)sulfonamido)-2-(6-azaspiro[2.5]octan-6-yl)benzamide |
| 25 | 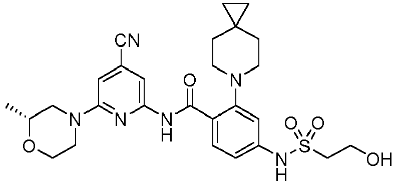 | (*R*)-*N-*(4-Cyano-6-(2-méthylmorpholino)pyridin-2-yl)-4-((2-hydroxyéthyl)sulfonamido)-2-(6-azaspiro[2.5]octan-6-yl)benzamide |
| 27-1 | 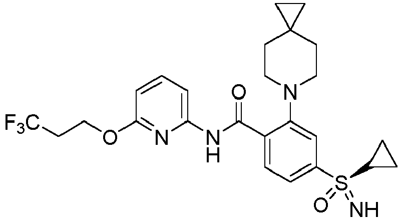 | 2-(6-Azaspiro[2.5]octan-6-yl)-4-(*S*-cyclopropylsulfonimidoyl)-*N*-(6-(3,3,3-trifluoropropoxy)-2-pyridinyl)benzamide |
| 27-2 | 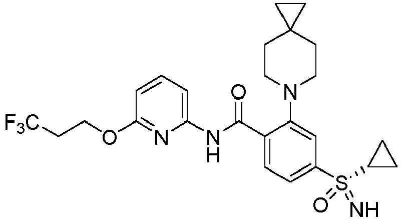 | 2-(6-Azaspiro[2.5]octan-6-yl)-4-(*R*-cyclopropylsulfonimidoyl)-*N*-(6-(3,3,3-trifluoropropoxy)-2-pyridinyl)benzamide |
; ou n'importe quel sel pharmaceutiquement acceptable de celui-ci.

18. Le composé de n'importe laquelle des revendications 1 à 17 pour une utilisation sous forme de médicament.

19. Une composition pharmaceutique comprenant le composé selon n'importe laquelle des revendications 1 à 17 ou le sel pharmaceutiquement acceptable de celui-ci, et un diluant ou véhicule pharmaceutiquement acceptable.

20. Le composé de n'importe laquelle des revendications 1 à 17 ou la composition comprenant le composé de la revendication 19 pour une utilisation dans une méthode de traitement d'une affection pouvant être traitée par des inhibiteurs de KIF18a, la méthode comprenant l'administration à un patient en ayant besoin d'une quantité thérapeutiquement efficace du composé ;
où ladite affection est un cancer sélectionné dans le groupe constitué (a) d'une tumeur solide ou dérivée d'une lignée hématologique sélectionnée parmi le cancer de la vessie, de l'endomètre, épidermoïde bronchique, du sein, du côlon, du rein, du foie, du poumon, du poumon à petites cellules, de l'oesophage, de la vésicule biliaire, du cerveau, de la tête et du cou, de l'ovaire, du pancréas, de l'estomac, du col de l'utérus, de la thyroïde, de la prostate et de la peau, (b) d'une tumeur hématopoïétique de lignée lymphoïde sélectionnée parmi la leucémie, la leucémie aiguë lymphoïde, la leucémie aiguë lymphoblastique, le lymphome à cellules B, le lymphome à cellules T, le lymphome de Hodgkin, le lymphome non hodgkinien, le lymphome à tricholeucocytes et le lymphome de Burkitt, (c) d'une tumeur hématopoïétique de lignée myéloïde sélectionnée parmi les leucémies myéloïdes aiguë et chronique, le syndrome myélodysplasique et la leucémie promyélocytaire, (d) d'une tumeur d'origine mésenchymateuse sélectionnée parmi le fibrosarcome et le rhabdomyosarcome, (e) d'une tumeur du système nerveux central et du système nerveux périphérique sélectionnée parmi l'astrocytome, le neuroblastome, le gliome et le schwannome, ou (f) d'un mélanome, séminome, tératocarcinome, ostéosarcome, xeroderma pigmentosum, kératoacanthome, cancer folliculaire de la thyroïde ou sarcome de Kaposi.

21. Le composé de n'importe laquelle des revendications 1 à 17 ou la composition comprenant le composé de la revendication 19 pour une utilisation dans une méthode de réduction de la taille d'une tumeur solide chez un sujet ; ou
dans une méthode de traitement d'un trouble de la prolifération cellulaire chez un sujet ; ou dans une méthode d'inhibition de KIF18A dans une cellule.
